Europäisches Patentamt

European Patent Office

Office européen des brevets

(19)

(11) **EP 1 568 373 A2**

(12) # EUROPEAN PATENT APPLICATION

(43) Date of publication:
**31.08.2005 Bulletin 2005/35**

(21) Application number: **05008363.3**

(22) Date of filing: **11.12.2000**

(51) Int Cl.[7]: **A61K 38/00**, A61K 38/03,
A61K 38/08, A61K 38/10,
A61K 38/16, C07K 7/00,
C07K 9/00, C07K 14/435,
C07H 21/02, C07H 21/04

(84) Designated Contracting States:
**AT BE CH CY DE DK ES FI FR GB GR IE IT LI LU
MC NL PT SE TR**

(30) Priority: **10.12.1999 US 458299**

(62) Document number(s) of the earlier application(s) in
accordance with Art. 76 EPC:
**00984214.7 / 1 239 866**

(71) Applicant: **Epimmune Inc.
San Diego, CA 92121 (US)**

(72) Inventors:
• **Fikes, John
San Diego, CA 92122 (US)**
• **Sette, Alessandro
La Jolla, CA 92037 (US)**
• **Sidney, John
San Diego, CA 92130 (US)**

• **Southwood, Scott
Santee, CA 92071 (US)**
• **Chesnut, Robert
Cardiff-by-the-sea, CA 92007 (US)**
• **Celis, Esteban
Rochester, MN 55902 (US)**
• **Keogh, Elissa
San Diego, CA 92122 (US)**

(74) Representative: **Cripps, Joanna Elizabeth et al
Mewburn Ellis LLP
York House
23 Kingsway
London WC2B 6HP (GB)**

Remarks:
This application was filed on 18 - 04 - 2005 as a
divisional application to the application mentioned
under INID code 62.

(54) **Inducing cellular immune responses to HER2/neu using peptide and nucleic acid compositions**

(57) This invention uses our knowledge of the mechanisms by which antigen is recognized by T cells to identify and prepare HER2/new epitopes, and to develop epitope-based vaccines directed towards HERS2/neu-bearing tumors. More specifically, this application communicates our discovery of pharmaceutical compositions and methods of use in the prevention and treatment of cancer.

EP 1 568 373 A2

**Description**

**I. BACKGROUND OF THE INVENTION**

A growing body of evidence suggests that cytotoxic T lymphocytes (CTL) are important in the immune response to tumor cells. CTL recognize peptide epitopes in the context of HLA class I molecules that are expressed on the surface of almost all nucleated cells. Following intracellular processing of endogenously synthesized tumor antigens, antigen-derived peptide epitopes bind to class I HLA molecules in the endoplasmic reticulum, and the resulting complex is then transported to the cell surface. CTL recognize the peptide-HLA class I complex, which then results in the destruction of the cell bearing the HLA-peptide complex directly by the CTL and/or via the activation of non-destructive mechanisms, *e.g.*, activation of lymphokines such as tumor necrosis factor-$\alpha$ (TNF-$\alpha$) or interferon-$\gamma$ (IFN$\gamma$) which enhance the immune response and facilitate the destruction of the tumor cell.

Tumor-specific helper T lymphocytes (HTLs) are also known to be important for maintaining effective antitumor immunity. Their role in antitumor immunity has been demonstrated in animal models in which these cells not only serve to provide help for induction of CTL and antibody responses, but also provide effector functions, which are mediated by direct cell contact and also by secretion of lymphokines (e.g., IFN$\gamma$ and TNF-$\alpha$).

A fundamental challenge in the development of an efficacious tumor vaccine is immune suppression or tolerance that can occur. There is therefore a need to establish vaccine embodiments that elicit immune responses of sufficient breadth and vigor to prevent progression and/or clear the tumor.

The epitope approach employed in the present invention represents a solution to this challenge, in that it allows the incorporation of various antibody, CTL and HTL epitopes, from discrete regions of a target tumor-associated antigen (TAA), and/or regions of other TAAs, in a single vaccine composition. Such a composition can simultaneously target multiple dominant and subdominant epitopes and thereby be used to achieve effective immunization in a diverse population.

HER2/neu (or erbB-2) is a 185 kD transmembrane protein with tyrosine kinase activity that has a structure similar to the epidermal growth factor receptor (Coussens et al., Science 230:113-119, 1985; Bargmann et al:, Nature 319:226-230, 1986; Yamamoto et al., Nature 319:230-234, 1986). Amplification of the Her2/neu gene and/or overexpression of the protein have been reported in many human adenocarcinomas of the breast, ovary, uterus, prostate, stomach, esophagus, pancreas, kidney, and lung *(see, e.g.*, Slamon *et al., Science* 235:177-182, 1987 and *Science* 244:707-712, 1989; *Borg et al., Cancer Res.* 50:4332-4337, 1990; Lukes *et al., Cancer* 73:2380-2385, 1994; Kuhn *et al., J. Urol.* 150:1427-1433, 1993; Sadasivan *et al., J. Urol.* 150:126-131, 1993; Yonemura *et al., Cancer Res.* 51: 1034-1038, 1991; Kameda *et al., Cancer Res.* 50:8002-8009, 1990; Houldsworth *et al.,* Cancer Res. 50:6417-6422, 1990; Yamanaka *et al., Human Path.* 24:1127-1134, 1993; Weidner *et al., Cancer Res.* 50:4504-4509, 1990; Kern *et al., Cancer Res.* 50:5184-5187, 1990; and Rachwal *et al., Br. J. Cancer* 72:56-64, 1995). This widespread expression on cancer cells makes HER2/neu an important target for immunotherapy.

The information provided in this section is intended to disclose the presently understood state of the art as of the filing date of the present application. Information is included in this section which was generated subsequent to the priority date of this application. Accordingly, information in this section is not intended, in any way, to delineate the priority date for the invention.

**II. SUMMARY OF THE INVENTION**

This invention applies our knowledge of the mechanisms by which antigen is recognized by T cells, for example, to develop epitope-based vaccines directed towards TAAs. More specifically, this application communicates our discovery of specific epitope pharmaceutical compositions and methods of use in the prevention and treatment of cancer.

Upon development of appropriate technology, the use of epitope-based vaccines has several advantages over current vaccines, particularly when compared to the use of whole antigens in vaccine compositions. For example, immunosuppressive epitopes that may be present in whole antigens can be avoided with the use of epitope-based vaccines. Such immunosuppressive epitopes may, *e.g.*, correspond to immunodominant epitopes in whole antigens, which may be avoided by selecting peptide epitopes from non-dominant regions *(see, e.g.,* Disis *et al., J. Immunol.* 156:3151-3158, 1996).

An additional advantage of an epitope-based vaccine approach is the ability to combine selected epitopes (CTL and HTL), and further, to modify the composition of the epitopes, achieving, for example, enhanced immunogenicity. Accordingly, the immune response can be modulated, as appropriate, for the target disease. Similar engineering of the response is not possible with traditional approaches.

Another major benefit of epitope-based immune-stimulating vaccines is their safety. The possible pathological side effects caused by infectious agents or whole protein antigens, which might have their own intrinsic biological activity, is eliminated.

An epitope-based vaccine also provides the ability to direct and focus an immune response to multiple selected antigens from the same pathogen (a "pathogen" may be an infectious agent or a tumor-associated molecule). Thus, patient-by-patient variability in the immune response to a particular pathogen may be alleviated by inclusion of epitopes from multiple antigens from the pathogen in a vaccine composition.

Furthermore, an epitope-based anti-tumor vaccine also provides the opportunity to combine epitopes derived from multiple tumor-associated molecules. This capability can therefore address the problem of tumor-to tumor variability that arises when developing a broadly targeted anti-tumor vaccine for a given tumor type and can also reduce the likelihood of tumor escape due to antigen loss. For example, a breast cancer tumor in one patient may express a target TAA that differs from a breast cancer tumor in another patient. Epitopes derived from multiple TAAs can be included in a polyepitopic vaccine that will target both breast cancer tumors.

One of the most formidable obstacles to the development of broadly efficacious epitope-based immunotherapeutics, however, has been the extreme polymorphism of HLA molecules. To date, effective non-genetically biased coverage of a population has been a task of considerable complexity; such coverage has required that epitopes be used that are specific for HLA molecules corresponding to each individual HLA allele. Impractically large numbers of epitopes would therefore have to be used in order to cover ethnically diverse populations. Thus, there has existed a need for peptide epitopes that are bound by multiple HLA antigen molecules for use in epitope-based vaccines. The greater the number of HLA antigen molecules bound, the greater the breadth of population coverage by the vaccine.

Furthermore, as described herein in greater detail, a need has existed to modulate peptide binding properties, *e.g.*, so that peptides that are able to bind to multiple HLA molecules do so with an affinity that will stimulate an immune response. Identification of epitopes restricted by more than one HLA allele at an affinity that correlates with immunogenicity is important to provide thorough population coverage, and to allow the elicitation of responses of sufficient vigor to prevent or clear an infection in a diverse segment of the population. Such a response can also target a broad array of epitopes. The technology disclosed herein provides for such favored immune responses.

In a preferred embodiment, epitopes for inclusion in vaccine compositions of the invention are selected by a process whereby protein sequences of known antigens are evaluated for the presence of motif or supermotif-bearing epitopes. Peptides corresponding to a motif- or supermotif-bearing epitope are then synthesized and tested for the ability to bind to the HLA molecule that recognizes the selected motif. Those peptides that bind at an intermediate or high affinity *i.e.,* an $IC_{50}$ (or a $K_D$ value) of 500 nM or less for HLA class I molecules or an $IC_{50}$ of 1000 nM or less for HLA class II molecules, are further evaluated for their ability to induce a CTL or HTL response. Immunogenic peptide epitopes are selected for inclusion in vaccine compositions.

Supermotif-bearing peptides may additionally be tested for the ability to bind to multiple alleles within the HLA supertype family. Moreover, peptide epitopes may be analogued to modify binding affinity and/or the ability to bind to multiple alleles within an HLA supertype.

The invention also includes embodiments comprising methods for monitoring or evaluating an immune response to a TAA in a patient having a known HLA-type. Such methods comprise incubating a T lymphocyte sample from the patient with a peptide composition comprising a TAA epitope that has an amino acid sequence described in, for example, Tables XXIII, XXIV, XXV, XXVI, XXVII, and XXXI which binds the product of at least one HLA allele present in the patient, and detecting for the presence of a T lymphocyte that binds to the peptide. A CTL peptide epitope can, for example, be used as a component of a tetrameric complex for this type of analysis.

An alternative modality for defining the peptide epitopes in accordance with the invention is to recite the physical properties, such as length; primary structure; or charge, which are correlated with binding to a particular allele-specific HLA molecule or group of allele-specific HLA molecules. A further modality for defining peptide epitopes is to recite the physical properties of an HLA binding pocket, or properties shared by several allele-specific HLA binding pockets (e.g. pocket configuration and charge distribution) and reciting that the peptide epitope fits and binds to the pocket or pockets.

As will be apparent from the discussion below, other methods and embodiments are also contemplated. Further, novel synthetic peptides produced by any of the methods described herein are also part of the invention.

## III. BRIEF DESCRIPTION OF THE FIGURES

not applicable

## IV. DETAILED DESCRIPTION OF THE INVENTION

The peptide epitopes and corresponding nucleic acid compositions of the present invention are useful for stimulating an immune response to a TAA by stimulating the production of CTL or HTL responses. The peptide epitopes, which are derived directly or indirectly from native TAA protein amino acid sequences, are able to bind to HLA molecules and stimulate an immune response to the TAA. The complete sequence of the TAA proteins to be analyzed can be obtained from GenBank. Peptide epitopes and analogs thereof can also be readily determined from sequence information that may subsequently be discovered for heretofore unknown variants of particular TAAs, as will be clear from the disclosure provided below.

A list of target TAA includes, but is not limited to, the following antigens: MAGE 1, MAGE 2, MAGE 3, MAGE-11, MAGE-A10, BAGE, GAGE, RAGE, MAGE-C1, LAGE-1, CAG-3, DAM, MUC1, MUC2, MUC18, NY-ESO-1, MUM-1, CDK4, BRCA2, NY-LU-1, NY-LU-7, NY-LU-12, CASP8, RAS, KIAA-2-5, SCCs, p53, p73, CEA, Her 2/neu, Melan-A, gp100, tyrosinase, TRP2, gp75/TRP1, kallikrein, PSM, PAP, PSA, PT1-1, B-catenin, PRAME, Telomerase, FAK, cyclin D1 protein, NOEY2, EGF-R, SART-1, CAPB, HPVE7, p15, Folate receptor CDC27, PAGE-1, and PAGE-4.

The peptide epitopes of the invention have been identified in a number of ways, as will be discussed below. Also discussed in greater detail is that analog peptides have been derived and the binding activity for HLA molecules modulated by modifying specific amino acid residues to create peptide analogs exhibiting altered immunogenicity. Further, the present invention provides compositions and combinations of compositions that enable epitope-based vaccines that are capable of interacting with HLA molecules encoded by various genetic alleles to provide broader population coverage than prior vaccines.

### IV.A. Definitions

The invention can be better understood with reference to the following definitions, which are listed alphabetically:

A "computer" or "computer system" generally includes: a processor; at least one information storage/retrieval apparatus such as, for example, a hard drive, a disk drive or a tape drive; at least one input apparatus such as, for example, a keyboard, a mouse, a touch screen, or a microphone; and display structure. Additionally, the computer may include a communication channel in communication with a network. Such a computer may include more or less than what is listed above.

A "construct" as used herein generally denotes a composition that does not occur in nature. A construct can be produced by synthetic technologies, *e.g.*, recombinant DNA preparation and expression or chemical synthetic techniques for nucleic or amino acids. A construct can also be produced by the addition or affiliation of one material with another such that the result is not found in nature in that form.

"Cross-reactive binding" indicates that a peptide is bound by more than one HLA molecule; a synonym is degenerate binding.

A "cryptic epitope" elicits a response by immunization with an isolated peptide, but the response is not cross-reactive *in vitro* when intact whole protein which comprises the epitope is used as an antigen.

A "dominant epitope" is an epitope that induces an immune response upon immunization with a whole native antigen (see, *e.g.,* Sercarz, *et al., Annu. Rev. Immunol.* 11:729-766, 1993). Such a response is cross-reactive *in vitro* with an isolated peptide epitope.

With regard to a particular amino acid sequence, an "epitope" is a set of amino acid residues which is involved in recognition by a particular immunoglobulin, or in the context of T cells, those residues necessary for recognition by T cell receptor proteins and/or Major Histocompatibility Complex (MHC) receptors. In an immune system setting, *in vivo* or *in vitro,* an epitope is the collective features of a molecule, such as primary, secondary and tertiary peptide structure, and charge, that together form a site recognized by an immunoglobulin, T cell receptor or HLA molecule. Throughout this disclosure epitope and peptide are often used interchangeably.

It is to be appreciated that protein or peptide molecules that comprise an epitope of the invention as well as additional amino acid(s) are within the bounds of the invention. In certain embodiments, there is a limitation on the length of a peptide of the invention which is not otherwise a construct as defined herein. An embodiment that is length-limited occurs when the protein/peptide comprising an epitope of the invention comprises a region (i.e., a contiguous series of amino acids) having 100% identity with a native sequence. In order to avoid a recited definition of epitope from reading, *e.g.*, on whole natural molecules, the length of any region that has 100% identity with a native peptide sequence is limited. Thus, for a peptide comprising an epitope of the invention and a region with 100% identity with a native peptide sequence (and which is not otherwise a construct), the region with 100% identity to a native sequence generally has a length of: less than or equal to 600 amino acids, often less than or equal to 500 amino acids, often less than or equal to 400 amino acids, often less than or equal to 250 amino acids, often less than or equal to 100 amino acids, often less than or equal to 85 amino acids, often less than or equal to 75 amino acids, often less than or equal to 65 amino acids, and often less than or equal to 50 amino acids. In certain embodiments, an "epitope" of the invention which is not a construct is comprised by a peptide having a region with less than 51 amino acids that has 100% identity to a native peptide sequence, in any increment of (50, 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5) down to 5 amino acids.

Certain peptide or protein sequences longer than 600 amino acids are within the scope of the invention. Such longer sequences are within the scope of the invention so long as they do not comprise any contiguous-sequence of more than 600 amino acids that have 100% identity with a native peptide sequence, or if longer than 600 amino acids, they are a construct. For any peptide that has five contiguous residues or less that correspond to a native sequence, there is no limitation on the maximal length of that peptide in order to fall within the scope of the invention. It is presently preferred that a CTL epitope of the invention be less than 600 residues long in any increment down to eight amino acid residues. "Human Leukocyte Antigen" or "HLA" is a human class I or class II Major Histocompatibility Complex (MHC) protein *(see, e.g.,* Stites, *et al.,* IMMUNOLOGY, 8[TH] ED., Lange Publishing, Los Altos, CA, 1994).

An "HLA supertype or family", as used herein, describes sets of HLA molecules grouped on the basis of shared peptide-binding specificities. HLA class I molecules that share somewhat similar binding affinity for peptides bearing certain amino acid motifs are grouped into HLA supertypes. The terms HLA superfamily, HLA supertype family, HLA family, and HLA xx-like molecules (where xx denotes a particular HLA type), are synonyms.

Throughout this disclosure, results are expressed in terms of "$IC_{50}$'s." $IC_{50}$ is the concentration of peptide in a binding assay at which 50% inhibition of binding of a reference peptide is observed. Given the conditions in which the assays are run (*i.e.*, limiting HLA proteins and labeled peptide concentrations), these values approximate $K_D$ values. Assays for determining binding are described in detail, *e.g.*, in PCT publications WO 94/20127 and WO 94/03205. It should be noted that $IC_{50}$ values can change, often dramatically, if the assay conditions are varied, and depending on the particular reagents used (e.g., HLA preparation, *etc.).* For example, excessive concentrations of HLA molecules will increase the apparent measured $IC_{50}$ of a given ligand.

Alternatively, binding is expressed relative to a reference peptide. Although as a particular assay becomes more, or less, sensitive, the $IC_{50}$'s of the peptides tested may change somewhat, the binding relative to the reference peptide will not significantly change. For example, in an assay run under conditions such that the $IC_{50}$ of the reference peptide increases 10-fold, the $IC_{50}$ values of the test peptides will also shift approximately 10-fold. Therefore, to avoid ambiguities, the assessment of whether a peptide is a good, intermediate, weak, or negative binder is generally based on its $IC_{50}$, relative to the $IC_{50}$ of a standard peptide.

Binding may also be determined using other assay systems including those using: live cells *(e.g.,* Ceppellini *et al., Nature* 339:392, 1989; Christnick *et al., Nature* 352:67, 1991; Busch *et al., Int. Immunol.* 2:443, 19990; Hill *et al., J. Immunol.* 147:189, 1991; del Guercio *et al., J. Immunol.* 154:685, 1995), cell free systems using detergent lysates (e.g., Cerundolo *et al., J. Immunol.* 21:2069, 1991), immobilized purified MHC *(e.g.,* Hill *et al., J. Immunol.* 152, 2890, 1994; Marshall *et al., J. Immunol.* 152:4946, 1994), ELISA systems (e.g., Reay *et al., EMBO J.* 11:2829, 1992), surface plasmon resonance (e.g., Khilko *et al., J. Biol. Chem,* 268:15425, 1993); high flux soluble phase assays (Hammer *et al., J. Exp. Med.* 180:2353, 1994), and measurement of class I MHC stabilization or assembly (e.g., Ljunggren *et al., Nature* 346:476, 1990; Schumacher *et al., Cell* 62:563, 1990; Townsend *et al., Cell* 62:285, 1990; Parker *et al., J. Immunol.* 149:1896, 1992).

As used herein, "high affinity" with respect to HLA class I molecules is defined as binding with an $IC_{50}$, or $K_D$ value, of 50 nM or less; "intermediate affinity" is binding with an $IC_{50}$ or $K_D$ value of between about 50 and about 500 nM. "High affinity" with respect to binding to HLA class II molecules is defined as binding with an $IC_{50}$ or $K_D$ value of 100 nM or less; "intermediate affinity" is binding with an $IC_{50}$ or $K_D$ value of between about 100 and about 1000 nM.

The terms "identical" or percent "identity," in the context of two or more peptide sequences, refer to two or more sequences or subsequences that are the same or have a specified percentage of amino acid residues that are the same, when compared and aligned for maximum correspondence over a comparison window, as measured using a sequence comparison algorithm or by manual alignment and visual inspection.

An "immunogenic peptide" or "peptide epitope" is a peptide that comprises an allele-specific motif or supermotif such that the peptide will bind an HLA molecule and induce a CTL and/or HTL response. Thus, immunogenic peptides of the invention are capable of binding to an appropriate HLA molecule and thereafter inducing a cytotoxic T cell response, or a helper T cell response, to the antigen from which the immunogenic peptide is derived.

The phrases "isolated" or "biologically pure" refer to material which is substantially or essentially free from components which normally accompany the material as it is found in its native state. Thus, isolated peptides in accordance with the invention preferably do not contain materials normally associated with the peptides in their *in situ* environment.

"Link" or "join" refers to any method known in the art for functionally connecting peptides, including, without limitation, recombinant fusion, covalent bonding, disulfide bonding, ionic bonding, hydrogen bonding, and electrostatic bonding.

"Major Histocompatibility Complex" or "MHC" is a cluster of genes that plays a role in control of the cellular interactions responsible for physiologic immune responses. In humans, the MHC complex is also known as the HLA complex. For a detailed description of the MHC and HLA complexes, see, Paul, FUNDAMENTAL IMMUNOLOGY, 3RD ED., Raven Press, New York, 1993.

The term "motif" refers to the pattern of residues in a peptide of defined length, usually a peptide of from about 8 to about 13 amino acids for a class I HLA motif and from about 6 to about 25 amino acids for a class II HLA motif, which is recognized by a particular HLA molecule. Peptide motifs are typically different for each protein encoded by each human HLA allele and differ in the pattern of the primary and secondary anchor residues.

A "negative binding residue" or "deleterious residue" is an amino acid which, if present at certain positions (typically not primary anchor positions) in a peptide epitope, results in decreased binding affinity of the peptide for the peptide's corresponding HLA molecule.

The term "peptide" is used interchangeably with "oligopeptide" in the present specification to designate a series of residues, typically L-amino acids, connected one to the other, typically by peptide bonds between the α-amino and carboxyl groups of adjacent amino acids. The preferred CTL-inducing peptides of the invention are 13 residues or less in length and usually consist of between about 8 and about 11 residues, preferably 9 or 10 residues. The preferred HTL-inducing oligopeptides are less than about 50 residues in length and usually consist of between about 6 and about 30 residues, more usually between about 12 and 25, and often between about 15 and 20 residues.

"Pharmaceutically acceptable" refers to a generally non-toxic, inert, and/or physiologically compatible composition.

A "pharmaceutical excipient" comprises a material such as an adjuvant, a carrier, pH-adjusting and buffering agents, tonicity adjusting agents, wetting agents, preservative, and the like.

A "primary anchor residue" is an amino acid at a specific position along a peptide sequence which is understood to provide a contact point between the immunogenic peptide and the HLA molecule. One to three, usually two, primary anchor residues within a peptide of defined length generally defines a "motif" for an immunogenic peptide. These residues are understood to fit in close contact with peptide binding grooves of an HLA molecule, with their side chains buried in specific pockets of the binding grooves themselves. In one embodiment, for example, the primary anchor residues are located at position 2 (from the amino terminal position) and at the carboxyl terminal position of a 9-residue peptide epitope in accordance with the invention. The primary anchor positions for each motif and supermotif are set forth in Table 1. For example, analog peptides can be created by altering the presence or absence of particular residues in these primary anchor positions. Such analogs are used to modulate the binding affinity of a peptide comprising a particular motif or supermotif.

"Promiscuous recognition" is where a distinct peptide is recognized by the same T cell clone in the context of various HLA molecules. Promiscuous recognition or binding is synonymous with cross-reactive binding.

A "protective immune response" or "therapeutic immune response" refers to a CTL and/or an HTL response to an antigen derived from an infectious agent or a tumor antigen, which prevents or at least partially arrests disease symptoms or progression. The immune response may also include an antibody response which has been facilitated by the stimulation of helper T cells.

The term "residue" refers to an amino acid or amino acid mimetic incorporated into an oligopeptide by an amide bond or amide bond mimetic.

A "secondary anchor residue" is an amino acid at a position other than a primary anchor position in a peptide which may influence peptide binding. A secondary anchor residue occurs at a significantly higher frequency amongst bound peptides than would be expected by random distribution of amino acids at one position. The secondary anchor residues are said to occur at "secondary anchor positions." A secondary anchor residue can be identified as a residue which is present at a higher frequency among high or intermediate affinity binding peptides, or a residue otherwise associated with high or intermediate affinity binding. For example, analog peptides can be created by altering the presence or absence of particular residues in these secondary anchor positions. Such analogs are used to finely modulate the binding affinity of a peptide comprising a particular motif or supermotif.

A "subdominant epitope" is an epitope which evokes little or no response upon immunization with whole antigens which comprise the epitope, but for which a response can be obtained by immunization with an isolated peptide, and this response (unlike the case of cryptic epitopes) is detected when whole protein is used to recall the response *in vitro* or *in vivo.*

A "supermotif" is a peptide binding specificity shared by HLA molecules encoded by two or more HLA alleles. Preferably, a supermotif-bearing peptide is recognized with high or intermediate affinity (as defined herein) by two or more HLA molecules.

"Synthetic peptide" refers to a peptide that is man-made using such methods as chemical synthesis or recombinant DNA technology.

As used herein, a "vaccine" is a composition that contains one or more peptides of the invention. There are numerous embodiments of vaccines in accordance with the invention, such as by a cocktail of one or more peptides; one or more epitopes of the invention comprised by a polyepitopic peptide; or nucleic acids that encode such peptides or polypeptides, *e.g.*, a minigene that encodes a polyepitopic peptide. The "one or more peptides" can include any whole unit integer from 1-150, *e.g.*, at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 55, 60, 65, 70, 75, 80, 85, 90, 95, 100, 105, 110, 115, 120, 125, 130, 135, 140, 145, or 150 or more peptides of the invention. The peptides or polypeptides can optionally be modified, such as by lipidation, addition of targeting or other sequences. HLA class I-binding peptides of the invention can be admixed with, or linked to, HLA class II-binding peptides, to facilitate activation of both cytotoxic T lymphocytes and helper T lymphocytes. Vaccines can also comprise peptide-pulsed antigen presenting cells, *e.g.*, dendritic cells.

The nomenclature used to describe peptide compounds follows the conventional practice wherein the amino group is presented to the left (the N-terminus) and the carboxyl group to the right (the C-terminus) of each amino acid residue. When amino acid residue positions are referred to in a peptide epitope they are numbered in an amino to carboxyl direction with position one being the position closest to the amino terminal end of the epitope, or the peptide or protein of which it may be a part. In the formulae representing selected specific embodiments of the present invention, the amino- and carboxyl-terminal groups, although not specifically shown, are in the form they would assume at physiologic pH values, unless otherwise specified. In the amino acid structure formulae, each residue is generally represented by standard three letter or single letter designations. The L-form of an amino acid residue is represented by a capital single letter or a capital first letter of a three-letter symbol, and the D-form for those amino acids having D-forms is represented by a lower case single letter or a lower case three letter symbol. Glycine has no asymmetric carbon atom and is simply referred to as "Gly" or G. The amino acid sequences of peptides set forth herein are generally designated using the standard single letter symbol. (A, Alanine; C, Cysteine; D, Aspartic Acid; E, Glutamic Acid; F, Phenylalanine; G, Glycine; H, Histidine; I, Isoleucine; K, Lysine; L, Leucine; M, Methionine; N, Asparagine; P, Proline; Q, Glutamine; R, Arginine; S, Serine; T, Threonine; V, Valine; W, Tryptophan; and Y, Tyrosine.) In addition to these symbols, "B"in the single letter abbreviations used herein designates α-amino butyric acid.

### IV.B. Stimulation of CTL and HTL responses

The mechanism by which T cells recognize antigens has been delineated during the past ten years. Based on our understanding of the immune system we have developed efficacious peptide epitope vaccine compositions that can induce a therapeutic or prophylactic immune response to a TAA in a broad population. For an understanding of the value and efficacy of the claimed compositions, a brief review of immunology-related technology is provided. The review is intended to disclose the presently understood state of the art as of the filing date of the present application. Information is included in this section which was generated subsequent to the priority date of this application. Accordingly, information in this section is not intended, in any way, to delineate the priority date for the invention.

A complex of an HLA molecule and a peptidic antigen acts as the ligand recognized by HLA-restricted T cells (Buus, *S: et al.*, *Cell* 47:1071, 1986; Babbitt, B. P. *et al.*, *Nature* 317:359, 1985; Townsend, A. and Bodmer, H., *Annu. Rev. Immunol.* 7:601, 1989; Germain, R. N., *Annu. Rev. Immunol.* 11:403, 1993). Through the study of single amino acid substituted antigen analogs and the sequencing of endogenously bound, naturally processed peptides, critical residues that correspond to motifs required for specific binding to HLA antigen molecules have been identified and are described herein and are set forth in Tables I, II, and III (*see* also, *e.g.*, Southwood, *et al., J. Immunol.* 160:3363, 1998; Rammensee, *et al., Immunogenetics* 41:178, 1995; Rammensee *et al.*, SYFPEITHI, access via web at :

http://134.2.96.221/scripts.hlaserver.dll/home.htm; Sette, A. and Sidney, J. *Curr. Opin. Immunol.* 10:478, 1998; Engelhard, V. H., *Curr. Opin. Immunol.* 6:13, 1994; Sette, A. and Grey, H. M., *Curr. Opin. Immunol.* 4:79, 1992; Sinigaglia, F. and Hammer, J. *Curr. Biol.* 6:52, 1994; Ruppert *et al.*, *Cell* 74:929-937, 1993; Kondo *et al., J. Immunol.* 155:4307-4312, 1995; Sidney *et al., J. Immunol.* 157:3480-3490, 1996; Sidney *et al., Human Immunol.* 45:79-93, 1996; Sette, A. and Sidney, J. *Immunogenetics 1999 Nov;50(3-4):201-12,* Review).

Furthermore, x-ray crystallographic analysis of HLA-peptide complexes has revealed pockets within the peptide binding cleft of HLA molecules which accommodate, in an allele-specific mode, residues borne by peptide ligands; these residues in turn determine the HLA binding capacity of the peptides in which they are present. *(See, e.g.,* Madden, D.R. *Annu. Rev. Immunol.* 13:587, 1995; Smith, *et al., Immunity* 4:203, 1996; Fremont *et al., Immunity* 8:305, 1998; Stern *et al., Structure* 2:245, 1994; Jones, E.Y. *Curr. Opin. Immunol.* 9:75, 1997; Brown, J. H. *et al., Nature* 364:33, 1993; Guo, H. C. *et al., Proc. Natl. Acad. Sci. USA* 90:8053, 1993; Guo, H. *C. et al., Nature* 360:364, 1992; Silver, M. L*. et al., Nature* 360:367, 1992; Matsumura, M. *et al., Science* 257:927, 1992; Madden *et al., Cell* 70:1035, 1992; Fremont, D. H. *et al., Science* 257:919, 1992; Saper, M. A. , Bjorkman, P. J. and Wiley, D. C., *J. Mol. Biol.* 219:277, 1991.)

Accordingly, the definition of class I and class II allele-specific HLA binding motifs, or class I or class II supermotifs allows identification of regions within a protein that have the potential of binding particular HLA molecules.

The present inventors have found that the correlation of binding affinity with immunogenicity, which is disclosed herein, is an important factor to be considered when evaluating candidate peptides. Thus, by a combination of motif searches and HLA-peptide binding assays, candidates for epitope-based vaccines have been identified. After determining their binding affinity, additional confirmatory work can be performed to select, amongst these vaccine candidates, epitopes with preferred characteristics in terms of population coverage, antigenicity, and immunogenicity.

Various strategies can be utilized to evaluate immunogenicity, including:

1) Evaluation of primary T cell cultures from normal individuals *(see, e.g.,* Wentworth, P. A. *et al., Mol. Immunol.* 32:603, 1995; Celis, E. *et al., Proc. Natl. Acad. Sci. USA* 91:2105, 1994; Tsai, V. *et al., J. Immunol.* 158:1796, 1997; Kawashima, I. *et al., Human Immunol.* 59:1, 1998); This procedure involves the stimulation of peripheral blood lymphocytes (PBL) from normal subjects with a test peptide in the presence of antigen presenting cells *in vitro* over a period of several weeks. T cells specific for the peptide become activated during this time and are detected using, *e.g.*, a $^{51}$Cr-release assay involving peptide sensitized target cells.

2) Immunization of HLA transgenic mice *(see, e.g.,* Wentworth, P. A. *et al., J. Immunol.* 26:97, 1996; Wentworth, P.A. *et al., Int. Immunol.* 8:651, 1996; Alexander,J. *et al., J. Immunol.* 159:4753, 1997); In this method, peptides in incomplete Freund's adjuvant are administered subcutaneously to HLA transgenic mice. Several weeks following immunization, splenocytes are removed and cultured *in vitro* in the presence of test peptide for approximately one week. Peptide-specific T cells are detected using, *e.g.*, a $^{51}$Cr-release assay involving peptide sensitized target cells and target cells expressing endogenously generated antigen.

3) Demonstration of recall T cell responses from patients who have been effectively vaccinated or who have a tumor; *(see, e.g.,* Rehermann, B. *et al., J. Exp. Med.* 181:1047, 1995; Doolan, D. L. *et al., Immunity* 7:97, 1997; Bertoni, R. *et al., J. Clin. Invest.* 100:503, 1997; Threlkeld, S. C. *et al., J. Immunol.* 159:1648, 1997; Diepolder, H. M. *et al., J. Virol.* 71:6011, 1997; Tsang *et al., J. Natf. Cancer Inst.* 87:982-990, 1995; Disis *et al., J. Immunol.* 156: 3151-3158, 1996). In applying this strategy, recall responses are detected by culturing PBL from patients with cancer who have generated an immune response "naturally", or from patients who were vaccinated with tumor antigen vaccines. PBL from subjects are cultured *in vitro* for 1-2 weeks in the presence of test peptide plus antigen presenting cells (APC) to allow activation of "memory" T cells, as compared to "naive" T cells. At the end of the culture period, T cell activity is detected using assays for T cell activity including $^{51}$Cr release involving peptide-sensitized targets, T cell proliferation, or lymphokine release.

The following describes the peptide epitopes and corresponding nucleic acids of the invention.

## IV.C. Binding Affinity of Peptide Epitopes for HLA Molecules

As indicated herein, the large degree of HLA polymorphism is an important factor to be taken into account with the epitope-based approach to vaccine development. To address this factor, epitope selection encompassing identification of peptides capable of binding at high or intermediate affinity to multiple HLA molecules is preferably utilized, most preferably these epitopes bind at high or intermediate affinity to two or more allele-specific HLA molecules.

CTL-inducing peptides of interest for vaccine compositions preferably include those that have an $IC_{50}$ or binding affinity value for class I HLA molecules of 500 nM or better (*i.e.,* the value is ≤ 500 nM). HTL-inducing peptides preferably include those that have an $IC_{50}$ or binding affinity value for class II HLA molecules of 1000 nM or better, (*i. e.,* the value is ≤ 1,000 nM). For example, peptide binding is assessed by testing the capacity of a candidate peptide to bind to a purified HLA molecule *in vitro.* Peptides exhibiting high or intermediate affinity are then considered for further analysis. Selected peptides are tested on other members of the supertype family. In preferred embodiments, peptides that exhibit cross-reactive binding are then used in cellular screening analyses or vaccines.

As disclosed herein, higher HLA binding affinity is correlated with greater immunogenicity. Greater immunogenicity can be manifested in several different ways. Immunogenicity corresponds to whether an immune response is elicited at all, and to the vigor of any particular response, as well as to the extent of a population in which a response is elicited. For example, a peptide might elicit an immune response in a diverse array of the population, yet in no instance produce a vigorous response. Moreover, higher binding affinity peptides lead to more vigorous immunogenic responses. As a result, less peptide is required to elicit a similar biological effect if a high or intermediate affinity binding peptide is used. Thus, in preferred embodiments of the invention, high or intermediate affinity binding epitopes are particularly useful.

The relationship between binding affinity for HLA class I molecules and immunogenicity of discrete peptide epitopes on bound antigens has been determined for the first time in the art by the present inventors. The correlation between binding affinity and immunogenicity was analyzed in two different experimental approaches *(see, e.g.,* Sette, *et al., J. Immunol.* 153:5586-5592, 1994). In the first approach, the immunogenicity of potential epitopes ranging in HLA binding affinity over a 10,000-fold range was analyzed in HLA-A*0201 transgenic mice. In the second approach, the antigenicity of approximately 100 different hepatitis B virus (HBV)-derived potential epitopes, all carrying A*0201 binding motifs, was assessed by using PBL from acute hepatitis patients. Pursuant to these approaches, it was determined that an affinity threshold value of approximately 500 nM (preferably 50 nM or less) determines the capacity of a peptide epitope to elicit a CTL response. These data are true for class I binding affinity measurements for naturally processed peptides and for synthesized T cell epitopes. These data also indicate the important role of determinant selection in the shaping of T cell responses *(see, e.g.,* Schaeffer *et al., Proc. Natl. Acad. Sci. USA* 86:4649-4653, 1989).

An affinity threshold associated with immunogenicity in the context of HLA class II DR molecules has also been delineated *(see, e.g.,* Southwood *et al. J. Immunology* 160:3363-3373,1998, and co-pending U.S.S.N. 09/009,953 filed 1/21/98). In order to define a biologically significant threshold of DR binding affinity, a database of the binding affinities of 32 DR-restricted epitopes for their restricting element (*i.e.*, the HLA molecule that binds the motif) was compiled. In approximately half of the cases (15 of 32 epitopes), DR restriction was associated with high binding affinities, *i.e.* binding affinity values of 100 nM or less. In the other half of the cases (16 of 32), DR restriction was associated with intermediate affinity (binding affinity values in the 100-1000 nM range). In only one of 32 cases was DR restriction associated with an $IC_{50}$ of 1000 nM or greater. Thus, 1000 nM can be defined as an affinity threshold associated with immunogenicity in the context of DR molecules.

In the case of tumor-associated antigens, many CTL peptide epitopes that have been shown to induce CTL that lyse peptide-pulsed target cells and tumor cell targets endogenously expressing the epitope exhibit binding affinity or $IC_{50}$ values of 200 nM or less. In a study that evaluated the association of binding affinity and immunogenicity of such TAA epitopes, 100% (10/10) of the high binders, *i.e.,* peptide epitopes binding at an affinity of 50 nM or less, were immunogenic and 80% (8/10) of them elicited CTLs that specifically recognized tumor cells. In the 51 to 200 nM range, very similar figures were obtained. CTL inductions positive for peptide and tumor cells were noted for 86% (6/7) and 71% (5/7) of the peptides, respectively. In the 201-500 nM range, most peptides (4/5 wildtype) were positive for induction of CTL recognizing wildtype peptide, but tumor recognition was not detected.

The binding affinity of peptides for HLA molecules can be determined as described in Example 1, below.

**IV.D. Peptide Epitope Binding Motifs and Supermotifs**

Through the study of single amino acid substituted antigen analogs and the sequencing of endogenously bound, naturally processed peptides, critical residues required for allele-specific binding to HLA molecules have been identified. The presence of these residues correlates with binding affinity for HLA molecules. The identification of motifs and/or supermotifs that correlate with high and intermediate affinity binding is an important issue with respect to the identification of immunogenic peptide epitopes for the inclusion in a vaccine. Kast *et al. (J. Immunol.* 152:3904-3912, 1994) have shown that motif-bearing peptides account for 90% of the epitopes that bind to allele-specific HLA class I molecules. In this study all possible peptides of 9 amino acids in length and overlapping by eight amino acids (240 peptides), which cover the entire sequence of the E6 and E7 proteins of human papillomavirus type 16, were evaluated for binding to five allele-specific HLA molecules that are expressed at high frequency among different ethnic groups. This unbiased set of peptides allowed an evaluation of the predictive value of HLA class I motifs. From the set of 240 peptides, 22 peptides were identified that bound to an allele-specific HLA molecule with high or intermediate affinity. Of these 22 peptides, 20 (*i.e.* 91%) were motif-bearing. Thus, this study demonstrates the value of motifs for the identification of peptide epitopes for inclusion in a vaccine: application of motif-based identification techniques will identify about 90% of the potential epitopes in a target antigen protein sequence.

Such peptide epitopes are identified in the Tables described below.

Peptides of the present invention also comprise epitopes that bind to MHC class II DR molecules. A greater degree of heterogeneity in both size and binding frame position of the motif, relative to the N and C termini of the peptide, exists for class II peptide ligands. This increased heterogeneity of HLA class II peptide ligands is due to the structure of the binding groove of the HLA class II molecule which, unlike its class I counterpart, is open at both ends. Crystallographic analysis of HLA class II DRB*0101-peptide complexes showed that the major energy of binding is contributed by peptide residues complexed with complementary pockets on the DRB*0101 molecules. An important anchor residue engages the deepest hydrophobic pocket *(see, e.g.,* Madden, D.R. *Ann. Rev. Immunol.* 13:587, 1995) and is referred to as position 1 (P1). P1 may represent the N-terminal residue of a class II binding peptide epitope, but more typically is flanked towards the N-terminus by one or more residues. Other studies have also pointed to an important role for the peptide residue in the 6th position towards the C-terminus, relative to PI, for binding to various DR molecules.

In the past few years evidence has accumulated to demonstrate that a large fraction of HLA class I and class II molecules can be classified into a relatively few supertypes, each characterized by largely overlapping peptide binding repertoires, and consensus structures of the main peptide binding pockets. Thus, peptides of the present invention are identified by any one of several HLA-specific amino acid motifs *(see, e.g.,* Tables I-III), or if the presence of the motif corresponds to the ability to bind several allele-specific HLA molecules, a supermotif. The HLA molecules that bind to peptides that possess a particular amino acid supermotif are collectively referred to as an HLA "supertype."

The peptide motifs and supermotifs described below, and summarized in Tables I-III, provide guidance for the identification and use of peptide epitopes in accordance with the invention.

Examples of peptide epitopes bearing a respective supermotif or motif are included in Tables as designated in the description of each motif or supermotif below. The Tables include a binding affinity ratio listing for some of the peptide epitopes. The ratio may be converted to $IC_{50}$ by using the following formula: $IC_{50}$ of the standard peptide/ratio = $IC_{50}$ of the test peptide (*i.e.*, the peptide epitope). The $IC_{50}$ values of standard peptides used to determine binding affinities for Class I peptides are shown in Table IV. The $IC_{50}$ values of standard peptides used to determine binding affinities for Class II peptides are shown in Table V. The peptides used as standards for the binding assays described herein are examples of standards; alternative standard peptides can also be used when performing binding studies.

To obtain the peptide epitope sequences listed in each of Tables VII-XX, the amino acid sequence of HER2/neu was evaluated for the presence of the designated supermotif or motif, *i.e.,* the amino acid sequence was searched for the presence of the primary anchor residues as set out in Table I (for Class I motifs) or Table III (for Class II motifs) for each respective motif or supermotif.

In the Tables, motif- and/or supermotif-bearing epitopes in the HER2/neu sequence are indicated by position number and length of the epitope with reference to the HER2/neu sequence and numbering provided below. The "pos" (position) column designates the amino acid position in the HER2/neu protein sequence that corresponds to the first amino acid residue of the epitope. The "number of amino acids" indicates the number of residues in the epitope sequence and hence the length of the epitope. For example, the first peptide epitope listed in Table VII is a sequence of 8 residues in length starting at position 66. Accordingly, the amino acid sequence of the epitope is PTNASLSF.

Binding data presented in Tables VII-XX is expressed as a relative binding ratio, *supra.*

HER2/neu amino acid sequence

```
1 MELAALCRWG LLLALLPPGA ASTQVCTGTD MKLRLPASPE THLDMLRHLY QGCQVVQGNL   60

  ELTYLPTNAS LSFLQDIQEV QGYVLIAHNQ VRQVPLQRLR IVRGTQLFED NYALAVLDNG  120

  DPLNNTTPVT GASPGGLREL QLRSLTEILK GGVLIQRNPQ LCYQDTILWK DIFHKNNQLA  180

  LTLIDTNRSR ACHPCSPMCK GSRCWGESSE DCQSLTRTVC AGGCARCKGP LPTDCCHEQC  240

  AAGCTGPKHS DCLACLHFNH SGICELHCPA LVTYNTDTFE SMPNPEGRYT FGASCVTACP  300

  YNYLSTDVGS CTLVCPLHNQ EVTAEDGTQR CEKCSKPCAR VCYGLGMEHL REVRAVTSAN  360

  IQEFAGCKKI FGSLAFLPES FDGDPASNTA PLQPEQLQVF ETLEEITGYL YISAWPDSLP  420

  DLSVFQNLQV IRGRILHNGA YSLTLQGLGI SWLGLRSLRE LGSGLALIHH NTHLCFVHTV  480

  PWDQLFRNPH QALLHTANRP EDECVGEGLA CHQLCARGHC WGPGPTQCVN CSQFLRGQEC  540

  VEECRVLQGL PREYVNARHC LPCHPECQPQ NGSVTCFGPE ADQCVACAHY KDPPFCVARC  600

  PSGVKPDLSY MPIWKFPDEE GACQPCPINC THSCVDLDDK GCPAEQRASP LTSIISAVVG  660

  ILLVVVLGVV FGILIKRRQQ KIRKYTMRRL LQETELVEPL TPSGAMPNQA QMRILKETEL  720

  RKVKVLGSGA FGTVYKGIWI PDGENVKIPV AIKVLRENTS PKANKEILDE AYVMAGVGSP  780

  YVSRLLGICL TSTVQLVTQL MPYGCLLDHV RENRGRLGSQ DLLNWCMQIA KGMSYLEDVR  840

  LVHRDLAARN VLVKSPNHVK ITDFGLARLL DIDETEYHAD GGKVPIKWMA LESILRRRFT  900

  HQSDVWSYGV TVWELMTFGA KPYDGIPARE IPDLLEKGER LPQPPICTID VYMIMVKCWM  960

  IDSECRPRFR ELVSEFSRMA RDPQRFVVIQ NEDLGPASPL DSTFYRSLLE DDDMGDLVDA 1020

  EEYLVPQQGF FCPDPAPGAG GMVHHRHRSS STRSGGGDLT LGLEPSEEEA PRSPLAPSEG 1080

  AGSDVFDGDL GMGAAKGLQS LPTHDPSPLQ RYSEDPTVPL PSETDGYVAP LTCSPQPEYV 1140

  NQPDVRPQPP SPREGPLPAA RPAGATLERP KTLSPGKNGV VKDVFAFGGA VENPEYLTPQ 1200

  GGAAPQPHPP PAFSPAFDNL YYWDQDPPER GAPPSTFKGT PTAENPEYLG LDVPV   1255
```

**HLA Class I Motifs Indicative of CTL Inducing Peptide Epitopes:**

The primary anchor residues of the HLA class I peptide epitope supermotifs and motifs delineated below are summarized in Table I. The HLA class I motifs set out in Table I(a) are those most particularly relevant to the invention claimed here. Primary and secondary anchor positions are summarized in Table II. Allele-specific HLA molecules that comprise HLA class I supertype families are listed in Table VI. In some cases, peptide epitopes may be listed in both a motif and a supermotif Table. The relationship of a particular motif and respective supermotif is indicated in the description of the individual motifs.

**IV.D.1. HLA-A1 supermotif**

The HLA-A1 supermotif is characterized by the presence in peptide ligands of a small (T or S) or hydrophobic (L, I, V, or M) primary anchor residue in position 2, and an aromatic (Y, F, or W) primary anchor residue at the C-terminal position of the epitope. The corresponding family of HLA molecules that bind to the A1 supermotif (*i.e.*, the HLA-A1 supertype) is comprised of at least: A*0101, A*2601, A*2602, A*2501, and A*3201 *(see, e.g.,* DiBrino, M. *et al., J. Immunol.* 151:5930, 1993; DiBrino, M. *et al., J. Immunol.* 152:620, 1994; Kondo, A. *et al.*, *Immunogenetics* 45:249, 1997). Other allele-specific HLA molecules predicted to be members of the A1 superfamily are shown in Table VI. Peptides binding to each of the individual HLA proteins can be modulated by substitutions at primary and/or secondary anchor positions, preferably choosing respective residues specified for the supermotif.

Representative peptide epitopes that comprise the A1 supermotif are set forth in Table VII.

### IV.D.2. HLA-A2 supermotif

Primary anchor specificities for allele-specific HLA-A2.1 molecules *(see, e.g.,* Falk *et al., Nature* 351:290-296, 1991; Hunt *et al., Science* 255:1261-1263, 1992; Parker *et al., J. Immunol.* 149:3580-3587, 1992; Ruppert *et al., Cell* 74:929-937, 1993) and cross-reactive binding among HLA-A2 and -A28 molecules have been described. *(See, e.g.,* Fruci *et al., Human Immunol.* 38:187-192, 1993; Tanigaki *et al., Human Immunol.* 39:155-162, 1994; Del Guercio *et al., J. Immunol.* 154:685-693, 1995; Kast *et al., J. Immunol.* 152:3904-3912, 1994 for reviews of relevant data.) These primary anchor residues define the HLA-A2 supermotif; which presence in peptide ligands corresponds to the ability to bind several different HLA-A2 and -A28 molecules. The HLA-A2 supermotif comprises peptide ligands with L, I, V, M, A, T, or Q as a primary anchor residue at position 2 and L, I, V, M, A, or T as a primary anchor residue at the C-terminal position of the epitope.

The corresponding family of HLA molecules (*i.e.*, the HLA-A2 supertype that binds these peptides) is comprised of at least: A*0201, A*0202, A*0203, A*0204, A*0205, A*0206, A*0207, A*0209, A*0214, A*6802, and A*6901. Other allele-specific HLA molecules predicted to be members of the A2 superfamily are shown in Table VI. As explained in detail below, binding to each of the individual allele-specific HLA molecules can be modulated by substitutions at the primary anchor and/or secondary anchor positions, preferably choosing respective residues specified for the supermotif.

Representative peptide epitopes that comprise an A2 supermotif are set forth in Table VIII. The motifs comprising the primary anchor residues V, A, T, or Q at position 2 and L, I, V, A, or T at the C-terminal position are those most particularly relevant to the invention claimed herein.

### IV.D.3. HLA-A3 supermotif

The HLA-A3 supermotif is characterized by the presence in peptide ligands of A, L, I, V, M, S, or, T as a primary anchor at position 2, and a positively charged residue, R or K, at the C-terminal position of the epitope, *e.g.,* in position 9 of 9-mers *(see, e.g.,* Sidney *et al., Hum. Immunol.* 45:79, 1996). Exemplary members of the corresponding family of HLA molecules (the HLA-A3 supertype) that bind the A3 supermotif include at least: A*0301, A*1101, A*3101, A*3301, and A*6801. Other allele-specific HLA molecules predicted to be members of the A3 supertype are shown in Table VI. As explained in detail below, peptide binding to each of the individual allele-specific HLA proteins can be modulated by substitutions of amino acids at the primary and/or secondary anchor positions of the peptide, preferably choosing respective residues specified for the supermotif.

Representative peptide epitopes that comprise the A3 supermotif are set forth in Table IX.

### IV.D.4. HLA-A24 supermotif

The HLA-A24 supermotif is characterized by the presence in peptide ligands of an aromatic (F, W, or Y) or hydrophobic aliphatic (L, I, V, M, or T) residue as a primary anchor in position 2, and Y, F, W, L, I, or M as primary anchor at the C-terminal position of the epitope (*see*, *e.g.*, Sette and Sidney, *Immunogenetics 1999 Nov;50(3-4):201-12,* Review). The corresponding family of HLA molecules that bind to the A24 supermotif (*i.e.*, the A24 supertype) includes at least: A*2402, A*3001, and A*2301. Other allele-specific HLA molecules predicted to be members of the A24 supertype are shown in Table VI. Peptide binding to each of the allele-specific HLA molecules can be modulated by substitutions at primary and/or secondary anchor positions, preferably choosing respective residues specified for the supermotif.

Representative peptide epitopes that comprise the A24 supermotif are set forth in Table X.

### IV.D.5. HLA-B7 supermotif

The HLA-B7 supermotif is characterized by peptides bearing proline in position 2 as a primary anchor, and a hydrophobic or aliphatic amino acid (L, I, V, M, A, F, W, or Y) as the primary anchor at the C-terminal position of the epitope. The corresponding family of HLA molecules that bind the B7 supermotif (*i.e.*, the HLA-B7 supertype) is comprised of at least twenty six HLA-B proteins comprising at least: B*0702, B*0703, B*0704, B*0705, B*1508, B*3501, B*3502, B*3503, B*3504, B*3505, B*3506, B*3507, B*3508, B*5101, B*5102, B*5103, B*5104, B*5105, B*5301, B*5401, B*5501, B*5502, B*5601; B*5602; B*6701; and B*7801 (*see; e.g.*; Sidney, *et al.*; *J. Immunol.* 154:24.7, 1995; Barber, *et al.*, *Curr. Biol.* 5:179, 1995; Hill, *et al., Nature* 360:434, 1992; Rammensee, *et al., Immunogenetics* 41:178, 1995 for reviews of relevant data). Other allele-specific HLA molecules predicted to be members of the B7 supertype are shown in Table VI. As explained in detail below, peptide binding to each of the individual allele-specific HLA proteins can be modulated by substitutions at the primary and/or secondary anchor positions of the peptide, preferably choosing respective residues specified for the supermotif.

Representative peptide epitopes that comprise the B7 supermotif are set forth in Table XI.

### IV.D.6. HLA-B27 supermotif

The HLA-B27 supermotif is characterized by the presence in peptide ligands of a positively charged (R, H, or K) residue as a primary anchor at position 2, and a hydrophobic (F, Y, L, W, M, I, A, or V) residue as a primary anchor at the C-terminal position of the epitope *(see, e.g.,* Sidney and Sette, *Immunogenetics 1999 Nov;50(3-4):201-12,* Review). Exemplary members of the corresponding family of HLA molecules that bind to the B27 supermotif (*i.e.,* the B27 supertype) include at least B*1401, B*1402, B*1509, B*2702, B*2703, B*2704, B*2705, B*2706, B*3801, B*3901, B*3902, and B*7301. Other allele-specific HLA molecules predicted to be members of the B27 supertype are shown in Table VI. Peptide binding to each of the allele-specific HLA molecules can be modulated by substitutions at primary and/or secondary anchor positions, preferably choosing respective residues specified for the supermotif.

Representative peptide epitopes that comprise the B27 supermotif are set forth in Table XII.

### IV.D.7. HLA-B44 supermotif

The HLA-B44 supermotif is characterized by the presence in peptide ligands of negatively charged (D or E) residues as a primary anchor in position 2, and hydrophobic residues (F, W, Y, L, I, M, V, or A) as a primary anchor at the C-terminal position of the epitope *(see, e.g.,* Sidney et al., *Immunol. Today 17:261,* 1996). Exemplary members of the corresponding family of HLA molecules that bind to the B44 supermotif (*i.e.,* the B44 supertype) include at least: B*1801, B*1802, B*3701, B*4001, B*4002, B*4006, B*4402, B*4403, and B*4404. Peptide binding to each of the allele-specific HLA molecules can be modulated by substitutions at primary and/or secondary anchor positions; preferably choosing respective residues specified for the supermotif.

### IV.D.8. HLA-B58 supermotif

The HLA-B58 supermotif is characterized by the presence in peptide ligands of a small aliphatic residue (A, S, or T) as a primary anchor residue at position 2, and an aromatic or hydrophobic residue (F, W, Y, L, I, V, M, or A) as a primary anchor residue at the C-terminal position of the epitope *(see, e.g.,* Sidney and Sette, *Immunogenetics 1999 Nov;50(3-4):201-12,* Review). Exemplary members of the corresponding family of HLA molecules that bind to the B58 supermotif (*i.e.,* the B58 supertype) include at least: B*1516, B*1517, B*5701, B*5702, and B*5801. Other allele-specific HLA molecules predicted to be members of the B58 supertype are shown in Table VI. Peptide binding to each of the allele-specific HLA molecules can be modulated by substitutions at primary and/or secondary anchor positions, preferably choosing respective residues specified for the supermotif.

Representative peptide epitopes that comprise the B58 supermotif are set forth in Table XIII.

### IV.D.9. HLA-B62 supermotif

The HLA-B62 supermotif is characterized by the presence in peptide ligands of the polar aliphatic residue Q or a hydrophobic aliphatic residue (L, V, M, I, or P) as a primary anchor in position 2, and a hydrophobic residue (F, W, Y, M, I, V, L, or A) as a primary anchor at the C-terminal position of the epitope *(see, e.g.,* Sidney and Sette, *Immunogenetics 1999 Nov;50(3-4):201-12,* Review). Exemplary members of the corresponding family of HLA molecules that bind to the B62 supermotif (*i.e.,* the B62 supertype) include at least: B*1501, B*1502, B*1513, and B5201. Other allele-specific HLA molecules predicted to be members of the B62 supertype are shown in Table VI. Peptide binding to each of the allele-specific HLA molecules can be modulated by substitutions at primary and/or secondary anchor positions, preferably choosing respective residues specified for the supermotif.

Representative peptide epitopes that comprise the B62 supermotif are set forth in Table XIV.

### IV.D.10. HLA-A1 motif

The HLA-A1 motif is characterized by the presence in peptide ligands of T, S, or M as a primary anchor residue at position 2 and the presence of Y as a primary anchor residue at the C-terminal position of the epitope. An alternative allele-specific A1 motif is characterized by a primary anchor residue at position 3 rather than position 2. This motif is characterized by the presence of D, E, A, or S as a primary anchor residue in position 3, and a Y as a primary anchor residue at the C-terminal position of the epitope *(see, e.g.,* DiBrino *et al.,* J. Immunol., 152:620, 1994; Kondo *et al., Immunogenetics* 45:249, 1997; and Kubo *et al., J. Immunol.* 152:3913, 1994 for reviews of relevant data). Peptide binding to HLA-A1 can be modulated by substitutions at primary and/or secondary anchor positions, preferably choosing respective residues specified for the motif.

Representative peptide epitopes that comprise either A1 motif are set forth in Table XV. Those epitopes comprising T, S, or M at position 2 and Y at the C-terminal position are also included in the listing of HLA-A1 supermotif-bearing peptide epitopes listed in Table VII, as these residues are a subset of the A1 supermotif primary anchors.

### IV.D.11. HLA-A*0201 motif

An HLA-A2*0201 motif was determined to be characterized by the presence in peptide ligands of L or M as a primary anchor residue in position 2, and L or V as a primary anchor residue at the C-terminal position of a 9-residue peptide *(see, e.g.,* Falk *et al.*, *Nature* 351:290-296, 1991) and was further found to comprise an I at position 2 and I or A at the C-terminal position of a nine amino acid peptide *(see, e.g.,* Hunt *et al.,* Science 255:1261-1263; March 6, 1992; Parker *et al., J. Immunol.* 149:3580-3587, 1992). The A*0201 allele-specific motif has also been defined by the present inventors to additionally comprise V, A, T, or Q as a primary anchor residue at position 2, and M or T as a primary anchor residue at the C-terminal position of the epitope *(see, e.g.,* Kast *et al., J. Immunol.* 152:3904-3912, 1994). Thus, the HLA-A*0201 motif comprises peptide ligands with L, I, V, M, A, T, or Q as primary anchor residues at position 2 and L, I, V, M; A, or T as a primary anchor residue at the C-terminal position of the epitope. The preferred and tolerated residues that characterize the primary anchor positions of the HLA-A*0201 motif are identical to the residues describing the A2 supermotif. (For reviews of relevant data, *see, e.g.,* del Guercio *et al., J. Immunol.* 154:685-693, 1995; Ruppert *et al., Cell* 74:929-937, 1993; Sidney *et al., Immunol. Today* 17:261-266, 1996; Sette and Sidney, *Curr. Opin. in Immunol.* 10:478-482, 1998). Secondary anchor residues that characterize the A*0201 motif have additionally been defined *(see, e.g.,* Ruppert *et al., Cell* 74:929-937, 1993). These are shown in Table II. Peptide binding to HLA-A*0201 molecules can be modulated by substitutions at primary and/or secondary anchor positions, preferably choosing respective residues specified for the motif.

Representative peptide epitopes that comprise an A*0201 motif are set forth in Table VIII. The A*0201 motifs comprising the primary anchor residues V, A, T, or Q at position 2 and L, I, V, A, or T at the C-terminal position are those most particularly relevant to the invention claimed herein.

### IV.D.12. HLA-A3 motif

The HLA-A3 motif is characterized by the presence in peptide ligands of L, M, V, I, S, A, T, F, C, G, or D as a primary anchor residue at position 2, and the presence of K, sY, R, H, F, or A as a primary anchor residue at the C-terminal position of the epitope *(see, e.g.,* DiBrino *et al., Proc. Natl. Acad. Sci USA* 90:1508, 1993; and Kubo *et al., J. Immunol.* 152:3913-3924, 1994). Peptide binding to HLA-A3 can be modulated by substitutions at primary and/or secondary anchor positions, preferably choosing respective residues specified for the motif.

Representative peptide epitopes that comprise the A3 motif are set forth in Table XVI. Those peptide epitopes that also comprise the A3 supermotif are also listed in Table IX. The A3 supermotif primary anchor residues comprise a subset of the A3- and A11-allele specific motif primary anchor residues.

### IV.D.13. HLA-A11 motif

The HLA-A11 motif is characterized by the presence in peptide ligands of V, T, M, L, I, S, A, G, N, C, D, or F as a primary anchor residue in position 2, and K, R, Y, or H as a primary anchor residue at the C-terminal position of the epitope *(see, e.g.,* Zhang *et al., Proc. Natl. Acad. Sci USA* 90:2217-2221, 1993; and Kubo *et al., J. Immunol.* 152:3913-3924, 1994). Peptide binding to HLA-A11 can be modulated by substitutions at primary and/or secondary anchor positions, preferably choosing respective residues specified for the motif.

Representative peptide epitopes that comprise the A11 motif are set forth in Table XVII; peptide epitopes comprising the A3 allele-specific motif are also present in this Table because of the extensive overlap between the A3 and A11 motif primary anchor specificities. Further, those peptide epitopes that comprise the A3 supermotif are also listed in Table IX.

### IV.D.14. HLA-A24 motif

The HLA-A24 motif is characterized by the presence in peptide ligands of Y, F, W, or M as a primary anchor residue in position 2, and F, L, I, or W as a primary anchor residue at the C-terminal position of the epitope (*see, e.g., Kondo et al.; J. Immunol.* 155:4307-4312, 1995; and Kubo *et al., J. Immunol.* 152:3913-3924, 1994). Peptide binding to HLA-A24 molecules can be modulated by substitutions at primary and/or secondary anchor positions; preferably choosing respective residues specified for the motif.

Representative peptide epitopes that comprise the A24 motif are set forth in Table XVIII. These epitopes are also listed in Table X, which sets forth HLA-A24-supermotif-bearing peptide epitopes, as the primary anchor residues characterizing the A24 allele-specific motif comprise a subset of the A24 supermotif primary anchor residues.

### Motifs Indicative of Class II HTL Inducing Peptide Epitopes

The primary and secondary anchor residues of the HLA class II peptide epitope supermotifs and motifs delineated below are summarized in Table III.

### IV.D.15. HLA DR-1-4-7 supermotif

Motifs have also been identified for peptides that bind to three common HLA class II allele-specific HLA molecules: HLA DRB1 *0401, DRB1*0101, and DRB1*0701 (*see, e.g.,* the review by Southwood *et al. J. Immunology* 160: 3363-3373,1998). Collectively, the common residues from these motifs delineate the HLA DR-1-4-7 supermotif. Peptides that bind to these DR molecules carry a supermotif characterized by a large aromatic or hydrophobic residue (Y, F, W, L, I, V, or M) as a primary anchor residue in position 1, and a small, non-charged residue (S, T, C, A, P, V, I, L, or M) as a primary anchor residue in position 6 of a 9-mer core region. Allele-specific secondary effects and secondary anchors for each of these HLA types have also been identified (Southwood *et al., supra). These are set forth in Table III. Peptide binding to HLA- DRB1*0401, DRB1 *0101, and/or DRB1*0701 can be modulated by substitutions at primary and/or secondary anchor positions, preferably choosing respective residues specified for the supermotif.

Potential epitope 9-mer core regions comprising the DR-1-4-7 supermotif, wherein position 1 of the supermotif is at position 1 of the nine-residue core, are set forth in Table XIX. Respective exemplary peptide epitopes of 15 amino acid residues in length, each of which comprise a nine residue core, are also shown, along with cross-reactive binding data for the exemplary 15-residue peptides.

### IV.D.16. HLA DR3 motifs

Two alternative motifs (*i.e.,* submotifs) characterize peptide epitopes that bind to HLA-DR3 molecules (*see, e.g.,* Geluk *et al., J. Immunol.* 152:5742, 1994). In the first motif (submotif DR3a) a large, hydrophobic residue (L, I, V, M, F, or Y) is present in anchor position 1 of a 9-mer core, and D is present as an anchor at position 4, towards the carboxyl terminus of the epitope. As in other class II motifs, core position 1 may or may not occupy the peptide N-terminal position.

The alternative DR3 submotif provides for lack of the large, hydrophobic residue at anchor position 1, and/or lack of the negatively charged or amide-like anchor residue at position 4, by the presence of a positive charge at position 6 towards the carboxyl terminus of the epitope. Thus, for the alternative allele-specific DR3 motif (submotif DR3b): L, I, V, M, F, Y, A, or Y is present at anchor position 1; D, N, Q, E, S, or T is present at anchor position 4; and K, R, or H is present at anchor position 6. Peptide binding to HLA-DR3 can be modulated by substitutions at primary and/or secondary anchor positions, preferably choosing respective residues specified for the motif.

Potential peptide epitope 9-mer core regions corresponding to a nine residue sequence comprising the DR3a submotif (wherein position 1 of the motif is at position 1 of the nine residue core) are set forth in Table XXa. Respective exemplary peptide epitopes of 15 amino acid residues in length, each of which comprise the nine residue core, are also shown in Table XXa along with binding data for the exemplary peptides.

Potential peptide epitope 9-mer core regions comprising the DR3b submotif and respective exemplary 15-mer peptides comprising the DR3 submotif-b epitope are set forth in Table XXb along with binding data for the exemplary peptides.

Each of the HLA class I or class II peptide epitopes set out in the Tables herein are deemed singly to be an inventive aspect of this application. Further, it is also an inventive aspect of this application that each peptide epitope may be used in combination with any other peptide epitope.

**IV.E. Enhancing Population Coverage of the Vaccine**

Vaccines that have broad population coverage are preferred because they are more commercially viable and generally applicable to the most people. Broad population coverage can be obtained using the peptides of the invention (and nucleic acid compositions that encode such peptides) through selecting peptide epitopes that bind to HLA alleles which, when considered in total, are present in most of the population. Table XXI lists the overall frequencies of the HLA class I supertypes in various ethnicities (Table XXIa) and the combined population coverage achieved by the A2-, A3-, and B7-supertypes (Table XXIb). The A2-, A3-, and B7 supertypes are each present on the average of over 40% in each of these five major ethnic groups. Coverage in excess of 80% is achieved with a combination of these supermotifs. These results suggest that effective and non-ethnically biased population coverage is achieved upon use of a limited number of cross-reactive peptides. Although the population coverage reached with these three main peptide specificities is high, coverage can be expanded to reach 95% population coverage and above, and more easily achieve truly multispecific responses upon use of additional supermotif or allele-specific motif bearing peptides.

The B44-, A1-, and A24-supertypes are each present, on average, in a range from 25% to 40% in these major ethnic populations (Table XXIa). While less prevalent overall, the B27-, B58-, and B62 supertypes are each present with a frequency >25% in at least one major ethnic group (Table XXIa). Table XXIb summarizes the estimated prevalence of combinations of HLA supertypes that have been identified in five major ethnic groups. The incremental coverage obtained by the inclusion of A1,- A24-, and B44-supertypes to the A2, A3, and B7 coverage and coverage obtained with all of the supertypes described herein, is shown.

The data presented herein, together with the previous definition of the A2-, A3-, and B7-supertypes, indicates that all antigens, with the possible exception of A29, B8, and B46, can be classified into a total of nine HLA supertypes. By including epitopes from the six most frequent supertypes, an average population coverags of 99% is obtained for five major ethnic groups.

**IV.F. Immune Response-Stimulating Peptide Analogs**

In general, CTL and HTL responses are not directed against all possible epitopes. Rather, they are restricted to a few "immunodominant" determinants (Zinkernagel, *et al., Adv. Immunol.* 27:5159, 1979; Bennink, *et al., J. Exp. Med.* 168:19351939, 1988; Rawle, *et al., J. Immunol.* 146:3977-3984, 1991). It has been recognized that immunodominance (Benacerraf, *et al., Science* 175:273-279, 1972) could be explained by either the ability of a given epitope to selectively bind a particular HLA protein (determinant selection theory) (Vitiello, *et al., J. Immunol.* 131:1635, 1983); Rosenthal, *et al., Nature* 267:156-158, 1977), or to be selectively recognized by the existing TCR (T cell receptor) specificities (repertoire theory) (Klein, J., IMMUNOLOGY, THE SCIENCE OF SELF/NONSELF DISCRIMINATION, John Wiley & Sons, New York, pp. 270-310, 1982). It has been demonstrated that additional factors, mostly linked to processing events, can also play a key role in dictating, beyond strict immunogenicity, which of the many potential determinants will be presented as immunodominant (Sercarz, *et al., Annu. Rev. Immunol.* 11:729-766, 1993).

Because tissue specific and developmental TAAs are expressed on normal tissue at least at some point in time or location within the body, it may be expected that T cells to them, particularly dominant epitopes, are eliminated during immunological surveillance and that tolerance is induced. However, CTL responses to tumor epitopes in both normal donors and cancer patient has been detected, which may indicate that tolerance is incomplete *(see, e.g.,* Kawashima *et al.,* Hum. Immunol. 59:1, 1998; Tsang, *J. Natl. Cancer Inst.* 87:82-90, 1995; *Rongcun et al., J. Immunol.* 163:1037, 1999). Thus, immune tolerance does not completely eliminate or inactivate CTL precursors capable of recognizing high affinity HLA class I binding peptides.

An additional strategy to overcome tolerance is to use analog peptides. Without intending to be bound by theory, it is believed that because T cells to dominant epitopes may have been clonally deleted, selecting subdominant epitopes may allow existing T cells to be recruited, which will then lead to a therapeutic or prophylactic response. However, the binding of HLA molecules to subdominant epitopes is often less vigorous than to dominant ones. Accordingly, there is a need to be able to modulate the binding affinity of particular immunogenic epitopes for one or more HLA molecules, and thereby to modulate the immune response elicited by the peptide, for example to prepare analog peptides which elicit a more vigorous response.

Although peptides with suitable cross-reactivity among all alleles of a superfamily are identified by the screening procedures described above, cross-reactivity is not always as complete as possible, and in certain cases procedures to increase cross-reactivity of peptides can be useful; moreover, such procedures can also be used to modify other properties of the peptides such as binding affinity or peptide stability. Having established the general rules that govern cross-reactivity of peptides for HLA alleles within a given motif or supermotif, modification (*i.e.,* analoging) of the structure of peptides of particular interest in order to achieve broader (or otherwise modified) HLA binding capacity can be performed. More specifically, peptides which exhibit the broadest cross-reactivity patterns, can be produced in accordance with the teachings herein. The present concepts related to analog generation are set forth in greater detail in co-pending U.S.S.N. 09/226,775 filed 1/6/99.

In brief, the strategy employed utilizes the motifs or supermotifs, which correlate with binding to certain HLA molecules. The motifs or supermotifs are defined by having primary anchors, and in many cases secondary anchors. Analog peptides can be created by substituting amino acid residues at primary anchor, secondary anchor, or at primary and secondary anchor positions. Generally, analogs are made for peptides that already bear a motif or supermotif. Preferred secondary anchor residues of supermotifs and motifs that have been defined for HLA class I and class II binding peptides are shown in Tables II and III, respectively.

For a number of the motifs or supermotifs in accordance with the invention, residues are defined which are deleterious to binding to allele-specific HLA molecules or members of HLA supertypes that bind the respective motif or supermotif (Tables II and III). Accordingly, removal of such residues that are detrimental to binding can be performed in accordance with the present invention. For example, in the case of the A3 supertype, when all peptides that have such deleterious residues are removed from the population of peptides used in the analysis, the incidence of cross-reactivity increased from 22% to 37% *(see, e.g.,* Sidney, J. *et al., Hu. Immunol.* 45:79, 1996). Thus, one strategy to improve the cross-reactivity of peptides within a given supermotif is simply to delete one or more of the deleterious residues present within a peptide and substitute a small "neutral" residue such as Ala (that may not influence T cell recognition of the peptide). An enhanced likelihood of cross-reactivity is expected if, together with elimination of detrimental residues within a peptide, "preferred" residues associated with high affinity binding to an allele-specific HLA molecule or to multiple HLA molecules within a superfamily are inserted.

To ensure that an analog peptide, when used as a vaccine, actually elicits a CTL response to the native epitope *in vivo* (or, in the case of class II epitopes, elicits helper T cells that cross-react with the wild type peptides), the analog peptide may be used to immunize T cells *in vitro* from individuals of the appropriate HLA allele. Thereafter, the immunized cells' capacity to induce lysis of wild type peptide sensitized target cells is evaluated. It will be desirable to use as antigen presenting cells, cells that have been either infected, or transfected with the appropriate genes, or, in the case of class II epitopes only, cells that have been pulsed with whole protein antigens, to establish whether endogenously produced antigen is also recognized by the relevant T cells.

Another embodiment of the invention is to create analogs of weak binding peptides, to thereby ensure adequate numbers of cross-reactive cellular binders. Class I binding peptides exhibiting binding affinities of 500-5000 nM, and carrying an acceptable but suboptimal primary anchor residue at one or both positions can be "fixed" by substituting preferred anchor residues in accordance with the respective supertype. The analog peptides can then be tested for crossbinding activity.

Another embodiment for generating effective peptide analogs involves the substitution of residues that have an adverse impact on peptide stability or solubility in, *e.g.*, a liquid environment. This substitution may occur at any position of the peptide epitope. For example, a cysteine can be substituted out in favor of $\alpha$-amino butyric acid ("B" in the single letter abbreviations for peptide sequences listed herein). Due to its chemical nature, cysteine has the propensity to form disulfide bridges and sufficiently alter the peptide structurally so as to reduce binding capacity. Substituting $\alpha$-amino butyric acid for cysteine not only alleviates this problem, but actually improves binding and crossbinding capability in certain instances *(see, e.g.,* the review by Sette *et al.,* In: Persistent Viral Infections, Eds. R. Ahmed and I. Chen, John Wiley & Sons, England, 1999).

Representative analog peptides are set forth in Tables XXII-XXVII. The Table indicates the length and sequence of the analog peptide as well as the motif or supermotif, if appropriate. The "source" column indicates the residues substituted at the indicated position numbers for the respective analog.

### IV.G. Computer Screening of Protein Sequences from Disease-Related Antigens for Supermotif- or Motif-Bearing Peptides

In order to identify supermotif- or motif-bearing epitopes in a target antigen, a native protein sequence, *e.g.*, a tumor-associated antigen, or sequences from an infectious organism, or a donor tissue for transplantation, is screened using a means for computing, such as an intellectual calculation or a computer, to determine the presence of a supermotif or motif within the sequence. The information obtained from the analysis of native peptide can be used directly to evaluate the status of the native peptide or may be utilized subsequently to generate the peptide epitope.

Computer programs that allow the rapid screening of protein sequences for the occurrence of the subject supermotifs or motifs are encompassed by the present invention; as are programs that permit the generation of analog peptides. These programs are implemented to analyze any identified amino acid sequence or operate on an unknown sequence and simultaneously determine the sequence and identify motif-bearing epitopes thereof; analogs can be simultaneously determined as well. Generally, the identified sequences will be from a pathogenic organism or a tumor-associated peptide. For example, the target TAA molecules include, without limitation, CEA, MAGE, p53 and HER2/neu.

It is important that the selection criteria utilized for prediction of peptide binding are as accurate as possible, to correlate most efficiently with actual binding. Prediction of peptides that bind, for example, to HLA-A*0201, on the basis of the presence of the appropriate.primary anchors, is positive at about a 30% rate *(see, e.g.,* Ruppert, J. *et al. Cell* 74:929, 1993). However, by extensively analyzing peptide-HLA binding data disclosed herein, data in related patent applications, and data in the art, the present inventors have developed a number of allele-specific polynomial algorithms that dramatically increase the predictive value over identification on the basis of the presence of primary anchor residues alone. These algorithms take into account not only the presence or absence of primary anchors, but also consider the positive or deleterious presence of secondary anchor residues (to account for the impact of different amino acids at different positions). The algorithms are essentially based on the premise that the overall affinity (or $\Delta G$) of peptide-HLA interactions can be approximated as a linear polynomial function of the type:

$$\Delta G = a_{1i} \times a_{2i} \times a_{3i}...\times a_{ni}$$

where $a_{ji}$ is a coefficient that represents the effect of the presence of a given amino acid (*j*) at a given position (i) along the sequence of a peptide of *n* amino acids. An important assumption of this method is that the effects at each position are essentially independent of each other. This assumption is justified by studies that demonstrated that peptides are bound to HLA molecules and recognized by T cells in essentially an extended conformation. Derivation of specific algorithm coefficients has been described, for example, in Gulukota, K. *et al., J. Mol. Biol.* 267:1258, 1997.

Additional methods to identify preferred peptide sequences, which also make use of specific motifs, include the use of neural networks and molecular modeling programs *(see, e.g.,* Milik *et al., Nature Biotechnology* 16:753, 1998, Altuvia *et al., Hum: Immunol.* 58:1, 1997; Altuvia *et al, J. Mol. Biol.* 249:244, 1995; Buus, S. *Curr. Opin. Immunol.* 11:209-213, 1999; Brusic, V. *et al., Bioinformatics* 14:121-130, 1998; Parker *et al., J. Immunol.* 152:163, 1993; Meister *et al., Vaccine* 13:581, 1995; Hammer *et al., J. Exp. Med.* 180:2353, 1994; Sturniolo *et al., Nature Biotechnol.* 17:555 1999).

For example, it has been shown that in sets of A*0201 motif-bearing peptides containing at least one preferred secondary anchor residue while avoiding the presence of any deleterious secondary anchor residues, 69% of the peptides will bind A*0201 with an $IC_{50}$ less than 500 nM (Ruppert, J. *et al. Cell* 74:929, 1993). These algorithms are also flexible in that cut-off scores may be adjusted to select sets of peptides with greater or lower predicted binding properties, as desired.

In utilizing computer screening to identify peptide epitopes, a protein sequence or translated sequence may be analyzed using software developed to search for motifs, for example the "FINDPATTERNS' program (Devereux, *et al. Nucl. Acids Res.* 12:387-395, 1984) or MotifSearch 1.4 software program (D. Brown, San Diego, CA) to identify potential peptide sequences containing appropriate HLA binding motifs. The identified peptides can be scored using customized polynomial algorithms to predict their capacity to bind specific HLA class I or class II alleles. As appreciated by one of ordinary skill in the art, a large array of computer programming software and hardware options are available in the relevant art which can be employed to implement the motifs of the invention in order to evaluate (e.g., without limitation, to identify epitopes, identify epitope concentration per peptide length, or to generate analogs) known or unknown peptide sequences.

In accordance with the procedures described above, HER2/neu peptide epitopes and analogs thereof that are able to bind HLA supertype groups or allele-specific HLA molecules have been identified (Tables VII-XX; Table XXII-XXXI).

**IV.H. Preparation of Peptide Epitopes**

Peptides in accordance with the invention can be prepared synthetically, by recombinant DNA technology or chemical synthesis, or from natural sources such as native tumors or pathogenic organisms. Peptide epitopes may be synthesized individually or as polyepitopic peptides. Although the peptide will preferably be substantially free of other naturally occurring host cell proteins and fragments thereof, in some embodiments the peptides may be synthetically conjugated to native fragments or particles.

The peptides in accordance with the invention can be a variety of lengths, and either in their neutral (uncharged) forms or in forms which are salts. The peptides in accordance with the invention are either free of modifications such as glycosylation, side chain oxidation, or phosphorylation; or they contain these modifications, subject to the condition that modifications do not destroy the biological activity of the peptides as described herein.

When possible, it may be desirable to optimize HLA class I binding epitopes of the invention, such as can be used in a polyepitopic construct, to a length of about 8 to about 13 amino acid residues, often 8 to 11, preferably 9 to 10. HLA class II binding peptide epitopes of the invention may be optimized to a length of about 6 to about 30 amino acids in length, preferably to between about 13 and about 20 residues. Preferably, the peptide epitopes are commensurate in size with endogenously processed pathogen-derived peptides or tumor cell peptides that are bound to the relevant HLA molecules, however, the identification and preparation of peptides that comprise epitopes of the invention can also be carried out using the techniques described herein.

In alternative embodiments, epitopes of the invention can be linked as a polyepitopic peptide, or as a minigene that encodes a polyepitopic peptide.

In another embodiment, it is preferred to identify native peptide regions that contain a high concentration of class I and/or class II epitopes. Such a sequence is generally selected on the basis that it contains the greatest number of epitopes per amino acid length. It is to be appreciated that epitopes can be present in a nested or overlapping manner, e.g. a 10 amino acid long peptide could contain two 9 amino acid long epitopes and one 10 amino acid long epitope; upon intracellular processing, each epitope can be exposed and bound by an HLA molecule upon administration of such a peptide. This larger, preferably multi-epitopic, peptide can be generated synthetically, recombinantly, or via cleavage from the native source.

The peptides of the invention can be prepared in a wide variety of ways. For the preferred relatively short size, the peptides can be synthesized in solution or on a solid support in accordance with conventional techniques. Various automatic synthesizers are commercially available and can be used in accordance with known protocols. *(See,* for example, Stewart & Young, SOLID PHASE PEPTIDE SYNTHESIS, 2D. ED., Pierce Chemical Co., 1984). Further, individual peptide epitopes can be joined using chemical ligation to produce larger peptides that are still within the bounds of the invention.

Alternatively, recombinant DNA technology can be employed wherein a nucleotide sequence which encodes an immunogenic peptide of interest is inserted into an expression vector, transformed or transfected into an appropriate host cell and cultivated under conditions suitable for expression. These procedures are generally known in the art, as described generally in Sambrook *et al.,* MOLECULAR CLONING, A LABORATORY MANUAL, Cold Spring Harbor Press, Cold Spring Harbor, New York (1989). Thus, recombinant polypeptides which comprise one or more peptide sequences of the invention can be used to present the appropriate T cell epitope.

The nucleotide coding sequence for peptide epitopes of the preferred lengths contemplated herein can be synthesized by chemical techniques, for example, the phosphotriester method of Matteucci, *et al., J. Am. Chem. Soc.* 103:3185 (1981). Peptide analogs can be made simply by substituting the appropriate and desired nucleic acid base (s) for those that encode the native peptide sequence; exemplary nucleic acid substitutions are those that encode an amino acid defined by the motifs/supermotifs herein. The coding sequence can then be provided with appropriate linkers and ligated into expression vectors commonly available in the art, and the vectors used to transform suitable hosts to produce the desired fusion protein. A number of such vectors and suitable host systems are now available. For expression of the fusion proteins, the coding sequence will be provided with operably linked start and stop codons, promoter and terminator regions and usually a replication system to provide an expression vector for expression in the desired cellular host. For example, promoter sequences compatible with bacterial hosts are provided in plasmids containing convenient restriction sites for insertion of the desired coding sequence. The resulting expression vectors are transformed into suitable bacterial hosts. Of course, yeast, insect or mammalian cell hosts may also be used, employing suitable vectors and control sequences.

### IV.I. Assays to Detect T-Cell Responses

Once HLA binding peptides are identified, they can be tested for the ability to elicit a T-cell response. The preparation and evaluation of motif-bearing peptides are described in PCT publications WO 94/20127 and WO 94/03205. Briefly, peptides comprising epitopes from a particular antigen are synthesized and tested for their ability to bind to the appropriate HLA proteins. These assays may involve evaluating the binding of a peptide of the invention to purified HLA class I molecules in relation to the binding of a radioiodinated reference peptide. Alternatively, cells expressing empty class I molecules (*i.e.* lacking peptide therein) may be evaluated for peptide binding by immunofluorescent staining and flow microfluorimetry. Other assays that may be used to evaluate peptide binding include peptide-dependent class I assembly assays and/or the inhibition of CTL recognition by peptide competition. Those peptides that bind to the class I molecule, typically with an affinity of 500 nM or less, are further evaluated for their ability to serve as targets for CTLs derived from infected or immunized individuals, as well as for their capacity to induce primary *in vitro* or *in vivo* CTL responses that can give rise to CTL populations capable of reacting with selected target cells associated with a disease. Corresponding assays are used for evaluation of HLA class II binding peptides. HLA class II motif-bearing peptides that are shown to bind, typically at an affinity of 1000 nM or less, are further evaluated for the ability to stimulate HTL responses.

Conventional assays utilized to detect T cell responses include proliferation assays, lymphokine secretion assays, direct cytotoxicity assays, and limiting dilution assays. For example, antigen-presenting cells that have been incubated with a peptide can be assayed for the ability to induce CTL responses in responder cell populations. Antigen-presenting cells can be normal cells such as peripheral blood mononuclear cells or dendritic cells. Alternatively, mutant non-human mammalian cell lines that are deficient in their ability to load class I molecules with internally processed peptides and that have been transfected with the appropriate human class I gene, may be used to test for the capacity of the peptide to induce *in vitro* primary CTL responses.

Peripheral blood mononuclear cells (PBMCs) may be used as the responder cell source of CTL precursors. The appropriate antigen-presenting cells are incubated with peptide, after which the peptide-loaded antigen-presenting cells are then incubated with the responder cell population under optimized culture conditions. Positive CTL activation can be determined by assaying the culture for the presence of CTLs that kill radio-labeled target cells, both specific peptide-pulsed targets as well as target cells expressing endogenously processed forms of the antigen from which the peptide sequence was derived.

More recently, a method has been devised which allows direct quantification of antigen-specific T cells by staining with Fluorescein-labelled HLA tetrameric complexes (Altman, J. D. *et al.*, *Proc. Natl. Acad. Sci. USA* 90:10330, 1993; Altman, J. D. *et al., Science* 274:94, 1996). Other relatively recent technical developments include staining for intracellular lymphokines, and interferon-$\gamma$ release assays or ELISPOT assays. Tetramer staining, intracellular lymphokine staining and ELISPOT assays all appear to be at least 10-fold more sensitive than more conventional assays (Lalvani, A. *et al., J. Exp. Med.* 186:859, 1997; Dunbar, P. R. *et al., Curr. Biol.* 8:413, 1998; Murali-Krishna, K. *et al., Immunity* 8:177, 1998).

HTL activation may also be assessed using such techniques known to those in the art such as T cell proliferation and secretion of lymphokines, *e.g.* IL-2 *(see, e.g.* Alexander *et al.,* Immunity 1:751-761, 1994).

Alternatively, immunization of HLA transgenic mice can be used to determine immunogenicity of peptide epitopes. Several transgenic mouse models including mice with human A2.1, A11 (which can additionally be used to analyze HLA-A3 epitopes), and B7 alleles have been characterized and others (e.g., transgenic mice for HLA-A1 and A24) are being developed. HLA-DR1 and HLA-DR3 mouse models have also been developed. Additional transgenic mouse models with other HLA alleles may be generated as necessary. Mice may be immunized with peptides emulsified in Incomplete Freund's Adjuvant and the resulting T cells tested for their capacity to recognize peptide-pulsed target cells and target cells transfected with appropriate genes. CTL responses may be analyzed using cytotoxicity assays described above. Similarly, HTL responses may be analyzed using such assays as T cell proliferation or secretion of lymphokines.

## IV.J. Use of Peptide Epitopes as Diagnostic Agents and for Evaluating Immune Responses

In one embodiment of the invention, HLA class I and class II binding peptides as described herein are used as reagents to evaluate an immune response. The immune response to be evaluated is induced by using as an immunogen any agent that may result in the production of antigen-specific CTLs or HTLs that recognize and bind to the peptide epitope(s) to be employed as the reagent. The peptide reagent need not be used as the immunogen. Assay systems that are used for such an analysis include relatively recent technical developments such as tetramers, staining for intracellular lymphokines and interferon release assays, or ELISPOT assays.

For example, peptides of the invention are used in tetramer staining assays to assess peripheral blood mononuclear cells for the presence of antigen-specific CTLs following exposure to a tumor cell antigen or an immunogen. The HLA-tetrameric complex is used to directly visualize antigen-specific CTLs *(see, e.g.,* Ogg *et al., Science* 279: 2103-2106, 1998; and Altman *et al., Science* 174:94-96, 1996) and determine the frequency of the antigen-specific CTL population in a sample of peripheral blood mononuclear cells. A tetramer reagent using a peptide of the invention is generated as follows: A peptide that binds to an HLA molecule is refolded in the presence of the corresponding HLA heavy chain and $\beta_2$-microglobulin to generate a trimolecular complex. The complex is biotinylated at the carboxyl terminal end of the heavy chain at a site that was previously engineered into the protein. Tetramer formation is then induced by the addition of streptavidin. By means of fluorescently labeled streptavidin, the tetramer can be used to stain antigen-specific cells. The cells can then be identified, for example, by flow cytometry. Such an analysis may be used for diagnostic or prognostic purposes. Cells identified by the procedure can also be used for therapeutic purposes.

Peptides of the invention are also used as reagents to evaluate immune recall responses *(see, e.g.,* Bertoni *et al., J. Clin. Invest.* 100:503-513, 1997 and Penna *et al., J. Exp. Med.* 174:1565-1570, 1991). For example, patient PBMC samples from individuals with cancer are analyzed for the presence of antigen-specific CTLs or HTLs using specific peptides. A blood sample containing mononuclear cells can be evaluated by cultivating the PBMCs and stimulating the cells with a peptide of the invention. After an appropriate cultivation period, the expanded cell population can be analyzed, for example, for CTL or for HTL activity.

The peptides are also used as reagents to evaluate the efficacy of a vaccine PBMCs obtained from a patient vaccinated with an immunogen are analyzed using, for example, either of the methods described above. The patient is HLA typed, and peptide epitope reagents that recognize the allele-specific molecules present in that patient are selected for the analysis. The immunogenicity of the vaccine is indicated by the presence of epitope-specific CTLs and/or HTLs in the PBMC sample.

The peptides of the invention are also used to make antibodies, using techniques well known in the art (see, *e.g. CURRENT PROTOCOLS IN IMMUNOLOGY,* Wiley/Greene, NY; and *Antibodies A Laboratory Manual,* Harlow and Lane, Cold Spring Harbor Laboratory Press, 1989), which may be useful as reagents to diagnose or monitor cancer. Such antibodies include those that recognize a peptide in the context of an HLA molecule, *i.e.,* antibodies that bind to a peptide-MHC complex.

**IV.K. Vaccine Compositions**

Vaccines and methods of preparing vaccines that contain an immunogenically effective amount of one or more peptides as described herein are further embodiments of the invention. Once appropriately immunogenic epitopes have been defined, they can be sorted and delivered by various means, herein referred to as "vaccine" compositions. Such vaccine compositions can include, for example, lipopeptides *(e.g.,*Vitiello, *A. et al., J. Clin. Invest.* 95:341, 1995), peptide compositions encapsulated in poly(DL-lactide-co-glycolide) ("PLG") microspheres *(see, e.g.,* Eldridge, *et al., Molec. Immunol.* 28:287-294, 1991: Alonso *et al., Vaccine* 12:299-306, 1994; Jones *et al., Vaccine* 13:675-681, 1995), peptide compositions contained in immune stimulating complexes (ISCOMS) *(see, e.g.,* Takahashi *et al., Nature* 344: 873-875, 1990; Hu *et al., Clin Exp Immunol.* 113:235-243, 1998), multiple antigen peptide systems (MAPs) *(see e.g.,* Tam, J. P., *Proc. Natl. Acad. Sci. U.S.A.* 85:5409-5413, 1988; Tam, J.P., *J. Immunol. Methods* 196:17-32, 1996), peptides formulated as multivalent peptides; peptides for use in ballistic delivery systems, typically crystallized peptides, viral delivery vectors (Perkus, M. E. *et al.,* In: *Concepts in vaccine development,* Kaufmann, S. H. E., ed., p. 379, 1996; Chakrabarti, S. *et al., Nature* 320:535, 1986; Hu, S. L. *et al., Nature* 320:537, 1986; Kieny, M.-P. *et al., AIDS Biol Technology* 4:790, 1986; Top, F. H. *et al., J. Infect. Dis.* 124:148, 1971; Chanda, P. *K. et al., Virology* 175:535, 1990), particles of viral or synthetic origin *(e.g.,* Kofler, N. *et al., J. Immunol. Methods.* 192:25, 1996; Eldridge, J. H. *et al., Sem. Hematol.* 30:16, 1993; Falo, L. D., Jr. *et al., Nature Med.* 7:649, 1995), adjuvants (Warren, H. S., Vogel, F. R., and Chedid, L. A. *Annu. Rev. Immunol.* 4:369, 1986; Gupta, R. K. *et al., Vaccine* 11:293, 1993), liposomes (Reddy, R. *et al., J. Immunol.* 148:1585, 1992; Rock, K. L., *Immunol. Today* 17:131, 1996), or, naked or particle absorbed cDNA (Ulmer, J. B. *et al., Science* 259:1745, 1993; Robinson, H. L., Hunt, L. A., and Webster, R. G., *Vaccine* 11:957, 1993; Shiver, J. W. *et al.,* In: *Concepts in vaccine development,* Kaufmann, S. H. E., ed., p. 423, 1996; Cease, K. B., and Berzofsky, J. A., *Annu. Rev. Immunol.* 12:923, 1994 and Eldridge, J. H. *et al., Sem. Hematol.* 30:16, 1993). Toxintargeted delivery technologies, also known as receptor mediated targeting, such as those of Avant Immunotherapeutics, Inc. (Needham, Massachusetts) may also be used.

Vaccines of the invention include nucleic acid-mediated modalities. DNA or RNA encoding one or more of the peptides of the invention can also be administered to a patient. This approach is described, for instance, in Wolff *et. al., Science* 247:1465 (1990) as well as U.S. Patent Nos. 5,580,859; 5,589,466; 5,804,566; 5,739,118; 5,736,524; 5,679,647; WO 98/04720; and in more detail below. Examples of DNA-based delivery technologies include "naked DNA", facilitated (bupivicaine, polymers, peptide-mediated) delivery, cationic lipid complexes, and particle-mediated ("gene gun") or pressure-mediated delivery *(see, e.g.*, U.S. Patent No. 5,922,687).

For therapeutic or prophylactic immunization purposes, the peptides of the invention can also be expressed by viral or bacterial vectors. Examples of expression vectors include attenuated viral hosts, such as vaccinia or fowlpox. As an example of this approach, vaccinia virus is used as a vector to express nucleotide sequences that encode the peptides of the invention. Upon introduction into a host bearing a tumor, the recombinant vaccinia virus expresses the immunogenic peptide, and thereby elicits a host CTL and/or HTL response. Vaccinia vectors and methods useful in immunization protocols are described in, *e.g.*, U.S. Patent No. 4,722,848. Another vector is BCG (Bacille Calmette Guerin). BCG vectors are described in Stover *et al., Nature* 351:456-460 (1991). A wide variety of other vectors useful for therapeutic administration or immunization of the peptides of the invention, e.g. adeno and adeno-associated virus vectors, retroviral vectors, *Salmonella typhi* vectors, detoxified anthrax toxin vectors, and the like, will be apparent to those skilled in the art from the description herein.

Furthermore, vaccines in accordance with the invention encompass compositions of one or more of the claimed peptides. A peptide can be present in a vaccine individually. Alternatively, the peptide can exist as a homopolymer comprising multiple copies of the same peptide, or as a heteropolymer of various peptides. Polymers have the advantage of increased immunological reaction and, where different peptide epitopes are used to make up the polymer, the additional ability to induce antibodies and/or CTLs that react with different antigenic determinants of the pathogenic organism or tumor-related peptide targeted for an immune response. The composition can be a naturally occurring region of an antigen or can be prepared, *e.g.*, recombinantly or by chemical synthesis.

Carriers that can be used with vaccines of the invention are well known in the art, and include, *e.g.*, thyroglobulin, albumins such as human serum albumin, tetanus toxoid, polyamino acids such as poly L-lysine, poly L-glutamic acid, influenza, hepatitis B virus core protein, and the like. The vaccines can contain a physiologically tolerable (*i.e.*, acceptable) diluent such as water, or saline, preferably phosphate buffered saline. The vaccines also typically include an adjuvant. Adjuvants such as incomplete Freund's adjuvant, aluminum phosphate, aluminum hydroxide, or alum are examples of materials well known in the art. Additionally, as disclosed herein, CTL responses can be primed by conjugating peptides of the invention to lipids, such as tripalmitoyl-S-glycerylcysteinlyseryl- serine ($P_3CSS$).

Upon immunization with a peptide composition in accordance with the invention, via injection, aerosol, oral, transdermal, transmucosal, intrapleural, intrathecal, or other suitable routes, the immune system of the host responds to the vaccine by producing large amounts of CTLs and/or HTLs specific for the desired antigen. Consequently, the host becomes at least partially immune to later infection, or at least partially resistant to developing an ongoing chronic infection, or derives at least some therapeutic benefit when the antigen was tumor-associated.

In some embodiments, it may be desirable to combine the class I peptide components with components that induce or facilitate neutralizing antibody and or helper T cell responses to the target antigen of interest. A preferred embodiment of such a composition comprises class I and class II epitopes in accordance with the invention. An alternative embodiment of such a composition comprises a class I and/or class II epitope in accordance with the invention, along with a cross-binding HLA class II epitope such as PADRE$^{TM}$ (Epimmune, San Diego, CA) molecule (described, for example, in U.S. Patent Number 5,736,142).

A vaccine of the invention can also include antigen-presenting cells (APC), such as dendritic cells (DC), as a vehicle to present peptides of the invention. Vaccine compositions can be created *in vitro,* following dendritic cell mobilization and harvesting, whereby loading of dendritic cells occurs *in vitro.* For example, dendritic cells are transfected, *e.g.*, with a minigene in accordance with the invention, or are pulsed with peptides. The dendritic cell can then be administered to a patient to elicit immune responses in *vivo.*

Vaccine compositions, either DNA- or peptide-based, can also be *administered in vivo* in combination with dendritic cell mobilization whereby loading of dendritic cells occurs *in vivo.*

Antigenic peptides are used to elicit a CTL and/or HTL response *ex vivo,* as well. The resulting CTL or HTL cells, can be used to treat tumors in patients that do not respond to other conventional forms of therapy, or will not respond to a therapeutic vaccine peptide or nucleic acid in accordance with the invention. *Ex vivo* CTL or HTL responses to a particular tumor-associated antigen are induced by incubating in tissue culture the patient's, or genetically compatible, CTL or HTL precursor cells together with a source of antigen-presenting cells, such as dendritic cells, and the appropriate immunogenic peptide. After an appropriate incubation time (typically about 7-28 days), in which the precursor cells are activated and expanded into effector cells, the cells are infused back into the patient, where they will destroy (CTL) or facilitate destruction (HTL) of their specific target cell (an infected cell or a tumor cell). Transfected dendritic cells may also be used as antigen presenting cells.

The vaccine compositions of the invention can also be used in combination with other treatments used for cancer, including use in combination with immune adjuvants such as IL-2, IL-12, GM-CSF, and the like.

Preferably, the following principles are utilized when selecting an array of epitopes for inclusion in a polyepitopic composition for use in a vaccine, or for selecting discrete epitopes to be included in a vaccine and/or to be encoded by nucleic acids such as a minigene. Examples of epitopes that can be utilized in a vaccine to treat or prevent cancer are provided in Tables XXIII-XXVII and XXXI. It is preferred that each of the following principles are balanced in order to make the selection. The multiple epitopes to be incorporated in a given vaccine composition can be, but need not be, contiguous in sequence in the native antigen from which the epitopes are derived.

1.) Epitopes are selected which, upon administration, mimic immune responses that have been observed to be correlated with tumor clearance. For HLA Class I this includes 3-4 epitopes that come from at least one TAA. For HLA Class II a similar rationale is employed; again 3-4 epitopes are selected from at least one TAA *(see e.g.,* Rosenberg *et al., Science* 278:1447-1450). Epitopes from one TAA may be used in combination with epitopes from one or more additional TAAs to produce a vaccine that targets tumors with varying expression patterns of frequently-expressed TAAs as described, *e.g.*, in Example 15.

2.) Epitopes are selected that have the requisite binding affinity established to be correlated with immunogenicity for HLA Class I an IC$_{50}$ of 500 nM or less, or for Class II an IC$_{50}$ of 1000 nM or less.

3.) Sufficient supermotif bearing-peptides, or a sufficient array of allele-specific motif-bearing peptides, are selected to give broad population coverage. For example, it is preferable to have at least 80% population coverage. A Monte Carlo analysis, a statistical evaluation known in the art, can be employed to assess the breadth, or redundancy of, population coverage.

4.) When selecting epitopes from cancer-related antigens it is often useful to select analogs because the patient may have developed tolerance to the native epitope. When selecting epitopes for infectious disease-related antigens it is preferable to select either native or analoged epitopes.

5.) Of particular relevance are epitopes referred to as "nested epitopes." Nested epitopes occur where at least two epitopes overlap in a given peptide sequence. A nested peptide sequence can comprise both HLA class I and HLA class II epitopes. When providing nested epitopes, a general objective is to provide the greatest number of epitopes per sequence. Thus, an aspect is to avoid providing a peptide that is any longer than the amino terminus of the amino terminal epitope and the carboxyl terminus of the carboxyl terminal epitope in the peptide. When providing a multi-epitopic sequence, such as a sequence comprising nested epitopes, it is generally important to screen the sequence in order to insure that it does not have pathological or other deleterious biological properties.

6.) If a polyepitopic protein is created, or when creating a minigene, an objective is to generate the smallest peptide that encompasses the epitopes of interest. This principle is similar, if not the same as that employed when selecting a peptide comprising nested epitopes. However, with an artificial polyepitopic peptide, the size minimization objective is balanced against the need to integrate any spacer sequences between epitopes in the polyepitopic protein. Spacer amino acid residues can, for example, be introduced to avoid junctional epitopes (an epitope recognized by the immune system, not present in the target antigen, and only created by the man-made juxtaposition of epitopes), or to facilitate cleavage between epitopes and thereby enhance epitope presentation. Junctional epitopes are generally to be avoided because the recipient may generate an immune response to that non-native epitope. Of particular concern is a junctional epitope that is a "dominant epitope." A dominant epitope may lead to such a zealous response that immune responses to other epitopes are diminished or suppressed.

### IV.K.1. Minigene Vaccines

A number of different approaches are available which allow simultaneous delivery of multiple epitopes. Nucleic acids encoding the peptides of the invention are a particularly useful embodiment of the invention. Epitopes for inclusion in a minigene are preferably selected according to the guidelines set forth in the previous section. A preferred means of administering nucleic acids encoding the peptides of the invention uses minigene constructs encoding a peptide comprising one or multiple epitopes of the invention.

The use of multi-epitope minigenes is described below and in, *e.g.*, co-pending application U.S.S.N. 09/311,784; Ishioka *et al., J. Immunol.* 162:3915-3925, 1999; An, L. and Whitton, J. L., *J. Virol.* 71:2292, 1997; Thomson, S. A. *et al., J. Immunol.* 157:822, 1996; Whitton, J. L. *et al., J. Virol.* 67:348, 1993; Hanke, R. *et al., Vaccine* 16:426, 1998. For example, a multi-epitope DNA plasmid encoding supermotif-and/or motif-bearing HER2/neu epitopes derived from multiple regions of HER2/neu,a universal helper T epitope, *e.g.*, PADRE™ (or multiple HTL epitopes from HER2/neu), and an endoplasmic reticulum-translocating signal sequence can be engineered. A vaccine may also comprise epitopes, in addition to HER2/neu epitopes, that are derived from other TAAs.

The immunogenicity of a multi-epitopic minigene can be tested in transgenic mice to evaluate the magnitude of CTL induction responses against the epitopes tested. Further, the immunogenicity of DNA-encoded epitopes *in vivo* can be correlated with the *in vitro* responses of specific CTL lines against target cells transfected with the DNA plasmid. Thus, these experiments can show that the minigene serves to both: 1.) generate a CTL response and 2.) that the induced CTLs recognized cells expressing the encoded epitopes.

For example, to create a DNA sequence encoding the selected epitopes (minigene) for expression in human cells, the amino acid sequences of the epitopes may be reverse translated. A human codon usage table can be used to guide the codon choice for each amino acid. These epitope-encoding DNA sequences may be directly adjoined, so that when translated, a continuous polypeptide sequence is created. To optimize expression and/or immunogenicity, additional elements can be incorporated into the minigene design. Examples of amino acid sequences that can be reverse translated and included in the minigene sequence include: HLA class I epitopes, HLA class II epitopes, a ubiquitination signal sequence, and/or an endoplasmic reticulum targeting signal. In addition, HLA presentation of CTL and HTL epitopes may be improved by including synthetic (e.g. poly-alanine) or naturally-occurring flanking sequences adjacent to the CTL or HTL epitopes; these larger peptides comprising the epitope(s) are within the scope of the invention.

The minigene sequence may be converted to DNA by assembling oligonucleotides that encode the plus and minus strands of the minigene. Overlapping oligonucleotides (30-100 bases long) may be synthesized, phosphorylated, purified and annealed under appropriate conditions using well known techniques. The ends of the oligonucleotides can be joined, for example, using T4 DNA ligase. This synthetic minigene, encoding the epitope polypeptide, can then be cloned into a desired expression vector.

Standard regulatory sequences well known to those of skill in the art are preferably included in the vector to ensure expression in the target cells. Several vector elements are desirable: a promoter with a down-stream cloning site for minigene insertion; a polyadenylation signal for efficient transcription termination; an *E. coli* origin of replication; and an *E. coli* selectable marker *(e.g.* ampicillin or kanamycin resistance). Numerous promoters can be used for this purpose, *e.g.,* the human cytomegalovirus (hCMV) promoter. See, *e.g.*, U.S. Patent Nos. 5,580,859 and 5,589,466 for other suitable promoter sequences.

Additional vector modifications may be desired to optimize minigene expression and immunogenicity. In some cases, introns are required for efficient gene expression, and one or more synthetic or naturally-occurring introns could be incorporated into the transcribed region of the minigene. The inclusion of mRNA stabilization sequences and sequences for replication in mammalian cells may also be considered for increasing minigene expression.

Once an expression vector is selected, the minigene is cloned into the polylinker region downstream of the promoter. This plasmid is transformed into an appropriate E. *coli* strain, and DNA is prepared using standard techniques. The orientation and DNA sequence of the minigene, as well as all other elements included in the vector, are confirmed using restriction mapping and DNA sequence analysis. Bacterial cells harboring the correct plasmid can be stored as a master cell bank and a working cell bank.

In addition, immunostimulatory sequences (ISSs or CpGs) appear to play a role in the immunogenicity of DNA vaccines. These sequences may be included in the vector, outside the minigene coding sequence, if desired to enhance immunogenicity.

In some embodiments, a bi-cistronic expression vector which allows production of both the minigene-encoded epitopes and a second protein (included to enhance or decrease immunogenicity) can be used. Examples of proteins or polypeptides that could beneficially enhance the immune response if co-expressed include cytokines (e.g., IL-2, IL-12, GM-CSF), cytokine-inducing molecules (e.g., LeIF), costimulatory molecules, or for HTL responses, pan-DR binding proteins (PADRE™, Epimmune, San Diego, CA). Helper (HTL) epitopes can be joined to intracellular targeting signals and expressed separately from expressed CTL epitopes; this allows direction of the HTL epitopes to a cell compartment different than that of the CTL epitopes. If required, this could facilitate more efficient entry of HTL epitopes into the HLA class II pathway, thereby improving HTL induction. In contrast to HTL or CTL induction, specifically decreasing the immune response by co-expression of immunosuppressive molecules *(e.g.* TGF-β) may be beneficial in certain diseases.

Therapeutic quantities of plasmid DNA can be produced for example, by fermentation in *E. coli,* followed by purification. Aliquots from the working cell bank are used to inoculate growth medium, and grown to saturation in shaker flasks or a bioreactor according to well known techniques. Plasmid DNA can be purified using standard bioseparation technologies such as solid phase anion-exchange resins supplied by QIAGEN, Inc. (Valencia, California). If required, supercoiled DNA can be isolated from the open circular and linear forms using gel electrophoresis or other methods.

Purified plasmid DNA can be prepared for injection using a variety of formulations. The simplest of these is reconstitution of lyophilized DNA in sterile phosphate-buffered saline (PBS). This approach, known as "naked DNA," is currently being used for intramuscular (IM) administration in clinical trials. To maximize the immunotherapeutic effects of minigene DNA vaccines, an alternative method for formulating purified plasmid DNA may be desirable. A variety of methods have been described, and new techniques may become available. Cationic lipids, glycolipids, and fusogenic liposomes can also be used in the formulation (see, *e.g.,* as described by WO 93/24640; Mannino & Gould-Fogerite, *BioTechniques* 6(7): 682 (1988); U.S. Pat No. 5,279,833; WO 91/06309; and Felgner, *et al., Proc. Nat'l Acad. Sci. USA* 84:7413 (1987). In addition, peptides and compounds referred to collectively as protective, interactive, non-condensing compounds (PINC) could also be complexed to purified plasmid DNA to influence variables such as stability, intramuscular dispersion, or trafficking to specific organs or cell types.

Target cell sensitization can be used as a functional assay for expression and HLA class I presentation of minigene-encoded CTL epitopes. For example, the plasmid DNA is introduced into a mammalian cell line that is suitable as a target for standard CTL chromium release assays. The transfection method used will be dependent on the final formulation. Electroporation can be used for "naked" DNA, whereas cationic lipids allow direct *in vitro* transfection. A plasmid expressing green fluorescent protein (GFP) can be co-transfected to allow enrichment of transfected cells using fluorescence activated cell sorting (FACS). These cells are then chromium-51 ($^{51}$Cr) labeled and used as target cells for epitope-specific CTL lines; cytolysis, detected by $^{51}$Cr release, indicates both production of, and HLA presentation of, minigene-encoded CTL epitopes. Expression of HTL epitopes may be evaluated in an analogous manner using assays to assess HTL activity.

*In vivo* immunogenicity is a second approach for functional testing of minigene DNA formulations. Transgenic mice expressing appropriate human HLA proteins are immunized with the DNA product. The dose and route of administration are formulation dependent (e.g., IM for DNA in PBS, intraperitoneal (IP) for lipid-complexed DNA). Twenty-one days after immunization, splenocytes are harvested and restimulated for one week in the presence of peptides encoding each epitope being tested. Thereafter, for CTL effector cells, assays are conducted for cytolysis of peptide-loaded, $^{51}$Cr-labeled target cells using standard techniques. Lysis of target cells that were sensitized by HLA loaded with peptide epitopes, corresponding to minigene-encoded epitopes, demonstrates DNA vaccine function for *in vivo* induction of CTLs. Immunogenicity of HTL epitopes is evaluated in transgenic mice in an analogous manner.

Alternatively, the nucleic acids can be administered using ballistic delivery as described, for instance, in U.S. Patent No. 5,204,253. Using this technique, particles comprised solely of DNA are administered. In a further alternative embodiment, DNA can be adhered to particles, such as gold particles.

Minigenes can also be delivered using other bacterial or viral delivery systems well known in the art, *e.g.*, an expression construct encoding epitopes of the invention can be incorporated into a viral vector such as vaccinia.

### IV.K.2. Combinations of CTL Peptides with Helper Peptides

Vaccine compositions comprising the peptides of the present invention, or analogs thereof, which have immunostimulatory activity may be modified to provide desired attributes, such as improved serum half-life, or to enhance immunogenicity.

For instance, the ability of a peptide to induce CTL activity can be enhanced by linking the peptide to a sequence which contains at least one epitope that is capable of inducing a T helper cell response. The use of T helper epitopes in conjunction with CTL epitopes to enhance immunogenicity is illustrated, for example, in the co-pending applications U.S.S.N. 08/820,360, U.S.S.N. 08/197,484, and U.S.S.N. 08/464,234.

Although a CTL peptide can be directly linked to a T helper peptide, often CTL epitope/HTL epitope conjugates are linked by a spacer molecule. The spacer is typically comprised of relatively small, neutral molecules, such as amino acids or amino acid mimetics, which are substantially uncharged under physiological conditions. The spacers are typically selected from, *e.g.*, Ala, Gly, or other neutral spacers of nonpolar amino acids or neutral polar amino acids. It will be understood that the optionally present spacer need not be comprised of the same residues and thus may be a hetero- or homo-oligomer. When present, the spacer will usually be at least one or two residues, more usually three to six residues and sometimes 10 or more residues. The CTL peptide epitope can be linked to the T helper peptide epitope either directly or via a spacer either at the amino or carboxy terminus of the CTL peptide. The amino terminus of either the immunogenic peptide or the T helper peptide may be acylated.

In certain embodiments, the T helper peptide is one that is recognized by T helper cells present in the majority of the population. This can be accomplished by selecting amino acid sequences that bind to many, most, or all of the HLA class II molecules. These are known as "loosely HLA-restricted" or "promiscuous" T helper sequences. Examples of peptides that are promiscuous include sequences from antigens such as tetanus toxoid at positions 830-843 (QYIKANSKFIGITE), *Plasmodium falciparum* circumsporozoite (CS) protein at positions 378-398 (DIEKKIAKMEKASSVFNVVNS), and *Streptococcus* 18kD protein at positions 116 (GAVDSILGGVATYGAA). Other examples include peptides bearing a DR 1-4-7 supermotif, or either of the DR3 motifs.

Alternatively, it is possible to prepare synthetic peptides capable of stimulating T helper lymphocytes, in a loosely HLA-restricted fashion, using amino acid sequences not found in nature *(see, e.g.,* PCT publication WO 95/07707). These synthetic compounds called Pan-DR-binding epitopes *(e.g.,* PADRE[TM], Epimmune, Inc., San Diego, CA) are designed to most preferrably bind most HLA-DR (human HLA class II) molecules. For instance, a pan-DR-binding epitope peptide having the formula: aKXVAAWTLKAAa, where "X" is either cyclohexylalanine, phenylalanine, or tyrosine, and "a" is either D-alanine or L-alanine, has been found to bind to most HLA-DR alleles, and to stimulate the response ofT helper lymphocytes from most individuals, regardless of their HLA type. An alternative of a pan-DR binding epitope comprises all "L" natural amino acids and can be provided in the form of nucleic acids that encode the epitope.

HTL peptide epitopes can also be modified to alter their biological properties. For example, they can be modified to include D-amino acids to increase their resistance to proteases and thus extend their serum half life, or they can be conjugated to other molecules such as lipids, proteins, carbohydrates, and the like to increase their biological activity. For example, a T helper peptide can be conjugated to one or more palmitic acid chains at either the amino or carboxyl termini.

### IV.K.3. Combinations of CTL Peptides with T Cell Priming Agents

In some embodiments it may be desirable to include in the pharmaceutical compositions of the invention at least one component which primes cytotoxic T lymphocytes. Lipids have been identified as agents capable of priming CTL *in vivo* against viral antigens. For example, palmitic acid residues can be attached to the $\varepsilon$-and $\alpha$- amino groups of a lysine residue and then linked, *e.g.*, via one or more linking residues such as Gly, Gly-Gly-, Ser, Ser-Ser, or the like, to an immunogenic peptide. The lipidated peptide can then be administered either directly in a micelle or particle, incorporated into a liposome, or emulsified in an adjuvant, *e.g.*, incomplete Freund's adjuvant. A preferred immunogenic composition comprises palmitic acid attached to $\varepsilon$- and $\alpha$- amino groups of Lys, which is attached via linkage, *e.g.*, Ser-Ser, to the amino terminus of the immunogenic peptide.

As another example of lipid priming of CTL responses, E. *coli* lipoproteins, such as tripalmitoyl-S-glyceryl-cysteinlyseryl- serine ($P_3CSS$) can be used to prime virus specific CTL when covalently attached to an appropriate peptide *(see,* e.g., Deres*, et al., Nature* 342:561, 1989). Peptides of the invention can be coupled to $P_3CSS$, for example, and the lipopeptide administered to an individual to specifically prime a CTL response to the target antigen. Moreover, because the induction of neutralizing antibodies can also be primed with $P_3CSS$-conjugated epitopes, two such compositions can be combined to more effectively elicit both humoral and cell-mediated responses.

CTL and/or HTL peptides can also be modified by the addition of amino acids to the termini of a peptide to provide for ease of linking peptides one to another, for coupling to a carrier support or larger peptide, for modifying the physical or chemical properties of the peptide or oligopeptide, or the like. Amino acids such as tyrosine, cysteine, lysine, glutamic or aspartic acid, or the like, can be introduced at the C- or N-terminus of the peptide or oligopeptide, particularly class I peptides. However, it is to be noted that modification at the carboxyl terminus of a CTL epitope may, in some cases, alter binding characteristics of the peptide. In addition, the peptide or oligopeptide sequences can differ from the natural sequence by being modified by terminal-$NH_2$ acylation, *e.g.*, by alkanoyl ($C_1$-$C_{20}$) or thioglycolyl acetylation,

terminal-carboxyl amidation, *e.g.*, ammonia, methylamine, *etc.* In some instances these modifications may provide sites for linking to a support or other molecule.

**IV.K.4. Vaccine Compositions Comprising DC Pulsed with CTL and/or HTL Peptides**

An embodiment of a vaccine composition in accordance with the invention comprises *ex vivo* administration of a cocktail of epitope-bearing peptides to PBMC, or isolated DC therefrom, from the patient's blood. A pharmaceutical to facilitate harvesting of DC can be used, such as Progenipoietin™ (Monsanto, St. Louis, MO) or GM-CSF/IL-4. After pulsing the DC with peptides and prior to reinfusion into patients, the DC are washed to remove unbound peptides. In this embodiment, a vaccine comprises peptide-pulsed DCs which present the pulsed peptide epitopes complexed with HLA molecules on their surfaces.

The DC can be pulsed *ex vivo* with a cocktail of peptides, some of which stimulate CTL response to one or more antigens of interest, *e.g.*, CEA, p53, Her2/neu, MAGE, prostate cancer-associated antigens and the like. Optionally, a helper T cell peptide such as a PADRE™ family molecule, can be included to facilitate the CTL response.

**IV.L. Administration of Vaccines for Therapeutic or Prophylactic Purposes**

The peptides of the present invention and pharmaceutical and vaccine compositions of the invention are typically used therapeutically to treat cancer. Vaccine compositions containing the peptides of the invention are typically administered to a cancer patient who has a malignancy associated with expression of one or more tumor-associated antigens. Alternatively, vaccine compositions can be administered to an individual susceptible to, or otherwise at risk for developing a particular type of cancer, *e.g.*, breast cancer.

In therapeutic applications, peptide and/or nucleic acid compositions are administered to a patient in an amount sufficient to elicit an effective CTL and/or HTL response to the tumor antigen and to cure or at least partially arrest or slow symptoms and/or complications. An amount adequate to accomplish this is defined as "therapeutically effective dose." Amounts effective for this use will depend on, *e.g.*, the particular composition administered, the manner of administration, the stage and severity of the disease being treated, the weight and general state of health of the patient, and the judgment of the prescribing physician.

As noted above, peptides comprising GTL and/or HTL epitopes of the invention induce immune responses when presented by HLA molecules and contacted with a CTL or HTL specific for an epitope comprised by the peptide. The manner in which the peptide is contacted with the CTL or HTL is not critical to the invention. For instance, the peptide can be contacted with the CTL or HTL either *in vivo* or *in vitro.* If the contacting occurs *in vivo,* the peptide itself can be administered to the patient, or other vehicles, *e.g.*, DNA vectors encoding one or more peptides, viral vectors encoding the peptide(s), liposomes and the like, can be used, as described herein.

When the peptide is contacted *in vitro,* the vaccinating agent can comprise a population of cells, *e.g.*, peptide-pulsed dendritic cells, or TAA-specific CTLs, which have been induced by pulsing antigen-presenting cells *in vitro* with the peptide. Such a cell population is subsequently administered to a patient in a therapeutically effective dose.

For pharmaceutical compositions, the immunogenic peptides of the invention, or DNA encoding them, are generally administered to an individual already diagnosed with cancer. The peptides or DNA encoding them can be administered individually or as fusions of one or more peptide sequences.

For therapeutic use, administration should generally begin at the first diagnosis of cancer. This is followed by boosting doses until at least symptoms are substantially abated and for a period thereafter. The embodiment of the vaccine composition (i.e., including, but not limited to embodiments such as peptide cocktails, polyepitopic polypeptides, minigenes, or TAA-specific CTLs) delivered to the patient may vary according to the stage of the disease. For example, a vaccine comprising TAA-specific CTLs may be more efficacious in killing tumor cells in patients with advanced disease than alternative embodiments.

The vaccine compositions of the invention may also be used therapeutically in combination with treatments such as surgery. An example is a situation in which a patient has undergone surgery to remove a primary tumor and the vaccine is then used to slow or prevent recurrence and/or metastasis.

Where susceptible individuals, *e.g.*, individuals who may be diagnosed as being genetically predisposed to developing a particular type of tumor, for example breast cancer, are identified prior to diagnosis of cancer, the composition can be targeted to them, thus minimizing the need for administration to a larger population.

The dosage for an initial immunization generally occurs in a unit dosage range where the lower value is about 1, 5, 50, 500, or 1,000 μg and the higher value is about 10,000; 20,000; 30,000; or 50,000 μg. Dosage values for a human typically range from about 500 μg to about 50,000 μg per 70 kilogram patient. Boosting dosages of between about 1.0 μg to about 50,000 μg of peptide pursuant to a boosting regimen over weeks to months may be administered depending upon the patient's response and condition as determined by measuring the specific activity of CTL and HTL obtained from the patient's blood.

Administration should continue until at least clinical symptoms or laboratory tests indicate that the tumor has been eliminated or that the tumor cell burden has been substantially reduced and for a period thereafter. The dosages, routes of administration, and dose schedules are adjusted in accordance with methodologies known in the art.

In certain embodiments, peptides and compositions of the present invention are employed in serious disease states, that is, life-threatening or potentially life threatening situations. In such cases, as a result of the minimal amounts of extraneous substances and the relative nontoxic nature of the peptides in preferred compositions of the invention, it is possible and may be felt desirable by the treating physician to administer substantial excesses of these peptide compositions relative to these stated dosage amounts.

The pharmaceutical compositions for therapeutic treatment are intended for parenteral, topical, oral, intrathecal, or local administration. Preferably, the pharmaceutical compositions are administered parentally, *e.g.*, intravenously, subcutaneously, intradermally, or intramuscularly. Thus, the invention provides compositions for parenteral administration which comprise a solution of the immunogenic peptides dissolved or suspended in an acceptable carrier, preferably an aqueous carrier. A variety of aqueous carriers may be used, *e.g.*, water, buffered water, 0.8% saline, 0.3% glycine, hyaluronic acid and the like. These compositions may be sterilized by conventional, well known sterilization techniques, or may be sterile filtered. The resulting aqueous solutions may be packaged for use as is, or lyophilized, the lyophilized preparation being combined with a sterile solution prior to administration. The compositions may contain pharmaceutically acceptable auxiliary substances as required to approximate physiological conditions, such as pH-adjusting and buffering agents, tonicity adjusting agents, wetting agents, preservatives, and the like, for example, sodium acetate, sodium lactate, sodium chloride, potassium chloride, calcium chloride, sorbitan monolaurate, triethanolamine oleate, *etc.*

The concentration of peptides of the invention in the pharmaceutical formulations can vary widely, *i.e.,* from less than about 0.1%, usually at or at least about 2% to as much as 20% to 50% or more by weight, and will be selected primarily by fluid volumes, viscosities, *etc.,* in accordance with the particular mode of administration selected.

A human unit dose form of the peptide composition is typically included in a pharmaceutical composition that comprises a human unit dose of an acceptable carrier, preferably an aqueous carrier, and is administered in a volume of fluid that is known by those of skill in the art to be used for administration of such compositions to humans *(see, e. g.*, Remington's Pharmaceutical Sciences, 17th Edition, A. Gennaro, Editor, Mack Publishing Co., Easton, Pennsylvania, 1985).

The peptides of the invention may also be administered via liposomes, which serve to target the peptides to a particular tissue, such as lymphoid tissue, or to target selectively to infected cells, as well as to increase the half-life of the peptide composition. Liposomes include emulsions, foams, micelles, insoluble monolayers, liquid crystals, phospholipid dispersions, lamellar layers and the like. In these preparations, the peptide to be delivered is incorporated as part of a liposome, alone or in conjunction with a molecule which binds to a receptor prevalent among lymphoid cells, such as monoclonal antibodies which bind to the CD45 antigen, or with other therapeutic or immunogenic compositions. Thus, liposomes either filled or decorated with a desired peptide of the invention can be directed to the site of lymphoid cells, where the liposomes then deliver the peptide compositions. Liposomes for use in accordance with the invention are formed from standard vesicle-forming lipids, which generally include neutral and negatively charged phospholipids and a sterol, such as cholesterol. The selection of lipids is generally guided by consideration of, *e.g.,* liposome size, acid lability and stability of the liposomes in the blood stream. A variety of methods are available for preparing liposomes, as described in, *e.g.,* Szoka, *et al., Ann. Rev. Biophys. Bioeng.* 9:467 (1980), and U.S. Patent Nos. 4,235,871, 4,501,728, 4,837,028, and 5,019,369.

For targeting cells of the immune system, a ligand to be incorporated into the liposome can include, *e.g.,* antibodies or fragments thereof specific for cell surface determinants of the desired immune system cells. A liposome suspension containing a peptide may be administered intravenously, locally, topically, *etc.* in a dose which varies according to, *inter alia,* the manner of administration, the peptide being delivered, and the stage of the disease being treated.

For solid compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient, that is, one or more peptides of the invention, and more preferably at a concentration of 25%-75%.

For aerosol administration, the immunogenic peptides are preferably supplied in finely divided form along with a surfactant and propellant. Typical percentages of peptides are 0.01%-20% by weight, preferably 1%-10%. The surfactant must, of course, be nontoxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. The surfactant may constitute 0.1%-20% by weight of the composition, preferably 0.25-5%. The balance of the composition is ordinarily propellant. A carrier can also be included, as desired, as with, *e.g.*, lecithin for intranasal delivery.

## IV.M. HLA EXPRESSION: IMPLICATIONS FOR T CELL-BASED IMMUNOTHERAPY

### Disease progression in cancer and infectious disease

It is well recognized that a dynamic interaction between exists between host and disease, both in the cancer and infectious disease settings. In the infectious disease setting, it is well established that pathogens evolve during disease. The strains that predominate early in HIV infection are different from the ones that are associated with AIDS and later disease stages (NS versus S strains). It has long been hypothesized that pathogen forms that are effective in establishing infection may differ from the ones most effective in terms of replication and chronicity.

Similarly, it is widely recognized that the pathological process by which an individual succumbs to a neoplastic disease is complex. During the course of disease, many changes occur in cancer cells. The tumor accumulates alterations which are in part related to dysfunctional regulation of growth.and differentiation, but also related to maximizing its growth potential, escape from drug treatment and/or the body's immunosurveillance. Neoplastic disease results in the accumulation of several different biochemical alterations of cancer cells, as a function of disease progression. It also results in significant levels of intra-and inter- cancer heterogeneity, particularly in the late, metastatic stage.

Familiar examples of cellular alterations affecting treatment outcomes include the outgrowth of radiation or chemotherapy resistant tumors during the course of therapy. These examples parallel the emergence of drug resistant viral strains as a result of aggressive chemotherapy, *e.g.*, of chronic HBV and HIV infection, and the current resurgence of drug resistant organisms that cause Tuberculosis and Malaria. It appears that significant heterogeneity of responses is also associated with other approaches to cancer therapy, including anti-angiogenesis drugs, passive antibody immunotherapy, and active T cell-based immunotherapy. Thus, in view of such phenomena, epitopes from multiple disease related antigens can be used in vaccines and therapeutics thereby counteracting the ability of diseased cells to mutate and escape treatment.

### The interplay between disease and the immune system

One of the main factors contributing to the dynamic interplay between host and disease is the immune response mounted against the pathogen, infected cell, or malignant cell. In many conditions such immune responses control the disease. Several animal model systems and prospective studies of natural infection in humans suggest that immune responses against a pathogen can control the pathogen, prevent progression to severe disease and/or eliminate the pathogen. A common theme is the requirement for a multispecific T cell response, and that narrowly focused responses appear to be less effective. These observations guide skilled artisan as to embodiments of methods and compositions of the present invention that provide for a broad immune response.

In the cancer setting there are several findings that indicate that immune responses can impact neoplastic growth:

First, the demonstration in many different animal models, that anti-tumor T cells, restricted by MHC class I, can prevent or treat tumors.

Second, encouraging results have come from immunotherapy trials.

Third, observations made in the course of natural disease correlated the type and composition of T cell infiltrate within tumors with positive clinical outcomes (Coulie PG*, et al.* Antitumor immunity at work in a melanoma patient In *Advances in Cancer Research*, 213-242, 1999).

Finally, tumors commonly have the ability to mutate, thereby changing their immunological recognition. For example, the presence of monospecific CTL was also correlated with control of tumor growth, until antigen loss emerged (Riker A, *et al.,* Immune selection after antigen-specific immunotherapy of melanoma *Surgery,* Aug: 126(2):112-20, 1999; Marchand M, *et al.,* Tumor regressions observed in patients with metastatic melanoma treated with an antigenic peptide encoded by gene MAGE-3 and presented by HLA-A1 *Int. J. Cancer* 80(2):219-30, Jan. 18, 1999). Similarly, loss of beta 2 microglobulin was detected in 5/13 lines established from melanoma patients after receiving immunotherapy at the NCI (Restifo NP, *et al.,* Loss of functional Beta2 - microglobulin in metastatic melanomas from five patients receiving immunotherapy *Journal of the National Cancer Institute,* Vol. 88 (2), 100-108, Jan. 1996). It has long been recognized that HLA class 1 is frequently altered in various tumor types. This has led to a hypothesis that this phenomenon might reflect immune pressure exerted on the tumor by means of class I restricted CTL. The extent and degree of alteration in HLA class I expression appears to be reflective of past immune pressures, and may also have prognostic value (van Duinen SG, *et al.,* Level of HLA antigens in locoregional metastases and clinical course of the disease in patients with melanoma *Cancer Research* 48, 1019-1025, Feb. 1988; Möller P, *et al.,* Influence of major histocompatibility complex class I and II antigens on survival in colorectal carcinoma *Cancer Research* 51, 729-736, Jan. 1991). Taken together, these observations provide a rationale for immunotherapy of cancer and infectious disease, and suggest that effective strategies need to account for the complex series of pathological changes associated with disease.

The three main types of alterations in HLA expression in tumors and their functional significance

The level and pattern of expression of HLA class I antigens in tumors has been studied in many different tumor types and alterations have been reported in all types of tumors studied. The molecular mechanisms underlining HLA class I alterations have been demonstrated to be quite heterogeneous. They include alterations in the TAP/processing pathways, mutations of β2-microglobulin and specific HLA heavy chains, alterations in the regulatory elements controlling over class I expression and loss of entire chromosome sections. There are several reviews on this topic, *see, e.g., :* Garrido F, *et al.,* Natural history of HLA expression during tumour development *Immunol Today* 14 (10):491-499, 1993; Kaklamanis L, *et al.,* Loss of HLA class-I alleles, heavy chains and β2-microglobulin in colorectal cancer *Int. J. Cancer,* 51(3):379-85, May 28,1992. There are three main types of HLA Class I alteration (complete loss, allele-specific loss and decreased expression). The functional significance of each alteration is discussed separately:

*Complete loss of HLA expression*

Complete loss of HLA expression can result from a variety of different molecular mechanisms, reviewed in (Algarra I, *et al.,* The HLA crossroad in tumor immunology *Human Immunology* 61, 65-73, 2000; Browning M, *et al.,* Mechanisms of loss of HLA class I expression on colorectal tumor cells *Tissue Antigens* 47:364-371, 1996; Ferrone S, et al., Loss of HLA class I antigens by melanoma cells: molecular mechanisms, functional significance and clinical relevance *Immunology Today,* 16(10): 487-494, 1995; Garrido F, *et al.,* Natural history of HLA expression during tumour development *Immunology Today* 14(10):491-499, 1993; Tait, BD, HLA Class I expression on human cancer cells: Implications for effective immunotherapy *Hum Immunol* 61, 158-165, 2000). In functional terms, this type of alteration has several important implications.

While the complete absence of class I expression will eliminate CTL recognition of those tumor cells, the loss of HLA class I will also render the tumor cells extraordinary sensitive to lysis from NK cells (Ohnmacht, GA, *et al.,* Heterogeneity in expression of human leukocyte antigens and melanoma-associated antigens in advanced melanoma *J Cellular Phys* 182:332-338, 2000; Liunggren HG, *et al.,* Host resistance directed selectively against H-2 deficient lymphoma variants: Analysis of the mechanism *J. Exp. Med.,* Dec 1; 162(6): 1745-59, 1985; Maio M, *et al.,* Reduction in susceptibility to natural killer cell-mediated lysis of human FO-1 melanoma cells after induction of HLA class I antigen expression by transfection with B2m gene *J. Clin. Invest.* 88(1):282-9, July 1991; Schrier PI, *et al.,* Relationship between myc oncogene activation and MHC class I expression *Adv. Cancer Res.,* 60:181-246, 1993).

The complementary interplay between loss of HLA expression and gain in NK sensitivity is exemplified by the classic studies of Coulie and coworkers (Coulie, PG, *et al.,* Antitumor immunity at work in a melanoma patient. In *Advances in Cancer Research,* 213-242, 1999) which described the evolution of a patient's immune response over the course of several years. Because of increased sensitivity to NK lysis, it is predicted that approaches leading to stimulation of innate immunity in general and NK activity in particular would be of special significance. An example of such approach is the induction of large amounts of dendritic cells (DC) by various hematopoietic growth factors, such as Flt3 ligand or ProGP. The rationale for this approach resides in the well known fact that dendritic cells produce large amounts of IL-12, one of the most potent stimulators for innate immunity and NK activity in particular. Alternatively, IL-12 is administered directly, or as nucleic acids that encode it. In this light, it is interesting to note that Flt3 ligand treatment results in transient tumor regression of a class I negative prostate murine cancer model (Ciavarra RP, *et al.,* Flt3-Ligand induces transient tumor regression in an ectopic treatment model of major histocompatibility complex-negative prostate cancer *Cancer Res* 60:2081-84, 2000). In this context, specific anti-tumor vaccines in accordance with the invention synergize with these types of hematopoietic growth factors to facilitate both CTL and NK cell responses, thereby appreciably impairing a cell's ability to mutate and thereby escape efficacious treatment. Thus, an embodiment of the present invention comprises a composition of the invention together with a method or composition that augments functional activity or numbers ofNK cells. Such an embodiment can comprise a protocol that provides a composition of the invention sequentially with an NK-inducing modality, or contemporaneous with an NK-inducing modality.

Secondly, complete loss of HLA frequently occurs only in a fraction of the tumor cells, while the remainder of tumor cells continue to exhibit normal expression. In functional terms, the tumor would still be subject, in part, to direct attack from a CTL response; the portion of cells lacking HLA subject to an NK response. Even if only a CTL response were used, destruction of the HLA expressing fraction of the tumor has dramatic effects on survival times and quality of life.

It should also be noted that in the case of heterogeneous HLA expression, both normal HLA-expressing as well as defective cells are predicted to be susceptible to immune destruction based on "bystander effects." Such effects were demonstrated, e.g., in the studies of Rosendahl and colleagues that investigated in vivo mechanisms of action of antibody targeted superantigens (Rosendahl A, *et al.,* Perforin and IFN-gamma are involved in the antitumor effects of antibody-targeted superantigens *J. Immunol.* 160(11):5309-13, June 1, 1998). The bystander effect is understood to be mediated by cytokines elicited from, *e.g.*, CTLs acting on an HLA-bearing target cell, whereby the cytokines are in the environment of other diseased cells that are concomitantly killed.

*Allele-specific loss*

One of the most common types of alterations in class I molecules is the selective loss of certain alleles in individuals heterozygous for HLA. Allele-specific alterations might reflect the tumor adaptation to immune pressure, exerted by an immunodominant response restricted by a single HLA restriction element. This type of alteration allows the tumor to retain class I expression and thus escape NK cell recognition, yet still be susceptible to a CTL-based vaccine in accordance with the invention which comprises epitopes corresponding to the remaining HLA type. Thus, a practical solution to overcome the potential hurdle of allele-specific loss relies on the induction of multispecific responses. Just as the inclusion of multiple disease-associated antigens in a vaccine of the invention guards against mutations that yield loss of a specific disease antigens, simultaneously targeting multiple HLA specificities and multiple disease-related antigens prevents disease escape by allele-specific losses.

*Decrease in expression (allele-specific or not)*

The sensitivity of effector CTL has long been demonstrated (Brower, RC, *et al.,* Minimal requirements for peptide mediated activation of CD8+CTL *Mol-Immunol,* 31;1285-93, 1994; Chriustnick, ET, *et al.* Low numbers of MHC class I-peptide complexes required to trigger a T cell response *Nature* 352:67-70, 1991; Sykulev, *Y, et al.,* Evidence that a single peptide-MHC complex on a target cell can elicit a cytolytic T cell response *Immunity,* 4(6):565-71, June 1996). Even a single peptide/MHC complex can result in tumor cells lysis and release of anti-tumor lymphokines. The biological significance of decreased HLA expression and possible tumor escape from immune recognition is not fully known. Nevertheless, it has been demonstrated that CTL recognition of as few as one MHC/peptide complex is sufficient to lead to tumor cell lysis.

Further, it is commonly observed that expression of HLA can be upregulated by gamma IFN, commonly secreted by effector CTL. Additionally, HLA class I expression can be induced in vivo by both alpha and beta IFN (Halloran, *et al.* Local T cell responses induce widespread MHC expression. *J Immunol* 148:3837, 1992; Pestka, S, *et al.,* Interferons and their actions *Annu. Rev. Biochem.* 56:727-77, 1987). Conversely, decreased levels of HLA class I expression also render cells more susceptible to NK lysis.

With regard to gamma IFN, Torres et al (Torres, MJ*, et al.,* Loss of an HLA haplotype in pancreas cancer tissue and its corresponding tumor derived cell line. *Tissue Antigens* 47:372-81, 1996) note that HLA expression is upregulated by gamma IFN in pancreatic cancer, unless a total loss of haplotype has occurred. Similarly, Rees and Mian note that allelic deletion and loss can be restored, at least partially, by cytokines such as IFN-gamma (Rees, R., *et al.* Selective MHC expression in tumours modulates adaptive and innate antitumour responses *Cancer Immunol Immunother* 48:374-81, 1999). It has also been noted that IFN-gamma treatment results in upregulation of class I molecules in the majority of the cases studied (Browning M*, et al.,* Mechanisms of loss of HLA class I expression on colorectal tumor cells. *Tissue Antigens* 47:364-71, 1996). Kaklamakis, et al. also suggested that adjuvant immunotherapy with IFN-gamma may be beneficial in the case of HLA class I negative tumors (Kaklamanis L, Loss of transporter in antigen processing I transport protein and major histocompatibility complex class I molecules in metastatic versus primary breast cancer. *Cancer Research* 55:5191-94, November 1995). It is important to underline that IFN-gamma production is induced and self-amplified by local inflammation/immunization (Halloran*, et al.* Local T cell responses induce widespread MHC expression *J. Immunol* 148:3837, 1992), resulting in large increases in MHC expressions even in sites distant from the inflammatory site.

Finally, studies have demonstrated that decreased HLA expression can render tumor cells more susceptible to NK lysis (Ohnmacht, GA, *et al.,* Heterogeneity in expression of human leukocyte antigens and melanoma-associated antigens in advanced melanoma *J Cellular Phys* 182:332-38, 2000; Liunggren HG, *et al.*, Host resistance directed selectively against H-2 deficient lymphoma variants: Analysis of the mechanism *J. Exp. Med.,* 162(6):1745-59, December 1, 1985; Maio M, *et al.,* Reduction in susceptibility to natural killer cell-mediated lysis of human FO-1 melanoma cells after induction of HLA class I antigen expression by transfection with β2m gene *J. Clin. Invest.* 88(1):282-9, July 1991; Schrier PI, *et al.,* Relationship between myc oncogene activation and MHC class I expression *Adv. Cancer Res.,* 60:181-246, 1993). If decreases in HLA expression benefit a tumor because it facilitates CTL escape, but render the tumor susceptible to NK lysis, then a minimal level of HLA expression that allows for resistance to NK activity would be selected for (Garrido F, *et al.*, Implications for immunosurveillance of altered HLA class I phenotypes in human tumours *Immonol Today* 18(2):89-96, February 1997). Therefore, a therapeutic compositions or methods in accordance with the invention together with a treatment to upregulate HLA expression and/or treatment with high affinity T-cells renders the tumor sensitive to CTL destruction.

Frequency of alterations in HLA expression

The frequency of alterations in class I expression is the subject of numerous studies (Algarra I, *et al.,* The HLA crossroad in tumor immunology *Human Immunology* 61, 65-73, 2000). Rees and Mian estimate allelic loss to occur overall in 3-20% of tumors, and allelic deletion to occur in 15-50% of tumors. It should be noted that each cell carries two separate sets of class I genes, each gene carrying one HLA-A and one HLA-B locus. Thus, fully heterozygous individuals carry two different HLA-A molecules and two different HLA-B molecules. Accordingly, the actual frequency of losses for any specific allele could be as little as one quarter of the overall frequency. They also note that, in general, a gradient of expression exists between normal cells, primary tumors and tumor metastasis. In a study from Natali and coworkers (Natali PG, *et al.,* Selective changes in expression of HLA class I polymorphic determinants in human solid tumors *PNAS USA* 86:6719-6723, September 1989), solid tumors were investigated for total HLA expression, using W6/32 antibody, and for allele-specific expression of the A2 antigen, as evaluated by use of the BB7.2 antibody. Tumor samples were derived from primary cancers or metastasis, for 13 different tumor types, and scored as negative if less than 20%, reduced if in the 30-80% range, and normal above 80%. All tumors, both primary and metastatic, were HLA positive with W6/32. In terms of A2 expression, a reduction was noted in 16.1 % of the cases, and A2 was scored as undetectable in 39.4 % of the cases. Garrido and coworkers (Garrido F, *et al.,* Natural history of HLA expression during tumour development *Immunol Today* 14(10):491-99, 1993) emphasize that HLA changes appear to occur at a particular step in the progression from benign to most aggressive. Jiminez *et al* (Jiminez P, *et al.,* Microsatellite instability analysis in tumors with different mechanisms for total loss of HLA expression. *Cancer Immunol* Immunother 48:684-90, 2000) have analyzed 118 different tumors (68 colorectal, 34 laryngeal and 16 melanomas). The frequencies reported for total loss of HLA expression were 11% for colon, 18% for melanoma and 13 % for larynx. Thus, HLA class I expression is altered in a significant fraction of the tumor types, possibly as a reflection of immune pressure, or simply a reflection of the accumulation of pathological changes and alterations in diseased cells.

Immunotherapy in the context of HLA loss

A majority of the tumors express HLA class I, with a general tendency for the more severe alterations to be found in later stage and less differentiated tumors. This pattern is encouraging in the context of immunotherapy, especially considering that: 1) the relatively low sensitivity of immunohistochemical techniques might underestimate HLA expression in tumors; 2) class I expression can be induced in tumor cells as a result of local inflammation and lymphokine release; and, 3) class I negative cells are sensitive to lysis by NK cells.

Accordingly, various embodiments of the present invention can be selected in view of the fact that there can be a degree of loss of HLA molecules, particularly in the context of neoplastic disease. For example, the treating physician can assay a patient's tumor to ascertain whether HLA is being expressed. If a percentage of tumor cells express no class-I HLA, then embodiments of the present invention that comprise methods or compositions that elicit NK cell responses can be employed. As noted herein, such NK-inducing methods or composition can comprise a Flt3 ligand or ProGP which facilitate mobilization of dendritic cells, the rationale being that dendritic cells produce large amounts of IL-12. IL-12 can also be administered directly in either amino acid or nucleic acid form. It should be noted that compositions in accordance with the invention can be administered concurrently with NK cell-inducing compositions, or these compositions can be administered sequentially.

In the context of allele-specific HLA loss, a tumor retains class I expression and may thus escape NK cell recognition, yet still be susceptible to a CTL-based vaccine in accordance with the invention which comprises epitopes corresponding to the remaining HLA type. The concept here is analogous to embodiments of the invention that include multiple disease antigens to guard against mutations that yield loss of a specific antigen. Thus, one can simultaneously target multiple HLA specificities and epitopes from multiple disease-related antigens to prevent tumor escape by allele-specific loss as well as disease-related antigen loss. In addition, embodiments of the present invention can be combined with alternative therapeutic compositions and methods. Such alternative compositions and methods comprise, without limitation, radiation, cytotoxic pharmaceuticals, and/or compositions/methods that induce humoral antibody responses.

Moreover, it has been observed that expression of HLA can be upregulated by gamma IFN, which is commonly secreted by effector CTL, and that HLA class I expression can be induced in vivo by both alpha and beta IFN. Thus, embodiments of the invention can also comprise alpha, beta and/or gamma IFN to facilitate upregualtion of HLA.

**IV.N. REPRIEVE PERIODS FROM THERAPIES THAT INDUCE SIDE EFFECTS: "Scheduled Treatment Interruptions or Drug Holidays"**

Recent evidence has shown that certain patients infected with a pathogen, whom are initially treated with a therapeutic regimen to reduce pathogen load, have been able to maintain decreased pathogen load when removed from the therapeutic regimen, *i.e.,* during a "drug holiday" (Rosenberg, E., *et al.,* Immune control of HIV-1 after early treatment of acute infection Nature 407:523-26, Sept. 28, 2000) As appreciated by those skilled in the art, many therapeutic regimens for both pathogens and cancer have numerous, often severe, side effects. During the drug holiday, the patient's immune system keeps the disease in check. Methods for using compositions of the invention are used in the context of drug holidays for cancer and pathogenic infection.

For treatment of an infection, where therapies are not particularly immunosuppressive, compositions of the invention are administered concurrently with the standard therapy. During this period, the patient's immune system is directed to induce responses against the epitopes comprised by the present inventive compositions. Upon removal from the treatment having side effects, the patient is primed to respond to the infectious pathogen should the pathogen load begin to increase. Composition of the invention can be provided during the drug holiday as well.

For patients with cancer, many therapies are immunosuppressive. Thus, upon achievement of a remission or identification that the patient is refractory to standard treatment, then upon removal from the immunosuppressive therapy, a composition in accordance with the invention is administered. Accordingly, as the patient's immune system reconstitutes, precious immune resources are simultaneously directed against the cancer. Composition of the invention can also be administered concurrently with an immunosuppressive regimen if desired.

**IV.O. Kits**

The peptide and nucleic acid compositions of this invention can be provided in kit form together with instructions for vaccine administration. Typically the kit would include desired peptide compositions in a container, preferably in unit dosage form and instructions for administration. An alternative kit would include a minigene construct with desired nucleic acids of the invention in a container, preferably in unit dosage form together with instructions for administration. Lymphokines such as IL-2 or IL-12 may also be included in the kit. Other kit components that may also be desirable include, for example, a sterile syringe, booster dosages, and other desired excipients.

**IV.P. Overview**

Epitopes in accordance with the present invention were successfully used to induce an immune response. Immune responses with these epitopes have been induced by administering the epitopes in various forms. The epitopes have been administered as peptides, as nucleic acids, and as viral vectors comprising nucleic acids that encode the epitope(s) of the invention. Upon administration of peptide-based epitope forms, immune responses have been induced by direct loading of an epitope onto an empty HLA molecule that is expressed on a cell, and via internalization of the epitope and processing via the HLA class I pathway; in either event, the HLA molecule expressing the epitope was then able to interact with and induce a CTL response. Peptides can be delivered directly or using such agents as liposomes. They can additionally be delivered using ballistic delivery, in which the peptides are typically in a crystalline form. When DNA is used to induce an immune response, it is administered either as naked DNA, generally in a dose range of approximately 1-5mg, or via the ballistic "gene gun" delivery, typically in a dose range of approximately 10-100 µg. The DNA can be delivered in a variety of conformations, *e.g.*, linear, circular *etc*. Various viral vectors have also successfully been used that comprise nucleic acids which encode epitopes in accordance with the invention.

Accordingly compositions in accordance with the invention exist in several-forms. Embodiments of each of these composition forms in accordance with the invention have been successfully used to induce an immune response.

One composition in accordance with the invention comprises a plurality of peptides. This plurality or cocktail of peptides is generally admixed with one or more pharmaceutically acceptable excipients. The peptide cocktail can comprise multiple copies of the same peptide or can comprise a mixture of peptides. The peptides can be analogs of naturally occurring epitopes. The peptides can comprise artificial amino acids and/or chemical modifications such as addition of a surface active molecule, *e.g.*, lipidation; acetylation, glycosylation, biotinylation, phosphorylation etc. The peptides can be CTL or HTL epitopes. In a preferred embodiment the peptide cocktail comprises a plurality of different CTL epitopes and at least one HTL epitope. The HTL epitope can be naturally or non-naturally (e.g., PADRE®, Epimmune Inc., San Diego, CA). The number of distinct epitopes in an embodiment of the invention is generally a whole unit integer from one through two hundred (*e.g.*, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 105,107,108,109,110,111,112,113,114,115,116,117,118,119,120,121,122,123,124,125,126, 127, 128, 129, 130, 131, 132, 133, 134, 135, 136, 137, 138, 139, 140, 141, 142, 143, 144, 145, 146, 147, 148,149,150,151,152,153,154,155,156,157,158,159,160,161,162,163,164,165,166,167,168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200).

An additional embodiment of a composition in accordance with the invention comprises a polypeptide multi-epitope construct, *i.e.,* a polyepitopic peptide. Polyepitopic peptides in accordance with the invention are prepared by use of technologies well-known in the art. By use of these known technologies, epitopes in accordance with the invention are connected one to another. The polyepitopic peptides can be linear or non-linear, *e.g.,* multivalent. These polyepitopic constructs can comprise artificial amino acids, spacing or spacer amino acids, flanking amino acids, or chemical modifications between adjacent epitope units. The polyepitopic construct can be a heteropolymer or a homopolymer. The polyepitopic constructs generally comprise epitopes in a quantity of any whole unit integer between 2-150 (e.g., 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or, 100). The polyepitopic construct can comprise CTL and/or HTL epitopes. One or more of the epitopes in the construct can be modified, *e.g.,* by addition of a surface active material, *e.g.* a lipid, or chemically modified, *e.g.,* acetylation, *etc.* Moreover, bonds in the multiepitopic construct can be other than peptide bonds, *e.g.,* covalent bonds, ester or ether bonds, disulfide bonds, hydrogen bonds, ionic bonds *etc.*

Alternatively, a composition in accordance with the invention comprises construct which comprises a series, sequence, stretch, *etc.,* of amino acids that have homology to (*i.e.,* corresponds to or is contiguous with) to a native sequence. This stretch of amino acids comprises at least one subsequence of amino acids that, if cleaved or isolated from the longer series of amino acids, functions as an HLA class I or HLA class II epitope in accordance with the invention. In this embodiment, the peptide sequence is modified, so as to become a construct as defined herein, by use of any number of techniques known or to be provided in the art. The polyepitopic constructs can contain homology to a native sequence in any whole unit integer increment from 70-100%, e.g., 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or, 100 percent.

A further embodiment of a composition in accordance with the invention is an antigen presenting cell that comprises one or more epitopes in accordance with the invention. The antigen presenting cell can be a "professional" antigen presenting cell, such as a dendritic cell. The antigen presenting cell can comprise the epitope of the invention by any means known or to be determined in the art. Such means include pulsing of dendritic cells with one or more individual epitopes or with one or more peptides that comprise multiple epitopes, by nucleic acid administration such as ballistic nucleic acid delivery or by other techniques in the art for administration of nucleic acids, including vector-based, e.g. viral vector, delivery of nucleic acids.

Further embodiments of compositions in accordance with the invention comprise nucleic acids that encode one or more peptides of the invention, or nucleic acids which encode a polyepitopic peptide in accordance with the invention. As appreciated by one of ordinary skill in the art, various nucleic acids compositions will encode the same peptide due to the redundancy of the genetic code. Each of these nucleic acid compositions falls within the scope of the present invention. This embodiment of the invention comprises DNA or RNA, and in certain embodiments a combination of DNA and RNA. It is to be appreciated that any composition comprising nucleic acids that will encode a peptide in accordance with the invention or any other peptide based composition in accordance with the invention, falls within the scope of this invention.

It is to be appreciated that peptide-based forms of the invention (as well as the nucleic acids that encode them) can comprise analogs of epitopes of the invention generated using priniciples already known, or to be known, in the art. Principles related to analoging are now known in the art, and are disclosed herein; moreover, analoging principles (heteroclitic analoging) are disclosed in co-pending application serial number U.S.S.N. 09/226,775 filed 6 January 1999. Generally the compositions of the invention are isolated or purified.

The invention will be described in greater detail by way of specific examples. The following examples are offered for illustrative purposes, and are not intended to limit the invention in any manner. Those of skill in the art will readily recognize a variety of non-critical parameters that can be changed or modified to yield alternative embodiments in accordance with the invention.

## V. EXAMPLES

The following examples illustrate identification, selection, and use of immunogenic Class I and Class II peptide epitopes for inclusion in vaccine compositions.

Example 1. HLA Class I and Class II Binding Assays

The following example of peptide binding to HLA molecules demonstrates quantification of binding affinities of HLA class I and class II peptides. Binding assays can be performed with peptides that are either motif-bearing or not motif-bearing.

HLA class I and class II binding assays using purified HLA molecules were performed in accordance with disclosed protocols (*e.g.,* PCT publications WO 94/20127 and WO 94/03205; Sidney *et al., Current Protocols in Immunology* 18.3.1 (1998); Sidney, *et al., J. Immunol.* 154:247 (1995); Sette, *et al., Mol. Immunol.* 31:813 (1994)). Briefly, purified MHC molecules (5 to 500nM) were incubated with various unlabeled peptide inhibitors and 1-10nM $^{125}$I-radiolabeled probe peptides as described. Following incubation, MHC-peptide complexes were separated from free peptide by gel filtration and the fraction of peptide bound was determined. Typically, in preliminary experiments, each MHC preparation was titered in the presence of fixed amounts of radiolabeled peptides to determine the concentration of HLA molecules necessary to bind 10-20% of the total radioactivity. All subsequent inhibition and direct binding assays were performed using these HLA concentrations.

Since under these conditions [label]<[HLA] and $IC_{50} \geq$ [HLA], the measured $IC_{50}$ values are reasonable approximations of the true $K_D$ values. Peptide inhibitors are typically tested at concentrations ranging from 120 µg/ml to 1.2 ng/ml, and are tested in two to four completely independent experiments. To allow comparison of the data obtained in different experiments, a relative binding figure is calculated for each peptide by dividing the $IC_{50}$ of a positive control for inhibition by the $IC_{50}$ for each tested peptide (typically unlabeled versions of the radiolabeled probe peptide). For database purposes, and inter-experiment comparisons, relative binding values are compiled. These values can subsequently be converted back into $IC_{50}$ nM values by dividing the $IC_{50}$ nM of the positive controls for inhibition by the relative binding of the peptide of interest. This method of data compilation has proven to be the most accurate and consistent for comparing peptides that have been tested on different days, or with different lots of purified MHC.

Binding assays as outlined above can be used to analyze supermotif and/or motif-bearing epitopes as, for example, described in Example 2.

Example 2. Identification of HLA Supermotif- and Motif-Bearing CTL Candidate Epitopes

Vaccine compositions of the invention may include multiple epitopes that comprise multiple HLA supermotifs or motifs to achieve broad population coverage. This example illustrates the identification of supermotif- and motif-bearing epitopes for the inclusion in such a vaccine composition. Calculation of population coverage is performed using the strategy described below.

*Computer searches and algorthims for identification of supermotif and/or motif-bearing epitopes*

The searches performed to identify the motif-bearing peptide sequences in Examples 2 and 5 employed protein sequence data for the tumor-associated antigen HER2/neu.

Computer searches for epitopes bearing HLA Class I or Class II supermotifs or motifs were performed as follows. All translated protein sequences were analyzed using a text string search software program, *e.g.*, MotifSearch 1.4 (D. Brown, San Diego) to identify potential peptide sequences containing appropriate HLA binding motifs; alternative programs are readily produced in accordance with information in the art in view of the motif/supermotif disclosure herein. Furthermore, such calculations can be made mentally. Identified A2-, A3-, and DR-supermotif sequences were scored using polynomial algorithms to predict their capacity to bind to specific HLA-Class I or Class II molecules. These polynomial algorithms take into account both extended and refined motifs (that is, to account for the impact of different amino acids at different positions), and are essentially based on the premise that the overall affinity (or $\Delta G$) of peptide-HLA molecule interactions can be approximated as a linear polynomial function of the type:

$$\text{"}\Delta G\text{"} = a_{1i} \times a_{2i} \times a_{3i} .... \times a_{ni}$$

where $a_{ji}$ is a coefficient which represents the effect of the presence of a given amino acid (*j*) at a given position (*i*) along the sequence of a peptide of n amino acids. The crucial assumption of this method is that the effects at each position are essentially independent of each other (i.e., independent binding of individual side-chains). When residue *j* occurs at position i in the peptide, it is assumed to contribute a constant amount $j_i$ to the free energy of binding of the peptide irrespective of the sequence of the rest of the peptide. This assumption is justified by studies from our laboratories that demonstrated that peptides are bound to MHC and recognized by T cells in essentially an extended conformation (data omitted herein).

The method of derivation of specific algorithm coefficients has been described in Gulukota *et al., J. Mol. Biol.*

267:1258-126, 1997; *(see also* Sidney *et al., Human Immunol.* 45:79-93, 1996; and Southwood *et al., J. Immunol.* 160: 3363-3373, 1998). Briefly, for all i positions, anchor and non-anchor alike, the geometric mean of the average relative binding (ARB) of all peptides carrying *j* is calculated relative to the remainder of the group, and used as the estimate of *$j_i$*. For Class II peptides, if multiple alignments are possible, only the highest scoring alignment is utilized, following an iterative procedure. To calculate an algorithm score of a given peptide in a test set, the ARB values corresponding to the sequence of the peptide are multiplied. If this product exceeds a chosen threshold, the peptide is predicted to bind. Appropriate thresholds are chosen as a function of the degree of stringency of prediction desired.

*Selection of HLA-A2 supertype cross-reactive peptides*

The complete protein sequence from HER2/neu was scanned, utilizing motif identification software, to identify 8-, 9-, 10-, and 11-mer sequences containing the HLA-A2-supermotif main anchor specificity.

A total of 623 HLA-A2 supermotif-positive sequences were identified. Of these, 73 scored positive in the A2 algorithm and the peptides corresponding to the sequences were then synthesized. An additional 90 A2 supermotif-bearing nonamers and decamers were also synthesized. These 163 peptides were then tested for their capacity to bind purified HLA-A*0201 molecules *in vitro* (HLA-A*0201 is considered a prototype A2 supertype molecule). Twenty of the peptides bound A*0201 with $IC_{50}$ values ≤500 nM.

The twenty A*0201-binding peptides were subsequently tested for the capacity to bind to additional A2-supertype molecules (A*0202, A*0203, A*0206, and A*6802). As shown in Table XXII, 9 of the 20 peptides were found to be A2-supertype cross-reactive binders, binding at least three of the five A2-supertype alleles tested.

*Selection of HLA-A3 supermotif-bearing epitopes*

The protein sequences scanned above are also examined for the presence of peptides with the HLA-A3-supermotif primary anchors using methodology similar to that performed to identify HLA-A2 supermotif-bearing epitopes.

Peptides corresponding to the supermotif-bearing sequences are then synthesized and tested for binding to HLA-A*0301 and HLA-A*1101 molecules, the two most prevalent A3-supertype alleles. The peptides that are found to bind one of the two alleles with binding affinities of ≤500 nM are then tested for binding cross-reactivity to the other common A3-supertype alleles (A*3101, A*3301, and A*6801) to identify those that can bind at least three of the five HLA-A3-supertype molecules tested. Examples of HLA-A3 cross-binding supermotif-bearing peptides identified in accordance with this procedure are provided in Table XXIII.

*Selection of HLA-B7 supermotif bearing epitopes*

The same target antigen protein sequences are also analyzed to identify HLA-B7-supermotif-bearing sequences. The corresponding peptides are then synthesized and tested for binding to HLA-B*0702, the most common B7-supertype allele (*i.e.*, the prototype B7 supertype allele). Those peptides that bind B*0702 with $IC_{50}$ of ≤500 nM are then tested for binding to other common B7-supertype molecules (B*3501, B*5101, B*5301, and B*5401) to identify those peptides that are capable of binding to three or more of the five B7-supertype alleles tested. Examples of HLA-B7 cross-binding supermotif-bearing peptides identified in accordance with this procedure are provided in Table XXIV.

*Selection of A1 and A24 motif-bearing epitopes*

To further increase population coverage, HLA-A1 and -A24 motif-bearing epitopes can also be incorporated into potential vaccine constructs. An analysis of the protein sequence data from the target antigen utilized above is also performed to identify HLA-A1- and A24-motif-containing conserved sequences. The corresponding peptide sequence are then synthesized and tested for binding to the appropriate allele-specific HLA molecule, HLA-A1 or HLA-24. Peptides are identified that bind to the allele-specific HLA molecules at an $IC_{50}$ of ≤500 nM. Examples of peptides identified in accordance with this procedure are provided in Tables XXV and XXVI.

Example 3. Confirmation of Immunogenicity

The nine cross-reactive candidate CTL A2-supermotif-bearing peptides identified in Example 2 were selected for *in vitro* immunogenicity testing. Testing was performed using the following methodology.

**Target Cell Lines for Cellular Screening:**

The .221A2.1 cell line, produced by transferring the HLA-A2.1 gene into the HLA-A, -B, -C null mutant human B-lymphoblastoid cell line 721.221, was used as the peptide-loaded target to measure activity of HLA-A2.1-restricted CTL. The colon adenocarcinoma cell lines SW403 and HT-29 were obtained from the American Type Culture Collection (ATCC) (Rockville, MD). The cell lines that were obtained from ATCC were maintained under the culture conditions recommended by the supplier. All other cell lines were grown in RPMI-1640 medium supplemented with antibiotics, sodium pyruvate, nonessential amino acids and 10% (v/v) heat inactivated FCS. The colon cancer cells were treated with 100U/ml IFNγ (Genzyme) for 48 hours at 37°C before use as targets in the $^{51}$Cr release and *in situ* IFNγ assays.

**Primary CTL Induction Cultures:**

*Generation of Dendritic Cells (DC):* PBMCs were thawed in RPMI with 30 μg/ml DNAse, washed twice and resuspended in complete medium (RPMI-1640 plus 5% AB human serum, non-essential amino acids, sodium pyruvate, L-glutamine and penicillin/strpetomycin). The monocytes were purified by plating 10 x 10$^6$ PBMC/well in a 6-well plate. After 2 hours at 37°C, the non-adherent cells were removed by gently shaking the plates and aspirating the supernatants. The wells were washed a total of three times with 3 ml RPMI to remove most of the non-adherent and loosely adherent cells. Three ml of complete medium containing 50 ng/ml of GM-CSF and 1,000 U/ml of IL 4 were then added to each well. DC were used for CTL induction cultures following 7 days of culture.

*Induction of CTL with DC and Peptide:* CD8+ T-cells were isolated by positive selection with Dynal immunomagnetic beads (Dynabeads® M-450) and the detacha-bead® reagent. Typically about 200-250×10$^6$ PBMC were processed to obtain 24×10$^6$ CD8$^+$ T-cells (enough for a 48-well plate culture). Briefly, the PBMCs were thawed in RPMI with 30μg/ml DNAse, washed once with PBS containing 1% human AB serum and resuspended in PBS/1% AB serum at a concentration of 20x10$^6$cells/ml. The magnetic beads were washed 3 times with PBS/AB serum, added to the cells (140μl beads/20×10$^6$ cells) and incubated for 1 hour at 4°C with continuous mixing. The beads and cells were washed 4x with PBS/AB serum to remove the nonadherent cells and resuspended at 100x10$^6$ cells/ml (based on the original cell number) in PBS/AB serum containing 100μl/ml detacha-bead® reagent and 30μg/ml DNAse. The mixture is incubated for 1 hour at room temperature with continuous mixing. The beads were washed again with PBS/AB/DNAse to collect the CD8+ T-cells. The DC were collected and centrifuged at 1300 rpm for 5-7 minutes, washed once with PBS with 1% BSA, counted and pulsed with 40μg/ml of peptide at a cell concentration of 1-2×10$^6$/ml in the presence of 3μg/ml ß$_2$- microglobulin for 4 hours at 20°C. The DC were then irradiated (4,200 rads), washed 1 time with medium and counted again.

*Setting up induction cultures:* 0.25 ml cytokine-generated DC (@1×10$^5$ cells/ml) were co-cultured with 0.25ml of CD8+ T-cells (@2x10$^6$ cell/ml) in each well of a 48-well plate in the presence of 10 ng/ml of IL-7. rHuman IL10 0 was added the next day at a final concentration of 10 ng/ml and rhuman IL2 was added 48 hours later at 10IU/ml.

*Restimulation of the induction cultures with peptide-pulsed adherent cells:* Seven and fourteen days after the primary induction the cells were restimulated with peptide-pulsed adherent cells. The PBMCS were thawed and washed twice with RPMI and DNAse. The cells were resuspended at 5×10$^6$ cells/ml and irradiated at ~4200 rads. The PBMCs were plated at 2×10$^6$ in 0.5ml complete medium per well and incubated for 2 hours at 37°C. The plates were washed twice with RPMI by tapping the plate gently to remove the nonadherent cells and the adherent cells pulsed with 10μg/ml of peptide in the presence of 3 μg/ml ß$_2$ microglobulin in 0.25ml RPMI/5%AB per well for 2 hours at 37°C. Peptide solution from each well was aspirated and the wells were washed once with RPMI. Most of the media was aspirated from the induction cultures (CD8+ cells) and brought to 0.5 ml with fresh media. The cells were then transferred to the wells containing the peptide-pulsed adherent cells. Twenty four hours later rhuman IL10 was added at a final concentration of 10ng/ml and rhuman IL2 was added the next day and again 2-3 days later at 50IU/ml (Tsai *et al., Critical Reviews in Immunology* 18(1-2):65-75, 1998). Seven days later the cultures were assayed for CTL activity in a $^{51}$Cr release assay. In some experiments the cultures were assayed for peptide-specific recognition in the in situ IFNγ ELISA at the time of the second restimulation followed by assay of endogenous recognition 7 days later. After expansion, activity was measured in both assays for a side by side comparison.

**Measurement of CTL lytic activity by $^{51}$Cr release.**

Seven days after the second restimulation, cytotoxicity was determined in a standard (5hr),$^{51}$Cr release assay by assaying individual wells at a single E:T. Peptide-pulsed targets were prepared by incubating the cells with 10μg/ml peptide overnight at 37°C.

Adherent target cells were removed from culture flasks with trypsin-EDTA. Target cells were labelled with 200μCi of $^{51}$Cr sodium chromate (Dupont, Wilmington, DE) for 1 hour at 37°C. Labelled target cells are resuspended at 10$^6$ per ml and diluted 1:10 with K562 cells at a concentration of 3.3×10$^6$/ml (an NK-sensitive erythroblastoma cell line used to reduce non-specific lysis). Target cells (100 μl) and 100μl of effectors were plated in 96 well round-bottom plates and incubated for 5 hours at 37°C. At that time, 100 μl of supernatant were collected from each well and percent lysis was determined according to the formula: [(cpm of the test sample- cpm of the spontaneous $^{51}$Cr release sample)

/(cpm of the maximal $^{51}$Cr release sample- cpm of the spontaneous $^{51}$Cr release sample)] x 100. Maximum and spontaneous release were determined by incubating the labelled targets with 1% Tritinn X-100 and media alone, respectively. A positive culture was defined as one in which the specific lysis (sample- background) was 10% or higher in the case of individual wells and was 15% or more at the 2 highest E:T ratios when expanded cultures were assayed.

### *In situ* Measurement of Human $\gamma$IFN Production as an Indicator of Peptide-specific and Endogenous Recognition

Immulon 2 plates were coated with mouse anti-human IFN$\gamma$ monoclonal antibody (4 $\mu$g/ml 0.1M NaHCO$_3$, pH8.2) overnight at 4°C. The plates were washed with Ca$^{2+}$, Mg$^{2+}$-free PBS/0.05% Tween 20 and blocked with PBS/ 10% FCS for 2 hours, after which the CTLs (100 $\mu$l/well) and targets (100 $\mu$l/well) were added to each well, leaving empty wells for the standards and blanks (which received media only). The target cells, either peptide-pulsed or endogenous targets, were used at a concentration of 1x10$^6$ cells/ml. The plates were incubated for 48 hours at 37°C with 5% CO$_2$.

Recombinant human IFN$\gamma$ was added to the standard wells starting at 400 pg or 1200pg/100$\mu$l/well and the plate incubated for 2 hours at 37°C. The plates were washed and 100 $\mu$l of biotinylated mouse anti-human IFN$\gamma$ monoclonal antibody (4$\mu$g/ml in PBS/3%FCS/0.05% Tween 20) were added and incubated for 2 hours at room temperature. After washing again, 100 $\mu$l HRP-streptavidin were added and the plates incubated for 1 hour at room temperature. The plates were then washed 6x with wash buffer, 100$\mu$l/well developing solution (TMB 1:1) were added, and the plates allowed to develop for 5-15 minutes. The reaction was stopped with 50 $\mu$l/well 1M H$_3$PO$_4$ and read at OD450. A culture was considered positive if it measured at least 50 pg of IFN$\gamma$/well above background and was twice the background level of expression.

**CTL Expansion.** Those cultures that demonstrated specific lytic activity against peptide-pulsed targets and/ or tumor targets were expanded over a two week period with anti-CD3. Briefly, $5 \times 10^4$ CD8+ cells were added to a T25 flask containing the following: 1x10$^6$ irradiated (4,200 rad) PBMC (autologous or allogeneic) per ml, 2x10$^5$ irradiated (8,000 rad) EBV- transformed cells per ml, and OKT3 (anti-CD3) at 30ng per ml in RPMI-1640 containing 10% (v/v) human AB serum, non-essential amino acids, sodium pyruvate, 25$\mu$M 2-mercaptoethanol, L-glutamine and penicillin/ streptomycin. rHuman IL2 was added 24 hours later at a final concentration of 200IU/ml and every 3 days thereafter with fresh media at 50IU/ml. The cells were split if the cell concentration exceeded 1x10$^6$/ml and the cultures were assayed between days 13 and 15 at E:T ratios of 30, 10, 3 and 1:1 in the $^{51}$Cr release assay or at $1 \times 10^6$/ml in the *in situ* IFN$\gamma$ assay using the same targets as before the expansion.

### *Immunogenicity of A2 supermotif-bearing peptides*

A2-supermotif cross-reactive binding peptides were tested in the cellular assay for the ability to induce peptide-specific CTL in normal individuals. In this analysis, a peptide was considered to be an epitope if it induced peptide-specific CTLs in at least 2 donors (unless otherwise noted) and if those CTLs also recognized the endogenously expressed peptide. Peptides that were able to induce a peptide-specific CTL response in at least 2 normal donors are shown in Table XXVII. Further analysis demonstrated those that also recognized target cells pulsed with the wild-type peptide and tumor targets that endogenously express HER2/neu (Table XXVII). An additional wild-type peptide, Her2/neu.5 was selected for evaluation based on its A2.1 binding affinity and, although it binds to only 2 HLA-A2 supertype molecules, it was capable of generating a strong CTL response that was both peptide- and tumor-specific.

Immunogenicity was additionally confirmed using PBMCs isolated from cancer patients. Briefly, PBMCs were isolated from two patients with ovarian cancer, re-stimulated with peptide-pulsed monocytes and assayed for the ability to recognize peptide-pulsed target cells as well as transfected cells endogenously expressing the antigen. These data indicated that Her2/neu.435 was recognized in 2 donors as well as Her2/neu.369, Her2/neu.952, and Her2/neu.48. Her2/neu.689 is also an epitope, but not a supertype binder. Of the other peptides tested, Her2/neu.665 and Her2/neu. 773 were recognized by CTLs from only one of the two patients and CTLs to Her2/neu.153 and Her2/neu.789 recognized peptide-pulsed targets only.

### *Evaluation of A*03/A11 immunogenicity*

HLA-A3 supermotif-bearing cross-reactive binding peptides are also evaluated for immunogenicity using methodology analogous for that used to evaluate the immunogenicity of the HLA-A2 supermotif peptides. Using this procedure, peptides that induce an immune response are identified. Examples of such peptides are shown in Table XXIII.

*Evaluation of immunogenicity of Motif/Supermotif-Bearing Peptides.*

Analogous methodology, as appreciated by one of ordinary skill in the art, is employed to determine immunogenicity of peptides bearing HLA class I motifs and/or supermotifs set out herein. Using such a prodcedure peptides that induce an immune response are identified.

Example 4. Implementation of the Extended Supermotif to Improve the Binding Capacity of Native Epitopes by Creating Analogs

HLA motifs and supermotifs (comprising primary and/or secondary residues) are useful in the identification and preparation of highly cross-reactive native peptides, as demonstrated herein. Moreover, the definition of HLA motifs and supermotifs also allows one to engineer highly cross-reactive epitopes by identifying residues within a native peptide sequence which can be analogued, or "fixed" to confer upon the peptide certain characteristics, e.g. greater cross-reactivity within the group of HLA molecules that comprise a supertype, and/or greater binding affinity for some or all of those HLA molecules. Examples of analog peptides that exhibit modulated binding affinity are set forth in this example and provided in Tables XXII through XXVII.

*Analoguing at Primary Anchor Residues*

Peptide engineering strategies were implemented to further increase the cross-reactivity of the epitopes identified above. On the basis of the data disclosed, *e.g.*, in related and co-pending U.S.S.N 09/226,775, the main anchors of A2-supermotif-bearing peptides are altered, for example, to introduce a preferred L, I, V, or M at position 2, and I or V at the C-terminus.

Peptides that exhibit at least weak A*0201 binding ($IC_{50}$ of 5000 nM or less), and carrying suboptimal anchor residues at either position 2, the C-terminal position, or both, can be fixed by introducing canonical substitutions (L at position 2 and V at the C-terminus). Those analogued peptides that show at least a three-fold increase in A*0201 binding and bind with an $IC_{50}$ of 500 nM, or less were then tested for A2 cross-reactive binding along with their wild-type (WT) counterparts. Analogued peptides that bind at least three of the five A2 supertype alleles were then selected for cellular screening analysis (Table XXVII).

Additionally, the selection of analogs for cellular screening analysis was further restricted by the capacity of the WT parent peptide to bind at least weakly, *i.e.,* bind at an $IC_{50}$ of 5000nM or less, to three of more A2 supertype alleles. The rationale for this requirement is that the WT peptides must be present endogenously in sufficient quantity to be biologically relevant. Analogued peptides have been shown to have increased immunogenicity and cross-reactivity by T cells specific for the WT epitope *(see, e.g.,* Parkhurst *et al., J. Immunol.* 157:2539, 1996; and Pogue *et al., Proc. Natl. Acad. Sci. USA* 92:8166, 1995).

In the cellular screening of these peptide analogs, it is important to demonstrate that analog-specific CTLs are also able to recognize the wild-type peptide and, when possible, tumor targets that endogenously express the epitope.

Of the 20 peptides identified in Example 2 that bound to HLA-A*0201 at a high affinity, 15 carried suboptimal primary anchor residues and met the criterion for analoguing at primary anchor residues by introducing a canonical substitution. Analogs of six of the A*0201-binding peptides were created and tested for primary binding to HLA-A*0201 and supertype binding (Table XXII). In 4 of 6 cases, binding to HLA-A*0201 was improved at least three-fold. In 4 cases, crossbinding capability was also improved. In one instance, peptide Her2/neu.153 did not show a three-fold increase in binding to HLA-A*0201, but crossbinding was improved.

Additionally, 22 peptides that weakly bound to HLA-A*0201 that carry suboptimal anchors were also identified and can also be analogued.

Two analogs of Her2/neu.5, two analogs of Her2/neu.369, one version of Her2/neu.952, and one version of Her2/neu.665 were selected for cellular screening studies. As shown in Table XXVII, both Her2/neu.369L2V9 and V2V9 induced peptide-specific CTLs and those CTLs also recognized the target tumor cells expressing that endogenously express the antigen. Her2neu.5B3V9 and Her2/neu.952L2B7V10 induced peptide-specific CTLs in at least 2 donors, but when the positive cultures were expanded, no wild-type peptide or endogenous recognition was observed.

The Her2/neu.665L2V9 analog exhibited binding to four of the five A2 supertype alleles tested, whereas the wildtype peptide only binds two of the five alleles. In the cellular screening analysis, a strong peptide-specific CTL response was observed. The positive cultures were expanded and assayed for peptide and endogenous recognition. Peptide-specific CTL activity was maintained in some of the cultures, but no corresponding endogenous recognition was observed.

Using methodology similar to that used to develop HLA-A2 analogs, analogs of HLA-A3 and HLA-B7 supermotif-bearing epitopes are also generated. For example, peptides binding at least weakly to 3/5 of the A3-supertype molecules can be engineered at primary anchor residues to possess a preferred residue (V, S, M, or A) at position 2. The analog peptides are then tested for the ability to bind A*03 and A*11 (prototype A3 supertype alleles). Those peptides that demonstrate ≤ 500 nM binding capacity are then tested for A3-supertype cross-reactivity. Examples of HLA-A3 supermotif analog peptides are provided in Table XXIII.

B7 supermotif-bearing peptides can, for example, be engineered to possess a preferred residue (V, I, L, or F) at the C-terminal primary anchor position *(see, e.g.* Sidney *et al. (J. Immunol.* 157:3480-3490, 1996). Analoged peptides are then tested for cross-reactive binding to B7 supertype alleles. Examples of B7-supermotif-bearing analog peptides are provided in Table XXIV.

Similarly, HLA-A1 and HLA-A24 motif-bearing peptides can be engineered at primary anchor residues to improvde binding to the allele-specific HLA molecule or to improve cross-reactive binding. Examples of analoged HLA-A1 and HLA-A24 motif-bearing peptides are provided in Tables XXV and XXVI.

Analoged peptides that exhibit improved binding and/or or cross-reactivity are evaluated for immunogenicity using methodology similar to that described for the analysis of HLA-A2 supermotif-bearing peptides. Using such a procedure, peptides that induce an immune response are identified, e.g. Table XXIII.

*Analoguing at Secondary Anchor Residues*

Moreover, HLA supermotifs are of value in engineering highly cross-reactive peptides and/or peptides that bind HLA molecules with increased affinity by identifying particular residues at secondary anchor positions that are associated with such properties. Examples of such analoged peptides are provided in Table XXIV.

For example, the binding capacity of a B7 supermotif-bearing peptide representing a discreet single amino acid substitution at position 1 can be analyzed. A peptide can, for example, be analogued to substitute L with F at position 1 and subsequently be evaluated for increased binding affinity/ and or increased cross-reactivity. This procedure will identify analogued peptides with modulated binding affinity.

Analoged peptides that exhibit improved binding and/or or cross-reactivity are evaluated for immunogenicity using methodology similar to that described for the analysis of HLA-A2 supermotif-bearing peptides. Using such a procedure, peptides that induce an immune response are identified.

*Other analoguing strategies*

Another form of peptide analoguing, unrelated to the anchor positions, involves the substitution of a cysteine with α-amino butyric acid. Due to its chemical nature, cysteine has the propensity to form disulfide bridges and sufficiently alter the peptide structurally so as to reduce binding capacity. Subtitution of α-amino butyric acid for cysteine not only alleviates this problem, but has been shown to improve binding and crossbinding capabilities in some instances (*see, e.g.*, the review by Sette *et al.,* In: Persistent Viral Infections, Eds. R. Ahmed and I. Chen, John Wiley & Sons, England, 1999).

Analoged peptides that exhibit improved binding and/or or cross-reactivity are evaluated for immunogenicity using methodology similar to that described for the analysis of HLA-A2 supermotif-bearing peptides. Using such a procedure, peptides that induce an immune response are identified.

This Example therefore demonstrates that by the use of even single amino acid substitutions, the binding affinity and/or cross-reactivity of peptide ligands for HLA supertype molecules is modulated.

Example 5. Identification of peptide epitope sequences with HLA-DR binding motifs

Peptide epitopes bearing an HLA class II supermotif or motif may also be identified as outlined below using methodology similar to that described in Examples 1-3.

*Selection of HLA-DR-supermotif-bearing epitopes*

To identify HLA class II HTL epitopes, the HER2/neu protein sequence was analyzed for the presence of sequences bearing an HLA-DR-motif or supermotif. Specifically, 15-mer sequences were selected comprising a DR-supermotif, further comprising a 9-mer core, and three-residue N- and C-terminal flanking regions (15 amino acids total).

Protocols for predicting peptide binding to DR molecules have been developed (Southwood *et al., J. Immunol.* 160:3363-3373, 1998). These protocols, specific for individual DR molecules, allow the scoring, and ranking, of 9-mer core regions. Each protocol not only scores peptide sequences for the presence of DR-supermotif primary anchors (i. e., at position 1 and position 6) within a 9-mer core, but additionally evaluates sequences for the presence of secondary anchors. Using allele specific selection tables (see, *e.g.*, Southwood *et al., ibid.),* it has been found that these protocols efficiently select peptide sequences with a high probability of binding a particular DR molecule. Additionally, it has been found that performing these protocols in tandem, specifically those for DR1, DR4w4, and DR7, can efficiently select DR cross-reactive peptides.

The HER2/neu-derived peptides identified above were tested for their binding capacity for various common HLA-DR molecules. All peptides were initially tested for binding to the DR molecules in the primary panel: DR1, DR4w4, and DR7. Peptides binding at least 2 of these 3 DR molecules with an $IC_{50}$ value of 1000 nM or less, were then tested for binding to DR5*0101, DRB 1*1501, DRB1*1101, DRB1*0802, and DRB1*1302. Peptides were considered to be cross-reactive DR supertype binders if they bound at an $IC_{50}$ value of 1000 nM or less to at least 5 of the 8 alleles tested.

Following the strategy outlined above, 188 DR supermotif-bearing sequences were identified within the HER2/neu protein sequence. Of those, 41 scored positive in 2 of the 3 combined DR 147 algorithms. These peptides were synthesized and tested for binding to HLA-DRB1*0101, DRB1*0401), DRB1*0701. Of the 41 peptides tested, 18 bound at least 2 of the 3 alleles (Table XXVIII).

These 18 peptides were then tested for binding to secondary DR supertype alleles: DRB5*0101, DRB1*1501, DRB1*1101, DRB1*0802, and DRB1*1302. Nine peptides were identified that bound at least 5 of the 8 alleles tested, of which 8 occurred in distinct, non-overlapping regions (Table XXIX).

*Selection of DR3 motif peptides*

Because HLA-DR3 is an allele that is prevalent in Caucasian, Black, and Hispanic populations, DR3 binding capacity is an important criterion in the selection of HTL epitopes. However, data generated previously indicated that DR3 only rarely cross-reacts with other DR alleles (Sidney *et al., J. Immunol.* 149:2634-2640, 1992; Geluk *et al., J. Immunol.* 152:5742-5748, 1994; Southwood *et al., J. Immunol.* 160:3363-3373, 1998). This is not entirely surprising in that the DR3 peptide-binding motif appears to be distinct from the specificity of most other DR alleles. For maximum efficiency in developing vaccine candidates it would be desirable for DR3 motifs to be clustered in proximity with DR supermotif regions. Thus, peptides shown to be candidates may also be assayed for their DR3 binding capacity. However, in view of the distinct binding specificity of the DR3 motif, peptides binding only to DR3 can also be considered as candidates for inclusion in a vaccine formulation.

To efficiently identify peptides that bind DR3, the HER2/neu protein sequence was analyzed for conserved sequences carrying one of the two DR3 specific binding motifs (Table III) reported by Geluk *et al. (J. Immunol.* 152: 5742-5748, 1994). Forty-six motif-positive peptides were identified. The corresponding peptides were then synthesized and tested for the ability to bind DR3 with an affinity of 1000 nM or better, *i.e.*, less than 1000 nM. Seven peptides were found that met this binding criterion (Table XXX), and thereby qualify as HLA class II high affinity binders.

Additionally, the 7 DR3 binders were tested for binding to the DR supertype alleles (Table XXXI). Four of the seven DR3 binders bound at least 3 other DR alleles, and one peptide, Her2/neu.886, was a cross-reactive supertype binder as well. Conversely, the DR supertype cross-reactive binding peptides were also tested for DR3 binding capacity. The cross-reactive DR supermotif-bearing peptides showed little capacity to bind DR3 molecules (Table XXXI).

DR3 binding epitopes identified in this manner may then be included in vaccine compositions with DR supermotif-bearing peptide epitopes.

In summary, 8 DR supertype cross-reactive binding peptides and 7 DR3 binding peptides were identified from the HER2/neu protein sequence, with one peptide shared between the two motifs. Of these, 5 DR supertype and 5 DR3-binding peptides were located in the intracellular domain.

Similarly to the case of HLA class I motif-bearing peptides, the class II motif-bearing peptides may be analogued to improve affinity or cross-reactivity. For example; aspartic acid at position 4 of the 9-mer core sequence is an optimal residue for DR3 binding, and substitution for that residue may improve DR 3 binding. Analoged peptides are evaluated for immunogenicity in accordance with the methodology of Example 6.

Example 6. Immunogenicity of HTL epitopes

This example determines immunogenic DR supermotif- and DR3 motif-bearing epitopes among those identified using the methodology in Example 5. Immunogenicity of HTL epitopes are evaluated in a manner analogous to the determination of immunogenicity of CTL epitopes by assessing the ability to stimulate HTL responses and/or by using appropriate transgenic mouse models. Immunogenicity is determined by screening for: 1.) *in vitro* primary induction using normal PBMC or 2.) recall responses from cancer patient PBMCs. Such a procedure identifies epitopes that induce an HTL response.

Example 7. Calculation of phenotypic frequencies of HLA-supertypes in various ethnic backgrounds to determine breadth of population coverage

This example illustrates the assessment of the breadth of population coverage of a vaccine composition comprised of multiple epitopes comprising multiple supermotifs and/or motifs.

In order to analyze population coverage, gene frequencies of HLA alleles were determined. Gene frequencies for each HLA allele were calculated from antigen or allele frequencies utilizing the binomial distribution formulae gf=1-(SQRT(1-af)) (see, *e.g., Sidney et al., Human Immunol.* 45:79-93, 1996). To obtain overall phenotypic frequencies, cumulative gene frequencies were calculated, and the cumulative antigen frequencies derived by the use of the inverse formula [af=1-(1-Cgf)$^2$].

Where frequency data was not available at the level of DNA typing, correspondence to the serologically defined antigen frequencies was assumed. To obtain total potential supertype population coverage no linkage disequilibrium was assumed, and only alleles confirmed to belong to each of the supertypes were included (minimal estimates). Estimates of total potential coverage achieved by inter-loci combinations were made by adding to the A coverage the proportion of the non-A covered population that could be expected to be covered by the B alleles considered (e.g., total=A+B*(1-A)). Confirmed members of the A3-like supertype are A3, A11, A31, A*3301, and A*6801. Although the A3-like supertype may also include A34, A66, and A*7401, these alleles were not included in overall frequency calculations. Likewise, confirmed members of the A2-like supertype family are A*0201, A*0202, A*0203, A*0204, A*0205, A*0206, A*0207, A*6802, and A*6901. Finally, the B7-like supertype-confirmed alleles are: B7, B*3501-03, B51, B*5301, B*5401, B*5501-2, B*5601, B*6701, and B*7801 (potentially also B*1401, B*3504-06, B*4201, and B*5602).

Population coverage achieved by combining the A2-, A3- and B7-supertypes is approximately 86% in five major ethnic groups (see Table XXI). Coverage may be extended by including peptides bearing the A1 and A24 motifs. On average, A1 is present in 12% and A24 in 29% of the population across five different major ethnic groups (Caucasian, North American Black, Chinese, Japanese, and Hispanic). Together, these alleles are represented with an average frequency of 39% in these same ethnic populations. The total coverage across the major ethnicities when A1 and A24 are combined with the coverage of the A2-, A3- and B7-supertype alleles is >95%. An analogous approach can be used to estimate population coverage achieved with combinations of class II motif-bearing epitopes.

Example 8. Recognition Of Generation Of Endogenous Processed Antigens After Priming

This example determines that CTL induced by native or analogued peptide epitopes identified and selected as described in Examples 1-6 recognize endogenously synthesized, *i.e.,* native antigens, using a transgenic mouse model.

Effector cells isolated from transgenic mice that are immunized with peptide epitopes (as described, e.g., in Wentworth et al., *Mol. Immunol.* 32:603, 1995), for example HLA-A2 supermotif-bearing epitopes, are re-stimulated *in vitro* using peptide-coated stimulator cells. Six days later, effector cells are assayed for cytotoxicity and the cell lines that contain peptide-specific cytotoxic activity are further re-stimulated. An additional six days later; these cell lines are tested for cytotoxic activity on $^{51}$Cr labeled Jurkat-A2.1/K$^b$ target cells in the absence or presence of peptide, and also tested on $^{51}$Cr labeled target cells bearing the endogenously synthesized antigen, *i.e.* cells that are stably transfected with TAA expression vectors.

The result will demonstrate that CTL lines obtained from animals primed with peptide epitope recognize endogenously synthesized antigen. The choice of transgenic mouse model to be used for such an analysis depends upon the epitope(s) that is being evaluated. In addition to HLA-A*0201/K$^b$ transgenic mice, several other transgenic mouse models including mice with human A11, which may also be used to evaluate A3 epitopes, and B7 alleles have been characterized and others (e.g., transgenic mice for HLA-A1 and A24) are being developed. HLA-DR1 and HLA-DR3 mouse models have also been developed, which may be used to evaluate HTL epitopes.

Example 9. Activity Of CTL-HTL Conjugated Epitopes In Transgenic Mice

This example illustrates the induction of CTLs and HTLs in transgenic mice by use of a tumor associated antigen CTL/HTL peptide conjugate whereby the vaccine composition comprises peptides to be administered to a cancer patient. The peptide composition can comprise multiple CTL and/or HTL epitopes and further, can comprise epitopes selected from multiple-tumor associated antigens. The epitopes are identified using methodology as described in Examples 1-6 This analysis demonstrates the enhanced immunogenicity that can be achieved by inclusion of one or more HTL epitopes in a vaccine composition. Such a peptide composition can comprise an HTL epitope conjugated to a preferred CTL epitope containing, for example, at least one CTL epitope selected from Tables XXVII and XXIII-XXVI, or other analogs of that epitope. The HTL epitope is, for example, selected from Table XXXI. The peptides may be lipidated, if desired.

Immunization procedures: Immunization of transgenic mice is performed as described (Alexander *et al., J. Immunol.* 159:4753-4761, 1997). For example, A2/K$^b$ mice, which are transgenic for the human HLA A2.1 allele and are useful for the assessment of the immunogenicity of HLA-A*0201 motif- or HLA-A2 supermotif-bearing epitopes, are primed subcutaneously (base of the tail) with 0.1 ml of peptide conjugate formulated in saline, or DMSO/saline.

Seven days after priming, splenocytes obtained from these animals are restimulated with syngenic irradiated LPS-activated lymphoblasts coated with peptide.

The target cells for peptide-specific cytotoxicity assays are Jurkat cells transfected with the HLA-A2.1/K$^b$chimeric gene (e.g., Vitiello *et al., J. Exp. Med.* 173:1007, 1991).

*In vitro* CTL activation: One week after priming, spleen cells (30x10$^6$ cells/flask) are co-cultured at 37°C with syngeneic, irradiated (3000 rads), peptide coated lymphoblasts (10×10$^6$ cells/flask) in 10 ml of culture medium/T25 flask. After six days, effector cells are harvested and assayed for cytotoxic activity.

Assay for cytotoxic activity: Target cells (1.0 to 1.5×10$^6$) are incubated at 37°C in the presence of 200 μl of $^{51}$Cr. After 60 minutes, cells are washed three times and resuspended in medium. Peptide is added where required at a concentration of 1 μg/ml. For the assay, 10$^4$ $^{51}$Cr-labeled target cells are added to different concentrations of effector cells (final volume of 200 μl) in U-bottom 96-well plates. After a 6 hour incubation period at 37°C, a 0.1 ml aliquot of supernatant is removed from each well and radioactivity is determined in a Micromedic automatic gamma counter. The percent specific lysis is determined by the formula: percent specific release = 100 x (experimental release-spontaneous release)/(maximum release-spontaneous release). To facilitate comparison between separate CTL assays run under the same conditions, % $^{51}$Cr release data is expressed as lytic units/10$^6$ cells. One lytic unit is arbitrarily defined as the number of effector cells required to achieve 30% lysis of 10,000 target cells in a 6 hour $^{51}$Cr release assay. To obtain specific lytic units/10$^6$, the lytic units/10$^6$ obtained in the absence of peptide is subtracted from the lytic units/10$^6$ obtained in the presence of peptide. For example, if 30% $^{51}$Cr release is obtained at the effector (E): target (T) ratio of 50:1 (i.e., 5×10$^5$ effector cells for 10,000 targets) in the absence of peptide and 5:1 (i.e., 5×10$^4$ effector cells for 10,000 targets) in the presence of peptide, the specific lytic units would be: [(1/50,000)-(1/500,000)] x 10$^6$= 18 LU.

The results are analyzed to assess the magnitude of the CTL responses of animals injected with the immunogenic CTL/HTL conjugate vaccine preparation. The magnitude and frequency of the response can also be compared to the the CTL response achieved using the CTL epitopes by themselves. Analyses similar to this may be performed to evaluate the immunogenicity of peptide conjugates containing multiple CTL epitopes and/or multiple HTL epitopes. In accordance with these procedures it is found that a CTL response is induced, and concomitantly that an HTL response is induced upon administration of such compositions.

Example 10. Selection of CTL and HTL epitopes for inclusion in a cancer vaccine.

This example illustrates the procedure for the selection of peptide epitopes for vaccine compositions of the invention. The peptides in the composition can be in the form of a nucleic acid sequence, either single or one or more sequences (i.e., minigene) that encodes peptide(s), or may be single and/or polyepitopic peptides.

The following principles are utilized when selecting an array of epitopes for inclusion in a vaccine composition. Each of the following principles is balanced in order to make the selection.

Epitopes are selected which, upon administration, mimic immune responses that have been observed to be correlated with tumor clearance. For example, a vaccine can include 3-4 epitopes that come from at least one TAA. Epitopes from one TAA can be used in combination with epitopes from one or more additional TAAs to produce a vaccine that targets tumors with varying expression patterns of frequently-expressed TAAs as described, e.g., in Example 15.

Epitopes are preferably selected that have a binding affinity (IC50) of 500 nM or less, often 200 nM or less, for an HLA class I molecule, or for a class II molecule, 1000 nM or less.

Sufficient supermotif bearing peptides, or a sufficient array of allele-specific motif bearing peptides, are selected to give broad population coverage. For example, epitopes are selected to provide at least 80% population coverage. A Monte Carlo analysis, a statistical evaluation known in the art, can be employed to assess breadth, or redundancy, of population coverage.

When selecting epitopes from cancer-related antigens it is often preferred to select analogs because the patient may have developed tolerance to the native epitope.

When creating a polyepitopic composition, e.g. a minigene, it is typically desirable to generate the smallest peptide possible that encompasses the epitopes of interest, although spacers or other flanking sequences can also be incorporated. The principles employed are often similar as those employed when selecting a peptide comprising nested epitopes. Additionally; however, upon determination of the nucleic acid sequence to be provided as a minigene, the peptide sequence encoded thereby is analyzed to determine whether any "junctional epitopes" have been created. A junctional epitope is a potential HLA binding epitope, as predicted, *e.g.,* by motif analysis. Junctional epitopes are generally to be avoided because the recipient may bind to an HLA molecule and generate an immune response to that epitope, which is not present in a native protein sequence.

CTL epitopes for inclusion in vaccine compositions are, for example, selected from those listed in Tables XXVII and XXIII-XXVI. Examples of HTL epitopes that can be included in vaccine compositions are provided in Table XXXI. A vaccine composition comprised of selected peptides, when administered, is safe, efficacious, and elicits an immune response that results in tumor cell killing and reduction of tumor size or mass.

Example 11. Construction of Minigene Multi-Epitope DNA Plasmids

This example provides general guidance for the construction of a minigene expression plasmid. Minigene plasmids may, of course, contain various configurations of CTL and/or HTL epitopes or epitope analogs as described herein. Expression plasmids have been constructed and evaluated as described, for example, in co-pending U.S.S. N. 09/311,784 filed 5/13/99.

A minigene expression plasmid may include multiple CTL and HTL peptide epitopes. In the present example, HLA-A2, -A3, -B7 supermotif-bearing peptide epitopes and HLA-A1 and -A24 motif-bearing peptide epitopes are used in conjunction with DR supermotif-bearing epitopes and/or DR3 epitopes. Preferred epitopes are identified, for example, in Tables XXVII, XXIII-XXVI, and XXXI. HLA class I supermotif or motif-bearing peptide epitopes derived from multiple TAAs are selected such that multiple supermotifs/motifs are represented to ensure broad population coverage. Similarly, HLA class II epitopes are selected from multiple tumor antigens to provide broad population coverage, *i.e.* both HLA DR-1-4-7 supermotif-bearing epitopes and HLA DR-3 motif-bearing epitopes are selected for inclusion in the minigene construct. The selected CTL and HTL epitopes are then incorporated into a minigene for expression in an expression vector.

This example illustrates the methods to be used for construction of such a minigene-bearing expression plasmid. Other expression vectors that may be used for minigene compositions are available and known to those of skill in the art.

The minigene DNA plasmid contains a consensus Kozak sequence and a consensus murine kappa Ig-light chain signal sequence followed by CTL and/or HTL epitopes selected in accordance with principles disclosed herein. The sequence encodes an open reading frame fused to the Myc and His antibody epitope tag coded for by the pcDNA 3.1 Myc-His vector.

Overlapping oligonucleotides, for example eight oligonucleotides, averaging approximately 70 nucleotides in length with 15 nucleotide overlaps, are synthesized and HPLC-purified. The oligonucleotides encode the selected peptide epitopes as well as appropriate linker nucleotides, Kozak sequence, and signal sequence. The final multiepitope minigene is assembled by extending the overlapping oligonucleotides in three sets of reactions using PCR. A Perkin/Elmer 9600 PCR machine is used and a total of 30 cycles are performed using the following conditions: 95°C for 15 sec, annealing temperature (5° below the lowest calculated Tm of each primer pair for 30 sec; and 72°C for 1 min.

For the first PCR reaction, 5 µg of each of two oligonucleotides are annealed and extended: Oligonucleotides 1+2, 3+4, 5+6, and 7+8 are combined in 100 µl reactions containing Pfu polymerase buffer (1x= 10 mM KCL, 10 mM $(NH_4)_2SO_4$, 20 mM Tris-chloride, pH 8.75, 2 mM $MgSO_4$, 0.1% Triton X-100, 100 µg/ml BSA), 0.25 mM each dNTP, and 2.5 U of *Pfu* polymerase. The full-length dimer products are gel-purified, and two reactions containing the product of 1+2 and 3+4, and the product of 5+6 and 7+8 are mixed, annealed, and extended for 10 cycles. Half of the two reactions are then mixed, and 5 cycles of annealing and extension carried out before flanking primers are added to amplify the full length product for 25 additional cycles. The full-length product is gel-purified and cloned into pCR-blunt (Invitrogen) and individual clones are screened by sequencing.

Example 12. The plasmid construct and the degree to which it induces immunogenicity.

The degree to which the plasmid construct prepared using the methodology outlined in Example 11 is able to induce immunogenicity is evaluated through *in vivo* injections into mice and subsequent in *vitro* assessment of CTL and HTL activity, which are analysed using cytotoxicity and proliferation assays, respectively, as detailed *e.g.*, in U.S. S.N. 09/311,784 filed 5/13/99 and Alexander *et al., Immunity* 1:751-761, 1994.

Alternatively, plasmid constructs can be evaluated *in vitro* by testing for epitope presentation by APC following transduction or transfection of the APC with an epitope-expressing nucleic acid construct. Such a study determines "antigenicity" and allows the use of human APC. The assay determines the ability of the epitope to be presented by the APC in a context that is recognized by a T cell by quantifying the density of epitope-HLA class I complexes on the cell surface. Quantitation can be performed by directly measuring the amount of peptide eluted from the APC *(see, e. g.,* Sijts *et al., J. Immunol.* 156:683-692, 1996; Demotz *et al., Nature* 342:682-684, 1989); or the number of peptide-HLA class I complexes can be estimated by measuring the amount of lysis or lymphokine release induced by infected or transfected target cells, and then determining the concentration of peptide necessary to obtained equivalent levels of lysis or lymphokine release *(see, e.g.,* Kageyama *et al., J. Immunol.* 154:567-576, 1995).

To assess the capacity of the pMin minigene construct (e.g., a pMin minigene construct generated as described in U.S.S.N. 09/311,784) to induce CTLs *in vivo,* HLA-A11/K[b] transgenic mice, for example, are immunized intramuscularly with 100 µg of naked cDNA. As a means of comparing the level of CTLs induced by cDNA immunization, a control group of animals is also immunized with an actual peptide composition that comprises multiple epitopes synthesized as a single polypeptide as they would be encoded by the minigene.

Splenocytes from immunized animals are stimulated twice with each of the respective compositions (peptide epitopes encoded in the minigene or the polyepitopic peptide), then assayed for peptide-specific cytotoxic activity in a $^{51}$Cr release assay. The results indicate the magnitude of the CTL response directed against the A3-restricted epitope, thus indicating the *in vivo* immunogenicity of the minigene vaccine and polyepitopic vaccine. It is, therefore, found that the minigene elicits immune responses directed toward the HLA-A3 supermotif peptide epitopes as does the polyepitopic peptide vaccine. A similar analysis is also performed using other HLA-A2 and HLA-B7 transgenic mouse models to assess CTL induction by HLA-A2 and HLA-B7 motif or supermotif epitopes.

To assess the capacity of a class II epitope encoding minigene to induce HTLs *in vivo,* I-A$^b$ restricted mice, for example, are immunized intramuscularly with 100 µg of plasmid DNA. As a means of comparing the level of HTLs induced by DNA immunization, a group of control animals is also immunized with an actual peptide composition emulsified in complete Freund's adjuvant. CD4+ T cells, *i.e.* HTLs, are purified from splenocytes of immunized animals and stimulated with each of the respective compositions (peptides encoded in the minigene). The HTL response is measured using a $^3$H-thymidine incorporation proliferation assay, *(see, e.g.,* Alexander et al. Immunity 1:751-761, 1994). The results indicate the magnitude of the HTL response, thus demonstrating the *in vivo* immunogenicity of the minigene.

DNA minigenes, constructed as described in Example 11, may also be evaluated as a vaccine in combination with a boosting agent using a prime boost protocol. The boosting agent may consist of recombinant protein *(e.g.,* Barnett *et al., Aids Res. and Human Retroviruses* 14, Supplement 3:S299-S309, 1998) or recombinant vaccinia, for example, expressing a minigene or DNA encoding the complete protein of interest *(see, e.g.,* Hanke *et al., Vaccine* 16:439-445, 1998; Sedegah *et al., Proc. Natl. Acad. Sci USA* 95:7648-53, 1998; Hanke and McMichael, *Immunol. Letters* 66:177-181, 1999; and Robinson *et al., Nature Med.* 5:526-34, 1999).

For example, the efficacy of the DNA minigene may be evaluated in transgenic mice. In this example, A2.1/K$^b$ transgenic mice are immunized IM with 100 µg of the DNA minigene encoding the immunogenic peptides. After an incubation period (ranging from 3-9 weeks), the mice are boosted IP with 10$^7$ pfu/mouse of a recombinant vaccinia virus expressing the same sequence encoded by the DNA minigene. Control mice are immunized with 100 µg of DNA or recombinant vaccinia without the minigene sequence, or with DNA encoding the minigene, but without the vaccinia boost. After an additional incubation period of two weeks, splenocytes from the mice are immediately assayed for peptide-specific activity in an ELISPOT assay. Additionally, splenocytes are stimulated *in vitro* with the A2-restricted peptide epitopes encoded in the minigene and recombinant vaccinia, then assayed for peptide-specific activity in an IFN-γ ELISA. It is found that the minigene utilized in a prime-boost mode elicits greater immune responses toward the HLA-A2 supermotif peptides than with DNA alone. Such an analysis is also performed using other HLA-A11 and HLA-B7 transgenic mouse models to assess CTL induction by HLA-A3 and HLA-B7 motif or supermotif epitopes.

Example 13. Peptide Composition for Prophylactic Uses

Vaccine compositions of the present invention are used to prevent cancer in persons who are at risk for developing a tumor. For example, a polyepitopic peptide epitope composition (or a nucleic acid comprising the same) containing multiple CTL and HTL epitopes such as those selected in Examples 9 and/or 10, which are also selected to target greater than 80% of the population, is administered to an individual at risk for a cancer, *e.g.*, breast cancer. The composition is provided as a single polypeptide that encompasses multiple epitopes. The vaccine is administered in an aqueous carrier comprised of Freunds Incomplete Adjuvant. The dose of peptide for the initial immunization is from about 1 to about 50,000 µg, generally 100-5,000 µg for a 70 kg patient. The initial administration of vaccine is followed by booster dosages at 4 weeks followed by evaluation of the magnitude of the immune response in the patient, by techniques that determine the presence of epitope-specific CTL populations in a PBMC sample. Additional booster doses are administered as required. The composition is found to be both safe and efficacious as a prophylaxis against cancer.

Alternatively, the polyepitopic peptide composition can be administered as a nucleic acid in accordance with methodologies known in the art and disclosed herein.

Example 14. Polyepitopic Vaccine Compositions Derived from Native TAA Sequences

A native TAA polyprotein sequence is screened, preferably using computer algorithms defined for each class I and/or class II supermotif or motif, to identify "relatively short" regions of the polyprotein that comprise multiple epitopes and is preferably less in length than an entire native antigen. This relatively short sequence that contains multiple distinct, even overlapping, epitopes is selected and used to generate a minigene construct. The construct is engineered to express the peptide, which corresponds to the native protein sequence. The "relatively short" peptide is generally less than 1000, 500, or 250 amino acids in length, often less than 100 amino acids in length, preferably less than 75 amino acids in length, and more preferably less than 50 amino acids in length. The protein sequence of the vaccine composition is selected because it has maximal number of epitopes contained within the sequence, *i.e.,* it has a high concentration of epitopes. As noted herein, epitope motifs may be nested or overlapping (*i.e.*, frame shifted relative to one another). For example, with frame shifted overlapping epitopes, two 9-mer epitopes and one 10-mer epitope can be present in a 10 amino acid peptide. Such a vaccine composition is administered for therapeutic or prophylactic purposes.

The vaccine composition will preferably include, for example, three CTL epitopes and at least one HTL epitope from TAAs. This polyepitopic native sequence is administered either as a peptide or as a nucleic acid sequence which encodes the peptide. Alternatively, an analog can be made of this native sequence, whereby one or more of the epitopes comprise substitutions that alter the cross-reactivity and/or binding affinity properties of the polyepitopic peptide.

The embodiment of this example provides for the possibility that an as yet undiscovered aspect of immune system processing will apply to the native nested sequence and thereby facilitate the production of therapeutic or prophylactic immune response-inducing vaccine compositions. Additionally such an embodiment provides for the possibility of motif-bearing epitopes for an HLA makeup that is presently unknown. Furthermore, this embodiment (absent analogs) directs the immune response to multiple peptide sequences that are actually present in native TAAs thus avoiding the need to evaluate any junctional epitopes. Lastly, the embodiment provides an economy of scale when producing nucleic acid vaccine compositions.

Related to this embodiment, computer programs can be derived in accordance with principles in the art, which identify in a target sequence, the greatest number of epitopes per sequence length.

Example 15. Polyepitopic Vaccine Compositions Directed To Multiple Tumors

The HER2/neu peptide epitopes of the present invention are used in conjunction with peptide epitopes from other target tumor antigens to create vaccine composition that is useful for the treatment of various types of tumors. For example, a set of TAA epitopes can be selected that allows the targeting of most common epithelial tumors *(see, e.g.*, Kawashima *et al.*, *Hum. Immunol.* 59:1-14, 1998). Such a composition includes epitopes from CEA, HER-2/neu, and MAGE2/3, all of which are expressed to appreciable degrees (20-60%) in frequently found tumors such as lung, breast, and gastrointestinal tumors.

The composition can be provided as a single polypeptide that incorporates the multiple epitopes from the various TAAs, or can be administered as a composition comprising one or more discrete epitopes. Alternatively, the vaccine can be administered as a minigene construct or as dendritic cells which have been loaded with the peptide epitopes *in vitro.*

Targeting multiple tumor antigens is also important to provide coverage of a large fraction of tumors of any particular type. A single TAA is rarely expressed in the majority of tumors of a given type. For example, approximately 50% of breast tumors express CEA, 20% express MAGE3, and 30% express HER-2/neu. Thus, the use of a single antigen for immunotherapy would offer only limited patient coverage. The combination of the three TAAs, however, would address approximately 70% of breast tumors. A vaccine composition comprising epitopes from multiple tumor antigens also reduces the potential for escape mutants due to loss of expression of an individual tumor antigen.

Example 16. Use of peptides to evaluate an immune response

Peptides of the invention may be used to analyze an immune response for the presence of specific CTL or HTL populations directed to a TAA. Such an analysis may be performed using multimeric complexes as described, *e. g.,* by Ogg *et al., Science* 279:2103-2106, 1998 and Greten *et al.*, *Proc. Natl. Acad. Sci. USA* 95:7568-7573, 1998. In the following example, peptides in accordance with the invention are used as a reagent for diagnostic or prognostic purposes, not as an immunogen.

In this example, highly sensitive human leukocyte antigen tetrameric complexes ("tetramers") are used for a cross-sectional analysis of, for example, tumor-associated antigen HLA-A*0201-specific CTL frequencies from HLA A*0201-positive individuals at different stages of disease or following immunization using a TAA peptide containing an A*0201 motif. Tetrameric complexes are synthesized as described (Musey *et al., N. Engl. J. Med.* 337:1267, 1997). Briefly, purified HLA heavy chain (A*0201 in this example) and β2-microglobulin are synthesized by means of a prokaryotic expression system. The heavy chain is modified by deletion of the transmembrane-cytosolic tail and COOH-terminal addition of a sequence containing a BirA enzymatic biotinylation site. The heavy chain, β2-microglobulin, and peptide are refolded by dilution. The 45-kD refolded product is isolated by fast protein liquid chromatography and then biotinylated by BirA in the presence of biotin (Sigma, St. Louis, Missouri), adenosine 5'triphosphate and magnesium. Streptavidin-phycoerythrin conjugate is added in a 1:4 molar ratio, and the tetrameric product is concentrated to 1 mg/ml. The resulting product is referred to as tetramer-phycoerythrin.

For the analysis of patient blood samples, approximately one million PBMCs are centrifuged at 300g for 5 minutes and resuspended in 50 μl of cold phosphate-buffered saline. Tri-color analysis is performed with the tetramer-phycoerythrin, along with anti-CD8-Tricolor, and anti-CD38. The PBMCs are incubated with tetramer and antibodies on ice for 30 to 60 min and then washed twice before formaldehyde fixation. Gates are applied to contain >99.98% of control samples. Controls for the tetramers include both A*0201-negative individuals and A*0201-positive uninfected donors. The percentage of cells stained with the tetramer is then determined by flow cytometry. The results indicate the number of cells in the PBMC sample that contain epitope-restricted CTLs, thereby readily indicating the extent of immune response to the TAA epitope, and thus the stage of tumor progression or exposure to a vaccine that elicits a protective or therapeutic response.

## Example 17. Use of Peptide Epitopes to Evaluate Recall Responses

The peptide epitopes of the invention are used as reagents to evaluate T cell responses, such as acute or recall responses, in patients. Such an analysis may be performed on patients who are in remission, have a tumor, or who have been vaccinated with a TAA vaccine.

For example, the class I restricted CTL response of persons who have been vaccinated may be analyzed. The vaccine may be any TAA vaccine. PBMC are collected from vaccinated individuals and HLA typed. Appropriate peptide epitopes of the invention that, optimally, bear supermotifs to provide cross-reactivity with multiple HLA supertype family members, are then used for analysis of samples derived from individuals who bear that HLA type.

PBMC from vaccinated individuals are separated on Ficoll-Histopaque density gradients (Sigma Chemical Co., St. Louis, MO), washed three times in HBSS (GIBCO Laboratories), resuspended in RPMI-1640 (GIBCO Laboratories) supplemented with L-glutamine (2mM), penicillin (50U/ml), streptomycin (50 μg/ml), and Hepes (10mM) containing 10% heat-inactivated human AB serum (complete RPMI) and plated using microculture formats. A synthetic peptide comprising an epitope of the invention is added at 10 μg/ml to each well and HBV core 128-140 epitope is added at 1 μg/ml to each well as a source ofT cell help during the first week of stimulation.

In the microculture format, $4 \times 10^5$ PBMC are stimulated with peptide in 8 replicate cultures in 96-well round bottom plate in 100 μl/well of complete RPMI. On days 3 and 10, 100 μl of complete RPMI and 20 U/ml final concentration of rIL-2 are added to each well. On day 7 the cultures are transferred into a 96-well flat-bottom plate and restimulated with peptide, rIL-2 and $10^5$ irradiated (3,000 rad) autologous feeder cells. The cultures are tested for cytotoxic activity on day 14. A positive CTL response requires two or more of the eight replicate cultures to display greater than 10% specific $^{51}$Cr release, based on comparison with uninfected control subjects as previously described (Rehermann, *et al., Nature Med.* 2:1104,1108, 1996; Rehermann *et al., J. Clin. Invest.* 97:1655-1665, 1996; and Rehermann *et al. J. Clin. Invest.* 98:1432-1440, 1996).

Target cell lines are autologous and allogeneic EBV-transformed B-LCL that are either purchased from the American Society for Histocompatibility and Immunogenetics (ASHI, Boston, MA) or established from the pool of patients as described (Guilhot, *et al. J. Virol.* 66:2670-2678, 1992).

Cytotoxicity assays are performed in the following manner. Target cells consist of either allogeneic HLA-matched or autologous EBV-transformed B lymphoblastoid cell line that are incubated overnight with the synthetic peptide epitope of the invention at 10 μM, and labeled with 100 μCi of $^{51}$Cr (Amersham Corp., Arlington Heights, IL) for 1 hour after which the y are washed four times with HBSS.

Cytolytic activity is determined in a standard 4 hour, split-well $^{51}$Cr release assay using U-bottomed 96 well plates containing 3,000 targets/well. Stimulated PBMC are tested at effector/target (E/T) ratios of 20-50:1 on day 14. Percent cytotoxicity is determined from the formula: 100 x [(experimental release-spontaneous release)/maximum release-spontaneous release)]. Maximum release is determined by lysis of targets by detergent (2% Triton X-100; Sigma Chemical Co., St. Louis, MO). Spontaneous release is <25% of maximum release for all experiments.

The results of such an analysis indicate the extent to which HLA-restricted CTL populations have been stimulated by previous exposure to the TAA or TAA vaccine.

The class II restricted HTL responses may also be analyzed. Purified PBMC are cultured in a 96-well flat bottom plate at a density of $1.5 \times 10^5$ cells/well and are stimulated with 10 μg/ml synthetic peptide, whole antigen, or PHA. Cells are routinely plated in replicates of 4-6 wells for each condition. After seven days of culture, the medium is removed and replaced with fresh medium containing 10U/ml IL-2. Two days later, 1 μCi $^3$H-thymidine is added to each well and incubation is continued for an additional 18 hours. Cellular DNA is then harvested on glass fiber mats and analyzed for $^3$H-thymidine incorporation. Antigen-specific T cell proliferation is calculated as the ratio of $^3$H-thymidine incorporation in the presence of antigen divided by the $^3$H-thymidine incorporation in the absence of antigen.

## Example 18. Induction Of Specific CTL Response In Humans

A human clinical trial for an immunogenic composition comprising CTL and HTL epitopes of the invention is set up as an IND Phase I, dose escalation study. Such a trial is designed, for example, as follows:

A total of about 27 subjects are enrolled and divided into 3 groups:

Group I: 3 subjects are injected with placebo and 6 subjects are injected with 5 µg of peptide composition;
Group II: 3 subjects are injected with placebo and 6 subjects are injected with 50 µg peptide composition;
Group III: 3 subjects are injected with placebo and 6 subjects are injected with 500 µg of peptide composition.

After 4 weeks following the first injection, all subjects receive a booster inoculation at the same dosage. Additional booster inoculations can be administered on the same schedule.

The endpoints measured in this study relate to the safety and tolerability of the peptide composition as well as its immunogenicity. Cellular immune responses to the peptide composition are an index of the intrinsic activity of the peptide composition, and can therefore be viewed as a measure of biological efficacy. The following summarize the clinical and laboratory data that relate to safety and efficacy endpoints.

Safety: The incidence of adverse events is monitored in the placebo and drug treatment group and assessed in terms of degree and reversibility.

Evaluation of Vaccine Efficacy: For evaluation of vaccine efficacy, subjects are bled before and after injection. Peripheral blood mononuclear cells are isolated from fresh heparinized blood by Ficoll-Hypaque density gradient centrifugation, aliquoted in freezing media and stored frozen. Samples are assayed for CTL and HTL activity.

The vaccine is found to be both safe and efficacious.

Example 19. Therapeutic Use in Cancer Patients

Evaluation of vaccine compositions are performed to validate the efficacy of the CTL-HTL peptide compositions in cancer patients. The main objectives of the trials are to determine an effective dose and regimen for inducing CTLs in cancer patients, to establish the safety of inducing a CTL and HTL response in these patients, and to see to what extent activation of CTLs improves the clinical picture of cancer patients, as manifested by a reduction in tumor cell numbers. Such a study is designed, for example, as follows:

The studies are performed in multiple centers. The trial design is an open-label, uncontrolled, dose escalation protocol wherein the peptide composition is administered as a single dose followed six weeks later by a single booster shot of the same dose. The dosages are 50, 500 and 5,000 micrograms per injection. Drug-associated adverse effects (severity and reversibility) are recorded.

There are three patient groupings. The first group is injected with 50 micrograms of the peptide composition and the second and third groups with 500 and 5,000 micrograms of peptide composition, respectively. The patients within each group range in age from 21-65, include both males and females (unless the tumor is sex-specific, *e.g.*, breast or prostate cancer), and represent diverse ethnic backgrounds.

Example 20. Induction of CTL Responses Using a Prime Boost Protocol

A prime boost protocol similar in its underlying principle to that used to evaluate the efficacy of a DNA vaccine in transgenic mice, which was described in Example 12, can also be used for the administration of the vaccine to humans. Such a vaccine regimen may include an initial administration of, for example, naked DNA followed by a boost using recombinant virus encoding the vaccine, or recombinant protein/polypeptide or a peptide mixture administered in an adjuvant.

For example, the initial immunization can be performed using an expression vector, such as that constructed in Example 11, in the form of naked nucleic acid administered IM (or SC or ID) in the amounts of 0.5-5 mg at multiple sites. The nucleic acid (0.1 to 1000 µg) can also be administered using a gene gun. Following an incubation period of 3-4 weeks, a booster dose is then administered. The booster can be recombinant fowlpox virus administered at a dose of $5-10^7$ to $5 \times 10^9$ pfu. An alternative recombinant virus, such as an MVA, canarypox, adenovirus, or adeno-associated virus, can also be used for the booster, or the polyepitopic protein or a mixture of the peptides can be administered. For evaluation of vaccine efficacy, patient blood samples will be obtained before immunization as well as at intervals following administration of the initial vaccine and booster doses of the vaccine. Peripheral blood mononuclear cells are isolated from fresh heparinized blood by Ficoll-Hypaque density gradient centrifugation, aliquoted in freezing media and stored frozen. Samples are assayed for CTL and HTL activity.

Analysis of the results will indicate that a magnitude of response sufficient to achieve protective immunity against cancer is generated.

Example 21 Administration of Vaccine Compositions Using Dendritic Cells

Vaccines comprising peptide epitopes of the invention can be administered using antigen-presenting cells, or "professional" APCs such as dendritic cells. In this example, the peptide-pulsed DC are administered to a patient to stimulate a CTL response *in vivo.* In this method, dendritic cells are isolated, expanded, and pulsed with a vaccine comprising peptide CTL and HTL epitopes of the invention. The dendritic cells are infused back into the patient to elicit CTL and HTL *responses in vivo.* The induced lymphocytes then destroy or facilitate destruction of the specific target tumor cells that bear the proteins from which the epitopes in the vaccine are derived.

For example, a cocktail of epitope-bearing peptides is administered *ex vivo* to PBMC, or isolated DC therefrom, from the patient's blood. A pharmaceutical to facilitate harvesting of DC can be used, such as Progenipoietin™ (Monsanto, St. Louis, MO) or GM-CSF/IL-4. After pulsing the DC with peptides and prior to reinfusion into patients, the DC are washed to remove unbound peptides.

As appreciated clinically, and readily determined by one of skill based on clinical outcomes, the number of dendritic cells reinfused into the patient can vary *(see, e.g., Nature Med.* 4:328, 1998; *Nature Med.* 2:52, 1996 and *Prostate* 32:272, 1997). Although 2-50 x $10^6$ dendritic cells per patient are typically administered, larger number of dendritic cells, such as $10^7$ or $10^8$ can also be provided. Such cell populations typically contain between 50-90% dendritic cells.

In some embodiments, peptide-loaded PBMC are injected into patients without purification of the DC. For example, PBMC containing DC generated after treatment with an agent such as Progenipoietin™ are injected into patients without purification of the DC. The total number of PBMC that are administered often ranges from $10^8$ to $10^{10}$. Generally, the cell doses injected into patients is based on the percentage of DC in the blood of each patient, as determined, for example, by immunofluorescence analysis with specific anti-DC antibodies. Thus, for example, if Progenipoietin™ mobilizes 2% DC in the peripheral blood of a given patient, and that patient is to receive $5 \times 10^6$ DC, then the patient will be injected with a total of 2.5 x $10^8$ peptide-loaded PBMC. The percent DC mobilized by an agent such as Progenipoietin™ is typically estimated to be between 2-10%, but can vary as appreciated by one of skill in the art.

*Ex vivo* activation of CTL/HTL responses

Alternatively, *ex vivo* CTL or HTL responses to a particular tumor-associated antigen can be induced by incubating in tissue culture the patient's, or genetically compatible, CTL or HTL precursor cells together with a source of antigen-presenting cells (APC), such as dendritic cells, and the appropriate immunogenic peptides. After an appropriate incubation time (typically about 7-28 days), in which the precursor cells are activated and expanded into effector cells, the cells are infused back into the patient, where they will destroy (CTL) or facilitate destruction (HTL) of their specific target cells, *i.e.*, tumor cells.

Example 22 Alternative Method of Identifying Motif-Bearing Peptides

Another way of identifying motif-bearing peptides is to elute them from cells bearing defined MHC molecules. For example, EBV transformed B cell lines used for tissue typing, have been extensively characterized to determine which HLA molecules they express. In certain cases these cells express only a single type of HLA molecule These cells can then be infected with a pathogenic organism or transfected with nucleic acids that express the tumor antigen of interest. Thereafter, peptides produced by endogenous antigen processing of peptides produced consequent to infection (or as a result of transfection) will bind to HLA molecules within the cell and be transported and displayed on the cell surface.

The peptides are then eluted from the HLA molecules by exposure to mild acid conditions and their amino acid sequence determined, *e.g.,* by mass spectral analysis *(e.g.,* Kubo *et al., J. Immunol.* 152:3913, 1994). Because, as disclosed herein, the majority of peptides that bind a particular HLA molecule are motif-bearing, this is an alternative modality for obtaining the motif-bearing peptides correlated with the particular HLA molecule expressed on the cell.

Alternatively, cell lines that do not express any endogenous HLA molecules can be transfected with an expression construct encoding a single HLA allele. These cells may then be used as described, *i.e.,* they may be infected with a pathogenic organism or transfected with nucleic acid encoding an antigen of interest to isolate peptides corresponding to the pathogen or antigen of interest that have been presented on the cell surface. Peptides obtained from such an analysis will bear motif(s) that correspond to binding to the single HLA allele that is expressed in the cell.

As appreciated by one in the art, one can perform a similar analysis on a cell bearing more than one HLA allele and subsequently determine peptides specific for each HLA allele expressed. Moreover, one of skill would also recognize that means other than infection or transfection, such as loading with a protein antigen, can be used to provide a source of antigen to the cell.

The above examples are provided to illustrate the invention but not to limit its scope. For example, the human terminology for the Major Histocompatibility Complex, namely HLA, is used throughout this document. It is to be appreciated that these principles can be extended to other species as well. Thus, other variants of the invention will be readily apparent to one of ordinary skill in the art and are encompassed by the appended claims. All publications, patents, and patent application cited herein are hereby incorporated by reference for all purposes.

The following numbered paragraphs (paras.) contain statements of broad combinations of the inventive technical features herein disclosed:-

1. An isolated prepared HER2/neu epiotpe consisting of a sequence selected from the group consisting of the sequences set out in Table XXIII, XXIV, XXV, XXVI, XXVII, and XXXI.

2. A composition of para. 1, wherein the epitope is admized or joined to a CTL epitope.

3. A composition of para. 2, wherein the CTL epitope is selected from the group set out in para. 1.

4. A composition of para. 1, wherein the epitope is admixed or joined to an HTL epitope.

5. A composition of para. 4, wherein the HTL epitope is selected from the group set out in para. 1.

6. A composition of para. 4, wherein the HTL epitope is a pan-DR binding molecule.

7. A composition of para. 1, comprising at least three epitopes selected from the group set out in para 1.

8. A composition of para. 1, further comprising a liposome, wherein the epitope is on or within the liposome.

9. A composition of para. 1, wherein the epitope is joined to a lipid.

10. A composition of para. 1, wherein the epitope is joined to a linker.

11. A composition of para. 1, wherein the epitope is bound to an HLA heavy chain, β2-microglobulin, and strepavidin complex, whereby a tetramer is formed.

12. A composition of para. 1, further comprising an antigen presenting cell, wherein the epitope is on or within the antigen presenting cell.

13. A composition of para. 12, wherein the epitope is bound to an HLA molecule on the antigen presenting cell, whereby when a cytotoxic lymphocyte (CTL) or helper T lymphocyte (HTL) is present that is restricted to the HLA molecule, a receptor on the CTL or HTL binds to a complex of the HLA molecule and the epitope.

14. A clonal cytotoxic T lymphocyte (CTL), wherein the CTL is cultured *in vitro* and binds to a complex of an epitope selected from the group set out in Tables XXIII, XXIV, XXV, XXVI, and XXVII, bound to an HLA molecule.

15. A peptide comprising at least a first and a second epitope, wherein the first epitope is selected from the group consisting of the sequences set out in Table XXIII, XXIV, XXV, XXVI, XXVII and XXXI;

16. A composition of para. 15, wherein the first and second epitope are selected from the group of para. 14.

17. A composition of para. 16, further comprising a third epitope selected from the group of para. 15.

18. A composition of para. 15, wherein the peptide is a heteropolymer.

19. A composition of para. 15, wherein the peptide is a homopolymer.

20. A composition of para. 15, wherein the second epitope is a CTL epitope.

21. A composition of para. 20, whereing the CTL epitope is from a tumor associated antigen that is not HER2/neu.

22. A composition of para. 15, wherein the second epitope is a PanDR binding molecule.

23. A composition of para. 1, wherein the first epitope is linked to an a linker sequence.

24. A vaccine composition comprising:

a unit dose of a peptide that comprises less than 50 contiguous amino acids that have 100% identity with a native peptide sequence of HER2/neu, the peptide comprising at least a first epitope selected from the group consisting of the sequences set out in Tables XXIII, XXIV, XXV, XXVI, XXVII, and XXXI, and; a pharmaceutical excipient.

25. A vaccine composition in accordance with para. 24, further comprising a second epitope.

26. A vaccine composition of para. 24, wherein the second epitope is a PanDR binding molecule.

27. A vaccine composition of para. 24, wherein the pharmaceutical excipient comprises an adjuvant.

28. An isolated nucleic acid encoding a peptide comprising an epitope consisting of a sequence selected from the group consisting of the sequence set out in Tables XXIII, XXIV, XXV, XXVI, XXVII, and XXXI.

29. An isolated nucleic acid encoding a peptide comprising at least a first and a second epitope, wherein the first epitope is selected from the group consisting of the sequences set out in Table XXIII, XXIV, XXV, XXVI, XXVII, and XXXI; and wherein the peptide comprises less than 50 contiguous amino acids that have 100% identity with a native peptide sequence.

30. An isolated nucleic acid of para. 29, wherein the peptide comprises at least two epitopes selected from the sequences set out in para. 29.

31. An isolated nucleic acid of para. 30, wherein the peptide comprises at least three epitopes selected from the sequences set out in para. 29.

32. An isolated nucleic acid of para. 29, wherein the second peptide is a CTL epitope.

33. An isolated nucleic acid of para. 32, wherein the CTL is from a tumor-assocaited antigen that is not HER2/neu.

34. An isolated nucleic acid of para. 20, wherein the second peptide is an HTL epitope.

TABLE I

| SUPERMOTIFS | POSITION | POSITION | POSITION |
|---|---|---|---|
| | 2 (Primary Anchor) | 3 (Primary Anchor) | C Terminus (Primary Anchor) |
| A1 | T, I, *L, V, M, S* | | F, W, Y |
| A2 | L, I, V, M, *A, T, Q* | | I, V, *M, A, T, L* |
| A3 | V, S, M, A, *T, L, I* | | **R,K** |
| A24 | Y, F, *W, 1, V, L, M, T* | | **F, I**, *Y, W, L, M* |
| B7 | **P** | | **V, I, L, F**, *M, W, Y, A* |
| B27 | **R, H, K** | | **F, Y, L**, *W, M, I, V, A* |
| B44 | **E,** *D* | | **F**, W, **L, I, M, V, A** |
| B58 | **A, T,** S | | **F, W, Y**, *L, I, V, M, A* |
| B62 | **Q, L**, *I, V, M, P* | | **F, W, Y**, *M, I, V, L, A* |
| | | | |
| MOTIFS | | | |
| A1 | **T, S, M** | | **Y** |
| A1 | | **D, E,** *A, S* | **Y** |

TABLE I   (continued)

| SUPERMOTIFS | POSITION | POSITION | POSITION |
|---|---|---|---|
| | 2 (Primary Anchor) | 3 (Primary Anchor) | C Terminus (Primary Anchor) |
| A2.1 | **L, M,** *V, Q, I, A, T* | | **V,** *L, I, M, A, T* |
| A3 | **L, M, V, I, S, A, T, F,** *C, G, D* | | **K, Y, R,** *H, F, A* |
| A11 | **V, T, M, L, I, S, A, G, N,** *C, D, F* | | **K,** *R, Y, H* |
| A24 | **Y, F, W,** *M* | | **F, L, I, W** |
| A*3101 | **M, V, T,** *A ,L, I, S* | | **R,** *K* |
| A*3301 | **M, V, A, L, F**, *I, S, T* | | **R, K** |
| A*6801 | **A, V, T,** *M, S, L, I* | | **R, K** |
| B*0702 | **P** | | **L, M, F,** *W, Y, A, I, V* |
| B*3501 | **P** | | **L, M, F, W, Y,** *I, V, A* |
| B51 | **P** | | **L, I, V, F,** ***W, Y, A, M*** |
| B*5301 | **P** | | **I, M, F, W, Y,** *A, L, V* |
| B*5401 | **P** | | **A, T, I, V,** *L, M, F, W, Y* |

Bolded residues are preferred, italicized residues are less preferred: A peptide is considered motif-bearing if it has primary anchors at each primary anchor position for a motif or supermotif as specified in the above table.

TABLE Ia

| SUPERMOTIFS | POSITION | POSITION | POSITION |
|---|---|---|---|
| | 2 (Primary Anchor) | 3 (Primary Anchor) | C Terminus (Primary Anchor) |
| A1 | **T, I,** *L, V, M, S* | | **F, W, Y** |
| A2 | **V,** *Q, A, T* | | **I, V,** *L, M, A, T* |
| A3 | **V, S, M, A**, *T, L, I* | | **R, K** |
| A24 | **Y, F,** *W, I, V, L, M, T* | | **F,I,** *Y, W, L, M* |
| B7 | **P** | | **V, I, L, F**, *M, W, Y, A* |
| B27 | **R, H, K** | | **F, Y, L,** *W, M, I, V, A* |
| B58 | **A, T, S** | | **F, W, Y,** *L, I, V, M, A* |
| B62 | **Q,L,**,*I,V,M,P* | | **F,W,Y,** *M.I, V, L, A* |
| | | | |
| MOTIFS | | | |
| A1 | **T,S,M** | | **Y** |
| A1 | | **D,E,**,*A, S* | **Y** |
| A2.1 | *V, Q, A, T** | | **V,** *L, I, M, A, T* |
| A3.2 | **L, M, V, I, S, A, T, F,** *C, G,* D | | **K, Y, R,** *H, F, A* |
| A11 | **V, T, M, L, I, S, A, G, N,** *C, D, F* | | **K,** *R, H, Y* |
| A24 | **Y ,F, W** | | **F,L,I,W** |

*If 2 is V, or Q, the C-term is not L

Bolded residues are preferred, italicized residues are less preferred: A peptide is considered motif-bearing if it has primary anchors at each primary anchor position for a motif or supermotif as specified in the above table.

EP 1 568 373 A2

TABLE II

POSITION

| | | 1 | 2 | 3 | 4 | 5 | 6 | 7 | 8 | C-terminus |
|---|---|---|---|---|---|---|---|---|---|---|

SUPERMOTIFS

| | | 1 | 2 | 3 | 4 | 5 | 6 | 7 | 8 | C-terminus |
|---|---|---|---|---|---|---|---|---|---|---|
| A1 | | | 1° Anchor T,I,L,V,M,S | | | | | | | | 1° Anchor F,W,Y |
| A2 | | | 1° Anchor L,I,V,M,A, T,Q | | | | | | | | 1° Anchor L,I,V,M,A,T |
| A3 | preferred | | 1° Anchor V,S,M,A,T, L,I | Y,F,W, (4/5) | | | | Y,F,W, (3/5) | Y,F,W, (4/5) | P, (4/5) | 1°Anchor R,K |
| | deleterious | D,E (3/5); P, (5/5) | | D,E, (4/5) | | | | | | | |
| A24 | | | 1° Anchor Y,F,W,I,V, L,M,T | | | | | | | | 1° Anchor F,I,Y,W,L,M |
| B7 | preferred | F,W,Y (5/5) L,I,V,M, (3/5) | 1°Anchor P | F,W,Y (4/5) | | | | | | F,W,Y, (3/5) | 1°Anchor V,I,L,F,M,W,Y,A |
| | deleterious | D E (2/5); P(5/5); G(4/5); A(3/5); Q,N, (3/5) | | | | | D,E, (3/5) | G, (4/5) | Q,N, (4/5) | D,E, (4/5) | |
| B27 | | | 1° Anchor R,H,K | | | | | | | | 1° Anchor F,Y,L,W,M,V,A |
| B44 | | | 1° Anchor E,D | | | | | | | | 1° Anchor F,W,Y,L,I,M,V,A |
| B58 | | | 1° Anchor A,T,S | | | | | | | | 1° Anchor F,W,Y,L,I,V,M,A |
| B62 | | | 1° Anchor Q,L,I,V,M, P | | | | | | | | 1° Anchor F,W,Y,M,I,V,L,A |

| | | POSITION | | | | | | | | |
|---|---|---|---|---|---|---|---|---|---|---|
| | | 1 | 2 | 3 | 4 | 5 | 6 | 7 | 8 | C-terminus |
| **MOTIFS** | | | | | | | | | | |
| A1 9-mer | preferred | G,F,Y,W, | 1°Anchor S,T,M, | D,E,A, | Y,F,W, | | P, | D,E,Q,N, | Y,F,W, | 1°Anchor Y |
| | deleterious | D,E, | | R,H,K,L,I,V M,P, | A, | G, | A, | | | |
| A1 9-mer | preferred | G,R,H,K | A,S,T,C,L,I V,M, | 1°Anchor D,E,A,S | G,S,T,C, | | A,S,T,C, | L,I,V,M, | D,E, | 1°Anchor Y |
| | deleterious | A | R,H,K,D,E, P,Y,F,W, | | D,E, | P,Q,N, | R,H,K, | P,G, | G,P, | |

POSITION

| | | 1 | 2 | 3 | 4 | 5 | 6 | 7 | 8 | 9 or C-terminus | C-terminus |
|---|---|---|---|---|---|---|---|---|---|---|---|
| A1 10-mer | preferred | Y,F,W, | 1°Anchor S,T,M | D,E,A,Q,N, | A, | Y,F,W,Q,N, | | P,A,S,T,C, | G,D,E, | P, | 1°Anchor Y |
| | deleterious | G,P, | | R,H,K,G,L,I V,M, | D,E, | R,H,K, | Q,N,A | R,H,K,Y,F, W, | R,H,K, | A | |
| A1 10-mer | preferred | Y,F,W, | S,T,C,L,I,V M, | 1°Anchor D,E,A,S | A, | Y,F,W, | | P,G, | G, | Y,F,W, | 1°Anchor Y |
| | deleterious | R,H,K, | R,H,K,D,E, P,Y,F,W, | | | P, | G, | | P,R,H,K, | Q,N, | |
| A2.1 9-mer | preferred | Y,F,W, | 1°Anchor L,M,I,V,Q, A,T | Y,F,W, | S,T,C, | Y,F,W, | | A, | P | 1°Anchor V,L,I,M,A,T | |
| | deleterious | D,E,P, | | D,E,R,K,H | | | R,K,H | D,E,R,K,H | | | |
| A2.1 10-mer | preferred | A,Y,F,W, | 1°Anchor L,M,I,V,Q, A,T | L,V,I,M, | G, | | G, | | F,Y,W,L, V,I,M, | 1°Anchor V,L,I,M,A,T | |
| | deleterious | D,E,P, | | D,E, | R,K,H,A, | P, | | R,K,H, | D,E,R,K, H, | R,K,H, | |

EP 1 568 373 A2

POSITION

| | | 1 | 2 | 3 | 4 | 5 | 6 | 7 | 8 | 9 or C-terminus | C-terminus |
|---|---|---|---|---|---|---|---|---|---|---|---|
| A3 | preferred | R,H,K, | 1°Anchor L,M,V,I,S, A,T,F,C,G D | Y,F,W, | P,R,H,K,Y, F,W, | A, | Y,F,W, | | P, | 1°Anchor K,Y,R,*H,F,A* | |
| | deleterious | D,E,P, | | D,E | | | | | | | |
| A11 | preferred | A, | 1°Anchor V,T,L,M,I, S,A,G,N,*C, D,F* | Y,F,W, | Y,FW, | A, | Y,F,W, | Y,FW, | P, | 1°Anchor K,,*RY,H* | |
| | deleterious | D,E,P, | | | | | | A | G, | | |
| A24 9-mer | preferred | Y,F,W,R,H,K, | 1°Anchor Y,F,W,*M* | | S,T,C | | | Y,F,W, | Y,F,W, | 1°Anchor F,L,I,W | |
| | deleterious | D,E,G, | | D,E, | G, | Q,N,P, | D,E,R,H,K, | G, | A,Q,N, | | |
| A24 10-mer | preferred | | 1°Anchor Y,F,W,*M* | | P, | Y,F,W,P, | | P, | | | 1°Anchor F,L,I,W |
| | deleterious | | | G,D,E | Q,N | R,H,K | D,E | A | Q,N, | D,E,A, | |
| A3101 | preferred | R,H,K, | 1°Anchor M,V,T,*A,L,* *I,S* | Y,F,W, | P, | | Y,F,W, | Y,F,W, | A,P, | 1°Anchor R,*K* | |
| | deleterious | D,E,P, | | D,E, | | A,D,E, | D,E, | D,E, | D,E, | | |

| | | | POSITION | | | | | | | | | |
|---|---|---|---|---|---|---|---|---|---|---|---|---|
| | | **1** | **2** | **3** | **4** | **5** | **6** | **7** | **8** | **9** or C-terminus | C-terminus |
| A3301 | preferred | | 1°Anchor M,V,A,L,F, I,S,T | Y,F,W | | | | A,Y,F,W | | 1°Anchor R,K | |
| | deleterious | G,P | | D,E | | | | | | | |
| A6801 | preferred | Y,F,W,S,T,C, | 1°Anchor A,V,T,M,S, L,I | | | Y,F,W,L,I, V,M | | Y,F,W, | P, | 1°Anchor R,K | |
| | deleterious | G,P, | | D,E,G, | | R,H,K, | | | A, | | |
| B0702 | preferred | R,H,K,F,W,Y, | 1°Anchor P, | R,H,K, | | R,H,K, | R,H,K, | R,H,K, | P,A, | 1°Anchor L,M,F,*W,Y,A,* *I,V* | |
| | deleterious | D,E,Q,N,P, | | D,E,P, | D,E, | D,E, | G,D,E, | Q,N, | D,E, | | |
| B3501 | preferred | F,W,Y,L,I,V,M; | 1°Anchor P | F,W,Y, | | | | F,W,Y, | | 1°Anchor L,M,F,W,Y,*I,* *V,A* | |
| | deleterious | A,G,P, | | | | G, | G, | | | | |

EP 1 568 373 A2

POSITION

| | | 1 | 2 | 3 | 4 | 5 | 6 | 7 | 8 | 9 or C-terminus | C-terminus |
|---|---|---|---|---|---|---|---|---|---|---|---|
| B51 | preferred | L,I,V,M,F,W,Y, | 1°Anchor P | F,W,Y, | S,T,C, | F,W,Y, | | G, | F,W,Y, | 1°Anchor L,I,V,F,*W*, *Y,A,M* | |
| | deleterious | A,G,P,D,E,R,H,K, S,T,C, | | | | D,E, | G, | D,E,Q,N, | G,D,E, | | |
| B5301 | preferred | L,I,V,M,F,W,Y, | 1°Anchor P | F,W,Y, | S,T,C, | F,W,Y, | | L,I,V,M,F, W,Y, | F,W,Y, | 1°Anchor I,M,F,W,Y, *A,L,V* | |
| | deleterious | A,G,P,Q,N, | | | | | G, | R,H,K,Q,N, | D,E, | | |
| B5401 | preferred | F,W,Y, | 1°Anchor P | F,W,Y,L,I,V M, | | L,I,V,M, | | A,L,I,V,M, | F,W,Y,A,P, | 1°Anchor A,T,I,V,*L, M,F,W,Y* | |
| | deleterious | G,P,Q,N,D,E, | | G,D,E,S,T,C, | | R,H,K,D,E, | D,E, | Q,N,D,G,E, | D,E; | | |

Italicized residues indicate less preferred or "tolerated" residues.
The information in Table II is specific for 9-mers unless otherwise specified.
Secondary anchor specificities are designated for each position independently.

EP 1 568 373 A2

**Table III**

POSITION

| MOTIFS | | 1° anchor 1 | 2 | 3 | 4 | 5 | 1° anchor 6 | 7 | 8 | 9 |
|---|---|---|---|---|---|---|---|---|---|---|
| DR4 | preferred | F, M, Y, *L, I, V, W,* | M, | T, | | I, | V, S, T, *C, P, A, L, I, M,* | M, H, | | M, H |
| | deleterious | | | | W, | | | R, | | W, D, E |
| DR1 | preferred | M, F, *L, I, V, W, Y,* | | | P, A, M, Q, | | V, M, A, T, *S, P, L, I, C,* | M, | | A, V, M |
| | deleterious | | C | C, H | F, D | C, W, D | | G, D, E, | D | |
| DR7 | preferred | M, F, *L, I, V, W, Y,.* | M, | W, | A, | | I, V, M, S, A, *C, T, P, L,* | M, | | I, V |
| | deleterious | | C, | | G, | | | G, R, D, | N | G |
| DR Supermotif | | M, F, *L, I, V, W, Y,* | | | | | V, M, S, T, A, *C, P, L, I,* | | | |

| DR3 MOTIFS | 1° anchor 1 | 2 | 3 | 1° anchor 4 | 5 | 1° anchor 6 |
|---|---|---|---|---|---|---|
| motif a preferred | L, I, V, M, F, Y, | | | D | | |
| motif b preferred | L, I, V, M, F, A, Y, | | | D, N, Q, E, S, T | | K, R, H |

Italicized residues indicate less preferred or "tolerated" residues. Secondary anchor specificities are designated for each position independently.

Table IV:

| HLA Class I Standard Peptide Binding Affinity. | | | |
|---|---|---|---|
| ALLELE | STANDARD PEPTIDE | SEQUENCE (SEQ ID NO:) | STANDARD BINDING AFFINITY (nM) |
| A*0101 | 944.02 | YLEPAIAKY | 25 |
| A*0201 | 941.01 | FLPSDYFPSV | 5.0 |
| A*0202 | 941.01 | FLPSDYFPSV | 4.3 |
| A*0203 | 941.01 | FLPSDYFPSV | 10 |
| A*0205 | 941.01 | FLPSDYFPSV | 4.3 |
| A*0206 | 941.01 | FLPSDYFPSV | 3.7 |
| A*0207 | 941.01 | FLPSDYFPSV | 23 |
| A*6802 | 1072.34 | YVIKVSARV | 8.0 |
| A*0301 | 941.12 | KVFPYALINK | 11 |
| A* 1101 | 940.06 | AVDLYHFLK | 6.0 |
| A*3101 | 941.12 | KVFPYALINK | 18 |
| A*3301 | 1083.02 | STLPETYVVRR | 29 |
| A*6801 | 941.12 | KVFPYALINK | 8.0 |
| A*2402 | 979.02 | AYIDNYNKF | 12 |
| B*0702 | 1075.23 | APRTLVYLL | 5.5 |
| B*3501 | 1021.05 | FPFKYAAAF | 7.2 |
| B51 | 1021.05 | FPFKYAAAF | 5.5 |
| B*5301 | 1021.05 | FPFKYAAAF | 9.3 |
| B*5401 | 1021.05 | FPFKYAAAF | 10 |

Table V:

| HLA Class II Standard Peptide Binding Affinity. | | | | |
|---|---|---|---|---|
| Allele | Nomenclature | Standard Peptide | Sequence (SEQ ID NO:) | Binding Affinity (nM) |
| DRB1*0101 | DR1 | 515.01 | PKYVKQNTLKLAT | 5.0 |
| DRB1*0301 | DR3 | 829.02 | YKTIAFDEEARR | 300 |
| DRB1*0401 | DR4w4 | 515.01 | PKYVKQNTLKLAT | 45 |
| DRB1*0404 | DR4w14 | 717.01 | YARFQSQTTLKQKT | 50 |
| DRB1*0405 | DR4w15 | 717.01 | YARFQSQTTLKQKT | 38 |
| DRB1*0701 | DR7 | 553.01 | QYIKANSKFIGITE | 25 |
| DRB1*0802 | DR8w2 | 553.01 | QYIKANSKFIGITE | 49 |
| DRB1*0803 | DR8w3 | 553.01 | QYIKANSKFIGITE | 1600 |
| DRB1*0901 | DR9 | 553.01 | QYIKANSKFIGITE | 75 |
| DRB1*1101 | DR5w11 | 553.01 | QYIKANSKFIGITE | 20 |
| DRB1*1201 | DR5w12 | 1200.05 | EALIHQLKINPYVLS | 298 |
| DRB1*1302 | DR6w19 | 650.22 | QYIKANAKFIGITE | 3.5 |
| DRB1*1501 | DR2w2β1 | 507.02 | GRTQDENPVVHFFKNIV TPRTPPP | 9.1 |

Table V:   (continued)

| HLA Class II Standard Peptide Binding Affinity. | | | | |
|---|---|---|---|---|
| Allele | Nomenclature | Standard Peptide | Sequence (SEQ ID NO:) | Binding Affinity (nM) |
| DRB3*0101 | DR52a | 511 | NGQIGNDPNRDIL | 470 |
| DRB4*0101 | DRw53 | 717.01 | YARFQSQTTLKQKT | 58 |
| DRB5*0101 | DR2w2β2 | 553.01 | QYIKANSKFIGITE | 20 |

## Table VI

| HLA-supertype | Allelle-specific HLA-supertype members | |
| --- | --- | --- |
| | Verified[a] | Predicted[b] |
| A1 | A*0101, A*2501, A*2601, A*2602, A*3201 | A*0102, A*2604, A*3601, A*4301, A*8001 |
| A2 | A*0201, A*0202, A*0203, A*0204, A*0205, A*0206, A*0207, A*0209, A*0214, A*6802, A*6901 | A*0208, A*0210, A*0211, A*0212, A*0213 |
| A3 | A*0301, A*1101, A*3101, A*3301, A*6801 | A*0302, A*1102, A*2603, A*3302, A*3303, A*3401, A*3402, A*6601, A*6602, A*7401 |
| A24 | A*2301, A*2402, A*3001 | A*2403, A*2404, A*3002, A*3003 |
| B7 | B*0702, B*0703, B*0704, B*0705, B*1508, B*3501, B*3502, B*3503, B*3503, B*3504, B*3505, B*3506, B*3507, B*3508, B*5101, B*5102, B*5103, B*5104, B*5105, B*5301, B*5401, B*5501, B*5502, B*5601, B*5602, B*6701, B*7801 | B*1511, B*4201, B*5901 |
| B27 | B*1401, B*1402, B*1509, B*2702, B*2703, B*2704, B*2705, B*2706, B*3801, B*3901, B*3902, B*7301 | B*2701, B*2707, B*2708, B*3802, B*3903, B*3904, B*3905, B*4801, B*4802, B*1510, B*1518, B*1503 |
| B44 | B*1801, B*1802, B*3701, B*4402, B*4403, B*4404, B*4001, B*4002, B*4006 | B*4101, B*4501, B*4701, B*4901, B*5001 |
| B58 | B*5701, B*5702, B*5801, B*5802, B*1516, B*1517 | |
| B62 | B*1501, B*1502, B*1513, B*5201 | B*1301, B*1302, B*1504, B*1505, B*1506, B*1507, B*1515, B*1520, B*1521, B*1512, B*1514, B*1510 |

a.  Verified alleles include alleles whose specificity has been determined by pool sequencing analysis, peptide binding assays, or by analysis of the sequences of CTL epitopes.

b.  Predicted alleles are alleles whose specificity is predicted on the basis of B and F pocket structure to overlap with the supertype specificity.

### Table VII
### HER2/NEU A01 Supermotif Peptides with Binding Data

| Position | No. of Amino Acids | A*0101 |
|---|---|---|
| 66 | 8 | |
| 272 | 8 | |
| 732 | 8 | |
| 899 | 8 | |
| 296 | 8 | 0.1000 |
| 916 | 8 | -0.0021 |
| 1241 | 8 | 0.0030 |
| 166 | 8 | |
| 369 | 8 | |
| 76 | 8 | |
| 434 | 8 | |
| 828 | 8 | -0.0021 |
| 945 | 8 | |
| 418 | 8 | |
| 1023 | 8 | |
| 2 | 8 | |
| 607 | 8 | |
| 402 | 8 | -0.0021 |
| 1016 | 8 | |
| 101 | 8 | |
| 479 | 8 | |
| 664 | 8 | |
| 724 | 8 | |
| 911 | 8 | |
| 1024 | 8 | |
| 1180 | 8 | |
| 603 | 8 | |
| 796 | 8 | |
| 952 | 8 | |
| 357 | 8 | |
| 892 | 8 | |
| 962 | 8 | |
| 997 | 8 | |
| 818 | 8 | |
| 906 | 8 | |
| 165 | 9 | |
| 356 | 9 | |
| 478 | 9 | |
| 910 | 9 | |
| 104 | 9 | 0.1800 |
| 401 | 9 | 0.0430 |
| 1131 | 9 | 0.1300 |
| 100 | 9 | |
| 373 | 9 | |
| 1023 | 9 | |
| 444 | 9 | |
| 513 | 9 | |
| 42 | 9 | 9.1000 |
| 546 | 9 | 0.0050 |
| 795 | 9 | 0.0024 |

## Table VII
### HER2/NEU A01 Supermotif Peptides with Binding Data

| Position | No. of Amino Acids | A*0101 |
|---|---|---|
| 869 | 9 | 7.6000 |
| 1119 | 9 | 0.0017 |
| 271 | 9 | |
| 663 | 9 | |
| 1179 | 9 | |
| 295 | 9 | 0.0042 |
| 978 | 9 | |
| 197 | 9 | |
| 281 | 9 | 0.0028 |
| 773 | 9 | 0.0400 |
| 915 | 9 | 0.0011 |
| 417 | 9 | |
| 608 | 9 | |
| 293 | 9 | 0.0550 |
| 727 | 9 | 0.0011 |
| 997 | 9 | 0.0290 |
| 1213 | 9 | 0.0430 |
| 525 | 10 | |
| 406 | 10 | |
| 443 | 10 | |
| 899 | 10 | 2.7000 |
| 1239 | 10 | 0.0630 |
| 467 | 10 | |
| 960 | 10 | |
| 154 | 10 | 0.0300 |
| 64 | 10 | |
| 270 | 10 | |
| 391 | 10 | |
| 607 | 10 | |
| 662 | 10 | |
| 890 | 10 | |
| 160 | 10 | |
| 473 | 10 | |
| 816 | 10 | |
| 265 | 10 | 0.0015 |
| 402 | 10 | 1.1000 |
| 868 | 10 | 1.3000 |
| 914 | 10 | 0.0082 |
| 1130 | 10 | 0.0072 |
| 355 | 10 | |
| 722 | 10 | |
| 909 | 10 | |
| 904 | 10 | |
| 950 | 10 | |
| 55 | 10 | 0.0180 |
| 545 | 10 | 0.0015 |
| 772 | 10 | 1.1000 |
| 826 | 10 | 0.3000 |
| 249 | 10 | |
| 372 | 10 | |

## Table VII
## HER2/NEU A01 Supermotif Peptides with Binding Data

| Position | No. of Amino Acids | A*0101 |
|---|---|---|
| 1077 | 10 | |
| 280 | 10 | 0.1800 |
| 334 | 10 | 0.0016 |
| 601 | 10 | 0.0010 |
| 1213 | 10 | 5.5000 |
| 40 | 11 | 0.2800 |
| 401 | 11 | 0.4400 |
| 1102 | 11 | 0.0160 |
| 405 | 11 | |
| 98 | 11 | |
| 466 | 11 | |
| 661 | 11 | |
| 442 | 11 | |
| 73 | 11 | |
| 153 | 11 | |
| 725 | 11 | |
| 476 | 11 | |
| 54 | 11 | |
| 793 | 11 | |
| 1117 | 11 | |
| 281 | 11 | |
| 959 | 11 | |
| 1013 | 11 | 0.0027 |
| 854 | 11 | |
| 976 | 11 | |
| 195 | 11 | |
| 1213 | 11 | |
| 293 | 11 | 0.1900 |

## Table VIII
### HER2/NEU A02 Supermotif with Binding Data

| Position | No. of Amino Acids | A*0201 | A*0202 | A*0203 | A*0206 | A*6802 |
|---|---|---|---|---|---|---|
| 1094 | 8 | | | | | |
| 1094 | 10 | | | | | |
| 4 | 8 | | | | | |
| 4 | 9 | | | | | |
| 4 | 10 | 0.0010 | | | | |
| 4 | 11 | | | | | |
| 1203 | 10 | | | | | |
| 1159 | 8 | | | | | |
| 1159 | 9 | 0.0001 | | | | |
| 20 | 8 | | | | | |
| 20 | 10 | | | | | |
| 751 | 9 | | | | | |
| 113 | 11 | | | | | |
| 5 | 8 | | | | | |
| 5 | 9 | 0.0310 | | | | |
| 5 | 10 | 0.0360 | 0.0022 | 0.8600 | 0.0019 | 0.0160 |
| 5 | 11 | | | | | |
| 890 | 11 | | | | | |
| 466 | 9 | 0.0210 | | | | |
| 14 | 8 | | | | | |
| 14 | 10 | 0.0001 | | | | |
| 270 | 9 | 0.0001 | | | | |
| 705 | 8 | | | | | |
| 705 | 10 | 0.0007 | | | | |
| 705 | 11 | | | | | |
| 710 | 9 | | | | | |
| 710 | 11 | | | | | |
| 1165 | 8 | | | | | |
| 1165 | 9 | | | | | |
| 1190 | 8 | | | | | |
| 1190 | 9 | | | | | |
| 115 | 9 | 0.0004 | | | | |
| 355 | 11 | | | | | |
| 657 | 8 | | | | | |
| 657 | 9 | 0.0007 | | | | |
| 657 | 10 | 0.0002 | | | | |
| 657 | 11 | | | | | |
| 587 | 11 | | | | | |
| 224 | 8 | | | | | |
| 224 | 10 | | | | | |
| 338 | 8 | | | | | |
| 338 | 10 | 0.0011 | | | | |
| 255 | 9 | | | | | |
| 789 | 8 | | | | | |
| 789 | 9 | 0.0340 | | | | |
| 789 | 10 | | | | | |
| 826 | 8 | | | | | |
| 826 | 11 | | | | | |
| 623 | 9 | | | | | |
| 567 | 8 | | | | | |

EP 1 568 373 A2

**Table VIII**

**HER2/NEU A02 Supermotif with Binding Data**

| Position | No. of Amino Acids | A*0201 | A*0202 | A*0203 | A*0206 | A*6802 |
|---|---|---|---|---|---|---|
| 567 | 9 | | | | | |
| 212 | 8 | | | | | |
| 212 | 10 | | | | | |
| 53 | 8 | | | | | |
| 53 | 10 | | | | | |
| 53 | 11 | | | | | |
| 244 | 10 | | | | | |
| 244 | 11 | | | | | |
| 26 | 8 | | | | | |
| 26 | 10 | | | | | |
| 630 | 8 | | | | | |
| 947 | 8 | | | | | |
| 947 | 9 | | | | | |
| 947 | 10 | | | | | |
| 596 | 9 | 0.0004 | | | | |
| 634 | 11 | | | | | |
| 540 | 8 | | | | | |
| 540 | 11 | | | | | |
| 504 | 11 | | | | | |
| 528 | 8 | | | | | |
| 295 | 10 | 0.0001 | | | | |
| 871 | 9 | | | | | |
| 171 | 9 | 0.0002 | | | | |
| 171 | 10 | | | | | |
| 171 | 11 | | | | | |
| 76 | 9 | 0.0001 | | | | |
| 76 | 10 | 0.0001 | | | | |
| 76 | 11 | | | | | |
| 845 | 8 | | | | | |
| 845 | 9 | 0.0002 | | | | |
| 636 | 9 | | | | | |
| 1089 | 10 | 0.0001 | | | | |
| 993 | 8 | | | | | |
| 993 | 11 | | | | | |
| 933 | 9 | 0.0002 | | | | |
| 821 | 9 | 0.0002 | | | | |
| 821 | 10 | | | | | |
| 421 | 8 | | | | | |
| 421 | 10 | 0.0003 | | | | |
| 421 | 11 | | | | | |
| 1016 | 9 | 0.0002 | | | | |
| 1016 | 10 | 0.0002 | | | | |
| 1013 | 8 | | | | | |
| 30 | 8 | | | | | |
| 1224 | 9 | | | | | |
| 483 | 10 | | | | | |
| 483 | 11 | | | | | |
| 165 | 8 | | | | | |
| 1183 | 8 | | | | | |
| 1183 | 9 | 0.0002 | | | | |

## Table VIII
## HER2/NEU A02 Supermotif with Binding Data

| Position | No. of Amino Acids | A*0201 | A*0202 | A*0203 | A*0206 | A*6802 |
|---|---|---|---|---|---|---|
| 1084 | 9 | | | | | |
| 1084 | 11 | | | | | |
| 307 | 8 | | | | | |
| 307 | 11 | | | | | |
| 838 | 9 | 0.0002 | | | | |
| 838 | 10 | | | | | |
| 838 | 11 | | | | | |
| 904 | 8 | | | | | |
| 904 | 9 | 0.0002 | | | | |
| 950 | 11 | | | | | |
| 580 | 9 | | | | | |
| 1069 | 8 | | | | | |
| 770 | 8 | | | | | |
| 766 | 8 | | | | | |
| 766 | 9 | | | | | |
| 766 | 10 | | | | | |
| 147 | 8 | | | | | |
| 147 | 9 | 0.0001 | | | | |
| 405 | 8 | | | | | |
| 405 | 10 | | | | | |
| 2 | 10 | 0.0001 | | | | |
| 2 | 11 | | | | | |
| 460 | 8 | | | | | |
| 460 | 9 | 0.0004 | | | | |
| 265 | 8 | | | | | |
| 265 | 9 | | | | | |
| 139 | 8 | | | | | |
| 139 | 10 | | | | | |
| 139 | 11 | | | | | |
| 719 | 8 | | | | | |
| 61 | 9 | | | | | |
| 61 | 11 | | | | | |
| 695 | 11 | | | | | |
| 971 | 9 | 0.0001 | | | | |
| 971 | 10 | 0.0001 | | | | |
| 238 | 8 | | | | | |
| 395 | 8 | | | | | |
| 395 | 9 | | | | | |
| 645 | 8 | | | | | |
| 645 | 10 | | | | | |
| 645 | 11 | | | | | |
| 1123 | 9 | | | | | |
| 1123 | 10 | | | | | |
| 717 | 9 | | | | | |
| 717 | 10 | | | | | |
| 693 | 8 | | | | | |
| 693 | 9 | | | | | |
| 874 | 11 | | | | | |
| 40 | 10 | | | | | |
| 401 | 10 | | | | | |

EP 1 568 373 A2

## Table VIII
### HER2/NEU A02 Supermotif with Binding Data

| Position | No. of Amino Acids | A*0201 | A*0202 | A*0203 | A*0206 | A*6802 |
|---|---|---|---|---|---|---|
| 79 | 8 | | | | | |
| 79 | 9 | | | | | |
| 352 | 8 | | | | | |
| 352 | 10 | | | | | |
| 321 | 8 | | | | | |
| 364 | 11 | | | | | |
| 376 | 11 | | | | | |
| 73 | 8 | | | | | |
| 534 | 8 | | | | | |
| 425 | 11 | | | | | |
| 476 | 10 | 0.0001 | | | | |
| 986 | 9 | 0.0002 | | | | |
| 1093 | 9 | 0.0001 | | | | |
| 1093 | 11 | | | | | |
| 1202 | 11 | | | | | |
| 19 | 9 | | | | | |
| 19 | 11 | | | | | |
| 621 | 8 | | | | | |
| 621 | 11 | | | | | |
| 729 | 10 | 0.0001 | | | | |
| 1080 | 11 | | | | | |
| 919 | 8 | | | | | |
| 919 | 10 | | | | | |
| 704 | 9 | 0.0002 | | | | |
| 704 | 11 | | | | | |
| 1231 | 10 | | | | | |
| 131 | 10 | 0.0001 | | | | |
| 1164 | 9 | | | | | |
| 1164 | 10 | 0.0002 | | | | |
| 1189 | 9 | 0.0002 | | | | |
| 1189 | 10 | | | | | |
| 439 | 10 | 0.0030 | | | | |
| 262 | 9 | | | | | |
| 262 | 10 | 0.0005 | | | | |
| 262 | 11 | | | | | |
| 787 | 8 | | | | | |
| 787 | 10 | 0.0004 | | | | |
| 787 | 11 | | | | | |
| 672 | 11 | | | | | |
| 660 | 8 | | | | | |
| 660 | 10 | 0.0007 | | | | |
| 660 | 11 | | | | | |
| 925 | 10 | 0.0001 | | | | |
| 925 | 11 | | | | | |
| 449 | 10 | 0.0003 | | | | |
| 737 | 10 | 0.0002 | | | | |
| 508 | 9 | 0.0120 | 0.0001 | 0.0790 | 0.0001 | 0.0044 |
| 461 | 9 | | | | | |
| 461 | 11 | | | | | |
| 865 | 8 | | | | | |

## Table VIII
### HER2/NEU A02 Supermotif with Binding Data

| Position | No. of Amino Acids | A*0201 | A*0202 | A*0203 | A*0206 | A*6802 |
|---|---|---|---|---|---|---|
| 865 | 11 | | | | | |
| 1062 | 9 | | | | | |
| 447 | 9 | 0.0018 | | | | |
| 344 | 10 | 0.0017 | | | | |
| 10 | 11 | | | | | |
| 549 | 9 | | | | | |
| 136 | 10 | 0.0001 | | | | |
| 136 | 11 | | | | | |
| 454 | 8 | | | | | |
| 346 | 8 | | | | | |
| 346 | 10 | | | | | |
| 346 | 11 | | | | | |
| 1091 | 8 | | | | | |
| 1091 | 11 | | | | | |
| 832 | 8 | | | | | |
| 832 | 10 | 0.0017 | | | | |
| 832 | 11 | | | | | |
| 537 | 10 | | | | | |
| 537 | 11 | | | | | |
| 28 | 8 | | | | | |
| 28 | 10 | | | | | |
| 1239 | 11 | | | | | |
| 104 | 10 | | | | | |
| 104 | 11 | | | | | |
| 732 | 9 | | | | | |
| 776 | 10 | 0.0001 | | | | |
| 776 | 11 | | | | | |
| 603 | 9 | | | | | |
| 603 | 11 | | | | | |
| 152 | 10 | 0.0036 | | | | |
| 909 | 8 | | | | | |
| 909 | 9 | | | | | |
| 668 | 8 | | | | | |
| 1179 | 8 | | | | | |
| 878 | 9 | 0.0002 | | | | |
| 473 | 8 | | | | | |
| 42 | 8 | | | | | |
| 349 | 8 | | | | | |
| 349 | 9 | | | | | |
| 349 | 11 | | | | | |
| 48 | 8 | | | | | |
| 48 | 9 | 0.0340 | | | | |
| 490 | 8 | | | | | |
| 901 | 10 | | | | | |
| 901 | 11 | | | | | |
| 495 | 11 | | | | | |
| 478 | 8 | | | | | |
| 858 | 9 | 0.0002 | | | | |
| 858 | 10 | | | | | |
| 809 | 9 | 0.0002 | | | | |

EP 1 568 373 A2

**Table VIII**
**HER2/NEU A02 Supermotif with Binding Data**

| Position | No. of Amino Acids | A*0201 | A*0202 | A*0203 | A*0206 | A*6802 |
|---|---|---|---|---|---|---|
| 86 | 9 | | | | | |
| 86 | 11 | | | | | |
| 829 | 8 | | | | | |
| 829 | 11 | | | | | |
| 654 | 8 | | | | | |
| 654 | 9 | 0.0005 | | | | |
| 654 | 10 | 0.0120 | | | | |
| 654 | 11 | | | | | |
| 767 | 8 | | | | | |
| 767 | 9 | 0.0210 | 0.0001 | 0.0024 | 0.0012 | 0.0003 |
| 767 | 11 | | | | | |
| 435 | 9 | 0.2100 | | | | |
| 435 | 10 | | | | | |
| 435 | 11 | | | | | |
| 673 | 10 | | | | | |
| 714 | 10 | 0.0001 | | | | |
| 148 | 8 | | | | | |
| 661 | 9 | 0.0020 | | | | |
| 661 | 10 | 0.0006 | | | | |
| 954 | 8 | | | | | |
| 361 | 10 | | | | | |
| 77 | 8 | | | | | |
| 77 | 9 | | | | | |
| 77 | 10 | | | | | |
| 77 | 11 | | | | | |
| 989 | 9 | | | | | |
| 861 | 9 | | | | | |
| 861 | 10 | | | | | |
| 406 | 9 | | | | | |
| 762 | 10 | | | | | |
| 369 | 9 | 0.1500 | | | | |
| 747 | 8 | | | | | |
| 747 | 9 | 0.0002 | | | | |
| 681 | 10 | 0.0001 | | | | |
| 681 | 11 | | | | | |
| 860 | 8 | | | | | |
| 860 | 10 | 0.0020 | | | | |
| 860 | 11 | | | | | |
| 32 | 10 | | | | | |
| 1171 | 10 | | | | | |
| 1171 | 11 | | | | | |
| 722 | 9 | | | | | |
| 724 | 10 | | | | | |
| 724 | 11 | | | | | |
| 753 | 11 | | | | | |
| 883 | 8 | | | | | |
| 883 | 9 | 0.0002 | | | | |
| 3 | 9 | | | | | |
| 3 | 10 | 0.0022 | | | | |
| 3 | 11 | | | | | |

70

EP 1 568 373 A2

## Table VIII
## HER2/NEU A02 Supermotif with Binding Data

| Position | No. of Amino Acids | A*0201 | A*0202 | A*0203 | A*0206 | A*6802 |
|---|---|---|---|---|---|---|
| 846 | 8 | | | | | |
| 509 | 8 | | | | | |
| 253 | 11 | | | | | |
| 465 | 8 | | | | | |
| 465 | 10 | | | | | |
| 13 | 8 | | | | | |
| 13 | 9 | | | | | |
| 13 | 11 | | | | | |
| 179 | 8 | | | | | |
| 1075 | 11 | | | | | |
| 866 | 10 | | | | | |
| 114 | 10 | 0.0002 | | | | |
| 85 | 10 | 0.0001 | | | | |
| 183 | 9 | | | | | |
| 467 | 8 | | | | | |
| 467 | 11 | | | | | |
| 674 | 9 | | | | | |
| 154 | 8 | | | | | |
| 12 | 9 | 0.0008 | | | | |
| 12 | 10 | 0.0006 | | | | |
| 869 | 11 | | | | | |
| 1008 | 10 | 0.0001 | | | | |
| 1008 | 11 | | | | | |
| 934 | 8 | | | | | |
| 785 | 9 | | | | | |
| 785 | 10 | 0.0490 | | | | |
| 11 | 10 | 0.0054 | | | | |
| 11 | 11 | | | | | |
| 822 | 8 | | | | | |
| 822 | 9 | 0.0046 | | | | |
| 15 | 9 | 0.0007 | | | | |
| 15 | 11 | | | | | |
| 690 | 8 | | | | | |
| 690 | 11 | | | | | |
| 662 | 8 | | | | | |
| 662 | 9 | 0.0001 | | | | |
| 800 | 8 | | | | | |
| 800 | 11 | | | | | |
| 74 | 11 | | | | | |
| 691 | 10 | | | | | |
| 691 | 11 | | | | | |
| 445 | 9 | | | | | |
| 445 | 11 | | | | | |
| 547 | 9 | | | | | |
| 547 | 11 | | | | | |
| 140 | 9 | | | | | |
| 140 | 10 | | | | | |
| 392 | 8 | | | | | |
| 392 | 11 | | | | | |
| 96 | 10 | | | | | |

## Table VIII
### HER2/NEU A02 Supermotif with Binding Data

| Position | No. of Amino Acids | A*0201 | A*0202 | A*0203 | A*0206 | A*6802 |
|---|---|---|---|---|---|---|
| 1109 | 9 | | | | | |
| 1109 | 10 | | | | | |
| 397 | 10 | | | | | |
| 397 | 11 | | | | | |
| 428 | 8 | | | | | |
| 428 | 9 | | | | | |
| 1131 | 10 | | | | | |
| 145 | 9 | | | | | |
| 145 | 10 | | | | | |
| 145 | 11 | | | | | |
| 181 | 11 | | | | | |
| 443 | 8 | | | | | |
| 443 | 11 | | | | | |
| 1197 | 8 | | | | | |
| 700 | 11 | | | | | |
| 215 | 11 | | | | | |
| 651 | 8 | | | | | |
| 651 | 9 | | | | | |
| 651 | 11 | | | | | |
| 790 | 8 | | | | | |
| 790 | 9 | | | | | |
| 790 | 11 | | | | | |
| 62 | 8 | | | | | |
| 62 | 10 | | | | | |
| 313 | 10 | 0.0002 | | | | |
| 313 | 11 | | | | | |
| 1017 | 8 | | | | | |
| 1017 | 9 | 0.0030 | | | | |
| 696 | 10 | | | | | |
| 696 | 11 | | | | | |
| 841 | 8 | | | | | |
| 841 | 11 | | | | | |
| 852 | 8 | | | | | |
| 852 | 10 | | | | | |
| 852 | 11 | | | | | |
| 972 | 8 | 0.0001 | | | | |
| 972 | 9 | 0.0001 | | | | |
| 796 | 11 | | | | | |
| 271 | 8 | | | | | |
| 663 | 8 | | | | | |
| 663 | 11 | | | | | |
| 774 | 9 | 0.0014 | | | | |
| 979 | 9 | 0.0001 | | | | |
| 979 | 10 | | | | | |
| 979 | 11 | | | | | |
| 953 | 8 | | | | | |
| 953 | 9 | 0.0051 | | | | |
| 45 | 11 | | | | | |
| 827 | 10 | | | | | |
| 916 | 11 | | | | | |

EP 1 568 373 A2

**Table VIII**
**HER2/NEU A02 Supermotif with Binding Data**

| Position | No. of Amino Acids | A*0201 | A*0202 | A*0203 | A*0206 | A*6802 |
|---|---|---|---|---|---|---|
| 1042 | 11 | | | | | |
| 68 | 10 | 0.0001 | | | | |
| 360 | 11 | | | | | |
| 59 | 9 | | | | | |
| 59 | 11 | | | | | |
| 427 | 9 | | | | | |
| 427 | 10 | 0.0001 | | | | |
| 708 | 8 | | | | | |
| 708 | 11 | | | | | |
| 319 | 10 | | | | | |
| 177 | 8 | | | | | |
| 177 | 10 | | | | | |
| 89 | 8 | | | | | |
| 89 | 11 | | | | | |
| 388 | 10 | | | | | |
| 275 | 8 | | | | | |
| 471 | 9 | | | | | |
| 471 | 10 | | | | | |
| 758 | 10 | | | | | |
| 758 | 11 | | | | | |
| 125 | 8 | | | | | |
| 745 | 8 | | | | | |
| 745 | 10 | 0.0001 | | | | |
| 745 | 11 | | | | | |
| 850 | 10 | 0.0001 | | | | |
| 1158 | 8 | | | | | |
| 1158 | 9 | | | | | |
| 1158 | 10 | 0.0001 | | | | |
| 643 | 9 | 0.0001 | | | | |
| 643 | 10 | | | | | |
| 1211 | 10 | 0.0001 | | | | |
| 1162 | 11 | | | | | |
| 269 | 8 | | | | | |
| 269 | 10 | | | | | |
| 1035 | 8 | | | | | |
| 1035 | 9 | | | | | |
| 927 | 8 | | | | | |
| 927 | 9 | 0.0001 | | | | |
| 385 | 8 | | | | | |
| 36 | 8 | | | | | |
| 36 | 10 | 0.0001 | | | | |
| 36 | 11 | | | | | |
| 996 | 8 | | | | | |
| 945 | 9 | | | | | |
| 945 | 10 | | | | | |
| 945 | 11 | | | | | |
| 885 | 10 | | | | | |
| 885 | 11 | | | | | |
| 627 | 9 | 0.0002 | | | | |
| 627 | 11 | | | | | |

### Table VIII
### HER2/NEU A02 Supermotif with Binding Data

| Position | No. of Amino Acids | A*0201 | A*0202 | A*0203 | A*0206 | A*6802 |
|---|---|---|---|---|---|---|
| 612 | 11 | | | | | |
| 1074 | 8 | | | | | |
| 999 | 10 | 0.0001 | | | | |
| 999 | 11 | | | | | |
| 316 | 8 | | | | | |
| 316 | 9 | | | | | |
| 122 | 8 | | | | | |
| 122 | 9 | | | | | |
| 122 | 11 | | | | | |
| 1156 | 8 | | | | | |
| 1156 | 10 | | | | | |
| 1156 | 11 | | | | | |
| 1119 | 10 | 0.0001 | | | | |
| 1119 | 11 | | | | | |
| 391 | 9 | 0.0002 | | | | |
| 95 | 8 | | | | | |
| 95 | 11 | | | | | |
| 1108 | 10 | | | | | |
| 1108 | 11 | | | | | |
| 1130 | 11 | | | | | |
| 699 | 8 | | | | | |
| 650 | 8 | | | | | |
| 650 | 9 | 0.0015 | | | | |
| 650 | 10 | 0.0003 | | | | |
| 159 | 8 | | | | | |
| 159 | 9 | | | | | |
| 159 | 10 | | | | | |
| 1135 | 11 | | | | | |
| 1205 | 8 | | | | | |
| 942 | 8 | | | | | |
| 942 | 10 | | | | | |
| 1147 | 11 | | | | | |
| 1026 | 11 | | | | | |
| 1241 | 9 | | | | | |
| 1241 | 11 | | | | | |
| 232 | 10 | | | | | |
| 232 | 11 | | | | | |
| 1102 | 8 | | | | | |
| 66 | 9 | | | | | |
| 525 | 11 | | | | | |
| 1116 | 9 | | | | | |
| 749 | 11 | | | | | |
| 128 | 10 | 0.0001 | | | | |
| 709 | 10 | | | | | |
| 828 | 9 | 0.0006 | | | | |
| 178 | 9 | | | | | |
| 160 | 8 | | | | | |
| 160 | 9 | 0.0001 | | | | |
| 106 | 8 | | | | | |
| 106 | 9 | 0.4600 | | | | |

<u>Table VIII</u>
<u>HER2/NEU A02 Supermotif with Binding Data</u>

| Position | No. of Amino Acids | A*0201 | A*0202 | A*0203 | A*0206 | A*6802 |
|---|---|---|---|---|---|---|
| 106 | 10 | 0.0140 | 0.0065 | 1.1000 | 0.0170 | 0.5400 |
| 106 | 11 | | | | | |
| 484 | 9 | 0.0062 | | | | |
| 484 | 10 | 0.0003 | | | | |
| 484 | 11 | | | | | |
| 799 | 8 | | | | | |
| 799 | 9 | 0.0230 | 0.0044 | 0.0880 | 0.0052 | 0.0031 |
| 396 | 8 | | | | | |
| 396 | 11 | | | | | |
| 141 | 8 | | | | | |
| 141 | 9 | 0.0008 | | | | |
| 711 | 8 | | | | | |
| 711 | 10 | 0.0001 | | | | |
| 1027 | 10 | | | | | |
| 679 | 8 | | | | | |
| 679 | 9 | | | | | |
| 24 | 8 | | | | | |
| 24 | 10 | 0.0001 | | | | |
| 398 | 9 | | | | | |
| 398 | 10 | | | | | |
| 429 | 8 | | | | | |
| 93 | 9 | | | | | |
| 93 | 10 | 0.0001 | | | | |
| 90 | 10 | 0.0001 | | | | |
| 54 | 9 | 0.0001 | | | | |
| 54 | 10 | | | | | |
| 190 | 9 | 0.0001 | | | | |
| 647 | 8 | | | | | |
| 647 | 9 | 0.0002 | | | | |
| 647 | 11 | | | | | |
| 354 | 8 | | | | | |
| 434 | 10 | 0.0180 | | | | |
| 434 | 11 | | | | | |
| 713 | 8 | | | | | |
| 713 | 11 | | | | | |
| 100 | 8 | | | | | |
| 816 | 8 | | | | | |
| 868 | 8 | | | | | |
| 784 | 8 | | | | | |
| 784 | 10 | | | | | |
| 784 | 11 | | | | | |
| 689 | 8 | | | | | |
| 689 | 9 | 0.0910 | | | | |
| 34 | 8 | | | | | |
| 34 | 10 | 0.0001 | | | | |
| 940 | 9 | 0.0002 | | | | |
| 940 | 10 | | | | | |
| 98 | 8 | | | | | |
| 98 | 10 | 0.0001 | | | | |
| 840 | 8 | | | | | |

Table VIII
HER2/NEU A02 Supermotif with Binding Data

| Position | No. of Amino Acids | A*0201 | A*0202 | A*0203 | A*0206 | A*6802 |
|---|---|---|---|---|---|---|
| 840 | 9 | 0.0001 | | | | |
| 978 | 10 | 0.0020 | | | | |
| 978 | 11 | | | | | |
| 678 | 9 | | | | | |
| 678 | 10 | | | | | |
| 92 | 8 | | | | | |
| 92 | 10 | | | | | |
| 92 | 11 | | | | | |
| 217 | 9 | | | | | |
| 340 | 8 | | | | | |
| 340 | 11 | | | | | |
| 545 | 11 | | | | | |
| 358 | 8 | | | | | |
| 656 | 8 | | | | | |
| 656 | 9 | | | | | |
| 656 | 10 | 0.0009 | | | | |
| 656 | 11 | | | | | |
| 413 | 10 | 0.0002 | | | | |
| 653 | 9 | 0.0720 | 0.0082 | 0.2900 | 0.0130 | 0.2700 |
| 653 | 10 | 0.0002 | | | | |
| 653 | 11 | | | | | |
| 893 | 8 | | | | | |
| 1007 | 8 | | | | | |
| 1007 | 11 | | | | | |
| 418 | 11 | | | | | |
| 1100 | 10 | 0.0059 | | | | |
| 457 | 9 | 0.0002 | | | | |
| 457 | 10 | | | | | |
| 457 | 11 | | | | | |
| 70 | 8 | | | | | |
| 70 | 11 | | | | | |
| 144 | 10 | 0.0150 | | | | |
| 144 | 11 | | | | | |
| 442 | 9 | 0.0003 | | | | |
| 214 | 8 | | | | | |
| 281 | 10 | | | | | |
| 819 | 9 | | | | | |
| 819 | 11 | | | | | |
| 532 | 10 | | | | | |
| 305 | 8 | | | | | |
| 305 | 9 | | | | | |
| 305 | 10 | 0.0001 | | | | |
| 1235 | 8 | | | | | |
| 1235 | 9 | | | | | |
| 1002 | 8 | | | | | |
| 22 | 8 | | | | | |
| 22 | 10 | | | | | |
| 1051 | 9 | | | | | |
| 1051 | 10 | | | | | |
| 1051 | 11 | | | | | |

## Table VIII
## HER2/NEU A02 Supermotif with Binding Data

| Position | No. of Amino Acids | A*0201 | A*0202 | A*0203 | A*0206 | A*6802 |
|---|---|---|---|---|---|---|
| 792 | 9 | | | | | |
| 792 | 10 | | | | | |
| 423 | 8 | | | | | |
| 423 | 9 | 0.0017 | | | | |
| 573 | 9 | | | | | |
| 297 | 8 | | | | | |
| 297 | 10 | | | | | |
| 1242 | 8 | | | | | |
| 1242 | 10 | 0.0001 | | | | |
| 496 | 10 | | | | | |
| 389 | 9 | 0.0002 | | | | |
| 389 | 11 | | | | | |
| 948 | 8 | | | | | |
| 948 | 9 | 0.0005 | | | | |
| 402 | 9 | 0.0018 | | | | |
| 402 | 11 | | | | | |
| 1166 | 8 | | | | | |
| 1060 | 11 | | | | | |
| 182 | 10 | | | | | |
| 444 | 10 | 0.0011 | | | | |
| 1172 | 9 | 0.0002 | | | | |
| 1172 | 10 | 0.0001 | | | | |
| 312 | 11 | | | | | |
| 686 | 9 | | | | | |
| 686 | 11 | | | | | |
| 526 | 10 | | | | | |
| 105 | 9 | | | | | |
| 105 | 10 | | | | | |
| 105 | 11 | | | | | |
| 798 | 9 | | | | | |
| 798 | 10 | | | | | |
| 23 | 9 | | | | | |
| 23 | 11 | | | | | |
| 218 | 8 | | | | | |
| 1117 | 8 | | | | | |
| 793 | 8 | | | | | |
| 793 | 9 | | | | | |
| 911 | 10 | | | | | |
| 733 | 8 | | | | | |
| 750 | 10 | | | | | |
| 597 | 8 | | | | | |
| 988 | 10 | | | | | |
| 430 | 11 | | | | | |
| 116 | 8 | | | | | |
| 116 | 11 | | | | | |
| 725 | 9 | | | | | |
| 725 | 10 | 0.0007 | | | | |
| 666 | 8 | | | | | |
| 666 | 9 | 0.0005 | | | | |
| 666 | 10 | | | | | |

EP 1 568 373 A2

## Table VIII
### HER2/NEU A02 Supermotif with Binding Data

| Position | No. of Amino Acids | A*0201 | A*0202 | A*0203 | A*0206 | A*6802 |
|---|---|---|---|---|---|---|
| 84 | 8 | | | | | |
| 84 | 11 | | | | | |
| 153 | 9 | 0.0290 | | | | |
| 546 | 10 | 0.0009 | | | | |
| 754 | 10 | | | | | |
| 851 | 9 | 0.0002 | | | | |
| 851 | 11 | | | | | |
| 773 | 10 | 0.0180 | | | | |
| 56 | 8 | | | | | |
| 56 | 10 | | | | | |
| 80 | 8 | | | | | |
| 794 | 8 | | | | | |
| 296 | 9 | | | | | |
| 296 | 11 | | | | | |
| 574 | 8 | | | | | |
| 129 | 9 | | | | | |
| 797 | 10 | | | | | |
| 797 | 11 | | | | | |
| 356 | 10 | | | | | |
| 910 | 8 | | | | | |
| 910 | 11 | | | | | |
| 272 | 11 | | | | | |
| 658 | 8 | | | | | |
| 658 | 9 | 0.0005 | | | | |
| 658 | 10 | 0.0009 | | | | |
| 987 | 8 | | | | | |
| 987 | 11 | | | | | |
| 1180 | 11 | | | | | |
| 665 | 9 | 0.3500 | 0.0001 | 0.0040 | 0.0086 | 0.0200 |
| 665 | 10 | 0.0027 | | | | |
| 665 | 11 | | | | | |
| 55 | 8 | | | | | |
| 55 | 9 | | | | | |
| 55 | 11 | | | | | |
| 664 | 10 | 0.0032 | | | | |
| 664 | 11 | | | | | |
| 739 | 8 | | | | | |
| 739 | 10 | | | | | |
| 452 | 10 | 0.0001 | | | | |
| 888 | 8 | | | | | |
| 411 | 9 | 0.0003 | | | | |
| 835 | 8 | | | | | |
| 1248 | 8 | | | | | |
| 64 | 8 | | | | | |
| 64 | 11 | | | | | |
| 303 | 10 | 0.0002 | | | | |
| 303 | 11 | | | | | |
| 1196 | 8 | | | | | |
| 1196 | 9 | 0.0001 | | | | |
| 409 | 11 | | | | | |

EP 1 568 373 A2

## Table VIII
## HER2/NEU A02 Supermotif with Binding Data

| Position | No. of Amino Acids | A*0201 | A*0202 | A*0203 | A*0206 | A*6802 |
|---|---|---|---|---|---|---|
| 952 | 9 | 0.0230 | 0.0001 | 0.0160 | 0.0014 | 0.0400 |
| 952 | 10 | 0.0600 | 0.0004 | 0.0300 | 0.0190 | 0.0011 |
| 163 | 10 | | | | | |
| 50 | 11 | | | | | |
| 289 | 8 | | | | | |
| 289 | 9 | | | | | |
| 289 | 10 | | | | | |
| 685 | 10 | | | | | |
| 83 | 9 | 0.0005 | | | | |
| 772 | 11 | | | | | |
| 554 | 8 | | | | | |
| 781 | 8 | | | | | |
| 781 | 10 | 0.0004 | | | | |
| 781 | 11 | | | | | |

## Table IX
## HER2/NEU A03 Supermotif with Binding Data

| Position | No. of Amino Acids | A*0301 | A*1101 | A*3101 | A*3301 | A*6801 |
|---|---|---|---|---|---|---|
| 241 | 8 | | | | | |
| 847 | 8 | | | | | |
| 1159 | 11 | | | | | |
| 890 | 8 | | | | | |
| 890 | 9 | 0.0013 | 0.0006 | | | |
| 492 | 8 | | | | | |
| 180 | 9 | 0.0004 | 0.0005 | | | |
| 180 | 11 | | | | | |
| 705 | 9 | 0.0004 | 0.0006 | | | |
| 37 | 11 | | | | | |
| 997 | 10 | 0.0003 | 0.0670 | 0.1200 | 0.0140 | 0.0520 |
| 240 | 9 | 0.0021 | 0.0021 | | | |
| 220 | 9 | -0.0002 | -0.0002 | | | |
| 805 | 10 | 0.0003 | 0.0001 | | | |
| 195 | 9 | -0.0008 | -0.0001 | | | |
| 26 | 9 | 0.0002 | 0.0005 | | | |
| 630 | 11 | | | | | |
| 947 | 11 | | | | | |
| 584 | 8 | | | | | |
| 596 | 10 | 0.0220 | 0.0042 | 0.0008 | 0.0064 | 0.0093 |
| 528 | 9 | 0.0015 | 0.0310 | 0.5300 | 0.5800 | 0.4400 |
| 845 | 10 | 0.0018 | 0.0007 | | | |
| 1089 | 8 | | | | | |
| 933 | 8 | | | | | |
| 821 | 11 | | | | | |
| 607 | 9 | 0.0005 | 0.0100 | 0.0002 | 0.0880 | 0.0310 |
| 962 | 9 | -0.0002 | -0.0002 | | | |
| 165 | 11 | | | | | |
| 1144 | 10 | 0.0003 | 0.0001 | | | |
| 950 | 8 | | | | | |
| 147 | 11 | | | | | |
| 930 | 8 | | | | | |
| 930 | 11 | | | | | |
| 914 | 8 | | | | | |
| 971 | 8 | | | | | |
| 971 | 11 | | | | | |
| 280 | 9 | 0.0003 | -0.0002 | | | |
| 207 | 11 | | | | | |
| 717 | 8 | | | | | |
| 874 | 10 | 0.0003 | 0.0001 | | | |
| 40 | 8 | | | | | |
| 321 | 10 | 0.0002 | 0.0001 | | | |
| 976 | 10 | -0.0002 | 0.0010 | | | |
| 729 | 8 | | | | | |
| 1038 | 9 | -0.0002 | 0.0043 | | | |
| 1038 | 11 | | | | | |
| 919 | 11 | | | | | |
| 704 | 10 | -0.0002 | 0.0041 | | | |
| 1231 | 8 | | | | | |
| 131 | 8 | | | | | |

Table IX

HER2/NEU A03 Supermotif with Binding Data

| Position | No. of Amino Acids | A*0301 | A*1101 | A*3101 | A*3301 | A*6801 |
|---|---|---|---|---|---|---|
| 1164 | 8 | | | | | |
| 672 | 10 | 0.0150 | 0.0014 | | | |
| 449 | 8 | | | | | |
| 449 | 11 | | | | | |
| 737 | 11 | | | | | |
| 508 | 10 | 0.0110 | 0.0001 | | | |
| 1062 | 11 | | | | | |
| 447 | 10 | 0.0037 | 0.0001 | | | |
| 344 | 8 | | | | | |
| 344 | 11 | | | | | |
| 549 | 10 | 0.0002 | 0.0003 | | | |
| 136 | 8 | | | | | |
| 346 | 9 | -0.0002 | -0.0002 | | | |
| 832 | 9 | -0.0002 | 0.0002 | | | |
| 1041 | 8 | | | | | |
| 727 | 10 | 0.0660 | 0.1300 | 0.0014 | -0.0013 | 0.0012 |
| 327 | 10 | 0.0210 | 0.6100 | 0.0140 | 0.0012 | 0.0100 |
| 776 | 9 | 0.0010 | 0.0066 | | | |
| 668 | 9 | 0.0047 | 0.0890 | 0.0019 | 0.0025 | 0.0011 |
| 668 | 10 | 0.0180 | 0.0330 | 0.0590 | 0.0140 | 0.4300 |
| 668 | 11 | | | | | |
| 878 | 10 | 0.0003 | 0.0008 | | | |
| 632 | 9 | -0.0002 | 0.0007 | | | |
| 478 | 10 | 0.0035 | 0.0720 | 0.9600 | 0.3300 | 2.0000 |
| 858 | 11 | | | | | |
| 809 | 8 | | | | | |
| 673 | 9 | 0.3800 | 0.0097 | 0.0760 | 0.0064 | 0.0001 |
| 673 | 11 | | | | | |
| 714 | 8 | | | | | |
| 714 | 9 | 0.0190 | 0.0023 | 0.0009 | 0.0010 | 0.0001 |
| 714 | 11 | | | | | |
| 148 | 10 | 0.0400 | 0.0005 | 0.7300 | 0.2400 | 0.0390 |
| 167 | 9 | 0.2800 | 0.3100 | 0.2200 | 0.0300 | 0.0046 |
| 450 | 10 | 0.0410 | 0.0027 | 2.6000 | 0.1300 | 0.1100 |
| 861 | 8 | | | | | |
| 747 | 10 | 0.0009 | 0.0099 | | | |
| 681 | 8 | 0.0010 | 0.0004 | | | |
| 681 | 9 | 0.7600 | 0.0018 | 1.1000 | 0.0072 | 0.0002 |
| 860 | 9 | 0.1700 | 0.2400 | 0.1800 | 0.0012 | 0.0049 |
| 753 | 10 | 0.3800 | 0.2200 | 0.0068 | 0.0012 | 0.0008 |
| 846 | 9 | 0.0580 | 0.0285 | -0.0005 | -0.0012 | 0.0160 |
| 509 | 9 | -0.0002 | 0.0003 | | | |
| 179 | 10 | -0.0002 | 0.0003 | | | |
| 85 | 8 | | | | | |
| 183 | 8 | | | | | |
| 674 | 8 | | | | | |
| 674 | 10 | 0.0002 | 0.0001 | | | |
| 674 | 11 | | | | | |
| 806 | 9 | 0.0370 | 0.0006 | 0.0360 | 0.0890 | 0.0014 |
| 806 | 11 | | | | | |

81

EP 1 568 373 A2

**Table IX**

**HER2/NEU A03 Supermotif with Binding Data**

| Position | No. of Amino Acids | A*0301 | A*1101 | A*3101 | A*3301 | A*6801 |
|---|---|---|---|---|---|---|
| 822 | 10 | 0.1400 | 0.1400 | 0.0100 | 0.0088 | 0.0086 |
| 1173 | 10 | -0.0002 | 0.0003 | | | |
| 422 | 11 | | | | | |
| 608 | 8 | | | | | |
| 181 | 8 | | | | | |
| 181 | 10 | 0.0002 | 0.0005 | | | |
| 841 | 9 | 0.0040 | 0.0014 | | | |
| 852 | 9 | 0.4800 | 0.0700 | 0.0990 | 0.0370 | 0.1100 |
| 972 | 10 | 0.0072 | 0.0330 | 0.3700 | 0.2300 | 0.2200 |
| 774 | 11 | | | | | |
| 889 | 8 | | | | | |
| 889 | 9 | 0.0034 | 0.0237 | 0.0940 | 0.2200 | 0.0630 |
| 889 | 10 | 0.0011 | 0.0003 | | | |
| 960 | 9 | 0.0017 | 0.0006 | | | |
| 960 | 11 | | | | | |
| 833 | 8 | | | | | |
| 360 | 9 | 0.0002 | 0.0036 | | | |
| 360 | 10 | 0.0003 | 0.0056 | | | |
| 427 | 8 | | | | | |
| 758 | 8 | | | | | |
| 745 | 9 | 0.0058 | 0.0007 | 0.0015 | 0.0820 | 0.1200 |
| 850 | 11 | | | | | |
| 1162 | 8 | | | | | |
| 1162 | 10 | -0.0002 | -0.0002 | | | |
| 927 | 11 | | | | | |
| 996 | 11 | | | | | |
| 999 | 8 | | | | | |
| 95 | 9 | 0.0002 | 0.0001 | | | |
| 1065 | 8 | | | | | |
| 1102 | 10 | 0.0003 | 0.0001 | | | |
| 749 | 8 | | | | | |
| 128 | 11 | | | | | |
| 491 | 9 | 0.0046 | 0.0010 | | | |
| 709 | 8 | | | | | |
| 178 | 11 | | | | | |
| 160 | 11 | | | | | |
| 141 | 10 | 0.2000 | 0.0130 | 0.0270 | 0.0047 | 0.0002 |
| 711 | 11 | | | | | |
| 24 | 9 | 0.0007 | 0.0520 | 0.0002 | 0.0006 | 0.0110 |
| 24 | 11 | | | | | |
| 93 | 8 | | | | | |
| 93 | 11 | | | | | |
| 90 | 9 | 0.0029 | 0.0005 | | | |
| 90 | 11 | | | | | |
| 190 | 11 | | | | | |
| 713 | 9 | 0.0007 | 0.0038 | | | |
| 713 | 10 | 0.0570 | 0.1100 | 0.0055 | 0.0013 | 0.0002 |
| 840 | 10 | 0.1800 | 0.0001 | 0.9500 | 0.0021 | 0.0036 |
| 978 | 8 | | | | | |
| 143 | 8 | | | | | |

EP 1 568 373 A2

## Table IX
### HER2/NEU A03 Supermotif with Binding Data

| Position | No. of Amino Acids | A*0301 | A*1101 | A*3101 | A*3301 | A*6801 |
|---|---|---|---|---|---|---|
| 217 | 10 | 0.0068 | 0.0130 | 0.4500 | 0.0220 | 0.0250 |
| 545 | 8 | | | | | |
| 358 | 11 | | | | | |
| 281 | 8 | | | | | |
| 208 | 10 | -0.0002 | 0.0020 | | | |
| 22 | 11 | | | | | |
| 423 | 10 | 0.0170 | 0.0750 | 0.0340 | 0.0390 | 0.2500 |
| 323 | 8 | | | | | |
| 323 | 11 | | | | | |
| 948 | 10 | 0.0130 | 0.1200 | 0.0018 | 0.0120 | 0.0250 |
| 166 | 10 | 0.0430 | 3.6000 | 0.0370 | 0.0420 | 0.0400 |
| 182 | 9 | 0.0004 | 0.0005 | | | |
| 1172 | 11 | | | | | |
| 218 | 9 | 0.0004 | 0.0230 | 0.1400 | 0.0890 | 0.0970 |
| 218 | 11 | | | | | |
| 479 | 9 | 0.0006 | 0.0072 | | | |
| 911 | 11 | | | | | |
| 597 | 9 | 0.0100 | -0.0002 | | | |
| 666 | 11 | | | | | |
| 84 | 9 | 0.0033 | 0.0007 | | | |
| 754 | 9 | 0.4000 | 0.0130 | 0.1400 | 0.1000 | 0.0001 |
| 851 | 10 | 0.0820 | 0.0072 | 0.0052 | 0.0032 | 0.0005 |
| 973 | 9 | -0.0002 | 0.0021 | | | |
| 322 | 9 | 0.0002 | 0.0140 | 0.0011 | 0.0037 | 0.1000 |
| 129 | 10 | 0.0002 | 0.0005 | | | |
| 669 | 8 | | | | | |
| 669 | 9 | 0.1100 | 0.7200 | 1.4000 | 0.3700 | 2.0000 |
| 669 | 10 | 0.0030 | 0.0160 | 0.0620 | 0.1500 | 0.5400 |
| 739 | 9 | 0.0002 | 0.0001 | | | |
| 452 | 8 | | | | | |
| 888 | 9 | -0.0002 | -0.0002 | | | |
| 888 | 10 | 0.0085 | 0.0016 | | | |
| 888 | 11 | | | | | |
| 959 | 8 | | | | | |
| 959 | 10 | -0.0002 | 0.0002 | | | |
| 835 | 10 | 0.0003 | 0.0001 | | | |
| 83 | 10 | 0.0043 | 0.0013 | | | |
| 1139 | 8 | | | | | |

**Table X**

**HER2/NEU A24 Supermotif Peptides with Binding Data**

| Position | No. of Amino Acids | A*2401 |
|---|---|---|
| 1216 | 8 | 0.0039 |
| 1186 | 11 | |
| 730 | 9 | 0.0002 |
| 730 | 10 | 0.0010 |
| 730 | 11 | 0.0008 |
| 1212 | 9 | 0.0011 |
| 1212 | 10 | |
| 1212 | 11 | |
| 113 | 11 | |
| 5 | 8 | |
| 5 | 9 | |
| 5 | 11 | |
| 890 | 10 | |
| 466 | 9 | |
| 466 | 11 | |
| 270 | 10 | |
| 705 | 8 | |
| 705 | 10 | 0.0002 |
| 705 | 11 | -0.0003 |
| 1165 | 9 | |
| 1190 | 8 | |
| 115 | 9 | |
| 355 | 10 | |
| 657 | 11 | |
| 414 | 9 | 0.0041 |
| 440 | 9 | 0.1300 |
| 440 | 11 | 0.0230 |
| 771 | 11 | |
| 475 | 8 | 0.0190 |
| 475 | 11 | 0.0003 |
| 255 | 9 | |
| 789 | 8 | |
| 826 | 8 | |
| 826 | 10 | |
| 826 | 11 | -0.0003 |
| 244 | 10 | |
| 26 | 8 | |
| 26 | 10 | |
| 630 | 8 | |
| 947 | 8 | |
| 947 | 9 | |
| 540 | 8 | |
| 540 | 11 | |
| 504 | 11 | |
| 528 | 8 | |
| 295 | 9 | |
| 295 | 10 | |
| 342 | 9 | 0.0180 |
| 162 | 8 | 0.0120 |
| 162 | 11 | 0.0016 |

84

## Table X
### HER2/NEU A24 Supermotif Peptides with Binding Data

| Position | No. of Amino Acids | A*2401 |
|---|---|---|
| 863 | 8 | 0.0005 |
| 863 | 10 | 0.0002 |
| 171 | 9 | |
| 171 | 11 | |
| 76 | 8 | |
| 76 | 10 | |
| 76 | 11 | |
| 845 | 8 | |
| 1089 | 10 | |
| 993 | 8 | |
| 933 | 9 | |
| 821 | 9 | |
| 421 | 8 | |
| 421 | 11 | |
| 607 | 8 | |
| 607 | 10 | |
| 1016 | 8 | |
| 1016 | 9 | |
| 1013 | 11 | |
| 165 | 8 | |
| 165 | 9 | |
| 1084 | 9 | |
| 307 | 11 | |
| 838 | 9 | |
| 904 | 10 | |
| 950 | 10 | |
| 950 | 11 | |
| 363 | 8 | -0.0003 |
| 363 | 9 | 0.0003 |
| 766 | 9 | |
| 147 | 8 | |
| 147 | 9 | |
| 405 | 8 | |
| 405 | 11 | |
| 2 | 8 | |
| 2 | 10 | |
| 2 | 11 | |
| 460 | 8 | |
| 460 | 9 | |
| 265 | 10 | |
| 914 | 10 | |
| 139 | 10 | |
| 139 | 11 | |
| 719 | 8 | |
| 61 | 11 | |
| 971 | 9 | |
| 1123 | 9 | |
| 717 | 10 | |
| 693 | 8 | |
| 40 | 10 | |

EP 1 568 373 A2

### Table X
### HER2/NEU A24 Supermotif Peptides with Binding Data

| Position | No. of Amino Acids | A*2401 |
|---|---|---|
| 40 | 11 | |
| 401 | 9 | |
| 401 | 10 | |
| 401 | 11 | |
| 79 | 8 | |
| 352 | 10 | |
| 876 | 11 | -0.0003 |
| 1022 | 9 | 0.0014 |
| 1022 | 10 | 0.0120 |
| 553 | 9 | 0.0061 |
| 73 | 11 | |
| 899 | 8 | |
| 899 | 10 | |
| 476 | 10 | |
| 476 | 11 | |
| 986 | 9 | |
| 1004 | 11 | |
| 262 | 10 | |
| 787 | 10 | |
| 672 | 11 | |
| 660 | 8 | |
| 925 | 10 | |
| 925 | 11 | |
| 449 | 10 | |
| 464 | 11 | |
| 865 | 8 | |
| 447 | 9 | |
| 136 | 10 | |
| 454 | 8 | |
| 1091 | 8 | -0.0003 |
| 1091 | 11 | -0.0003 |
| 832 | 10 | |
| 28 | 8 | |
| 1239 | 10 | |
| 1239 | 11 | |
| 104 | 9 | |
| 104 | 11 | |
| 732 | 8 | |
| 732 | 9 | |
| 776 | 10 | |
| 776 | 11 | |
| 603 | 8 | |
| 603 | 9 | |
| 603 | 11 | |
| 152 | 10 | |
| 909 | 8 | |
| 909 | 10 | |
| 668 | 8 | |
| 1179 | 9 | |
| 408 | 8 | 0.0044 |

## Table X
## HER2/NEU A24 Supermotif Peptides with Binding Data

| Position | No. of Amino Acids | A*2401 |
|---|---|---|
| 257 | 10 | 0.0002 |
| 473 | 10 | |
| 42 | 8 | |
| 42 | 9 | |
| 478 | 8 | |
| 478 | 9 | |
| 858 | 9 | |
| 809 | 9 | |
| 370 | 8 | 0.0120 |
| 172 | 8 | -0.0003 |
| 172 | 10 | 0.0022 |
| 654 | 8 | |
| 654 | 9 | |
| 654 | 10 | |
| 767 | 8 | |
| 435 | 9 | |
| 435 | 11 | |
| 673 | 10 | |
| 148 | 8 | |
| 661 | 11 | |
| 954 | 8 | 0.0210 |
| 861 | 9 | |
| 861 | 10 . | |
| 406 | 10 | |
| 101 | 8 | |
| 738 | 11 | 0.0027 |
| 369 | 8 | |
| 369 | 9 | |
| 747 | 9 | |
| 681 | 10 | |
| 681 | 11 | |
| 860 | 10 | |
| 860 | 11 | |
| 722 | 10 . | |
| 724 | 8 | |
| 883 | 9 | |
| 887 | 8 | 0.0080 |
| 887 | 9 | 0.0150 |
| 684 | 8 | 0.0024 |
| 107 | 8 | 0.0006 |
| 107 | 11 | 0.0006 |
| 485 | 9 | 0.0002 |
| 485 | 10 | 0.0014 |
| 467 | 8 | |
| 467 | 10 | |
| 674 | 9 | |
| 154 | 8 | |
| 154 | 10 | |
| 869 | 9 | |
| 1008 | 10 | |

## Table X
## HER2/NEU A24 Supermotif Peptides with Binding Data

| Position | No. of Amino Acids | A*2401 |
|---|---|---|
| 934 | 8 | |
| 822 | 8 | |
| 690 | 11 | |
| 662 | 10 | |
| 800 | 8 | 0.0076 |
| 915 | 9 | 0.0001 |
| 1131 | 9 | |
| 145 | 10 | |
| 145 | 11 | |
| 443 | 8 | |
| 443 | 10 | |
| 443 | 11 | |
| 651 | 11 | |
| 790 | 11 | |
| 62 | 10 | |
| 1017 | 8 | |
| 696 | 11 | |
| 852 | 10 | |
| 1024 | 8 | |
| 972 | 8 | |
| 796 | 8 | |
| 796 | 11 | |
| 271 | 9 | |
| 663 | 9 | |
| 663 | 11 | |
| 410 | 10 | 0.0840 |
| 960 | 10 | |
| 953 | 8 | |
| 953 | 9 | |
| 916 | 8 | |
| 916 | 11 | |
| 360 | 11 | |
| 427 | 9 | |
| 427 | 10 | |
| 388 | 10 | |
| 275 | 8 | |
| 758 | 10 | |
| 758 | 11 | |
| 745 | 8 | |
| 745 | 11 | |
| 824 | 10 | 0.0002 |
| 945 | 8 | |
| 945 | 9 | |
| 945 | 10 | |
| 945 | 11 | |
| 885 | 10 | |
| 885 | 11 | |
| 627 | 11 | |
| 999 | 10 | |
| 999 | 11 | |

88

## Table X
### HER2/NEU A24 Supermotif Peptides with Binding Data

| Position | No. of Amino Acids | A*2401 |
|---|---|---|
| 1119 | 9 | |
| 391 | 10 | |
| 1130 | 10 | |
| 699 | 8 | |
| 197 | 9 | 0.0011 |
| 1241 | 8 | |
| 1241 | 9 | |
| 1241 | 11 | |
| 1102 | 8 | |
| 1102 | 11 | |
| 66 | 8 | |
| 66 | 9 | |
| 525 | 10 | |
| 525 | 11 | |
| 128 | 10 | |
| 922 | 10 | 0.0005 |
| 780 | 9 | 0.1700 |
| 780 | 11 | 0.0320 |
| 828 | 8 | |
| 828 | 9 | |
| 513 | 9 | |
| 160 | 8 | |
| 160 | 9 | |
| 160 | 10 | |
| 106 | 9 | |
| 484 | 10 | |
| 484 | 11 | |
| 799 | 8 | |
| 799 | 9 | |
| 396 | 8 | |
| 396 | 11 | |
| 141 | 8 | |
| 141 | 9 | |
| 795 | 9 | |
| 711 | 10 | |
| 24 | 8 | |
| 24 | 10 | |
| 398 | 9 | |
| 429 | 8 | |
| 93 | 9 | |
| 90 | 10 | |
| 54 | 9 | |
| 54 | 11 | |
| 968 | 9 | 0.0180 |
| 898 | 9 | 0.0110 |
| 898 | 11 | |
| 985 | 10 | 0.0002 |
| 434 | 8 | |
| 434 | 10 | |
| 713 | 8 | |

89

EP 1 568 373 A2

## Table X
### HER2/NEU A24 Supermotif Peptides with Binding Data

| Position | No. of Amino Acids | A*2401 |
|---|---|---|
| 100 | 8 | |
| 100 | 9 | |
| 816 | 8 | |
| 816 | 10 | |
| 868 | 10 | |
| 689 | 8 | |
| 34 | 10 | |
| 940 | 10 | |
| 98 | 10 | |
| 98 | 11 | |
| 978 | 9 | 0.0032 |
| 340 | 8 | |
| 340 | 11 | |
| 545 | 10 | |
| 8 | 8 | 0.0250 |
| 8 | 9 | 1.3000 |
| 1111 | 10 | 0.0120 |
| 653 | 9 | |
| 653 | 10 | |
| 653 | 11 | |
| 373 | 9 | |
| 1007 | 8 | |
| 1007 | 11 | |
| 418 | 8 | |
| 418 | 11 | |
| 1100 | 10 | |
| 457 | 9 | |
| 457 | 11 | |
| 70 | 8 | |
| 144 | 11 | |
| 442 | 9 | |
| 442 | 11 | |
| 281 | 9 | 0.0001 |
| 281 | 11 | 0.0180 |
| 305 | 9 | |
| 1002 | 8 | |
| 22 | 10 | |
| 1051 | 9 | |
| 1051 | 11 | |
| 792 | 9 | |
| 792 | 10 | |
| 423 | 9 | |
| 451 | 8 | -0.0003 |
| 451 | 11 | 0.0036 |
| 907 | 9 | 0.1200 |
| 907 | 10 | 0.0630 |
| 834 | 8 | 0.0059 |
| 609 | 8 | 0.3200 |
| 917 | 10 | 0.0002 |
| 948 | 8 | |

90

## Table X
### HER2/NEU A24 Supermotif Peptides with Binding Data

| Position | No. of Amino Acids | A*2401 |
|---|---|---|
| 166 | 8 | |
| 402 | 8 | |
| 402 | 9 | |
| 402 | 10 | |
| 402 | 11 | |
| 1166 | 8 | |
| 444 | 9 | |
| 444 | 10 | |
| 686 | 11 | -0.0003 |
| 1117 | 11 | |
| 479 | 8 | |
| 793 | 8 | |
| 793 | 9 | |
| 793 | 11 | |
| 911 | 8 | |
| 733 | 8 | |
| 63 | 9 | 0.0380 |
| 63 | 11 | 8.9000 |
| 273 | 10 | 0.0074 |
| 1085 | 8 | |
| 399 | 8 | -0.0003 |
| 399 | 11 | |
| 424 | 8 | -0.0003 |
| 116 | 8 | |
| 725 | 11 | |
| 666 | 8 | |
| 666 | 9 | |
| 666 | 10 | |
| 153 | 9 | |
| 153 | 11 | |
| 546 | 9 | |
| 851 | 11 | |
| 773 | 9 | 0.0001 |
| 296 | 8 | |
| 296 | 9 | |
| 129 | 9 | |
| 797 | 10 | |
| 797 | 11 | |
| 356 | 9 | |
| 910 | 9 | |
| 272 | 8 | |
| 272 | 11 | |
| 658 | 10 | |
| 987 | 8 | |
| 1180 | 8 | |
| 665 | 9 | |
| 665 | 10 | |
| 665 | 11 | |
| 55 | 8 | |
| 55 | 10 | |

## Table X
## HER2/NEU A24 Supermotif Peptides with Binding Data

| Position | No. of Amino Acids | A*2401 |
|---|---|---|
| 55 | 11 | |
| 664 | 8 | |
| 664 | 10 | |
| 664 | 11 | |
| 905 | 9 | 0.0800 |
| 905 | 11 | 0.0920 |
| 951 | 9 | 0.1600 |
| 951 | 10 | 0.0220 |
| 951 | 11 | 1.8000 |
| 739 | 10 | |
| 452 | 10 | |
| 888 | 8 | -0.0003 |
| 959 | 11 | 0.0011 |
| 411 | 9 | |
| 64 | 8 | |
| 64 | 10 | |
| 64 | 11 | |
| 303 | 11 | |
| 1023 | 8 | |
| 1023 | 9 | |
| 409 | 11 | |
| 952 | 8 | 0.0009 |
| 952 | 9 | |
| 952 | 10 | 0.0019 |
| 772 | 10 | 0.0001 |
| 554 | 8 | |
| 781 | 8 | |
| 781 | 10 | |

## Table XI
## HER2/NEU B07 Supermotif Peptides with Binding Data

| Position | No. of Amino Acids | B*0702 |
|---|---|---|
| 1036 | 8 | 0.0063 |
| 390 | 8 | -0.0006 |
| 390 | 10 | 0.0001 |
| 390 | 11 | 0.0011 |
| 1129 | 11 | -0.0002 |
| 1204 | 9 | 0.0056 |
| 1204 | 10 | 0.0530 |
| 1076 | 10 | 0.0002 |
| 1076 | 11 | 0.0006 |
| 612 | 10 | 0.1500 |
| 1032 | 8 | -0.0002 |
| 1032 | 11 | -0.0002 |
| 626 | 10 | 0.0002 |
| 315 | 8 | -0.0006 |
| 315 | 10 | 0.0001 |
| 600 | 9 | 0.0140 |
| 600 | 11 | 0.0300 |
| 299 | 10 | 0.0016 |
| 1034 | 9 | 0.0001 |
| 1034 | 10 | 0.0002 |
| 384 | 9 | 0.0004 |
| 121 | 9 | 0.0002 |
| 982 | 8 | -0.0006 |
| 1105 | 8 | -0.0006 |
| 698 | 8 | -0.0002 |
| 698 | 9 | 0.0110 |
| 995 | 10 | 0.0510 |
| 995 | 11 | 0.0036 |
| 578 | 8 | -0.0006 |
| 578 | 9 | 0.0001 |
| 578 | 11 | -0.0003 |
| 522 | 8 | -0.0006 |
| 246 | 8 | 0.0092 |
| 246 | 9 | 0.0001 |
| 246 | 11 | 0.0006 |
| 1155 | 9 | 0.0900 |
| 1155 | 11 | 0.0160 |
| 524 | 11 | 0.0005 |
| 564 | 11 | -0.0002 |
| 1208 | 9 | 0.0093 |
| 1208 | 10 | 0.0018 |
| 926 | 9 | 0.0006 |
| 926 | 10 | 0.0004 |
| 740 | 9 | 0.0001 |
| 740 | 11 | 0.0023 |
| 931 | 11 | -0.0002 |
| 748 | 8 | 0.0120 |
| 336 | 8 | -0.0006 |
| 336 | 10 | 0.0370 |
| 605 | 9 | 0.0720 |

## Table XI
## HER2/NEU B07 Supermotif Peptides with Binding Data

| Position | No. of Amino Acids | B*0702 |
|---|---|---|
| 605 | 10 | 0.0001 |
| 921 | 8 | 0.0150 |
| 921 | 11 | 0.0430 |
| 1157 | 9 | 0.0027 |
| 1157 | 11 | 0.0140 |
| 35 | 9 | 0.0002 |
| 35 | 11 | -0.0002 |
| 419 | 10 | 0.0003 |
| 377 | 10 | 0.0001 |
| 16 | 10 | 0.0002 |
| 941 | 9 | 0.0280 |
| 941 | 11 | 0.0032 |
| 550 | 8 | 0.0012 |
| 1120 | 8 | -0.0006 |
| 1120 | 9 | 0.0002 |
| 1120 | 10 | 0.0001 |
| 231 | 11 | -0.0003 |
| 1101 | 9 | 0.0460 |
| 65 | 9 | 0.0260 |
| 65 | 10 | 0.0190 |
| 282 | 8 | -0.0006 |
| 282 | 10 | 0.0001 |
| 706 | 9 | 0.0090 |
| 706 | 10 | 0.0490 |
| 801 | 10 | 0.0085 |
| 284 | 8 | -0.0002 |
| 284 | 10 | 0.0001 |
| 1245 | 9 | 0.0001 |
| 1245 | 11 | -0.0002 |
| 1193 | 11 | -0.0003 |
| 488 | 10 | 0.0005 |
| 158 | 10 | 0.0001 |
| 158 | 11 | -0.0002 |
| 1210 | 8 | -0.0002 |
| 1210 | 11 | -0.0002 |
| 1227 | 11 | -0.0003 |
| 17 | 9 | 0.0001 |
| 944 | 8 | -0.0006 |
| 944 | 9 | 0.0001 |
| 944 | 10 | 0.0004 |
| 944 | 11 | 0.0064 |
| 1209 | 8 | -0.0002 |
| 1209 | 9 | 0.0002 |
| 1149 | 9 | 0.0054 |
| 1149 | 11 | 0.4500 |
| 1233 | 11 | -0.0003 |
| 393 | 8 | -0.0002 |
| 393 | 11 | -0.0002 |
| 1136 | 10 | 0.0001 |
| 1206 | 8 | 0.0002 |

EP 1 568 373 A2

## Table XI
## HER2/NEU B07 Supermotif Peptides with Binding Data

| Position | No. of Amino Acids | B*0702 |
|---|---|---|
| 1206 | 11 | 0.0003 |
| 943 | 9 | 0.0001 |
| 943 | 10 | 0.0001 |
| 943 | 11 | 0.0020 |
| 1148 | 10 | 0.0014 |
| 568 | 10 | 0.0004 |
| 499 | 11 | -0.0002 |
| 966 | 8 | 0.0410 |
| 966 | 11 | 1.3000 |
| 1214 | 8 | -0.0002 |
| 1214 | 9 | 0.0001 |
| 1214 | 10 | 0.0001 |
| 38 | 8 | 0.0014 |
| 38 | 9 | 0.0005 |
| 133 | 8 | 0.0550 |
| 133 | 10 | 0.0580 |
| 1174 | 8 | 0.0230 |
| 1174 | 11 | -0.0002 |
| 760 | 8 | 0.0580 |
| 760 | 9 | 0.1200 |
| 1073 | 9 | 0.0030 |
| 998 | 8 | -0.0006 |
| 998 | 11 | 0.0640 |
| 649 | 9 | 0.0150 |
| 649 | 10 | 0.0900 |
| 649 | 11 | 0.0250 |
| 196 | 10 | 0.0021 |
| 855 | 10 | 0.0016 |
| 1151 | 9 | 0.6400 |
| 1151 | 10 | 0.4600 |
| 779 | 8 | 0.0440 |
| 779 | 10 | 0.1000 |
| 701 | 10 | 0.0001 |
| 1240 | 9 | |
| 1240 | 10 | 0.0002 |
| 127 | 11 | -0.0002 |
| 884 | 8 | 1.4000 |
| 884 | 11 | 0.0017 |
| 1118 | 10 | 0.0001 |
| 1118 | 11 | -0.0002 |
| 94 | 8 | 0.0020 |
| 94 | 9 | 0.0077 |
| 415 | 8 | 0.0200 |
| 415 | 10 | 0.0044 |
| 415 | 11 | 0.0005 |

## Table XII

### HER2/NEU B27 Supermotif Peptides

| Position | No. of Amino Acids |
|---|---|
| 87 | 10 |
| 588 | 8 |
| 598 | 11 |
| 980 | 10 |
| 928 | 8 |
| 867 | 11 |
| 1160 | 8 |
| 339 | 9 |
| 511 | 11 |
| 367 | 8 |
| 367 | 10 |
| 367 | 11 |
| 965 | 8 |
| 544 | 9 |
| 7 | 10 |
| 7 | 10 |
| 808 | 11 |
| 936 | 9 |
| 1229 | 8 |
| 939 | 9 |
| 939 | 11 |
| 173 | 9 |
| 173 | 11 |
| 969 | 8 |
| 969 | 11 |
| 486 | 8 |
| 486 | 9 |
| 1176 | 9 |
| 882 | 10 |
| 882 | 8 |
| 433 | 10 |
| 433 | 9 |
| 815 | 11 |
| 815 | 8 |
| 815 | 9 |
| 469 | 11 |
| 174 | 8 |
| 174 | 10 |
| 843 | 11 |
| 468 | 10 |
| 675 | 9 |
| 675 | 8 |
| 886 | 11 |
| 886 | 9 |
| 431 | 10 |
| 682 | 11 |
| 682 | 9 |
| 248 | 10 |
| 248 | 9 |

## Table XII

### HER2/NEU B27 Supermotif Peptides

| Position | No. of Amino Acids |
|---|---|
| 368 | 9 |
| 368 | 10 |
| 676 | 10 |
| 266 | 9 |
| 256 | 8 |
| 256 | 11 |
| 436 | 8 |
| 436 | 10 |
| 458 | 8 |
| 458 | 10 |
| 458 | 11 |
| 137 | 9 |
| 99 | 9 |
| 99 | 10 |
| 33 | 11 |
| 455 | 11 |
| 142 | 8 |
| 712 | 9 |
| 687 | 10 |
| 259 | 8 |
| 857 | 8 |
| 857 | 10 |
| 764 | 9 |
| 764 | 10 |
| 813 | 11 |
| 813 | 11 |
| 1207 | 11 |
| 761 | 8 |
| 247 | 10 |
| 551 | 11 |
| 967 | 10 |
| 680 | 8 |
| 680 | 11 |
| 646 | 9 |
| 646 | 10 |
| 984 | 11 |
| 97 | 11 |
| 156 | 8 |
| 1110 | 11 |
| 721 | 11 |
| 683 | 8 |
| 683 | 9 |
| 897 | 10 |
| 688 | 9 |
| 677 | 9 |
| 677 | 11 |
| 896 | 11 |
| 335 | 9 |
| 335 | 11 |
| 189 | 10 |

## Table XII
## HER2/NEU B27 Supermotif Peptides

| Position | No. of Amino Acids |
|---|---|
| 783 | 8 |
| 977 | 10 |
| 1103 | 10 |
| 472 | 11 |
| 41 | 9 |
| 41 | 10 |
| 900 | 9 |
| 1052 | 8 |
| 1052 | 10 |
| 842 | 11 |
| 477 | 9 |
| 477 | 10 |
| 746 | 10 |
| 859 | 8 |
| 859 | 11 |
| 604 | 8 |
| 604 | 10 |
| 604 | 11 |
| 853 | 9 |
| 723 | 9 |
| 353 | 9 |
| 810 | 8 |
| 102 | 11 |
| 839 | 8 |
| 91 | 9 |
| 91 | 11 |
| 169 | 11 |
| 877 | 10 |
| 1005 | 10 |

## Table XIII
## HER2/NEU B58 Supermotif Peptides

| Position | No. of Amino Acids |
|---|---|
| 1094 | 8 |
| 4 | 8 |
| 4 | 9 |
| 4 | 10 |
| 1203 | 11 |
| 1159 | 9 |
| 293 | 9 |
| 293 | 11 |
| 69 | 9 |
| 37 | 9 |
| 37 | 10 |
| 132 | 9 |
| 132 | 11 |
| 997 | 8 |
| 997 | 9 |
| 648 | 8 |
| 648 | 11 |
| 21 | 11 |
| 1165 | 9 |
| 587 | 9 |
| 587 | 11 |
| 224 | 8 |
| 338 | 8 |
| 338 | 10 |
| 334 | 8 |
| 334 | 10 |
| 195 | 11 |
| 1133 | 8 |
| 531 | 11 |
| 244 | 10 |
| 26 | 8 |
| 26 | 10 |
| 630 | 8 |
| 947 | 8 |
| 947 | 9 |
| 947 | 10 |
| 962 | 8 |
| 962 | 11 |
| 417 | 8 |
| 417 | 9 |
| 1001 | 8 |
| 1001 | 9 |
| 165 | 8 |
| 165 | 9 |
| 580 | 11 |
| 770 | 8 |
| 892 | 8 |
| 280 | 10 |
| 207 | 9 |
| 1123 | 9 |

EP 1 568 373 A2

98

## Table XIII
## HER2/NEU B58 Supermotif Peptides

| Position | No. of Amino Acids |
|---|---|
| 717 | 9 |
| 717 | 10 |
| 693 | 8 |
| 874 | 11 |
| 40 | 10 |
| 40 | 11 |
| 401 | 9 |
| 401 | 10 |
| 401 | 11 |
| 364 | 8 |
| 364 | 11 |
| 1213 | 8 |
| 1213 | 9 |
| 1213 | 10 |
| 1213 | 11 |
| 976 | 11 |
| 899 | 8 |
| 899 | 10 |
| 1093 | 9 |
| 621 | 8 |
| 729 | 10 |
| 729 | 11 |
| 1080 | 11 |
| 919 | 8 |
| 704 | 9 |
| 704 | 11 |
| 292 | 10 |
| 131 | 10 |
| 1164 | 10 |
| 1189 | 8 |
| 1189 | 9 |
| 439 | 10 |
| 309 | 9 |
| 1082 | 9 |
| 1082 | 11 |
| 727 | 8 |
| 727 | 9 |
| 372 | 10 |
| 778 | 8 |
| 778 | 9 |
| 778 | 11 |
| 818 | 8 |
| 818 | 10 |
| 28 | 8 |
| 1239 | 10 |
| 1239 | 11 |
| 104 | 9 |
| 104 | 11 |
| 732 | 8 |
| 732 | 9 |

EP 1 568 373 A2

## Table XIII
## HER2/NEU B58 Supermotif Peptides

| Position | No. of Amino Acids |
|---|---|
| 878 | 9 |
| 878 | 11 |
| 249 | 8 |
| 249 | 10 |
| 495 | 11 |
| 478 | 8 |
| 478 | 9 |
| 86 | 9 |
| 86 | 11 |
| 829 | 8 |
| 829 | 11 |
| 655 | 8 |
| 655 | 9 |
| 655 | 10 |
| 655 | 11 |
| 412 | 8 |
| 412 | 11 |
| 450 | 9 |
| 861 | 9 |
| 861 | 10 |
| 406 | 10 |
| 762 | 11 |
| 854 | 8 |
| 854 | 11 |
| 1171 | 10 |
| 1171 | 11 |
| 3 | 9 |
| 3 | 10 |
| 3 | 11 |
| 846 | 8 |
| 253 | 11 |
| 374 | 8 |
| 465 | 10 |
| 1075 | 11 |
| 114 | 10 |
| 71 | 10 |
| 1173 | 8 |
| 1173 | 9 |
| 304 | 10 |
| 304 | 11 |
| 422 | 9 |
| 422 | 10 |
| 608 | 9 |
| 1131 | 9 |
| 1131 | 10 |
| 145 | 9 |
| 145 | 10 |
| 145 | 11 |
| 443 | 8 |
| 443 | 10 |

**Table XIII**

**HER2/NEU B58 Supermotif Peptides**

| Position | No. of Amino Acids |
|---|---|
| 443 | 11 |
| 651 | 8 |
| 651 | 9 |
| 651 | 11 |
| 790 | 8 |
| 790 | 11 |
| 62 | 10 |
| 774 | 8 |
| 774 | 9 |
| 889 | 11 |
| 979 | 8 |
| 979 | 9 |
| 979 | 10 |
| 979 | 11 |
| 833 | 9 |
| 833 | 10 |
| 916 | 8 |
| 916 | 11 |
| 68 | 10 |
| 388 | 10 |
| 275 | 8 |
| 471 | 10 |
| 758 | 10 |
| 758 | 11 |
| 1158 | 10 |
| 643 | 9 |
| 1215 | 8 |
| 1215 | 9 |
| 1211 | 10 |
| 1211 | 11 |
| 269 | 11 |
| 1035 | 8 |
| 1035 | 9 |
| 927 | 8 |
| 927 | 9 |
| 385 | 8 |
| 36 | 10 |
| 36 | 11 |
| 36 | 9 |
| 996 | 10 |
| 996 | 11 |
| 1065 | 9 |
| 1077 | 10 |
| 1077 | 8 |
| 1121 | 11 |
| 1121 | 11 |
| 702 | 8 |
| 601 | 10 |
| 601 | 11 |

101

## Table XIII
## HER2/NEU B58 Supermotif Peptides

| Position | No. of Amino Acids |
|---|---|
| 1150 | 8 |
| 1241 | 8 |
| 1241 | 9 |
| 1241 | 11 |
| 1102 | 8 |
| 1102 | 11 |
| 66 | 8 |
| 66 | 9 |
| 525 | 10 |
| 525 | 11 |
| 902 | 9 |
| 902 | 11 |
| 1099 | 11 |
| 190 | 9 |
| 647 | 8 |
| 647 | 9 |
| 354 | 8 |
| 354 | 11 |
| 1053 | 9 |
| 1053 | 11 |
| 1006 | 9 |
| 456 | 10 |
| 143 | 11 |
| 188 | 11 |
| 656 | 8 |
| 656 | 9 |
| 656 | 10 |
| 656 | 11 |
| 413 | 10 |
| 208 | 8 |
| 1049 | 11 |
| 1050 | 10 |
| 305 | 9 |
| 305 | 10 |
| 1002 | 8 |
| 22 | 10 |
| 1051 | 9 |
| 1051 | 11 |
| 792 | 9 |
| 792 | 10 |
| 297 | 8 |
| 1242 | 8 |
| 1242 | 10 |
| 496 | 10 |
| 389 | 9 |
| 389 | 11 |
| 357 | 8 |
| 652 | 8 |
| 652 | 10 |
| 652 | 11 |

102

## Table XIII
## HER2/NEU B58 Supermotif Peptides

| Position | No. of Amino Acids |
|---|---|
| 759 | 9 |
| 759 | 10 |
| 791 | 10 |
| 791 | 11 |
| 585 | 11 |
| 597 | 8 |
| 782 | 9 |
| 296 | 8 |
| 296 | 9 |
| 129 | 9 |
| 797 | 10 |
| 797 | 11 |
| 356 | 9 |
| 910 | 9 |
| 272 | 8 |
| 272 | 11 |
| 906 | 8 |
| 906 | 10 |
| 906 | 11 |
| 1112 | 9 |
| 441 | 8 |
| 441 | 10 |
| 289 | 8 |

## Table XIV
## HER2/NEU B62 Supermotif Peptides

| Position | No. of Amino Acids |
|----------|--------------------|
| 890 | 10 |
| 466 | 11 |
| 270 | 10 |
| 705 | 8 |
| 705 | 10 |
| 1036 | 8 |
| 390 | 10 |
| 390 | 11 |
| 1129 | 11 |
| 1204 | 10 |
| 1076 | 10 |
| 1076 | 11 |
| 355 | 10 |
| 657 | 8 |
| 657 | 9 |
| 657 | 10 |
| 255 | 9 |
| 789 | 9 |
| 826 | 8 |
| 826 | 10 |
| 1032 | 11 |
| 626 | 10 |
| 315 | 8 |
| 600 | 11 |
| 299 | 10 |
| 567 | 8 |
| 567 | 11 |
| 212 | 8 |
| 596 | 9 |
| 295 | 9 |
| 76 | 8 |
| 76 | 9 |
| 76 | 11 |
| 845 | 9 |
| 821 | 9 |
| 421 | 10 |
| 421 | 11 |
| 607 | 8 |
| 607 | 10 |
| 1016 | 8 |
| 1016 | 10 |
| 1013 | 11 |
| 1034 | 9 |
| 1034 | 10 |
| 121 | 9 |
| 982 | 8 |
| 1105 | 8 |
| 582 | 9 |
| 1183 | 9 |
| 1084 | 9 |

104

## Table XIV
## HER2/NEU B62 Supermotif Peptides

| Position | No. of Amino Acids |
|---|---|
| 307 | 8 |
| 904 | 9 |
| 904 | 10 |
| 950 | 10 |
| 950 | 11 |
| 766 | 8 |
| 766 | 9 |
| 147 | 9 |
| 405 | 8 |
| 405 | 11 |
| 2 | 8 |
| 460 | 9 |
| 265 | 8 |
| 265 | 10 |
| 914 | 10 |
| 139 | 10 |
| 971 | 9 |
| 698 | 9 |
| 645 | 10 |
| 645 | 11 |
| 79 | 8 |
| 352 | 10 |
| 73 | 8 |
| 73 | 11 |
| 534 | 8 |
| 425 | 11 |
| 476 | 11 |
| 262 | 11 |
| 787 | 8 |
| 787 | 11 |
| 672 | 11 |
| 660 | 10 |
| 660 | 11 |
| 737 | 10 |
| 865 | 8 |
| 344 | 10 |
| 346 | 8 |
| 346 | 11 |
| 832 | 8 |
| 832 | 11 |
| 995 | 10 |
| 995 | 11 |
| 578 | 8 |
| 522 | 8 |
| 524 | 11 |
| 537 | 10 |
| 603 | 8 |
| 603 | 9 |
| 603 | 11 |
| 909 | 8 |

## Table XIV
## HER2/NEU B62 Supermotif Peptides

| Position | No. of Amino Acids |
|---|---|
| 909 | 10 |
| 668 | 8 |
| 1179 | 9 |
| 473 | 8 |
| 473 | 10 |
| 42 | 9 |
| 349 | 8 |
| 48 | 8 |
| 48 | 9 |
| 564 | 11 |
| 1208 | 10 |
| 512 | 10 |
| 901 | 8 |
| 901 | 10 |
| 654 | 8 |
| 654 | 11 |
| 767 | 8 |
| 767 | 11 |
| 673 | 10 |
| 714 | 10 |
| 148 | 8 |
| 661 | 9 |
| 661 | 10 |
| 661 | 11 |
| 954 | 8 |
| 740 | 9 |
| 740 | 11 |
| 361 | 10 |
| 361 | 11 |
| 77 | 8 |
| 77 | 10 |
| 155 | 9 |
| 101 | 8 |
| 369 | 8 |
| 747 | 8 |
| 336 | 8 |
| 605 | 9 |
| 605 | 10 |
| 921 | 11 |
| 722 | 10 |
| 724 | 8 |
| 724 | 11 |
| 85 | 10 |
| 467 | 10 |
| 467 | 11 |
| 674 | 9 |
| 154 | 10 |
| 869 | 9 |
| 1008 | 11 |
| 785 | 10 |

## Table XIV
## HER2/NEU B62 Supermotif Peptides

| Position | No. of Amino Acids |
|---|---|
| 822 | 8 |
| 15 | 11 |
| 690 | 8 |
| 662 | 8 |
| 662 | 9 |
| 662 | 10 |
| 800 | 11 |
| 915 | 9 |
| 35 | 11 |
| 16 | 10 |
| 941 | 9 |
| 941 | 11 |
| 1120 | 8 |
| 1120 | 9 |
| 65 | 9 |
| 74 | 10 |
| 74 | 11 |
| 445 | 8 |
| 547 | 9 |
| 547 | 9 |
| 140 | 8 |
| 392 | 9 |
| 392 | 10 |
| 1109 | 10 |
| 397 | 8 |
| 428 | 10 |
| 313 | 9 |
| 1017 | 11 |
| 696 | 11 |
| 841 | 8 |
| 852 | 10 |
| 852 | 8 |
| 1024 | 8 |
| 972 | 8 |
| 796 | 9 |
| 271 | 9 |
| 663 | 8 |
| 663 | 9 |
| 663 | 11 |
| 960 | 8 |
| 953 | 10 |
| 953 | 9 |
| 45 | 11 |
| 282 | 8 |
| 282 | 10 |
| 706 | 9 |
| 801 | 10 |
| 827 | 9 |
| 360 | 11 |
| 427 | 9 |

## Table XIV
## HER2/NEU B62 Supermotif Peptides

| Position | No. of Amino Acids |
|---|---|
| 284 | 8 |
| 1245 | 9 |
| 1245 | 11 |
| 158 | 10 |
| 177 | 8 |
| 745 | 8 |
| 745 | 10 |
| 850 | 10 |
| 945 | 8 |
| 945 | 9 |
| 945 | 10 |
| 945 | 11 |
| 885 | 10 |
| 627 | 9 |
| 122 | 8 |
| 1119 | 9 |
| 1119 | 10 |
| 391 | 9 |
| 391 | 10 |
| 95 | 8 |
| 1108 | 11 |
| 1130 | 10 |
| 1130 | 11 |
| 699 | 8 |
| 650 | 9 |
| 650 | 10 |
| 197 | 9 |
| 1210 | 8 |
| 1227 | 11 |
| 17 | 9 |
| 944 | 8 |
| 944 | 9 |
| 944 | 10 |
| 944 | 11 |
| 1209 | 9 |
| 159 | 9 |
| 159 | 11 |
| 569 | 9 |
| 1135 | 11 |
| 1205 | 9 |
| 942 | 8 |
| 942 | 10 |
| 942 | 11 |
| 828 | 8 |
| 513 | 9 |
| 160 | 8 |
| 160 | 10 |
| 106 | 11 |
| 396 | 11 |
| 141 | 8 |

## Table XIV
## HER2/NEU B62 Supermotif Peptides

| Position | No. of Amino Acids |
|---|---|
| 795 | 9 |
| 393 | 8 |
| 1136 | 10 |
| 1206 | 8 |
| 943 | 9 |
| 943 | 10 |
| 943 | 11 |
| 568 | 10 |
| 679 | 9 |
| 24 | 8 |
| 398 | 9 |
| 93 | 9 |
| 93 | 10 |
| 54 | 11 |
| 434 | 8 |
| 713 | 11 |
| 100 | 9 |
| 816 | 10 |
| 868 | 10 |
| 784 | 11 |
| 689 | 9 |
| 940 | 10 |
| 98 | 11 |
| 978 | 9 |
| 978 | 10 |
| 978 | 11 |
| 966 | 8 |
| 966 | 11 |
| 678 | 8 |
| 678 | 10 |
| 92 | 10 |
| 92 | 11 |
| 340 | 8 |
| 545 | 10 |
| 545 | 11 |
| 653 | 9 |
| 373 | 9 |
| 1007 | 8 |
| 418 | 8 |
| 70 | 8 |
| 70 | 11 |
| 144 | 10 |
| 442 | 9 |
| 442 | 11 |
| 281 | 9 |
| 281 | 11 |
| 1214 | 8 |
| 1214 | 9 |
| 1214 | 10 |
| 38 | 8 |

### Table XIV
### HER2/NEU B62 Supermotif Peptides

| Position | No. of Amino Acids |
| --- | --- |
| 1174 | 8 |
| 1174 | 11 |
| 760 | 8 |
| 998 | 8 |
| 649 | 10 |
| 649 | 11 |
| 196 | 10 |
| 855 | 10 |
| 779 | 10 |
| 819 | 9 |
| 819 | 11 |
| 532 | 10 |
| 423 | 8 |
| 423 | 9 |
| 948 | 8 |
| 948 | 9 |
| 166 | 8 |
| 402 | 8 |
| 402 | 10 |
| 402 | 11 |
| 444 | 9 |
| 1172 | 9 |
| 1172 | 10 |
| 312 | 11 |
| 1240 | 9 |
| 526 | 9 |
| 105 | 8 |
| 23 | 9 |
| 1117 | 11 |
| 479 | 8 |
| 793 | 9 |
| 793 | 11 |
| 911 | 8 |
| 733 | 8 |
| 725 | 10 |
| 725 | 11 |
| 666 | 8 |
| 666 | 10 |
| 84 | 8 |
| 84 | 11 |
| 153 | 11 |
| 546 | 9 |
| 546 | 10 |
| 851 | 9 |
| 851 | 11 |
| 773 | 9 |
| 773 | 10 |
| 884 | 11 |
| 1118 | 10 |
| 1118 | 11 |

## Table XV

## HER2/NEU B62 Supermotif Peptides

| Position | No. of Amino Acids |
|---|---|
| 94 | 8 |
| 94 | 9 |
| 56 | 9 |
| 794 | 8 |
| 794 | 10 |
| 658 | 8 |
| 658 | 9 |
| 1180 | 8 |
| 665 | 9 |
| 665 | 11 |
| 55 | 10 |
| 664 | 8 |
| 664 | 10 |
| 739 | 8 |
| 739 | 10 |
| 959 | 11 |
| 415 | 10 |
| 415 | 11 |
| 835 | 8 |
| 1248 | 8 |
| 64 | 10 |
| 1023 | 8 |
| 1023 | 9 |
| 952 | 8 |
| 952 | 9 |
| 952 | 10 |
| 163 | 10 |
| 163 | 11 |
| 83 | 9 |
| 772 | 10 |
| 772 | 11 |
| 781 | 8 |

## Table XV
## HER2/NEU A01 Motif Peptides with Binding Data

| Position | No. of Amino Acids | A*0101 |
|---|---|---|
| 1212 | 10 | 0.0010 |
| 1212 | 11 | 0.0140 |
| 293 | 9 | 0.0550 |
| 293 | 11 | 0.1900 |
| 997 | 9 | 0.0290 |
| 826 | 10 | 0.3000 |
| 600 | 11 | |
| 334 | 10 | 0.0016 |
| 1013 | 11 | 0.0027 |
| 1105 | 8 | |
| 580 | 11 | 0.1000 |
| 280 | 10 | 0.1800 |
| 40 | 11 | 0.2800 |
| 401 | 9 | 0.0430 |
| 401 | 11 | 0.4400 |
| 279 | 11 | 0.0049 |
| 291 | 11 | 0.0100 |
| 1213 | 9 | 0.0430 |
| 1213 | 10 | 5.5000 |
| 899 | 10 | 2.7000 |
| 292 | 10 | 0.0012 |
| 1188 | 9 | |
| 995 | 11 | |
| 727 | 9 | 0.0011 |
| 1239 | 10 | 0.0630 |
| 104 | 9 | 0.1800 |
| 42 | 9 | 9.1000 |
| 901 | 8 | -0.0021 |
| 333 | 11 | -0.0017 |
| 403 | 9 | 0.0057 |
| 726 | 10 | 0.0010 |
| 869 | 9 | 7.6000 |
| 915 | 9 | 0.0011 |
| 1120 | 8 | |
| 74 | 10 | 0.0015 |
| 1131 | 9 | 0.1300 |
| 1014 | 10 | 0.0120 |
| 916 | 8 | -0.0021 |
| 764 | 9 | 0.0017 |
| 996 | 10 | 0.0150 |
| 601 | 10 | 0.0010 |
| 1241 | 8 | 0.0030 |
| 1102 | 11 | 0.0160 |
| 828 | 8 | -0.0021 |
| 728 | 8 | -0.0021 |
| 281 | 9 | 0.0028 |
| 1214 | 8 | |
| 1214 | 9 | |
| 1132 | 8 | -0.0021 |
| 1103 | 10 | 0.0015 |

111

## Table XV
## HER2/NEU A01 Motif Peptides with Binding Data

| Position | No. of Amino Acids | A*0101 |
|----------|--------------------|--------|
| 402 | 8 | -0.0021 |
| 402 | 10 | 1.1000 |
| 399 | 11 | 0.0045 |
| 773 | 9 | 0.0400 |
| 296 | 8 | 0.1000 |

## Table XVI
## HER2/NEU A03 Motif Peptides with Binding Data

| Position | No. of Amino Acids | A*0301 |
|---|---|---|
| 241 | 8 | |
| 241 | 9 | |
| 1094 | 11 | |
| 4 | 11 | |
| 1203 | 10 | |
| 1203 | 11 | |
| 847 | 8 | |
| 1159 | 11 | |
| 586 | 10 | |
| 191 | 10 | |
| 510 | 8 | |
| 510 | 10 | |
| 622 | 11 | |
| 879 | 9 | |
| 581 | 8 | |
| 581 | 9 | |
| 581 | 10 | |
| 581 | 11 | |
| 1186 | 11 | |
| 1212 | 10 | |
| 1212 | 11 | 0.0003 |
| 1163 | 9 | |
| 365 | 11 | |
| 242 | 8 | |
| 221 | 8 | |
| 1039 | 8 | |
| 1039 | 9 | |
| 1039 | 10 | |
| 775 | 10 | |
| 5 | 10 | |
| 890 | 8 | |
| 890 | 9 | 0.0013 |
| 890 | 10 | |
| 466 | 8 | |
| 466 | 11 | |
| 492 | 8 | |
| 14 | 8 | |
| 180 | 9 | 0.0004 |
| 180 | 11 | |
| 270 | 10 | |
| 705 | 9 | 0.0004 |
| 293 | 9 | 0.0008 |
| 293 | 11 | |
| 37 | 11 | |
| 997 | 8 | |
| 997 | 9 | 0.0002 |
| 997 | 10 | 0.0003 |
| 648 | 10 | |
| 355 | 10 | |
| 355 | 11 | |

## Table XVI
### HER2/NEU A03 Motif Peptides with Binding Data

| Position | No. of Amino Acids | A*0301 |
|---|---|---|
| 240 | 9 | 0.0021 |
| 240 | 10 | |
| 220 | 9 | -0.0002 |
| 587 | 9 | |
| 235 | 8 | |
| 576 | 11 | |
| 252 | 9 | |
| 805 | 10 | 0.0003 |
| 826 | 10 | 0.0003 |
| 334 | 10 | 0.0003 |
| 195 | 9 | -0.0008 |
| 244 | 11 | |
| 26 | 9 | 0.0002 |
| 630 | 11 | |
| 947 | 11 | |
| 311 | 8 | |
| 584 | 8 | |
| 596 | 10 | 0.0220 |
| 634 | 11 | |
| 504 | 9 | |
| 528 | 9 | 0.0015 |
| 295 | 9 | 0.0002 |
| 234 | 8 | |
| 234 | 9 | |
| 251 | 8 | |
| 251 | 10 | |
| 211 | 11 | |
| 1011 | 10 | |
| 638 | 10 | |
| 638 | 11 | |
| 1012 | 9 | |
| 1087 | 8 | |
| 1087 | 9 | |
| 1087 | 10 | |
| 382 | 9 | |
| 742 | 10 | |
| 880 | 8 | |
| 880 | 11 | |
| 326 | 8 | |
| 326 | 11 | |
| 871 | 8 | |
| 871 | 9 | |
| 171 | 10 | |
| 76 | 8 | |
| 845 | 10 | 0.0018 |
| 636 | 9 | |
| 1089 | 8 | |
| 933 | 8 | |
| 821 | 10 | |
| 821 | 11 | |

114

## Table XVI
## HER2/NEU A03 Motif Peptides with Binding Data

| Position | No. of Amino Acids | A*0301 |
|---|---|---|
| 607 | 9 | 0.0005 |
| 607 | 10 | |
| 1016 | 8 | |
| 1013 | 8 | |
| 1013 | 11 | |
| 30 | 8 | |
| 962 | 8 | |
| 962 | 9 | -0.0002 |
| 417 | 9 | |
| 165 | 9 | |
| 165 | 10 | |
| 165 | 11 | |
| 185 | 9 | |
| 1183 | 8 | |
| 1084 | 11 | |
| 838 | 10 | |
| 838 | 11 | |
| 1144 | 10 | 0.0003 |
| 950 | 8 | |
| 580 | 9 | |
| 580 | 10 | |
| 580 | 11 | |
| 1069 | 8 | |
| 543 | 10 | |
| 503 | 8 | |
| 503 | 10 | |
| 210 | 8 | |
| 1010 | 11 | |
| 501 | 10 | |
| 325 | 9 | |
| 837 | 8 | |
| 837 | 11 | |
| 363 | 9 | |
| 975 | 11 | |
| 1079 | 8 | |
| 507 | 10 | |
| 507 | 11 | |
| 1154 | 8 | |
| 1154 | 10 | |
| 286 | 8 | |
| 766 | 10 | |
| 147 | 11 | |
| 930 | 8 | |
| 930 | 11 | |
| 405 | 10 | |
| 460 | 10 | |
| 460 | 11 | |
| 265 | 10 | 0.0002 |
| 914 | 8 | |
| 914 | 10 | 0.0002 |

**Table XVI**
**HER2/NEU A03 Motif Peptides with Binding Data**

| Position | No. of Amino Acids | A*0301 |
|---|---|---|
| 61 | 9 | |
| 695 | 11 | |
| 971 | 8 | |
| 971 | 10 | |
| 971 | 11 | |
| 379 | 8 | |
| 892 | 8 | |
| 892 | 10 | |
| 280 | 9 | 0.0003 |
| 280 | 10 | 0.0003 |
| 207 | 11 | |
| 717 | 8 | |
| 874 | 10 | 0.0003 |
| 40 | 8 | |
| 40 | 9 | |
| 40 | 11 | |
| 401 | 9 | 0.0002 |
| 401 | 11 | . |
| 79 | 9 | |
| 79 | 10 | |
| 352 | 8 | |
| 321 | 10 | 0.0002 |
| 364 | 8 | |
| 1031 | 9 | |
| 595 | 11 | |
| 1086 | 9 | |
| 1086 | 10 | |
| 1086 | 11 | |
| 381 | 10 | |
| 1030 | 10 | |
| 918 | 11 | |
| 291 | 8 | |
| 291 | 11 | |
| 1187 | 10 | |
| 671 | 8 | |
| 671 | 11 | |
| 577 | 10 | |
| 371 | 11 | |
| 376 | 11 | |
| 73 | 11 | |
| 1213 | 9 | 0.0002 |
| 1213 | 10 | 0.0005 |
| 976 | 10 | -0.0002 |
| 976 | 11 | |
| 899 | 10 | 0.0003 |
| 476 | 11 | |
| 1202 | 11 | |
| 729 | 8 | |
| 1038 | 8 | |
| 1038 | 9 | -0.0002 |

## Table XVI
## HER2/NEU A03 Motif Peptides with Binding Data

| Position | No. of Amino Acids | A*0301 |
|---|---|---|
| 1038 | 10 | |
| 1038 | 11 | |
| 919 | 10 | |
| 919 | 11 | |
| 704 | 10 | -0.0002 |
| 1231 | 8 | |
| 292 | 10 | 0.0003 |
| 131 | 8 | |
| 1164 | 8 | |
| 1189 | 8 | |
| 366 | 10 | |
| 366 | 11 | |
| 804 | 8 | |
| 804 | 11 | |
| 641 | 8 | |
| 1088 | 8 | |
| 1088 | 9 | |
| 1015 | 9 | |
| 383 | 8 | |
| 1029 | 8 | |
| 1029 | 11 | |
| 1201 | 8 | |
| 1188 | 9 | 0.0003 |
| 881 | 10 | |
| 135 | 9 | |
| 1040 | 8 | |
| 1040 | 9 | |
| 262 | 9 | |
| 672 | 10 | 0.0150 |
| 449 | 8 | |
| 449 | 11 | |
| 737 | 11 | |
| 508 | 9 | |
| 508 | 10 | 0.0110 |
| 464 | 10 | |
| 1062 | 9 | |
| 1062 | 11 | |
| 447 | 10 | 0.0037 |
| 344 | 8 | |
| 344 | 11 | |
| 10 | 11 | |
| 549 | 9 | |
| 549 | 10 | 0.0002 |
| 549 | 11 | |
| 1097 | 8 | |
| 136 | 8 | |
| 346 | 9 | -0.0002 |
| 346 | 10 | |
| 832 | 9 | -0.0002 |
| 1041 | 8 | |

Table XVI

HER2/NEU A03 Motif Peptides with Binding Data

| Position | No. of Amino Acids | A*0301 |
|---|---|---|
| 309 | 10 | |
| 727 | 9 | 0.0028 |
| 727 | 10 | 0.0660 |
| 462 | 8 | |
| 462 | 9 | |
| 372 | 10 | |
| 572 | 10 | |
| 28 | 10 | 0.0002 |
| 1239 | 10 | 0.0001 |
| 104 | 9 | |
| 104 | 10 | 0.0210 |
| 327 | 10 | 0.0010 |
| 776 | 9 | |
| 603 | 8 | 0.0047 |
| 989 | 10 | 0.0180 |
| 668 | 9 | |
| 668 | 10 | |
| 668 | 11 | 0.0003 |
| 1179 | 8 | |
| 1179 | 9 | |
| 878 | 10 | 0.0370 |
| 267 | 8 | -0.0002 |
| 1104 | 8 | |
| 1104 | 9 | |
| 257 | 11 | 0.0035 |
| 42 | 9 | |
| 349 | 11 | |
| 632 | 9 | |
| 249 | 9 | |
| 249 | 10 | |
| 260 | 8 | |
| 260 | 11 | |
| 478 | 9 | |
| 478 | 10 | |
| 858 | 10 | |
| 858 | 11 | |
| 809 | 8 | |
| 263 | 8 | |
| 872 | 8 | |
| 961 | 9 | |
| 961 | 10 | |
| 961 | 8 | |
| 184 | 10 | |
| 184 | 9 | |
| 949 | 9 | |
| 172 | 9 | 0.3800 |
| 767 | 9 | |
| 673 | 11 | |
| 673 | 8 | |
| 714 | | |

## Table XVI
### HER2/NEU A03 Motif Peptides with Binding Data

| Position | No. of Amino Acids | A*0301 |
|---|---|---|
| 714 | 9 | 0.0190 |
| 714 | 11 | |
| 148 | 10 | 0.0400 |
| 661 | 11 | |
| 894 | 8 | |
| 167 | 8 | |
| 167 | 9 | 0.2800 |
| 450 | 10 | 0.0410 |
| 861 | 8 | |
| 406 | 9 | |
| 101 | 8 | |
| 762 | 10 | |
| 762 | 11 | |
| 333 | 8 | |
| 333 | 11 | |
| 957 | 10 | |
| 170 | 11 | |
| 591 | 8 | |
| 591 | 9 | |
| 1182 | 9 | |
| 615 | 8 | |
| 640 | 8 | |
| 640 | 9 | |
| 150 | 8 | |
| 1096 | 9 | |
| 831 | 10 | |
| 228 | 10 | |
| 1238 | 11 | |
| 369 | 8 | |
| 747 | 10 | 0.0009 |
| 681 | 8 | 0.0010 |
| 681 | 9 | 0.7600 |
| 860 | 8 | |
| 860 | 9 | 0.1700 |
| 32 | 11 | |
| 854 | 11 | |
| 722 | 9 | |
| 722 | 10 | |
| 724 | 8 | |
| 753 | 10 | 0.3800 |
| 753 | 11 | |
| 883 | 8 | |
| 846 | 9 | 0.0580 |
| 509 | 8 | |
| 509 | 9 | -0.0002 |
| 509 | 11 | |
| 253 | 8 | |
| 374 | 8 | |
| 465 | 9 | |
| 13 | 8 | |

## Table XVI
### HER2/NEU A03 Motif Peptides with Binding Data

| Position | No. of Amino Acids | A*0301 |
|---|---|---|
| 13 | 9 | |
| 179 | 10 | -0.0002 |
| 6 | 9 | |
| 161 | 10 | 0.0081 |
| 637 | 8 | |
| 637 | 11 | |
| 768 | 8 | |
| 807 | 8 | |
| 807 | 10 | |
| 870 | 8 | |
| 870 | 9 | |
| 870 | 10 | |
| 43 | 8 | |
| 107 | 9 | |
| 485 | 8 | |
| 485 | 11 | |
| 448 | 9 | |
| 1061 | 10 | |
| 345 | 10 | |
| 345 | 11 | |
| 994 | 11 | |
| 726 | 10 | 0.0003 |
| 726 | 11 | |
| 461 | 9 | |
| 461 | 10 | |
| 667 | 10 | |
| 667 | 11 | |
| 85 | 8 | |
| 183 | 8 | |
| 183 | 9 | |
| 183 | 11 | |
| 467 | 10 | |
| 674 | 8 | |
| 674 | 10 | 0.0002 |
| 674 | 11 | |
| 154 | 10 | 0.0012 |
| 12 | 9 | |
| 12 | 10 | |
| 806 | 9 | 0.0370 |
| 806 | 11 | |
| 869 | 9 | 0.0003 |
| 869 | 10 | |
| 869 | 11 | |
| 11 | 10 | |
| 11 | 11 | |
| 822 | 9 | |
| 822 | 10 | 0.1400 |
| 662 | 10 | |
| 800 | 10 | |
| 915 | 9 | 0.0002 |

120

EP 1 568 373 A2

## Table XVI
## HER2/NEU A03 Motif Peptides with Binding Data

| Position | No. of Amino Acids | A*0301 |
|---|---|---|
| 1173 | 10 | -0.0002 |
| 422 | 11 | |
| 608 | 8 | |
| 608 | 9 | |
| 1131 | 9 | 0.0001 |
| 181 | 8 | |
| 181 | 10 | 0.0002 |
| 181 | 11 | |
| 1197 | 8 | |
| 700 | 11 | |
| 215 | 11 | |
| 62 | 8 | |
| 696 | 10 | |
| 841 | 8 | |
| 841 | 9 | 0.0040 |
| 852 | 9 | 0.4800 |
| 1024 | 8 | |
| 972 | 9 | |
| 972 | 10 | 0.0072 |
| 796 | 8 | |
| 271 | 9 | |
| 663 | 9 | |
| 774 | 8 | |
| 774 | 11 | |
| 889 | 8 | |
| 889 | 9 | 0.0034 |
| 889 | 10 | 0.0011 |
| 889 | 11 | |
| 979 | 8 | |
| 1014 | 10 | 0.0002 |
| 960 | 9 | 0.0017 |
| 960 | 10 | |
| 960 | 11 | |
| 833 | 8 | |
| 833 | 11 | |
| 916 | 8 | |
| 556 | 9 | |
| 571 | 11 | |
| 1178 | 8 | |
| 1178 | 9 | |
| 1178 | 10 | |
| 360 | 9 | 0.0002 |
| 360 | 10 | 0.0003 |
| 59 | 11 | |
| 427 | 8 | |
| 427 | 11 | |
| 471 | 8 | |
| 758 | 8 | |
| 125 | 8 | |
| 745 | 9 | 0.0058 |

121

## Table XVI
## HER2/NEU A03 Motif Peptides with Binding Data

| Position | No. of Amino Acids | A*0301 |
|---|---|---|
| 850 | 9 | |
| 850 | 11 | |
| 1158 | 8 | |
| 1215 | 8 | |
| 1211 | 11 | |
| 1162 | 8 | |
| 1162 | 10 | -0.0002 |
| 269 | 11 | |
| 1035 | 10 | |
| 1035 | 11 | |
| 927 | 11 | |
| 996 | 9 | |
| 996 | 10 | 0.0003 |
| 996 | 11 | |
| 625 | 8 | |
| 194 | 10 | |
| 741 | 11 | |
| 932 | 9 | |
| 606 | 10 | |
| 606 | 11 | |
| 416 | 10 | |
| 1143 | 11 | |
| 1037 | 8 | |
| 1037 | 9 | |
| 1037 | 10 | |
| 1037 | 11 | |
| 134 | 10 | |
| 1175 | 8 | |
| 1175 | 11 | |
| 945 | 8 | |
| 612 | 11 | |
| 1074 | 8 | |
| 999 | 8 | |
| 316 | 9 | |
| 122 | 11 | |
| 1156 | 8 | |
| 1156 | 10 | |
| 1119 | 9 | 0.0002 |
| 1119 | 11 | |
| 230 | 8 | |
| 391 | 10 | |
| 95 | 9 | 0.0002 |
| 1130 | 10 | 0.0002 |
| 650 | 8 | |
| 1065 | 8 | |
| 1077 | 10 | |
| 1121 | 9 | |
| 702 | 9 | |
| 601 | 10 | 0.0003 |
| 1150 | 10 | |

122

## Table XVI
## HER2/NEU A03 Motif Peptides with Binding Data

| Position | No. of Amino Acids | A*0301 |
|---|---|---|
| 1150 | 11 | |
| 1234 | 10 | |
| 1241 | 8 | |
| 232 | 10 | |
| 232 | 11 | |
| 1102 | 10 | 0.0003 |
| 1102 | 11 | |
| 66 | 8 | |
| 525 | 10 | |
| 749 | 8 | |
| 128 | 11 | |
| 491 | 9 | 0.0046 |
| 709 | 8 | |
| 239 | 10 | |
| 239 | 11 | |
| 583 | 8 | |
| 583 | 9 | |
| 527 | 8 | |
| 527 | 10 | |
| 75 | 9 | |
| 820 | 11 | |
| 1225 | 8 | |
| 164 | 10 | |
| 164 | 11 | |
| 1028 | 9 | |
| 1200 | 9 | |
| 446 | 11 | |
| 548 | 10 | |
| 548 | 11 | |
| 57 | 8 | |
| 81 | 8 | |
| 828 | 8 | |
| 178 | 11 | |
| 160 | 11 | |
| 106 | 8 | |
| 106 | 10 | |
| 484 | 9 | |
| 799 | 11 | |
| 141 | 10 | 0.2000 |
| 795 | 9 | 0.0110 |
| 711 | 11 | |
| 213 | 9 | |
| 24 | 9 | 0.0007 |
| 24 | 11 | |
| 429 | 9 | |
| 93 | 8 | |
| 93 | 11 | |
| 90 | 9 | 0.0029 |
| 90 | 11 | |
| 54 | 11 | |

## Table XVI
### HER2/NEU A03 Motif Peptides with Binding Data

| Position | No. of Amino Acids | A*0301 |
|---|---|---|
| 190 | 11 | |
| 647 | 11 | |
| 354 | 11 | |
| 330 | 10 | |
| 330 | 11 | |
| 844 | 11 | |
| 968 | 9 | |
| 968 | 11 | |
| 898 | 11 | |
| 1230 | 8 | |
| 1230 | 9 | |
| 536 | 10 | |
| 432 | 9 | |
| 432 | 10 | |
| 103 | 10 | 0.0003 |
| 103 | 11 | |
| 434 | 8 | |
| 713 | 9 | 0.0007 |
| 713 | 10 | 0.0570 |
| 100 | 9 | |
| 868 | 10 | 0.0017 |
| 868 | 11 | |
| 34 | 9 | |
| 98 | 11 | |
| 840 | 8 | |
| 840 | 9 | |
| 840 | 10 | 0.1800 |
| 978 | 8 | |
| 978 | 9 | 0.0001 |
| 456 | 11 | |
| 143 | 8 | |
| 1072 | 10 | |
| 217 | 9 | |
| 217 | 10 | 0.0068 |
| 340 | 10 | |
| 545 | 8 | |
| 545 | 10 | 0.0350 |
| 358 | 8 | |
| 358 | 11 | |
| 310 | 9 | |
| 633 | 8 | |
| 294 | 8 | |
| 294 | 10 | |
| 250 | 8 | |
| 250 | 9 | |
| 250 | 11 | |
| 380 | 11 | |
| 728 | 8 | |
| 728 | 9 | |
| 703 | 8 | |

124

## Table XVI
## HER2/NEU A03 Motif Peptides with Binding Data

| Position | No. of Amino Acids | A*0301 |
|---|---|---|
| 703 | 11 | |
| 261 | 10 | |
| 463 | 8 | |
| 463 | 11 | |
| 602 | 9 | |
| 893 | 9 | |
| 373 | 9 | |
| 418 | 8 | |
| 457 | 10 | |
| 214 | 8 | |
| 281 | 8 | |
| 281 | 9 | 0.0002 |
| 281 | 11 | |
| 208 | 10 | -0.0002 |
| 1235 | 9 | |
| 22 | 11 | |
| 423 | 10 | 0.0170 |
| 573 | 9 | |
| 323 | 8 | |
| 323 | 11 | |
| 1132 | 8 | |
| 233 | 9 | |
| 233 | 10 | |
| 29 | 9 | |
| 278 | 11 | |
| 290 | 9 | |
| 1236 | 8 | |
| 130 | 9 | |
| 245 | 10 | |
| 27 | 8 | |
| 27 | 11 | |
| 407 | 8 | |
| 948 | 10 | 0.0130 |
| 166 | 8 | |
| 166 | 9 | |
| 166 | 10 | 0.0430 |
| 402 | 8 | |
| 402 | 10 | 0.0001 |
| 1060 | 11 | |
| 182 | 9 | 0.0004 |
| 182 | 10 | |
| 1172 | 11 | |
| 357 | 8 | |
| 357 | 9 | |
| 218 | 8 | |
| 218 | 9 | 0.0004 |
| 218 | 11 | |
| 1117 | 11 | |
| 479 | 8 | |
| 479 | 9 | 0.0006 |

125

EP 1 568 373 A2

**Table XVI**
**HER2/NEU A03 Motif Peptides with Binding Data**

| Position | No. of Amino Acids | A*0301 |
|---|---|---|
| 793 | 11 | |
| 911 | 8 | |
| 911 | 10 | |
| 911 | 11 | |
| 585 | 11 | |
| 597 | 9 | 0.0100 |
| 219 | 8 | |
| 219 | 10 | |
| 314 | 11 | |
| 25 | 8 | |
| 25 | 10 | |
| 341 | 9 | |
| 341 | 11 | |
| 635 | 10 | |
| 1085 | 10 | |
| 1085 | 11 | |
| 399 | 11 | |
| 670 | 8 | |
| 670 | 9 | |
| 424 | 9 | |
| 424 | 11 | |
| 505 | 8 | |
| 308 | 11 | |
| 777 | 8 | |
| 988 | 10 | |
| 430 | 8 | |
| 430 | 11 | |
| 725 | 11 | |
| 666 | 11 | |
| 84 | 9 | 0.0033 |
| 153 | 11 | |
| 546 | 9 | 0.0012 |
| 754 | 9 | 0.4000 |
| 754 | 10 | |
| 851 | 8 | |
| 851 | 10 | 0.0820 |
| 773 | 9 | 0.0580 |
| 973 | 8 | |
| 973 | 9 | -0.0002 |
| 296 | 8 | |
| 322 | 9 | 0.0002 |
| 574 | 8 | |
| 129 | 10 | 0.0002 |
| 356 | 9 | |
| 356 | 10 | |
| 910 | 9 | |
| 910 | 11 | |
| 272 | 8 | |
| 669 | 8 | |
| 669 | 9 | 0.1100 |

127

## Table XVI
## HER2/NEU A03 Motif Peptides with Binding Data

| Position | No. of Amino Acids | A*0301 |
|---|---|---|
| 669 | 10 | 0.0030 |
| 987 | 11 | |
| 1180 | 8 | |
| 1180 | 11 | |
| 55 | 10 | 0.0024 |
| 664 | 8 | |
| 825 | 11 | |
| 1223 | 8 | |
| 1223 | 10 | |
| 482 | 9 | |
| 482 | 11 | |
| 739 | 9 | 0.0002 |
| 452 | 8 | |
| 888 | 9 | -0.0002 |
| 888 | 10 | 0.0085 |
| 888 | 11 | |
| 959 | 8 | |
| 959 | 10 | -0.0002 |
| 959 | 11 | |
| 803 | 9 | |
| 343 | 9 | |
| 908 | 11 | |
| 835 | 9 | |
| 835 | 10 | 0.0003 |
| 64 | 10 | |
| 1196 | 8 | |
| 1196 | 9 | |
| 1023 | 8 | |
| 1023 | 9 | |
| 289 | 10 | |
| 83 | 10 | 0.0043 |
| 772 | 10 | 0.0100 |
| 554 | 11 | |
| 1139 | 8 | |

### Table XVII
### HER2/NEU A11 Motif Peptides with Binding Data

| Position | No. of Amino Acids | A*1101 |
|---|---|---|
| 241 | 8 | |
| 241 | 9 | |
| 1094 | 11 | |
| 847 | 8 | |
| 1159 | 11 | |
| 191 | 10 | |
| 510 | 8 | |
| 510 | 10 | |
| 622 | 11 | |
| 879 | 9 | |
| 581 | 9 | |
| 581 | 10 | |
| 581 | 11 | |
| 1186 | 11 | |
| 1212 | 10 | 0.0003 |
| 1212 | 11 | |
| 1163 | 9 | |
| 242 | 8 | |
| 221 | 8 | |
| 1039 | 8 | |
| 1039 | 9 | |
| 1039 | 10 | |
| 775 | 10 | |
| 890 | 8 | |
| 890 | 9 | 0.0006 |
| 466 | 8 | |
| 492 | 8 | |
| 180 | 9 | 0.0005 |
| 180 | 11 | |
| 705 | 9 | 0.0006 |
| 359 | 10 | |
| 359 | 11 | |
| 763 | 10 | |
| 293 | 9 | 0.0074 |
| 293 | 11 | |
| 37 | 11 | |
| 997 | 9 | 0.0004 |
| 997 | 10 | 0.0670 |
| 240 | 9 | 0.0021 |
| 240 | 10 | |
| 220 | 9 | -0.0002 |
| 252 | 9 | |
| 805 | 10 | 0.0001 |
| 826 | 10 | 0.0001 |
| 334 | 10 | 0.0002 |
| 195 | 9 | -0.0001 |
| 26 | 9 | 0.0005 |
| 630 | 11 | |
| 947 | 11 | |
| 311 | 8 | |

## TableXVII
## HER2/NEU A11 Motif Peptides with Binding Data

| Position | No. of Amino Acids | A*1101 |
|---|---|---|
| 584 | 8 | |
| 596 | 10 | 0.0042 |
| 504 | 9 | |
| 528 | 9 | 0.0310 |
| 295 | 9 | 0.0004 |
| 251 | 10 | |
| 638 | 10 | |
| 1087 | 10 | |
| 880 | 8 | |
| 326 | 8 | |
| 326 | 11 | |
| 871 | 8 | |
| 76 | 8 | |
| 845 | 10 | 0.0007 |
| 1089 | 8 | |
| 933 | 8 | |
| 821 | 11 | |
| 607 | 9 | 0.0100 |
| 1016 | 8 | |
| 1013 | 11 | |
| 962 | 9 | -0.0002 |
| 165 | 10 | |
| 165 | 11 | |
| 185 | 9 | |
| 1144 | 10 | 0.0001 |
| 950 | 8 | |
| 580 | 10 | |
| 580 | 11 | |
| 543 | 10 | |
| 503 | 10 | |
| 210 | 8 | |
| 325 | 9 | |
| 837 | 8 | |
| 975 | 11 | |
| 507 | 11 | |
| 1154 | 8 | |
| 147 | 11 | |
| 930 | 8 | |
| 930 | 11 | |
| 460 | 10 | |
| 460 | 11 | |
| 265 | 10 | 0.0002 |
| 914 | 8 | |
| 914 | 10 | 0.0002 |
| 971 | 8 | |
| 971 | 11 | |
| 757 | 9 | |
| 744 | 10 | |
| 892 | 10 | |
| 280 | 9 | -0.0002 |

## Table XVII
## HER2/NEU A11 Motif Peptides with Binding Data

| Position | No. of Amino Acids | A*1101 |
|---|---|---|
| 280 | 10 | 0.0003 |
| 207 | 11 | |
| 717 | 8 | |
| 874 | 10 | 0.0001 |
| 40 | 8 | |
| 40 | 9 | |
| 40 | 11 | |
| 401 | 9 | 0.0002 |
| 401 | 11 | |
| 79 | 10 | |
| 321 | 10 | 0.0001 |
| 595 | 11 | |
| 1086 | 11 | |
| 291 | 11 | |
| 1187 | 10 | |
| 671 | 8 | |
| 671 | 11 | |
| 73 | 11 | |
| 258 | 10 | |
| 1213 | 9 | 0.0002 |
| 1213 | 10 | 0.0010 |
| 976 | 10 | 0.0010 |
| 899 | 10 | 0.0005 |
| 729 | 8 | |
| 1038 | 8 | |
| 1038 | 9 | 0.0043 |
| 1038 | 10 | |
| 1038 | 11 | |
| 919 | 11 | |
| 704 | 10 | 0.0041 |
| 1231 | 8 | |
| 292 | 10 | 0.0001 |
| 131 | 8 | |
| 1164 | 8 | |
| 1189 | 8 | |
| 804 | 8 | |
| 804 | 11 | |
| 1088 | 9 | |
| 1015 | 9 | |
| 1201 | 8 | |
| 1188 | 9 | 0.0001 |
| 135 | 9 | |
| 1040 | 8 | |
| 1040 | 9 | |
| 672 | 10 | 0.0014 |
| 449 | 8 | |
| 449 | 11 | |
| 737 | 11 | |
| 508 | 10 | 0.0001 |
| 464 | 10 | |

## TableXVII
## HER2/NEU A11 Motif Peptides with Binding Data

| Position | No. of Amino Acids | A*1101 |
|---|---|---|
| 1062 | 11 | |
| 447 | 10 | 0.0001 |
| 344 | 8 | |
| 344 | 11 | |
| 549 | 10 | 0.0003 |
| 549 | 11 | |
| 1097 | 8 | |
| 136 | 8 | |
| 346 | 9 | -0.0002 |
| 832 | 9 | 0.0002 |
| 1041 | 8 | |
| 309 | 10 | |
| 727 | 9 | 0.0001 |
| 727 | 10 | 0.1300 |
| 462 | 8 | |
| 462 | 9 | |
| 1239 | 10 | 0.0022 |
| 104 | 9 | 0.0280 |
| 327 | 10 | 0.6100 |
| 776 | 9 | 0.0066 |
| 603 | 8 | |
| 668 | 9 | 0.0890 |
| 668 | 10 | 0.0330 |
| 668 | 11 | |
| 878 | 10 | 0.0008 |
| 267 | 8 | |
| 1104 | 8 | |
| 1104 | 9 | |
| 257 | 11 | |
| 42 | 9 | 0.0002 |
| 88 | 11 | |
| 470 | 9 | |
| 632 | 9 | 0.0007 |
| 249 | 9 | |
| 260 | 8 | |
| 478 | 10 | 0.0720 |
| 858 | 11 | |
| 809 | 8 | |
| 961 | 8 | |
| 961 | 10 | |
| 184 | 10 | |
| 949 | 9 | |
| 673 | 9 | 0.0097 |
| 673 | 11 | |
| 714 | 8 | |
| 714 | 9 | 0.0023 |
| 714 | 11 | |
| 148 | 10 | 0.0005 |
| 894 | 8 | |
| 167 | 8 | |

## Table XVII
### HER2/NEU A11 Motif Peptides with Binding Data

| Position | No. of Amino Acids | A*1101 |
|---|---|---|
| 167 | 9 | 0.3100 |
| 450 | 10 | 0.0027 |
| 861 | 8 | |
| 762 | 11 | |
| 333 | 8 | |
| 333 | 11 | |
| 957 | 10 | |
| 591 | 9 | |
| 640 | 8 | |
| 150 | 8 | |
| 1096 | 9 | |
| 831 | 10 | |
| 228 | 10 | |
| 1238 | 11 | |
| 747 | 10 | 0.0099 |
| 681 | 8 | 0.0004 |
| 681 | 9 | 0.0018 |
| 860 | 9 | 0.2400 |
| 32 | 11 | |
| 753 | 10 | 0.2200 |
| 846 | 9 | 0.0285 |
| 509 | 9 | 0.0003 |
| 509 | 11 | |
| 253 | 8 | |
| 465 | 9 | |
| 179 | 10 | 0.0003 |
| 161 | 10 | 0.0063 |
| 637 | 11 | |
| 807 | 8 | |
| 807 | 10 | |
| 870 | 8 | |
| 870 | 9 | |
| 43 | 8 | |
| 485 | 11 | |
| 448 | 9 | |
| 345 | 10 | |
| 726 | 10 | 0.0003 |
| 726 | 11 | |
| 461 | 9 | |
| 461 | 10 | |
| 667 | 10 | |
| 667 | 11 | |
| 85 | 8 | |
| 183 | 8 | |
| 183 | 11 | |
| 674 | 8 | |
| 674 | 10 | 0.0001 |
| 674 | 11 | |
| 154 | 10 | 0.0002 |
| 806 | 9 | 0.0006 |

## TableXVII
## HER2/NEU A11 Motif Peptides with Binding Data

| Position | No. of Amino Acids | A*1101 |
|---|---|---|
| 806 | 11 | |
| 869 | 9 | 0.0001 |
| 869 | 10 | |
| 822 | 10 | 0.1400 |
| 800 | 10 | |
| 915 | 9 | 0.0003 |
| 823 | 9 | |
| 1173 | 10 | 0.0003 |
| 422 | 11 | |
| 608 | 8 | |
| 1131 | 9 | 0.0061 |
| 181 | 8 | |
| 181 | 10 | 0.0005 |
| 841 | 9 | 0.0014 |
| 852 | 9 | 0.0700 |
| 972 | 10 | 0.0330 |
| 796 | 8 | |
| 774 | 8 | |
| 774 | 11 | |
| 889 | 8 | |
| 889 | 9 | 0.0237 |
| 889 | 10 | 0.0003 |
| 1014 | 10 | 0.0002 |
| 960 | 9 | 0.0006 |
| 960 | 11 | |
| 833 | 8 | |
| 833 | 11 | . |
| 916 | 8 | |
| 556 | 9 | |
| 360 | 9 | 0.0036 |
| 360 | 10 | 0.0056 |
| 427 | 8 | |
| 427 | 11 | |
| 471 | 8 | |
| 758 | 8 | |
| 745 | 9 | 0.0007 |
| 850 | 9 | |
| 850 | 11 | |
| 1215 | 8 | |
| 1211 | 11 | |
| 1162 | 8 | |
| 1162 | 10 | -0.0002 |
| 1035 | 10 | |
| 1035 | 11 | |
| 927 | 11 | |
| 996 | 10 | 0.0001 |
| 996 | 11 | |
| 625 | 8 | |
| 194 | 10 | |
| 932 | 9 | |

133

EP 1 568 373 A2

## Table XVII
## HER2/NEU A11 Motif Peptides with Binding Data

| Position | No. of Amino Acids | A*1101 |
|---|---|---|
| 606 | 10 | |
| 1143 | 11 | |
| 1037 | 8 | |
| 1037 | 9 | |
| 1037 | 10 | |
| 1037 | 11 | |
| 134 | 10 | |
| 1175 | 8 | |
| 945 | 8 | |
| 999 | 8 | |
| 1119 | 9 | 0.0002 |
| 230 | 8 | |
| 95 | 9 | 0.0001 |
| 1130 | 10 | 0.0002 |
| 707 | 10 | |
| 1065 | 8 | |
| 601 | 10 | 0.0003 |
| 1241 | 8 | |
| 1102 | 10 | 0.0001 |
| 1102 | 11 | |
| 749 | 8 | |
| 128 | 11 | |
| 491 | 9 | 0.0010 |
| 709 | 8 | |
| 239 | 10 | |
| 239 | 11 | |
| 583 | 8 | |
| 583 | 9 | |
| 527 | 10 | |
| 75 | 9 | |
| 164 | 11 | |
| 1200 | 9 | |
| 446 | 11 | |
| 548 | 11 | |
| 57 | 8 | |
| 81 | 8 | |
| 828 | 8 | |
| 178 | 11 | |
| 160 | 11 | |
| 799 | 11 | |
| 141 | 10 | 0.0130 |
| 795 | 9 | 0.0039 |
| 711 | 11 | |
| 426 | 9 | |
| 24 | 9 | 0.0520 |
| 24 | 11 | |
| 429 | 9 | |
| 93 | 8 | |
| 93 | 11 | |
| 90 | 9 | 0.0005 |

134

EP 1 568 373 A2

## Table XVII
## HER2/NEU A11 Motif Peptides with Binding Data

| Position | No. of Amino Acids | A*1101 |
|---|---|---|
| 90 | 11 | |
| 54 | 11 | |
| 190 | 11 | |
| 330 | 11 | |
| 844 | 11 | |
| 968 | 11 | |
| 898 | 11 | |
| 1230 | 9 | |
| 536 | 10 | |
| 432 | 10 | |
| 103 | 10 | 0.0015 |
| 434 | 8 | |
| 713 | 9 | 0.0038 |
| 713 | 10 | 0.1100 |
| 868 | 10 | 0.0001 |
| 868 | 11 | |
| 34 | 9 | |
| 840 | 10 | 0.0001 |
| 978 | 8 | |
| 487 | 9 | |
| 849 | 10 | |
| 143 | 8 | |
| 217 | 10 | 0.0130 |
| 340 | 10 | |
| 545 | 8 | |
| 545 | 10 | 0.0050 |
| 358 | 11 | |
| 310 | 9 | |
| 633 | 8 | |
| 294 | 8 | |
| 294 | 10 | |
| 250 | 8 | |
| 250 | 11 | |
| 728 | 8 | |
| 728 | 9 | |
| 703 | 11 | |
| 463 | 8 | |
| 463 | 11 | |
| 602 | 9 | |
| 893 | 9 | |
| 281 | 8 | |
| 281 | 9 | 0.0003 |
| 208 | 10 | 0.0020 |
| 22 | 11 | |
| 423 | 10 | 0.0750 |
| 323 | 8 | |
| 323 | 11 | |
| 1132 | 8 | |
| 278 | 11 | |
| 130 | 9 | |

EP 1 568 373 A2

### Table XVII
### HER2/NEU A11 Motif Peptides with Binding Data

| Position | No. of Amino Acids | A*1101 |
|---|---|---|
| 27 | 8 | |
| 948 | 10 | 0.1200 |
| 166 | 9 | |
| 166 | 10 | 3.6000 |
| 402 | 8 | |
| 402 | 10 | 0.0001 |
| 182 | 9 | 0.0005 |
| 1172 | 11 | |
| 186 | 8 | |
| 218 | 9 | 0.0230 |
| 218 | 11 | |
| 1117 | 11 | |
| 479 | 9 | 0.0072 |
| 793 | 11 | |
| 911 | 11 | |
| 597 | 9 | -0.0002 |
| 219 | 8 | |
| 219 | 10 | |
| 25 | 8 | |
| 25 | 10 | |
| 341 | 9 | |
| 341 | 11 | |
| 399 | 11 | |
| 670 | 8 | |
| 670 | 9 | |
| 424 | 9 | |
| 424 | 11 | |
| 505 | 8 | |
| 308 | 11 | |
| 777 | 8 | |
| 430 | 8 | |
| 725 | 11 | |
| 666 | 11 | |
| 84 | 9 | 0.0007 |
| 153 | 11 | |
| 546 | 9 | 0.0002 |
| 754 | 9 | 0.0130 |
| 851 | 8 | |
| 851 | 10 | 0.0072 |
| 773 | 9 | 0.0079 |
| 555 | 10 | |
| 529 | 8 | |
| 973 | 9 | 0.0021 |
| 296 | 8 | |
| 322 | 9 | 0.0140 |
| 129 | 10 | 0.0005 |
| 669 | 8 | |
| 669 | 9 | 0.7200 |
| 669 | 10 | 0.0160 |
| 55 | 10 | 0.0110 |

## Table XVII
## HER2/NEU A11 Motif Peptides with Binding Data

| Position | No. of Amino Acids | A*1101 |
|---|---|---|
| 825 | 11 | |
| 1223 | 8 | |
| 482 | 9 | |
| 739 | 9 | 0.0001 |
| 452 | 8 | |
| 888 | 9 | -0.0002 |
| 888 | 10 | 0.0016 |
| 888 | 11 | |
| 959 | 8 | |
| 959 | 10 | 0.0002 |
| 803 | 9 | |
| 343 | 9 | |
| 835 | 9 | |
| 835 | 10 | 0.0001 |
| 83 | 10 | 0.0013 |
| 772 | 10 | 0.0120 |
| 554 | 11 | |
| 1139 | 8 | |

138

<u>TableXVIII</u>
<u>HER2/NEU A24 Motif Peptides with Binding Data</u>

| Position | No. of Amino Acids | A*2401 |
|---|---|---|
| 1216 | 8 | 0.0039 |
| 730 | 9 | 0.0002 |
| 730 | 10 | 0.0010 |
| 730 | 11 | 0.0008 |
| 1212 | 9 | 0.0011 |
| 705 | 10 | 0.0002 |
| 705 | 11 | -0.0003 |
| 414 | 9 | 0.0041 |
| 440 | 9 | 0.1300 |
| 440 | 11 | 0.0230 |
| 475 | 8 | 0.0190 |
| 475 | 11 | 0.0003 |
| 826 | 11 | -0.0003 |
| 342 | 9 | 0.0180 |
| 162 | 8 | 0.0120 |
| 162 | 11 | 0.0016 |
| 863 | 8 | 0.0005 |
| 863 | 10 | 0.0002 |
| 363 | 8 | -0.0003 |
| 363 | 9 | 0.0003 |
| 876 | 11 | -0.0003 |
| 1022 | 9 | 0.0014 |
| 1022 | 10 | 0.0120 |
| 553 | 9 | 0.0061 |
| 1091 | 8 | -0.0003 |
| 1091 | 11 | -0.0003 |
| 832 | 10 | |
| 408 | 8 | 0.0044 |
| 257 | 10 | 0.0002 |
| 370 | 8 | 0.0120 |
| 172 | 8 | -0.0003 |
| 172 | 10 | 0.0022 |
| 954 | 8 | 0.0210 |
| 738 | 11 | 0.0027 |
| 887 | 8 | 0.0080 |
| 887 | 9 | 0.0150 |
| 684 | 8 | 0.0024 |
| 107 | 8 | 0.0006 |
| 107 | 11 | 0.0006 |
| 485 | 9 | 0.0002 |
| 485 | 10 | 0.0014 |
| 800 | 8 | 0.0076 |
| 410 | 10 | 0.0840 |
| 197 | 9 | 0.0011 |
| 922 | 10 | 0.0005 |
| 780 | 9 | 0.1700 |
| 780 | 11 | 0.0320 |
| 711 | 10 | |
| 968 | 9 | 0.0180 |
| 898 | 9 | 0.0110 |

## Table XVIII
### HER2/NEU A24 Motif Peptides with Binding Data

| Position | No. of Amino Acids | A*2401 |
|---|---|---|
| 985 | 10 | 0.0002 |
| 978 | 9 | 0.0032 |
| 8 | 8 | 0.0250 |
| 8 | 9 | 1.3000 |
| 1111 | 10 | 0.0120 |
| 281 | 11 | 0.0180 |
| 451 | 8 | -0.0003 |
| 451 | 11 | 0.0036 |
| 907 | 9 | 0.1200 |
| 834 | 8 | 0.0059 |
| 609 | 8 | 0.3200 |
| 917 | 10 | 0.0002 |
| 686 | 11 | -0.0003 |
| 63 | 9 | 0.0380 |
| 63 | 11 | 8.9000 |
| 399 | 8 | -0.0003 |
| 424 | 8 | -0.0003 |
| 905 | 9 | 0.0800 |
| 905 | 11 | 0.0920 |
| 951 | 9 | 0.1600 |
| 951 | 11 | 1.8000 |
| 888 | 8 | -0.0003 |
| 959 | 11 | 0.0011 |
| 952 | 8 | 0.0009 |
| 952 | 10 | 0.0019 |

## Table XIX
### HER2/NEU DR Super Motif Peptides with Binding Data

| Core Sequence | Exemplary Sequence | Position | DR1 | DR2w81 | DR2w2β2 | DR3 | DR4w4 | DR4w15 | DR5w11 | DR5w12 | SEQ ID NO. |
|---|---|---|---|---|---|---|---|---|---|---|---|
| YNYLSTDVG | ACPYNYLSTDVGSCT | 298 | | | | | | | | | 3720 |
| VLRENTSPK | AIKVLRENTSPKANK | 751 | | | | 0.0075 | | | | | 3721 |
| LQSLPTHDP | AKGLQSLPTHDPSPL | 1095 | | | | | | | | | 3722 |
| YDGIPAREI | AKPYDGIPAREIPDL | 920 | | | | | | | | | 3723 |
| LDIDETEYH | ARLLDIDETEYHADG | 867 | 0.0001 | -0.0006 | -0.0007 | 0.3100 | -0.0055 | | -0.0008 | | 3724 |
| VLVKSPNHV | ARNVLVKSPNHVKIT | 848 | | | | | | | | | 3725 |
| LTSHSAVV | ASPLTSHSAVVGIL | 648 | 0.0890 | 0.0950 | 0.0037 | 0.0010 | -0.0025 | | -0.0005 | | 3726 |
| LTLQGLGIS | AYSLTLQGLGISWLG | 440 | | | | | | | | | 3727 |
| MAGVGSPYV | AYVMAGVGSPYVSRL | 771 | | | | | | | | | 3728 |
| IFGSLAFLP | CKKIFGSLAFLPESF | 367 | 0.2400 | 0.0070 | 0.0016 | 0.0010 | -0.0025 | | -0.0005 | | 3729 |
| FNHSGICEL | CLHFNHSGICELHCP | 255 | | | | | | | | | 3730 |
| IAKGMSYLE | CMQIAKGMSYLEDVR | 826 | | | | | | | | | 3731 |
| LVTYNTDTF | CPALVTYNTDTFESM | 268 | | | | | | | | | 3732 |
| LTRTVCAGG | CQSLTRTVCAGGCAR | 212 | | | | | | | | | 3733 |
| LDDKGCPAE | CVDLDDKGCPAEQRA | 634 | | | | | | | | | 3734 |
| LGMEHLREV | CYGLGMEHLREVRAV | 342 | | | | 0.0083 | | | | | 3735 |
| YVMAGVGSP | DEAYVMAGVGSPYVS | 769 | 0.0500 | 0.0029 | 0.0240 | 0.0010 | 0.2300 | | 0.0027 | | 3736 |
| VGEGLACHQ | DECVGEGLACHQLCA | 502 | | | | | | | | | 3737 |
| LGMGAAKGL | DGDLGMGAAKGLQSL | 1087 | | | | | | | | | 3738 |
| VAPLTCSPQ | DGYVAPLTCSPQPEY | 1125 | | | | | | | | | 3739 |
| LGLEPSEEE | DLTLGLEPSEEEAPR | 1058 | | | | | -0.0025 | | | | 3740 |
| LRLPASPET | DMKLRLPASPETHLD | 30 | 0.0010 | | | | -0.0025 | | | | 3741 |
| FCVARCPSG | DPPFCVARCPSGVKP | 592 | | | | | | | | | 3742 |
| FYRSLLEDD | DSTFYRSLLEDDDMG | 1001 | | | | | | | | | 3743 |
| LVHRDLAAR | DVRLVHRDLAARNVL | 838 | | | | | | | | | 3744 |
| MIMVKCWMI | DVYMIMVKCWMIDSE | 950 | 0.0280 | 0.0047 | 0.0042 | 0.0010 | 0.0570 | | 0.0220 | | 3745 |
| VLQGLPREY | ECRVLQGLPREYVNA | 543 | | | | | | | | | 3746 |
| YALAVLDNG | EDNYALAVLDNGDPL | 109 | | | | | | | | | 3747 |
| LPAARPAGA | EGPLPAARPAGATLE | 1154 | | | | | | | | | 3748 |
| YTFGASCVT | EGRYTFGASCVTACP | 286 | | | | | | | | | 3749 |
| YLPTNASLS | ELTYLPTNASLSFLQ | 61 | | | | | | | | | 3750 |
| LRRRFTHQS | ESILRRRFTHQSDVW | 892 | | | | | | | | | 3751 |
| LRKVKVLGS | ETELRKVKVLGSGAF | 717 | 0.0160 | 0.0019 | 0.0052 | 0.0045 | 0.0350 | | 0.0061 | | 3752 |
| LVEPLTPSG | ETELVEPLTPSGAMP | 693 | 0.0060 | | | | -0.0025 | | | | 3753 |
| LVSEFSRMA | FRELVSEFSRMARDP | 969 | | | | 0.0710 | | | | | 3754 |
| IQNEDLGPA | FVVIQNEDLGPASPL | 986 | | | | -0.0025 | | | | | 3755 |
| LERPKTLSP | GATLERPKTLSPGKN | 1164 | | | | | | | | | 3756 |
| VVQGNLELT | GCQVVQGNLELTYLP | 52 | | | | | | | | | 3757 |
| LNNTTPVTG | GDPLNNTTPVTGASP | 120 | | | | | | | | | 3758 |
| LACHQLCAR | GEGLACHQLCARGHC | 506 | | | | | | | | | 3759 |
| VKIPVAIKV | GENVKIPVAIKVLRE | 743 | 0.0630 | 0.0047 | 0.0034 | 0.0098 | -0.0032 | | -0.0005 | | 3760 |
| LPQPPICTI | GERLPQPPICTIDVY | 938 | -0.0005 | | | | -0.0032 | | | | 3761 |
| VPIKWMALE | GGKVPIKWMALESIL | 881 | | | | | | | | | 3762 |
| LIQRNPQLC | GGVLIQRNPQLCYQD | 151 | | | | | | | | | 3763 |
| WGPGPTQCV | GHCWGPGPTQCVNCS | 518 | | | | | | | | | 3764 |
| WLGLRSLRE | GISWLGLRSLRELGS | 449 | | | | | | | | | 3765 |
| LIHHNTHLC | GLALIHHNTHLCFVH | 464 | | | | | | | | | 3766 |
| ISWLGLRSL | GLGISWLGLRSLREL | 447 | | | | | | | | | 3767 |
| LALLPTGAA | GLLLALLPTGAASTQ | 10 | | | | | | | | | 3768 |
| VFDGDLGMG | GSDVFDGDLGMGAAK | 1082 | | | | | | | | | 3769 |

**TableXIX**
**HER2/NEU DR Super Motif Peptides with Binding Data**

| Core Sequence | Exemplary Sequence | DR6w19 | DR7 | DR8w2 | DR9 | DRw53 | SEQ ID NO. |
|---|---|---|---|---|---|---|---|
| YNYLSTDVG | ACPYNYLSTDVGSCT | | | | | | 3720 |
| VLRENTSPK | AIKVLRENTSPKANK | | | | | | 3721 |
| LQSLPTHDP | AKGLQSLPTHDPSPL | | | | | | 3722 |
| YDGIPAREI | AKPYDGIPAREIPDL | | | | | | 3723 |
| LDIDETEYH | ARLLDIDETEYHADG | -0.0001 | -0.0017 | -0.0009 | | | 3724 |
| VLVKSPNHV | ARNVLVKSPNIIVKIT | | | | | | 3725 |
| LTSHSAVV | ASPLTSHSAVVGIL | 0.0480 | 0.0350 | -0.0004 | | | 3726 |
| LTLQGLGIS | AYSLTLQGLGISWLG | | | | | | 3727 |
| MAGVGSPYV | AYVMAGVGSPYYSRL | | | | | | 3728 |
| IFGSLAFLP | CKKIFGSLAFLPESF | 0.0034 | 0.0270 | -0.0004 | | | 3729 |
| FNHSGICEL | CLHFNHSGICELHCP | | | | | | 3730 |
| IAKGMSYLE | CMQIAKGMSYLEDVR | | | | | | 3731 |
| LVTYNTDTF | CPALVTYNTDTFESM | | | | | | 3732 |
| LTRTVCAGG | CQSLTRTVCAGGCAR | | | | | | 3733 |
| LDDKGCPAE | CVDLDDKGCPAEQRA | | | | | | 3734 |
| LGMEHLREV | CYGLGMEHLREVRAV | | | | | | 3735 |
| YVMAGVGSP | DEAYVMAGVGSPYVS | 0.0020 | 0.2000 | 0.0570 | | | 3736 |
| VGEGLACHQ | DECVGEGLACHQLCA | | | | | | 3737 |
| LGMGAAKGL | DGDLGMGAAKGLQSL | | | | | | 3738 |
| VAPLTCSPQ | DGYVAPLTCSPQPEY | | | | | | 3739 |
| LGLEPSEEE | DLTLGLEPSEEEAPR | | | | | | 3740 |
| LRLPASPET | DMKLRLPASPETHLD | | -0.0013 | | | | 3741 |
| FCVARCPSG | DPPFCVARCPSGVKP | | | | | | 3742 |
| FYRSLLEDD | DSTFYRSLLEDDDMG | | | | | | 3743 |
| LVHRDLAAR | DVRLVHRDLAARNVL | | | | | | 3744 |
| MIMVKCWMI | DVYMIMVKCWMIDSE | -0.0003 | 0.1300 | 0.0450 | | | 3745 |
| VLQGLPREY | ECRVLQGLPREYVNA | | | | | | 3746 |
| YALAVLDNG | EDNYALAVLDNGDPL | | | | | | 3747 |
| LPAARPAGA | EGPLPAARPAGATLE | | | | | | 3748 |
| YTFGASCVT | EGRYTFGASCVTACP | | | | | | 3749 |
| YLPTNASLS | ELTYLPTNASLSFLQ | | | | | | 3750 |
| LRRRFTHQS | ESILRRRFTHQSDVW | | | | | | 3751 |
| LRKVKVLGS | ETELRKVKVLGSGAF | 0.0014 | 0.0380 | 0.0250 | | | 3752 |
| LVEPLTPSG | ETELVEPLTPSGAMP | | -0.0013 | | | | 3753 |
| LVSEFSRMA | FRELVSEFSRMARDP | | | | | | 3754 |
| IQNEDLGPA | FVVIQNEDLGPASPL | | | | | | 3755 |
| LERPKTLSP | GATLERPKTLSPGKN | | | | | | 3756 |
| VVQGNLELT | GCQVVQGNLELTYLP | | | | | | 3757 |
| LNNTTPVTG | GDPLNNTTPVTGASP | | | | | | 3758 |
| LACHQLCAR | GEGLACHQLCARGHC | | | | | | 3759 |
| VKIPVAIKV | GENVKIPVAIKVLRE | 0.0004 | 0.0310 | 0.0010 | | | 3760 |
| LPQPPICTI | GERLPQPPICTIDVY | | -0.0011 | | | | 3761 |
| VPIKWMALE | GGKVPIKWMALESIL | | | | | | 3762 |
| LIQRNPQLC | GGVLIQRNPQLCYQD | | | | | | 3763 |
| WGPGPTQCV | GHCWGPGPTQCVNCS | | | | | | 3764 |
| WLGLRSLRE | GISWLGLRSLRELGS | | | | | | 3765 |
| LHHHNTHLC | GLALHHHNTHLCFVH | | | | | | 3766 |
| ISWLGLRSL | GLGISWLGLRSLREL | | | | | | 3767 |
| LALLPPGAA | GLLLALLPPGAASTQ | | | | | | 3768 |
| VFDGDLGMG | GSDVFDGDLGMGAAK | | | | | | 3769 |

141

| Core Sequence | Exemplary Sequence | Position | DR1 | DR2w01 | DR2w2β2 | DR3 | DR4w4 | DR4w15 | DR5w11 | DR5w12 | SEQ ID NO. |
|---|---|---|---|---|---|---|---|---|---|---|---|
| YVSRLLGIC | GSPYVSRLLGICLTS | 778 | | | | | | | | | 3770 |
| MKLRLPASP | GTDMKLRLPASPETH | 28 | 0.0010 | | | | -0.0025 | | | | 3771 |
| YKGIWIPDG | GTVYKGIWIPDGENV | 732 | | | | | | | | | 3772 |
| VWELMTFGA | GVTVWELMTFGAKPY | 909 | 1.4000 | | | | 0.0330 | | | | 3773 |
| YISAWPDSL | GYLYISAWPDSLPDL | 408 | | | | | | | | | 3774 |
| FVHTVPWDQ | HLCFVHTVPWDQLFR | 473 | | | | | | | | | 3775 |
| VRQVPLQRL | HNQVRQVPLQRLRIV | 88 | | | | | | | | | 3776 |
| LAARNVLVK | HRDLAARNVLVKSPN | 843 | | | | | | | | | 3777 |
| ICELHCPAL | HSGICELHCPALVTY | 260 | | | | | | | | | 3778 |
| TDFGLARL | HVKITDFGLARLLDI | 858 | | | | | | | | | 3779 |
| LHCPALVTY | ICELHCPALVTYNTD | 263 | | | | | | | | | 3780 |
| IDVYMIMVK | ICTIDVYMIMVKCWM | 946 | | | | | | | | | 3781 |
| LRENTSPKA | IKVLRENTSPKANKE | 752 | -0.0005 | | | | -0.0032 | | | | 3782 |
| MALESILRR | IKWMALESILRRRFT | 886 | 0.9500 | | | | 0.0400 | | | | 3783 |
| VVVLGVVFG | ILLVVVLGVVFGILI | 661 | | | | | | | | | 3784 |
| VQGYVLIAH | IQEVQGYVLIAHNQV | 77 | | | | | | | | | 3785 |
| YTMRRLLQE | IRKYTMRRLLQETEL | 682 | | | | | | | | | 3786 |
| VVGILLVVV | ISAVVGILLVVVLGV | 655 | | | | | | | | | 3787 |
| WPDSLPDLS | ISAWPDSLPDLSVFQ | 412 | | | | | | | | | 3788 |
| LGLRSLREL | ISWLGLRSLRELGSG | 450 | | | | | | | | | 3789 |
| FGLARLLDI | ITDFGLARLLDIDET | 861 | 0.0048 | | | | -0.0032 | | | | 3790 |
| YLYISAWPD | ITGYLYISAWPDSLP | 406 | | | | | | | | | 3791 |
| MIDSECRPR | KCWMIDSECRPRFRE | 957 | | | | | | | | | 3792 |
| FAFGGAVEN | KDVFAFGGAVENPEY | 1182 | | | | | | | | | 3793 |
| LDEAYVMAG | KEILDEAYVMAGVGS | 765 | 0.0036 | | | | 0.0073 | | | | 3794 |
| LPTDCCHEQ | KGPLPTDCCHEQCAA | 228 | | | | -0.0027 | | | | | 3795 |
| VAIKVLREN | KIPVAIKVLRENTSP | 747 | | | | | | | | | 3796 |
| LSYMPIWKF | KPDLSYMPIWKFPDE | 605 | 0.0330 | | | | -0.0025 | | | | 3797 |
| VLGSGAFGT | KVKVLGSGAFGTVYK | 722 | | | | | | | | | 3798 |
| IKWMALESI | KVPIKWMALESILRR | 883 | 2.2000 | 2.7000 | 2.1000 | 0.0620 | 0.0690 | | 0.0073 | | 3799 |
| LCRWGLLLA | LAALCRWGLLLALLP | 3 | | | | | | | | | 3800 |
| LHFNHSGIC | LACLHFNHSGICELH | 253 | | | | | | | | | 3801 |
| LPPGAASTQ | LALLPPGAASTQVCT | 13 | | | | | | | | | 3802 |
| LDNGDPLNN | LAVLDNGDPLNNTTP | 114 | | | | | | | | | 3803 |
| WGLLLALLP | LCRWGLLLALLPGA | 6 | 0.0940 | | | | -0.0025 | | | | 3804 |
| VFGILIKRR | LGVVFGILIKRRQQK | 667 | | | | | | | | | 3805 |
| LLPPGAAST | LLALLPPGAASTQVC | 12 | | | | | | | | | 3806 |
| ICLTSTVQL | LLGICLTSTVQLVTQ | 785 | | | | | | | | | 3807 |
| WCMQIAKGM | LLNWCMQIAKGMSYL | 822 | 0.8400 | 0.0057 | 1.2000 | 0.0093 | 0.0011 | | 0.4000 | | 3808 |
| VVLGVVFGI | LLVVVLGVVFGILIK | 662 | -0.0008 | | | | -0.0025 | | | | 3809 |
| LGISWLGLR | LQGLGISWLGLRSLR | 445 | | | | | | | | | 3810 |
| LPREYVNAR | LQGLPREYVNARHCL | 547 | | | | -0.0027 | | | | | 3811 |
| YSEDPTVPL | LQRYSEDPTVPLPSE | 1109 | | | | 0.0270 | | | | | 3812 |
| LPTHDPSPL | LQSLPTHDPSPLQRY | 1098 | | | | | | | | | 3813 |
| IRGRILIING | LQVIRGRILIINGAYS | 428 | | | | | | | | | 3814 |
| LGSGLALIH | LRELGSGLALIHHINT | 458 | 0.0310 | | | | -0.0025 | | | | 3815 |
| LQLRSLTEI | LRELQLRSLTEILKG | 137 | | | | | | | | | 3816 |
| VRAVTSANI | LREVRAVTSANIQEF | 350 | | | | | | | | | 3817 |
| VRGTQLFED | LRIVRGTQLFEDNYA | 99 | | | | | | | | | 3818 |
| VKVLGSGAF | LRKVKVLGSGAFGTV | 720 | | | | | | | | | 3819 |

EP 1 568 373 A2

## Table XIX
### HER2/NEU DR Super Motif Peptides with Binding Data

| Core Sequence | Exemplary Sequence | DR6w19 | DR7 | DR8w2 | DR9 | DRw53 | SEQ ID NO. |
|---|---|---|---|---|---|---|---|
| YVSRLLGIC | GSPYVSRLLGICLTS | | | | | | 3770 |
| MKLRLPASP | GTDMKLRLPASPETH | | -0.0013 | | | | 3771 |
| YKGIWIPDG | GTVYKGIWIPDGENV | | | | | | 3772 |
| VWELMTFGA | GVTVWELMTFGAKPY | | 0.0170 | | | | 3773 |
| YISAWPDSL | GVLYISAWPDSLPDL | | | | | | 3774 |
| FVIITVPWDQ | HLCFVIITVPWDQLFR | | | | | | 3775 |
| VRQVPLQRL | HNQVRQVPLQRLRIV | | | | | | 3776 |
| LAARNVLVK | HRDLAARNVLVKSPN | | | | | | 3777 |
| ICELHCPAL | HSGICELHCPALVTY | | | | | | 3778 |
| ITDFGLARL | HVKITDFGLARLLDI | | | | | | 3779 |
| LHCPALVTY | ICELHCPALVTYNTD | | | | | | 3780 |
| IDVYMIMVK | ICTIDVYMIMVKCWM | | | | | | 3781 |
| LRENTSPKA | IKVLRENTSPKANKE | | -0.0011 | | | | 3782 |
| MALESILRR | IKWMALESILRRRFT | | 0.0040 | | | | 3783 |
| VVVLGVVFG | ILLVVVLGVVFGIEI | | | | | | 3784 |
| VQGYVLIAH | IQEVQGYVLIAHNQV | | | | | | 3785 |
| YTMRRLLQE | IRKYTMRRLLQETEL | | | | | | 3786 |
| VVGILLVVV | ISAVVGILLVVVLGV | | | | | | 3787 |
| WPDSLPDLS | ISAWPDSLPDLSVFQ | | | | | | 3788 |
| LGLRSLREL | ISWLGLRSLRELGSG | | | | | | 3789 |
| FGLARLLDI | ITDFGLARLLDIDET | | -0.0011 | | | | 3790 |
| YLYISAWPD | ITGYLYISAWPDSLP | | | | | | 3791 |
| MIDSECRPR | KCWMIDSECRPRFRE | | | | | | 3792 |
| FAFGGAVEN | KDVFAFGGAVENPEY | | | | | | 3793 |
| LDEAYVMAG | KEILDEAYVMAGVGS | | -0.0011 | | | | 3794 |
| LPTDCCHEQ | KGPLPTDCCHEQCAA | | | | | | 3795 |
| VAIKVLREN | KIPVAIKVLRENTSP | | | | | | 3796 |
| LSYMPIWKF | KPDLSYMPIWKFPDE | | 0.0029 | | | | 3797 |
| VLGSGAFGT | KVKVLGSGAFGTVYK | | | | | | 3798 |
| IKWMALESI | KVPIKWMALESILRR | 0.0031 | 0.0190 | 0.0079 | | | 3799 |
| LCRWGLLLA | LAALCRWGLLLALLP | | | | | | 3800 |
| LHFNHSGIC | LACLHFNHSGICELH | | | | | | 3801 |
| LPPGAASTQ | LALLPPGAASTQVCT | | | | | | 3802 |
| LDNGDPLNN | LAVLDNGDPLNNTTP | | | | | | 3803 |
| WGLLLALLP | LCRWGLLLALLPPGA | | 0.0021 | | | | 3804 |
| VFGILIKRR | LGVVFGILIKRRQQK | | | | | | 3805 |
| LLPPGAAST | LLALLPPGAASTQVC | | | | | | 3806 |
| ICLTSTVQL | LLGICLTSTVQLVTQ | | | | | | 3807 |
| WCMQIAKGM | LLNWCMQIAKGMSYL | 0.0390 | 0.1200 | 0.4100 | | | 3808 |
| VVLGVVFGI | LLVVVLGVVFGILIK | | 0.0019 | | | | 3809 |
| LGISWLGLR | LQGLGISWLGLRSLR | | | | | | 3810 |
| LPREYVNAR | LQGLPREYVNARHCL | | | | | | 3811 |
| YSEDPTVPL | LQRYSEDPTVPLPSE | | | | | | 3812 |
| LPTHDPSPL | LQSLPTHDPSPLQRY | | | | | | 3813 |
| IRGRILHNG | LQVIRGRILHNGAYS | | | | | | 3814 |
| LGSGLALHH | LRELGSGLALHHNT | | -0.0013 | | | | 3815 |
| LQLRSLTEI | LRELQLRSLTEILKG | | | | | | 3816 |
| VRAVTSANI | LREVRAVTSANIQEF | | | | | | 3817 |
| VRGTQLFED | LRIVRGTQLFEDNYA | | | | | | 3818 |
| VKVLGSGAF | LRKVKVLGSGAFGTV | | | | | | 3819 |

EP 1 568 373 A2

143

## Table XIX
### HER2/NEU DR Super Motif Peptides with Binding Data

| Core Sequence | Exemplary Sequence | Position | DR1 | DR2w01 | DR2w2B2 | DR3 | DR4w4 | DR4w15 | DR5w11 | DR5w12 | SEQ ID NO. |
|---|---|---|---|---|---|---|---|---|---|---|---|
| LRELGSGLA | LRSLRELGSGLALIH | 455 | | | | | | | | | 3820 |
| FQNLQVIRG | LSVFQNLQVIRGRIL | 422 | 0.1800 | 0.0280 | 0.0740 | 0.0010 | 0.0670 | | 0.0100 | | 3821 |
| ILKGGVLIQ | LTEILKGGVLIQRNP | 145 | | | | | | | | | 3822 |
| IDTNRSRAC | LTLIDTNRSRACHPC | 181 | | | | | | | | | 3823 |
| HSAVVGIL | LTSHSAVVGILLVV | 651 | 0.1900 | | | | -0.0025 | | | | 3824 |
| LPTNASLSF | LTYLPTNASLSFLQD | 62 | 0.4900 | 0.0100 | 0.0560 | 0.0150 | 0.3300 | | 0.0041 | | 3825 |
| WIQQDPPERG | LYYWDQDPPERGAPP | 1220 | | | | | | | | | 3826 |
| VGSPYVSRL | MAGVGSPYVSRLLGI | 774 | | | | | | | | | 3827 |
| LREVRAVTS | MEHLREVRAVTSANI | 347 | | | | | | | | | 3828 |
| VKCWMIDSE | MIMVKCWMIDSECRP | 953 | | | | | | | | | 3829 |
| LKETELRKV | MRILKETELRKVKVL | 712 | | | | | | | | | 3830 |
| LEDVRLVHR | MSYLEDVRLVHRDLA | 833 | | | | | | | | | 3831 |
| LGPASPLDS | NEDLGPASPLDSTFY | 991 | | | | | | | | | 3832 |
| VTCFGPEAD | NGSVTCFGPEADQCV | 571 | | | | | | | | | 3833 |
| VKDVFAFGG | NGVVKDVFAFGGAVE | 1178 | | | | | | | | | 3834 |
| LTYLPTNAS | NLELTYLPTNASLSF | 59 | 0.4700 | 0.0280 | 0.0090 | 0.0010 | 0.3800 | | 0.0050 | | 3835 |
| VIRGRILHN | NLQVIRGRILHNGAY | 427 | | | | | | | | | 3836 |
| YWDQDPPER | NLYYWDQDPPERGAP | 1219 | | | | | | | | | 3837 |
| LALTLIDTN | NNQLALTLIDTNRSR | 176 | | | | | | | | | 3838 |
| LCYQDTILW | NPQLCYQDTILWKDI | 158 | | | | | | | | | 3839 |
| LCFVHTVPW | NTHLCFVHTVPWDQL | 471 | | | | | | | | | 3840 |
| INCTHSCVD | PCPINCTHSCVDLDD | 625 | | | | | | | | | 3841 |
| LPDLSVFQN | PDSLPDLSVFQNLQV | 416 | | | | | | | | | 3842 |
| LQVFETLEE | PEQLQVFETLEETG | 394 | | | | | | | | | 3843 |
| FDGDPASNT | PESFDGDPASNTAPL | 378 | | | | | -0.0027 | | | | 3844 |
| VNQPDVRPQ | PEYVNQPDVRPQPPS | 1137 | | | | | | | | | 3845 |
| VARCPSGVK | PFCVARCPSGVKPDL | 594 | | | | | | | | | 3846 |
| LRELQLRSL | PGGLRELQLRSLTEI | 134 | | | | | | | | | 3847 |
| WMALESILR | PIKWMALESILRRRF | 885 | 0.7900 | | | | 0.0350 | | | | 3848 |
| VKPDLSYMP | PSGVKPDLSYMPIWK | 601 | | | | | -0.0027 | | | | 3849 |
| FKGTPTAEN | PSTFKGTPTAENPEY | 1234 | -0.0005 | | | | -0.0032 | | | | 3850 |
| YLSTDVGSC | PYNYLSTDVGSCTLV | 300 | | | | | | | | | 3851 |
| ILWKDIFHK | QDTILWKDIFHKNNQ | 164 | | | | | | | | | 3852 |
| VEECRVLQG | QECVEECRVLQGLPR | 538 | | | | | | | | | 3853 |
| FCPDPAPGA | QGFFCPDPAPGAGGM | 1028 | -0.0005 | | | | 0.0230 | | | | 3854 |
| LELTYLPTN | QGNLELTYLPTNASL | 57 | | | | | | | | | 3855 |
| LTLIDTNRS | QLALTLIDTNRSRAC | 178 | | | | | | | | | 3856 |
| YQDTILWKD | QLCYQDTILWKDIFH | 160 | | | | | | | | | 3857 |
| VRPQPPSPR | QPDVRPQPPSPREGP | 1142 | 0.0007 | | | | -0.0032 | | | | 3858 |
| ICTIDVYMI | QPPICTIDVYMIMVK | 943 | 0.0670 | 0.0540 | 0.0027 | 0.0976 | 0.0060 | | 0.0046 | | 3859 |
| FFCPDPAPG | QQGFFCPDPAPGAGG | 1027 | | | | | | | | | 3860 |
| IRKYTMRRL | QQKIRKYTMRRLLQE | 679 | | | | | | | | | 3861 |
| VWSYGVTVW | QSDVWSYGVTVWELM | 902 | | | | | | | | | 3862 |
| LQRLRIVRG | QVPLQRLRIVRGTQL | 93 | | | | | | | | | 3863 |
| VNARHCLPC | REYVNARHCLPCHPE | 552 | | | | | | | | | 3864 |
| ILHNGAYSL | RGIRILHNGAYSLTLQ | 432 | | | | | | | | | 3865 |
| LGSQDLLNW | RGRLGSQDLLNWCMQ | 814 | | | | | | | | | 3866 |
| YQGCQVVQG | RHLYQGCQVVQGNLE | 47 | | | | | | | | | 3867 |
| FRELVSEFS | RPRFRELVSEFSRMA | 966 | | | | | | | | | 3868 |
| LQETELVEP | RRLLQETELVEPLTP | 688 | | | | | | | | | 3869 |

## Table XIX.
## HER2/NEU DR Super Motif Peptides with Binding Data

| Core Sequence | Exemplary Sequence | DR6w19 | DR7 | DR8w2 | DR9 | DRw53 | SEQ ID NO. |
|---|---|---|---|---|---|---|---|
| LRELGSGLA | LRSLRELGSGLALII | | | | | | 3820 |
| FQNLQVIRG | LSVFQNLQVIRGRIL | 0.0057 | 0.2900 | 0.0330 | | | 3821 |
| ILKGGVLIQ | LTEILKGGVLIQRNP | | | | | | 3822 |
| IDTNRSRAC | LTLIDTNRSRACIIPC | | | | | | 3823 |
| IISAVVGIL | LTSIISAVVGILLVV | | 0.0049 | | | | 3824 |
| LPTNASLSF | LTYLPTNASLSFLQD | 0.0280 | 0.3200 | 0.0054 | | | 3825 |
| WDQDPPERG | LYYWDQDPPERGAPP | | | | | | 3826 |
| VGSPYVSRL | MAGVGSPYVSRLLGI | | | | | | 3827 |
| LREVRAVTS | MEILREVRAVTSANI | | | | | | 3828 |
| VKCWMIDSE | MIMVKCWMIDSECRP | | | | | | 3829 |
| LKETELRKV | MRILKETELRKVKVL | | | | | | 3830 |
| LEDVRLVIIR | MSYLEDVRLVIIRDLA | | | | | | 3831 |
| LGPASPLDS | NEDLGPASPLDSTFY | | | | | | 3832 |
| VTCFGPEAD | NGSVTCFGPEADQCV | | | | | | 3833 |
| VKDVFAFGG | NGVVKDVFAFGGAVE | | | | | | 3834 |
| LTYLPTNAS | NLELTYLPTNASLSF | 0.0017 | 0.0680 | 0.0220 | | | 3835 |
| VIRGRILIIN | NLQVIRGRILIINGAY | | | | | | 3836 |
| YWDQDPPER | NLYYWDQDPPERGAP | | | | | | 3837 |
| LALTLIDTN | NNQLALTLIDTNRSR | | | | | | 3838 |
| LCYQDTILW | NPQLCYQDTILWKDI | | | | | | 3839 |
| LCFVIITVPW | NTIILCFVIITVPWDQL | | | | | | 3840 |
| INCTIISCVD | PCPINCTIISCVDLDD | | | | | | 3841 |
| LPDLSVFQN | PDSLPDLSVFQNLQV | | | | | | 3842 |
| LQVFETLEE | PEQLQVFETLEEIITG | | | | | | 3843 |
| FDGDPASNT | PESFDGDPASNTAPL | | | | | | 3844 |
| VNQPDVRPQ | PEYVNQPDVRPQPPS | | | | | | 3845 |
| VARCPSGVK | PFCVARCPSGVKPDL | | | | | | 3846 |
| LRELQLRSL | PGGLRELQLRSLTEI | | | | | | 3847 |
| WMALESILR | PIKWMALESILRRRF | | 0.0078 | | | | 3848 |
| VKPDLSYMP | PSGVKPDLSYMPIWK | | | | | | 3849 |
| FKGTPTAEN | PSTFKGTPTAENPEY | | -0.0011 | | | | 3850 |
| YLSTDVGSC | PYNYLSTDVGSCTLV | | | | | | 3851 |
| ILWKDIFIIK | QDTILWKDIFIIKNNQ | | | | | | 3852 |
| VEECRVLQG | QECVEECRVLQGLPR | | | | | | 3853 |
| FCPDPAPGA | QGFFCPDPAPGAGGM | | -0.0011 | | | | 3854 |
| LELTYLPTN | QGNLELTYLPTNASL | | | | | | 3855 |
| LTLIDTNRS | QLALTLIDTNRSRAC | | | | | | 3856 |
| YQDTILWKD | QLCYQDTILWKDIFII | | | | | | 3857 |
| VRPQPPSPR | QPDVRPQPPSPREGP | | -0.0011 | | | | 3858 |
| ICTIDVYMI | QPPICTIDVYMIMVK | 0.0013 | 0.1000 | 0.0051 | | | 3859 |
| FFCPDPAPG | QQGFFCPDPAPGAGG | | | | | | 3860 |
| IRKYTMRRL | QQKIRKYTMRRLLQE | | | | | | 3861 |
| VWSYGVTVW | QSDVWSYGVTVWELM | | | | | | 3862 |
| LQRLRIVRG | QVPLQRLRIVRGTQL | | | | | | 3863 |
| VNARIICLPC | REYVNARIICLPCIIPE | | | | | | 3864 |
| ILIINGAYSL | RGRILIINGAYSLTLQ | | | | | | 3865 |
| LGSQDLLNW | RGRLGSQDLLNWCMQ | | | | | | 3866 |
| YQGCQVVQG | RIILYQGCQVVQGNLE | | | | | | 3867 |
| FRELVSEFS | RPRFRELVSEFSRMA | | | | | | 3868 |
| LQETELVEP | RRLLQETELVEPLTP | | | | | | 3869 |

145

## Table XIX:
## HER2/NEU DR Super Motif Peptides with Binding Data

| Core Sequence | Exemplary Sequence | Position | DR1 | DR2w01 | DR2w2B2 | DR3 | DR4w4 | DR4w15 | DR5w11 | DR5w12 | SEQ ID NO. |
|---|---|---|---|---|---|---|---|---|---|---|---|
| LEDDDMGDL | RSLLEDDDMGDLVDA | 1006 | | | | 0.0080 | | | | | 3870 |
| LLLALLPPG | RWGLLLALLPPGAAS | 8 | 12.0000 | 0.1300 | 0.0270 | 0.0010 | 0.2800 | | 0.0710 | | 3871 |
| FGASCVTAC | RYTFGASCVTACPYN | 288 | | | | | | | | | 3872 |
| VGILLVVVL | SAVVGILLVVVLGVV | 656 | | | | | | | | | 3873 |
| WSYGVTVWE | SDVWSYGVTVWELMT | 903 | | | | | | | | | 3874 |
| LQGLGISWL | SLTLQGLGISWLGLR | 442 | | | | | | | | | 3875 |
| LLNWCMQIA | SQDLLNWCMQIAKGM | 819 | | | | | | | | | 3876 |
| LRGQECVEE | SQFLRGQECVEECRV | 532 | | | | | | | | | 3877 |
| LGICLTSTV | SRLLGICLTSTVQLV | 783 | 0.3500 | 0.0220 | -0.0007 | 0.0062 | 0.1200 | | 0.0140 | | 3878 |
| VGSCTLVCP | STDVGSCTLVCPLIIN | 305 | | | | | | | | | 3879 |
| VTVWELMTF | SYGVTVWELMTFGAK | 907 | | | | | | | | | 3880 |
| LQPEQLQVF | TAPLQPEQLQVFETL | 389 | | | | 0.0023 | | | | | 3881 |
| YVAPLTCSP | TDGYVAPLTCSPQPE | 1124 | -0.0005 | | | | -0.0032 | | | | 3882 |
| LKGGVLIQR | TEILKGGVLIQRNPQ | 146 | | | | | | | | | 3883 |
| VEPLTPSGA | TELVEPLTPSGAMPN | 694 | | | | | | | | | 3884 |
| VYMIMVKCW | TIDVYMIMVKCWMID | 948 | | | | | | | | | 3885 |
| FEDNYALAV | TQLFEDNYALAVLDN | 105 | 0.0530 | | | | -0.0025 | | | | 3886 |
| MPYGCLLDI | TQLMPYGCLLDIIVRE | 798 | | | | | | | | | 3887 |
| VCAGGCARC | TRTVCAGGCARCKGP | 216 | | | | | | | | | 3888 |
| VTGASPGGL | TTPVTGASPGGLREL | 126 | | | | | | | | | 3889 |
| LVTQLMPYG | TVQLVTQLMPYGCLL | 793 | 0.2300 | 0.7500 | 0.0009 | 0.0010 | 0.0460 | | -0.0005 | | 3890 |
| LIINQEVTAE | VCPLIINQEVTAEDGT | 314 | -0.0004 | | | | -0.0025 | | | | 3891 |
| LTPSGAMPN | VEPLTPSGAMPNQAQ | 697 | -0.0008 | | | | -0.0025 | | | | 3892 |
| LLVVVLGVV | VGILLVVVLGVVFGI | 659 | | | | | | | | | 3893 |
| VPWDQLFRN | VHTVPWDQLFRNPHQ | 477 | | | | 0.0220 | | | | | 3894 |
| VVFGILIKR | VLGVVFGILIKRRQQ | 666 | 0.0700 | 0.0110 | 0.0620 | 0.0021 | 0.0029 | | 0.4700 | | 3895 |
| VTQLMPYGC | VQLVTQLMPYGCLLD | 794 | | | | | | | | | 3896 |
| VTSANIQEF | VRAVTSANIQEFAGC | 353 | | | | | | | | | 3897 |
| VHRDLAARN | VRLVHRDLAARNVLV | 839 | 0.0340 | 0.0064 | 0.0033 | 0.3400 | 0.0150 | | 0.2700 | | 3898 |
| VPLQRLRIV | VRQVPLQRLRIVRGT | 91 | | | | | | | | | 3899 |
| LLGICLTST | VSRLLGICLTSTVQL | 782 | | | | | | | | | 3900 |
| LMPYGCLLD | VTQLMPYGCLLDHVR | 797 | -0.0004 | | | | -0.0025 | | | | 3901 |
| ILLVVVLGV | VVGILLVVVLGVVFG | 658 | | | | | | | | | 3902 |
| LMTFGAKPY | VWELMTFGAKPYDGI | 912 | 0.0870 | 0.0990 | 0.1000 | 0.0010 | 0.0550 | | 0.0054 | | 3903 |
| LLALLPPGA | WGLLLALLPPGAAST | 9 | 5.1000 | 0.2100 | 0.0110 | 0.0013 | 1.3000 | | 0.2500 | | 3904 |
| IPAREIPDL | YDGIPAREIPDLLEK | 923 | | | | | | | | | 3905 |
| MVKCWMIDS | YMIMVKCWMIDSECR | 952 | | | | | | | | | 3906 |
| IAIINQVRQV | YVLIAIINQVRQVPLQ | 83 | | | | | | | | | 3907 |

146

## Table XIX
## HER2/NEU DR Super Motif Peptides with Binding Data

| Core Sequence | Exemplary Sequence | DR6w19 | DR7 | DR8w2 | DR9 | DRw53 | SEQ ID NO. |
|---|---|---|---|---|---|---|---|
| LEDDDMGDL | RSLLEDDDMGDLVDA | | | | | | 3870 |
| LLLALLPPG | RWGLLLALLPPGAAS | -0.0003 | -0.0013 | 0.1200 | | | 3871 |
| FGASCVTAC | RYTFGASCVTACPYN | | | | | | 3872 |
| VGILLVVVL | SAVVGILLVVVLGVV | | | | | | 3873 |
| WSYGVTVWE | SDVWSYGVTVWELMT | | | | | | 3874 |
| LQGLGISWL | SLTLQGLGISWLGLR | | | | | | 3875 |
| LLNWCMQIA | SQDLLNWCMQIAKGM | | | | | | 3876 |
| LRGQECVEE | SQFLRGQECVEECRV | | | | | | 3877 |
| LGICLTSTV | SRLLGICLTSTVQLV | 0.3400 | 0.5600 | 0.0009 | | | 3878 |
| VGSCTLVCP | STDVGSCTLVCPLIIN | | | | | | 3879 |
| VTVWELMTF | SYGVTVWELMTFGAK | | | | | | 3880 |
| LQPEQLQVF | TAPLQPEQLQVFETL | | | | | | 3881 |
| YVAPLTCSP | TDGYVAPLTCSPQPE | | -0.0011 | | | | 3882 |
| LKGGVLIQR | TEILKGGVLIQRNPQ | | | | | | 3883 |
| VEPLTPSGA | TELVEPLTPSGAMPN | | | | | | 3884 |
| VYMIMVKCW | TDVYMIMVKCWMID | | | | | | 3885 |
| FEDNYALAV | TQLFEDNYALAVLDN | | 0.0160 | | | | 3886 |
| MPYGCLLDH | TQLMPYGCLLDHVRE | | | | | | 3887 |
| VCAGGCARC | TRTVCAGGCARCKGP | | | | | | 3888 |
| VTGASPGGL | TTPVTGASPGGLREL | | | | | | 3889 |
| LVTQLMPYG | TVQLVTQLMPYGCLL | 0.0031 | 0.0100 | 0.0069 | | | 3890 |
| LHNQEVTAE | VCPLHNQEVTAEDGT | | -0.0013 | | | | 3891 |
| LTPSGAMPN | VEPLTPSGAMPNQAQ | | -0.0011 | | | | 3892 |
| LLVVVLGVV | VGILLVVVLGVVFGI | | | | | | 3893 |
| VPWDQLFRN | VHTVPWDQLFRNPHQ | | | | | | 3894 |
| VVFGILIKR | VLGVVFGILIKRRQQ | 0.0150 | 0.0320 | 0.6400 | | | 3895 |
| VTQLMPYGC | VQLVTQLMPYGCLLD | | | | | | 3896 |
| VTSANIQEF | VRAVTSANIQEFAGC | | | | | | 3897 |
| VHRDLAARN | VRLVHRDLAARNVLV | 0.0430 | 0.0230 | 0.1000 | | | 3898 |
| VPLQRLRIV | VRQVPLQRLRIVRGT | | | | | | 3899 |
| LLGICLTST | VSRLLGICLTSTVQL | | | | | | 3900 |
| LMPYGCLLD | VTQLMPYGCLLDHVR | | | | | | 3901 |
| ILLVVVLGV | VVGILLVVVLGVVFG | | -0.0013 | | | | 3902 |
| LMTFGAKPY | VWELMTFGAKPYDGI | 0.0004 | 0.0370 | 0.0089 | | | 3903 |
| LLALLPPGA | WGLLLALLPPGAAST | -0.0003 | -0.0013 | 0.4500 | | | 3904 |
| IPAREIPDL | YDGIPAREIPDLLEK | | | | | | 3905 |
| MVKCWMIDS | YMIMVKCWMIDSECR | | | | | | 3906 |
| IAHNQVRQV | YVLIAHNQVRQVPLQ | | | | | | 3907 |

147

Table XXa
HER2/NEU DR 3a Motif Peptides with Binding Data

| Core Sequence | Exemplary Sequence | Position | DR1 | DR2w2B1 | DR2w2B2 | DR3 | DR4w4 | DR4w15 | DR5w11 | DR5w12 | SEQ ID NO. |
|---|---|---|---|---|---|---|---|---|---|---|---|
| VLRENTSPK | AIKVLRENTSPKANK | 751 | 0.0001 | -0.0006 | -0.0007 | 0.0075 | -0.0055 | | -0.0008 | | 3908 |
| LDIDETEYH | ARLLDIDETEYHADG | 867 | | | | 0.3100 | | | | | 3909 |
| LGMEHLREV | CYGLGMEHLREVRAV | 342 | | | | 0.0083 | | | | | 3910 |
| LGLEPSEEE | DLTLGLEPSEEEAPR | 1058 | | | | -0.0025 | | | | | 3911 |
| YYWDQDPPE | DNLYYWDQDPPERGA | 1218 | | | | -0.0025 | | | | | 3912 |
| LWKDIFHKN | DTILWKDIFHKNNQL | 165 | | | | -0.0027 | | | | | 3913 |
| YHADGGKVP | ETEYHADGGKVPIKW | 874 | | | | -0.0027 | | | | | 3914 |
| LVSEFSRMA | FRELVSEFSRMARDP | 969 | | | | 0.0710 | | | | | 3915 |
| MARDPQRFV | FSRMARDPQRFVVIQ | 976 | | | | 0.1600 | | | | | 3916 |
| IQNEDLGPA | FVVIQNEDLGPASPL | 986 | | | | -0.0025 | | | | | 3917 |
| VDAEEYLVP | GDLVDAEEYLVPQQG | 1015 | | | | 0.0250 | | | | | 3918 |
| LFEDNYALA | GTQLFEDNYALAVLD | 104 | | | | 0.2200 | 0.0400 | | | | 3919 |
| MALESILRR | IKWMALESILRRRFT | 886 | 0.9500 | | | | | | | | 3920 |
| FPDEEGACQ | IWKFPDEEGACQPCP | 613 | | | | -0.0027 | | | | | 3921 |
| LPTDCCHEQ | KGPLPTDCCHEQCAA | 228 | | | | -0.0027 | | | | | 3922 |
| VVKDVFAFG | KNGVVKDVFAFGGAV | 1177 | | | | -0.0025 | | | | | 3923 |
| LPREYVNAR | LQGLPREYVNARIICL | 547 | | | | -0.0027 | | | | | 3924 |
| YSEDPTVPL | LQRYSEDPTVPLPSE | 1109 | | | | 0.0270 | | | | | 3925 |
| YNTDTFESM | LVTYNTDTFESMINP | 271 | | | | -0.0027 | | | | | 3926 |
| LLQETELVE | MRRLLQETELVEPLT | 687 | | | | 0.0047 | | | | | 3927 |
| ILDEAYVMA | NKEILDEAYVMAGVG | 764 | | | | -0.0027 | | | | | 3928 |
| VTAEDGTQR | NQFVTAEDGTQRCEK | 319 | | | | -0.0027 | | | | | 3929 |
| FDGDPASNT | PESFDGDPASNTAPL | 378 | | | | -0.0027 | | | | | 3930 |
| VKPDLSYMP | PSGVKPDLSYMPIWK | 601 | | | | -0.0027 | | | | | 3931 |
| FCPDPAPGA | QGFFCPDPAPGAGGM | 1028 | -0.0005 | | | 0.3200 | 0.0230 | | | | 3932 |
| ILKETELRK | QMRILKETELRKVKV | 711 | 0.0419 | 0.0150 | 0.5900 | 0.0080 | -0.0055 | | 0.0041 | | 3933 |
| LEDUDMGDL | RSLLEDDDMGDLVDA | 1006 | | | | -0.0025 | | | | | 3934 |
| FDGDLGMGA | SDVFDGDLGMGAAKG | 1083 | | | | -0.0027 | | | | | 3935 |
| FLPESFDGD | SLAFLPESFDGDPAS | 373 | | | | 0.0520 | | | | | 3936 |
| FLQDIQEVQ | SLSFLQDIQEVQGYV | 70 | | | | 0.0023 | | | | | 3937 |
| LQPEQLQVF | TAPLQPEQLQVFETL | 389 | | | | -0.0025 | | | | | 3938 |
| LPSETDGYV | TVPLPSETDGYVAPL | 1117 | | | | 0.0220 | | | | | 3939 |
| VPWDQLFRN | VHTVPWDQLFRNPHQ | 477 | 0.0340 | 0.0064 | 0.0033 | 0.3400 | 0.0150 | | 0.2700 | | 3940 |
| VHRDLAARN | VRLVHRDLAARNVLV | 839 | | | | -0.0027 | | | | | 3941 |
| FGPEADQCV | VTCFGPEADQCVACA | 574 | | | | 0.0059 | | | | | 3942 |
| LSTDVGSCT | YNYLSTDVGSCTLVC | 301 | | | | 0.0630 | | | | | 3943 |
| LEDDDMGD | YRSLLEDDDMGDLVD | 1005 | | | | | | | | | 3944 |

148

## Table XXa
## HER2/NEU DR 3a Motif Peptides with Binding Data

| Core Sequence | Exemplary Sequence | DR6w19 | DR7 | DR8w2 | DR9 | DRw53 | SEQ ID NO. |
|---|---|---|---|---|---|---|---|
| VLRENTSPK | AIKVLRENTSPKANK | | | | | | 3908 |
| LDIDETEYH | ARLLDIDETEYHADG | -0.0001 | -0.0017 | -0.0009 | | | 3909 |
| LGMEHLREV | CYGLGMEHLREVRAV | | | | | | 3910 |
| LGLEPSEEE | DLTLGLEPSEEEAPR | | | | | | 3911 |
| YYWDQDPPE | DNLYYWDQDPPERGA | | | | | | 3912 |
| LWKDIFHKN | DTILWKDIFHKNNQL | | | | | | 3913 |
| YHADGGKVP | ETEYHADGGKVPIKW | | | | | | 3914 |
| LVSEFSRMA | FRELVSEFSRMARDP | | | | | | 3915 |
| MARDPQRFV | FSRMARDPQRFVVIQ | | | | | | 3916 |
| IQNEDLGPA | FVVIQNEDLGPASPL | | | | | | 3917 |
| VDAEEYLVP | GDLVDAEEYLVPQQG | | | | | | 3918 |
| LFEDNYALA | GTQLFEDNYALAVLD | | | | | | 3919 |
| MALESILRR | IKWMALESILRRRFT | | 0.0040 | | | | 3920 |
| FPDEEGACQ | IWKFPDEEGACQPCP | | | | | | 3921 |
| LPTDCCHEQ | KGPLPTDCCHEQCAA | | | | | | 3922 |
| VVKDVFAFG | KNGVVKDVFAFGGAV | | | | | | 3923 |
| LPREYVNAR | LQGLPREYVNARIICL | | | | | | 3924 |
| YSEDPTVPL | LQRYSEDPTVPLPSE | | | | | | 3925 |
| YNTDTFESM | LVTYNTDTFESMPNP | | | | | | 3926 |
| LLQETELVE | MRRLLQETELVEPLT | | | | | | 3927 |
| ILDEAYVMA | NKEILDEAYVMAGVG | | | | | | 3928 |
| VTAEDGTQR | NQEVTAEDGTQRCEK | | | | | | 3929 |
| FDGDPASNT | PESFDGDPASNTAPL | | | | | | 3930 |
| VKPDLSYMP | PSGVKPDLSYMPIWK | | | | | | 3931 |
| FCPDPAPGA | QGFFCPDPAPGAGGM | | -0.0011 | | | | 3932 |
| ILKETELRK | QMRILKETELRKVKV | 0.0008 | 0.0130 | 0.0064 | | | 3933 |
| LEDDDMGDL | RSLLEDDDMGDLVDA | | | | | | 3934 |
| FDGDLGMGA | SDVFDGDLGMGAAKG | | | | | | 3935 |
| FLPESFDGD | SLAFLPESFDGDPAS | | | | | | 3936 |
| FLQDIQEVQ | SLSFLQDIQEVQGYV | | | | | | 3937 |
| LQFEQLQVF | TAPLQPEQLQVFETL | | | | | | 3938 |
| LPSETDGYV | TVPLPSETDGYVAPL | | | | | | 3939 |
| VPWDQLFRN | VHTVPWDQLFRNPHQ | | | | | | 3940 |
| VHRDLAARN | VRLVHRDLAARNVLV | 0.0430 | 0.0230 | 0.1000 | | | 3941 |
| FGPEADQCV | VTCFGPEADQCVACA | | | | | | 3942 |
| LSTDVGSCT | YNYLSTDVGSCTLVC | | | | | | 3943 |
| LLEDDDMGD | YRSLLEDDDMGDLVD | | | | | | 3944 |

EP 1 568 373 A2

150

### Table XXb
### HER2/NEU DR 3b Motif Peptides with Binding Data

| Core Sequence | Exemplary Sequence | Position | DR1 | DR2w2B1 | DR2w2B2 | DR3 | DR4w4 | DR4w15 | DR5w11 | DR5w12 | SEQ ID NO. |
|---|---|---|---|---|---|---|---|---|---|---|---|
| LIDTNRSRA | ALTLIDTNRSRACIIP | 180 | | | | 0.0350 | | | | | 3945 |
| IDSECRPRF | CWMIDSECRPRFREL | 958 | 0.0036 | -0.0006 | 0.0150 | 0.4500 | -0.0055 | | -0.0008 | | 3946 |
| YLEDVRLVH | GMSYLEDVRLVHRDL | 832 | | | | 0.1800 | | | | | 3947 |
| VDLDDKGCP | IISCVDLDDKGCPAEQ | 632 | | | | -0.0027 | | | | | 3948 |
| IIIINTHLCF | LALIIIINTHLCFVIIT | 465 | 0.0140 | 0.0990 | 0.0009 | 0.3100 | -0.0055 | | 0.0025 | | 3949 |
| AAPQPHPPP | QGGAAPQPHPPPAFS | 1200 | | | | -0.0025 | | | | | 3950 |
| ASPETHLDM | RLPASPETHLDMLRH | 34 | | | | -0.0027 | | | | | 3951 |
| AHNQVRQVP | VLIAHNQVRQVPLQR | 84 | | | | 0.0290 | | | | | 3952 |
| LFRNPHQAL | WDQLFRNPHQALLIIT | 482 | -0.0001 | 0.0015 | -0.0007 | 0.9000 | -0.0055 | | -0.0008 | | 3953 |

## Table XXb
### HER2/NEU DR 3b Motif Peptides with Binding Data

| Core Sequence | Exemplary Sequence | DR6w19 | DR7 | DR8w2 | DR9 | DRw53 | SEQ ID NO. |
|---|---|---|---|---|---|---|---|
| LIDTNRSRA | ALTLIDTNRSRACHP | | | | | | 3945 |
| IDSECRPRF | CWMIDSECRPRFREL | -0.0001 | -0.0014 | 0.0028 | | | 3946 |
| YLEDVRLVII | GMSYLEDVRLVHRDL | | | | | | 3947 |
| VDLDDKGCP | HSCVDLDDKGCPAEQ | | | | | | 3948 |
| HHHNTHLCF | LALHHHNTHLCFVHT | 0.7500 | 0.0200 | 0.0330 | | | 3949 |
| AAPOPHPPP | QGGAAPQPHPPPAFS | | | | | | 3950 |
| ASPETHLDM | RLPASPETHLDMLRH | | | | | | 3951 |
| AHNQVRQVP | VLIAHNQVRQVPLQR | | | | | | 3952 |
| LFRNPHOAL | WDQLFRNPHQALLHT | 0.0410 | -0.0017 | -0.0009 | | | 3953 |

TABLE XXI:

| Population coverage with combined HLA Supertypes | | | | | | |
|---|---|---|---|---|---|---|
| | PHENOTYPIC FREQUENCY | | | | | |
| HLA-SUPERTYPES | Caucasian | North American Black | Japanese | Chinese | Hispanic | Average |
| a. Individual Supertypes | | | | | | |
| A2 | 45.8 | 39.0 | 42.4 | 45.9 | 43.0 | 43.2 |
| A3 | 37.5 | 42.1 | 45.8 | 52.7 | 43.1 | 44.2 |
| B7 | 43.2 | 55.1 | 57.1 | 43.0 | 49.3 | 49.5 |
| A1 | 47.1 | 16.1 | 21.8 | 14.7 | 26.3 | 25.2 |
| A24 | 23.9 | 38.9 | 58.6 | 40.1 | 38.3 | 40.0 |
| B44 | 43.0 | 21.2 | 42.9 | 39.1 | 39.0 | 37.0 |
| B27 | 28.4 | 26.1 | 13.3 | 13.9 | 35.3 | 23.4 |
| B62 | 12.6 | 4.8 | 36.5 | 25.4 | 11.1 | 18.1 |
| B58 | 10.0 | 25.1 | 1.6 | 9.0 | 5.9 | 10.3 |
| b. Combined Supertypes | | | | | | |
| A2, A3, B7 | 84.3 | 86.8 | 89.5 | 89.8 | 86.8 | 87.4 |
| A2, A3, B7, A24, B44, A1 | 99.5 | 98.1 | 100.0 | 99.5 | 99.4 | 99.3 |
| A2, A3, B7, A24, B44, A1, B27, B62, B58 | 99.9 | 99.6 | 100.0 | 99.8 | 99.9 | 99.8 |

# Table XXII. Crossbinding data of A2 supermotif peptides

| Source | AA | Sequence | A*0201 nM | A*0202 nM | A*0203 nM | A*0206 nM | A*6802 nM | No. A2 Alleles Crossbound |
|---|---|---|---|---|---|---|---|---|
| Her2/neu.5 | 9 | ALCRWGLLL | 100 | -- | 278 | -- | -- | 2 |
| Her2/neu.5 | 10 | ALCRWGLLLA | 139 | 1955 | 12 | 1947 | 2500 | 2 |
| Her2/neu.48 | 9 | HLYQGCQVV | 139 | 307 | 13 | 514 | 1143 | 3 |
| Her2/neu.106 | 9 | QLFEDNYAL | 17 | 226 | 11 | 463 | 2105 | 4 |
| Her2/neu.106 | 10 | QLFEDNYALA | 357 | 662 | 9.1 | 218 | 74 | 4 |
| Her2/neu.144 | 10 | SLTEILKGGV | 238 | -- | 22 | -- | -- | 2 |
| Her2/neu.153 | 9 | VLIQRNPQL | 23 | 3909 | 3.3 | 1057 | -- | 2 |
| Her2/neu.369 | 9 | KIFGSLAFL | 36 | 9.0 | 19 | 23 | 3333 | 4 |
| Her2/neu.435 | 9 | ILHNGAYSL | 75 | 358 | 100 | 569 | -- | 3 |
| Her2/neu.466 | 9 | ALIHHNTHL | 278 | 1265 | 10 | 1762 | -- | 2 |
| Her2/neu.508 | 9 | GLACHQLCA | 417 | -- | 127 | -- | 9091 | 2 |
| Her2/neu.653 | 9 | SIISAVVGI | 69 | 524 | 35 | 285 | 148 | 4 |
| Her2/neu.665 | 9 | VVLGVVFGI | 14 | -- | 2500 | 430 | 2000 | 2 |
| Her2/neu.689 | 9 | RLLQETELV | 21 | -- | 625 | 34 | -- | 2 |
| Her2/neu.767 | 9 | ILDEAYVMA | 238 | -- | 4167 | 3083 | -- | 1 |
| Her2/neu.773 | 10 | VMAGVGSPYV | 200 | 391 | 13 | 3700 | -- | 3 |
| Her2/neu.789 | 9 | CLTSTVQLV | 208 | 457 | 6.7 | 308 | 8000 | 4 |
| Her2/neu.799 | 9 | QLMPYGCLL | 217 | 977 | 114 | 712 | -- | 2 |
| Her2/neu.952 | 10 | YMIMVKCWMI | 20 | 307 | 83 | 116 | 267 | 5 |
| Her2/neu.952 | 9 | YMIMVKCWM | 217 | -- | 625 | 2643 | 1000 | 1 |

-- indicates binding affinity =10,000nM.

EP 1 568 373 A2

## Table XXII. A2 supermotif analog peptides

| Source | AA | Sequence | A*0201 nM | A*0202 nM | A*0203 nM | A*0206 nM | A*6802 nM | No. A2 Alleles Crossbound |
|---|---|---|---|---|---|---|---|---|
| Her2/neu.5 | 9 | ALCRWGLLL | 100 | -- | 278 | -- | -- | 2 |
| Her2/neu.5.T2B3V9 | 9 | ATBRWGLLV | 16667 | 215 | 323 | 2177 | 1739 | 2 |
| Her2/neu.5V2B3V9 | 9 | AVBRWGLLV | 10000 | 215 | 141 | 2177 | 4706 | 2 |
| Her2/neu.5.B3 | 9 | ALBRWGLLL | 238 | 1 | 12 | 6167 | 7273 | 3 |
| Her2/neu.5B3V9 | 9 | ALBRWGLLV | 18 | 33 | 4.2 | 285 | -- | 4 |
| Her2/neu.5M2B3V9 | 9 | AMBRWGLLV | 36 | 473 | 16 | 726 | -- | 3 |
| Her2/neu.153 | 9 | VLIQRNPQL | 23 | 3909 | 3.3 | 1057 | -- | 2 |
| Her2/neu.153V9 | 9 | VLIQRNPQV | 55 | 768 | 135 | 385 | -- | 3 |
| Her2/neu.369 | 9 | KIFGSLAFL | 36 | 9.0 | 19 | 23 | 3333 | 4 |
| Her2/neu.369V2V9 | 9 | KVFGSLAFV | 20 | 19.0 | 769 | 15 | 29 | 4 |
| Her2/neu.369T2V9 | 9 | KTFGSLAFV | 35 | 13.0 | 1010 | 14 | 17 | 4 |
| Her2/neu.369L2V9 | 9 | KLFGSLAFV | 5.8 | 7.5 | 19 | 17 | 1270 | 4 |
| Her2/neu.653 | 9 | SIISAVVGI | 69 | 524 | 35 | 285 | 148 | 4 |
| Her2/neu.653.L2V9 | 9 | SLISAVVGV | 7.1 | 10 | 16 | 20 | 110 | 5 |
| Her2/neu.665 | 9 | VVLGVVFGI | 14 | -- | 2500 | 430 | 2000 | 2 |
| Her2/neu.665V2V9 | 9 | VVLGVVFGV | | | | | | |
| Her2/neu.665L2V9 | 9 | VLLGVVFGV | 2.4 | 17 | 14 | 6.0 | 8000 | 4 |
| Her2/neu.952 | 10 | YMIMVKCWMI | 20 | 307 | 83 | 116 | 267 | 5 |
| Her2/neu.952L2V10 | 10 | YLIMVKCWMV | 13 | 56 | 116 | 18 | 84 | 5 |
| Her2/neu.952L2B7V10 | 10 | YLIMVKBWMV | 7.2 | 66 | 77 | 11 | 851 | 4 |

-- indicates binding affinity =10,000nM.

EP 1 568 373 A2

Table XXIII. HLA-A3 Supermotif-bearing Peptides

| AA | Sequence | Source | A*0301 nM | A*1101 nM | A*3101 nM | A*3301 nM | A*6801 nM | No. of A3 Alleles Crossbound | CTL Wildtype | CTL Tumor | Published CTL Wildtype | Published CTL Tumor |
|---|---|---|---|---|---|---|---|---|---|---|---|---|
| 10 | QLRSLTEILK | Her2/neu.141 | 55 | 462 | 667 | 6170 | -- | 2 | | | | |
| 10 | ILKGGVLIQR | Her2/neu.148 | 275 | -- | 25 | 121 | 205 | 4 | | | | |
| 10 | IVKGGVLIQR | Her2/neu.148.V2 | 275 | 7500 | 72 | 126 | 29 | 4 | | | | |
| 10 | IVKGGVLIQK | Her2/neu.148.V2K10 | 26 | 102 | 450 | 6591 | 27 | 4 | | | | |
| 10 | TILWKDIFHK | Her2/neu.166 | 256 | 1.7 | 487 | 690 | 200 | 4 | | | | |
| 10 | TVLWKDIFHR | Her2/neu.166.V2R10 | 8462 | 286 | 600 | 76 | 42 | 3 | | | | |
| 9 | ILWKDIFHK | Her2/neu.167 | 39 | 19 | 82 | 967 | 1739 | 3 | | | | |
| 9 | IVWKDIFHK | Her2/neu.167.V2 | 23 | 40 | 247 | 853 | 178 | 4 | | | | |
| 9 | IVWKDIFHR | Her2/neu.167.V2R9 | 143 | 286 | 6.0 | 16 | 15 | 5 | | | | |
| 10 | RTVCAGGCAR | Her2/neu.217 | 1618 | 462 | 40 | 1318 | 320 | 3 | | | | |
| 9 | TVCAGGCAR | Her2/neu.218 | -- | 261 | 129 | 326 | 83 | 4 | | | | |
| 9 | TVBAGGBAR | Her2/neu.218.B3B7 | 314 | 111 | 247 | 242 | 8.0 | 5 | | | | |
| 9 | TVBAGGBAK | Her2/neu.218.B3B7K9 | 24 | 29 | -- | -- | 7.3 | 3 | | | | |
| 10 | IVWLGLRSLR | Her2/neu.450.V2 | 234 | 1936 | 11 | 193 | 7.3 | 4 | | | | |
| 10 | IVWLGLRSLK | Her2/neu.450.V2K10 | 3.9 | 128 | 273 | 2071 | 12 | 4 | | | | |
| 10 | ISWLGLRSLR | Her2/neu.450 | 268 | 2222 | 6.9 | 223 | 73 | 4 | | | | |
| 10 | HTVPWDQLFR | Her2/neu.478 | 3143 | 83 | 19 | 88 | 4.0 | 4 | | | | |
| 10 | HVVPWDQLFR | Her2/neu.478.V2 | 7333 | 1333 | 391 | 193 | 3.6 | 3 | | | | |
| 10 | HVVPWDQLFK | Her2/neu.478.V2K10 | 180 | 375 | -- | -- | 8.9 | 3 | | | | |
| 9 | CVNCSQFLR | Her2/neu.528 | 7333 | 194 | 34 | 50 | 18 | 4 | | | | |
| 9 | BVNBSQFLR | Her2/neu.528.B1B4 | 177 | 80 | 38 | 58 | 10 | 5 | | | | |
| 9 | BVNBSQFLK | Her2/neu.528.B1B4K9 | 34 | 22 | 60 | 4265 | 15 | 4 | | | | |
| 10 | GVVFGILIKR | Her2/neu.668 | 611 | 182 | 305 | 2071 | 19 | 3 | | | | |
| 9 | VVFGILIKR | Her2/neu.669 | 100 | 8.3 | 13 | 78 | 4.0 | 5 | 1/3 | 0/1 | | |
| 10 | VVFGILIKRR | Her2/neu.669 | 3667 | 375 | 290 | 193 | 15 | 4 | | | | |

-- indicates binding affinity >10,000nM.

EP 1 568 373 A2

## Table XXIII. HLA-A3 Supermotif-bearing Peptides

| AA | Sequence | Source | A*0301 nM | A*1101 nM | A*3101 nM | A*3301 nM | A*6801 nM | No. of A3 Alleles Crossbound | CTL Wildtype | CTL Tumor | Published CTL Wildtype | Published CTL Tumor |
|---|---|---|---|---|---|---|---|---|---|---|---|---|
| 9 | VVFGILIKK | Her2/neu.669.K9 | 22 | 19 | 3750 | -- | 35 | 3 | | | | |
| 9 | KIRKYTMRR | Her2/neu.681 | 15 | -- | 16 | 4028 | -- | 2 | 3/3 | 2/3 | + [1] | + |
| 9 | VLRENTSPK | Her2/neu.754 | 28 | 462 | 129 | 290 | -- | 4 | 4/7 | 3/6 | + [1] | + |
| 9 | VVRENTSPR | Her2/neu.754.V2R9 | 200 | 5455 | 375 | 126 | 178 | 4 | | | | |
| 9 | LLDHVRENR | Her2/neu.806 | 297 | -- | 500 | 326 | 5714 | 3 | | | | |
| 9 | LVARNVLVK | Her2/neu.846.V2 | 42 | 214 | 9000 | -- | 205 | 3 | | | | |
| 9 | LAARNVLVK | Her2/neu.846 | 190 | 211 | -- | -- | 500 | 3 | | | | |
| 9 | LVKSPNHVK | Her2/neu.852 | 23 | 86 | 182 | 784 | 73 | 4 | | | + [1] | |
| 9 | LVKSPNHVR | Her2/neu.852.R9 | 7857 | -- | 198 | 107 | 50 | 3 | | | | |
| 9 | KITDFGLAR | Her2/neu.860 | 65 | 25 | 100 | -- | 1633 | 3 | | | | |
| 9 | KVTDFGLAR | Her2/neu.860.V2 | 201 | 76 | 106 | -- | 133 | 4 | | | | |
| 9 | MVLESILRR | Her2/neu.889.V2 | 216 | 273 | 207 | 153 | 22 | 5 | | | | |
| 9 | MVLESILRK | Her2/neu.889.V2K9 | 61 | 16 | -- | 2636 | 381 | 3 | | | | |
| 9 | MALESILRR | Her2/neu.889 | 3235 | 253 | 192 | 132 | 127 | 4 | | | | |
| 10 | LVSEFSRMAR | Her2/neu.972 | 1528 | 182 | 49 | 126 | 36 | 4 | | | | |
| 10 | LVSEFSRMAK | Her2/neu.972.K10 | 250 | 71 | 2250 | 5273 | 62 | 3 | | | | |
| 10 | AVPLDSTFYR | Her2/neu.997.V2 | -110000 | 88 | 30000 | 2636 | 73 | 2 | | | | |
| 10 | AVPLDSTFYK | Her2/neU.997.V2K10 | 550 | 33 | 1500 | 22308 | 229 | 2 | | | | |
| 10 | ASPLDSTFYR | Her2/neu.997 | -- | 90 | 150 | 2071 | 154 | 3 | | | | |

1) Kawashima et al., Cancer Research 59:431, 1999

-- indicates binding affinity >10,000nM.

EP 1 568 373 A2

### Table XXIV. B7 Supermotif Peptides

| AA | Sequence | Source | B*0702 nM | B*3501 nM | B*5101 nM | B*5301 nM | B*5401 nM | No. of B7 Alleles Crossbound |
|---|---|---|---|---|---|---|---|---|
| 9 | LPTNASLSF | Her2/neu.65 | 212 | 114 | 809 | 34 | -- | 3 |
| 10 | LPTNASLSFL | Her2/neu.65 | 290 | -- | 2500 | -- | -- | 1 |
| 9 | FPTNASLSF | Her2/neu.65.F1 | 1.1 | 7.9 | 85 | 6.2 | 556 | 4 |
| 9 | FPTNASLSI | Her2/neu.65.F1I9 | 4.6 | 300 | 8.7 | 14 | 2.8 | 5 |
| 10 | FPTNASLSFI | Her2/neu.65.F1I10 | 120 | 424 | 23 | 85 | 53 | 5 |
| 10 | FPTNASLSFL | Her2/neu.65.F1 | 18 | 200 | 262 | 172 | 119 | 5 |
| 9 | FPLNNTTPI | Her2/neu.121.F1I9 | 1.6 | 200 | 13 | 52 | 1.6 | 5 |
| 9 | FPLNNTTPV | Her2/neu.121.F1 | 0.20 | 40 | 7.1 | 1069 | 1.1 | 4 |
| 10 | SPGGLRELQL | Her2/neu.133 | 95 | -- | -- | -- | -- | 1 |
| 8 | SPGGLREL | Her2/neu.133 | 100 | -- | -- | -- | -- | 1 |
| 10 | FPGGLRELQI | Her2/neu.133.F1I10 | 306 | -- | 1774 | 310 | 476 | 3 |
| 10 | FPMCKGSRCI | Her2/neu.196.F1 | 183 | -- | 500 | 6643 | 303 | 3 |
| 10 | MPNPEGRYTI | Her2/neu.282.I10 | 34 | 111 | 13 | 9.3 | 46 | 5 |
| 10 | FPNPEGRYTI | Her2/neu.282.F1 | 324 | 24 | 9.3 | 6.6 | 3.2 | 5 |
| 10 | CPLHNQEVTI | Her2/neu.315.I10 | 458 | -- | 42 | 274 | 556 | 3 |
| 10 | KPCARVCYGL | Her2/neu.336 | 149 | -- | -- | -- | -- | 1 |
| 10 | FPCARVCYGL | Her2/neu.336.F1 | 190 | 450 | 1000 | 1691 | 36 | 3 |
| 8 | WPDSLPDI | Her2/neu.415.I8 | 71 | -- | 5000 | -- | -- | 1 |
| 10 | FPHQALLHTA | Her2/neu.488.F1 | 393 | 379 | 4231 | -- | 1.3 | 3 |
| 10 | FPHQALLHTI | Her2/neu.488.F1I10 | 86 | 655 | 19 | 42 | 4.0 | 4 |
| 11 | CPSGVKPDLSY | Her2/neu.600 | 183 | 3600 | -- | -- | -- | 1 |
| 11 | CPSGVKPDLSI | Her2/neu.600.I11 | 290 | -- | 138 | 344 | 625 | 3 |
| 9 | FPSGVKPDI | Her2/neu.600.F1I9 | 6.3 | 9000 | 204 | 930 | 44 | 3 |
| 11 | FPSGVKPDLSI | Her2/neu.600.F1I11 | 196 | 3273 | 153 | 211 | 208 | 4 |
| 9 | KPDLSYMPI | Her2/neu.605 | 76 | -- | 2500 | -- | -- | 1 |
| 9 | FPDLSYMPI | Her2/neu.605.F1 | 22 | 167 | 10 | 72 | 31 | 5 |

## Table XXIV. B7 Supermotif Peptides

| AA | Sequence | Source | B*0702 nM | B*3501 nM | B*5101 nM | B*5301 nM | B*5401 nM | No. of B7 Alleles Crossbound |
|----|----------|--------|-----------|-----------|-----------|-----------|-----------|------------------------------|
| 10 | CPAEQRASPL | Her2/neu.642 | 37 | -- | -- | -- | -- | 1 |
| 10 | FPAEQRASPI | Her2/neu.642.F1I10 | 3.1 | -- | 98 | 4895 | 56 | 3 |
| 10 | FPAEQRASPL | Her2/neu.642.F1 | 1.4 | 248 | 859 | 8455 | 286 | 3 |
| 10 | SPLTSIISAV | Her2/neu.649 | 61 | -- | -- | -- | 667 | 1 |
| 9 | SPLTSIISI | Her2/neu.649.I9 | 86 | -- | 275 | 3444 | 217 | 3 |
| 9 | FPLTSIISA | Her2/neu.649.F1 | 290 | 21 | 663 | 3000 | 1.4 | 3 |
| 9 | FPLTSIISI | Her2/neu.649.F1I9 | 212 | 118 | 13 | 28 | 1.5 | 5 |
| 10 | FPLTSIISAI | Her2/neu.649.F1I10 | 229 | 327 | 26 | 194 | 3.3 | 5 |
| 10 | FPLTSIISAV | Her2/neu.649.F1 | 220 | 300 | 63 | 2906 | 1.2 | 4 |
| 11 | FPLTSIISAVI | Her2/neu.649.F1I11 | 367 | 96 | 4.6 | 66 | 2.2 | 5 |
| 9 | FPLTPSGAI | Her2/neu.698.F1I9 | 0.90 | 2057 | 9.2 | 1632 | 1.9 | 3 |
| 9 | FPLTPSGAM | Her2/neu.698.F1 | 2.0 | 16 | 71 | 1525 | 91 | 4 |
| 10 | FPSGAMPNQI | Her2/neu.701.F1 | 229 | 6546 | 131 | 775 | 100 | 3 |
| 11 | MPNQAQMRILI | Her2/neu.706.I11 | 344 | 1800 | 37 | 12 | 303 | 4 |
| 9 | FPNQAQMRI | Her2/neu.706.F1 | 68 | 108 | 12 | 18 | 4.5 | 5 |
| 10 | FPNQAQMRII | Her2/neu.706.F1I10 | 290 | 514 | 76 | 490 | 59 | 4 |
| 10 | FPNQAQMRIL | Her2/neu.706.F1 | 81 | 200 | 458 | 443 | 31 | 5 |
| 8 | FPKANKEI | Her2/neu.760F1 | 0.16 | -- | 2500 | -- | 3125 | 1 |
| 9 | FPKANKEII | Her2/neu.760.F1I9 | 8.5 | -- | 55 | 7154 | 39 | 3 |
| 9 | FPKANKEIL | Her2/neu.760.F1 | 6.7 | 4500 | 190 | -- | 77 | 3 |
| 8 | FPYVSRLL | Her2/neu.779.F1 | 61 | 600 | 6.9 | 581 | 172 | 3 |
| 10 | FPYVSRLLGI | Her2/neu.779.F1 | 112 | 3600 | 7.9 | 358 | 9.1 | 4 |
| 11 | MPYGCLLDHVI | Her2/neu.801.I11 | 66 | 4.2 | 2.4 | 1.9 | 7.7 | 5 |
| 8 | FPIKWMAI | Her2/neu.884.F1I8 | 22 | 1143 | 122 | 930 | 33 | 3 |
| 8 | FPIKWMAL | Her2/neu.884.F1 | 0.60 | 248 | 306 | 547 | 40 | 4 |
| 8 | KPYDGIPA | Her2/neu.921 | 367 | -- | -- | 490 | 29 | 3 |

EP 1 568 373 A2

## Table XXIV. B7 Supermotif Peptides

| AA | Sequence | Source | B*0702 nM | B*3501 nM | B*5101 nM | B*5301 nM | B*5401 nM | No. of B7 Alleles Crossbound |
|---|---|---|---|---|---|---|---|---|
| 8 | KPYDGIPI | Her2/neu.921.I8 | 115 | -- | 74 | 5167 | 303 | 3 |
| 8 | FPYDGIPA | Her2/neu.921.F1 | 423 | 206 | 157 | 6200 | 1.0 | 4 |
| 8 | FPYDGIPI | Her2/neu.921.F1I8 | 177 | 379 | 10 | 344 | 56 | 5 |
| 11 | FPYDGIPAREI | Her2/neu.921.F1I11 | 141 | 2880 | 50 | 465 | 15 | 4 |
| 9 | LPQPPICTI | Her2/neu.941 | 196 | -- | 4.2 | 28 | .19 | 4 |
| 9 | FPQPPICTI | Her2/neu.941.F1 | 20 | 360 | 17 | 11 | 1.7 | 5 |
| 11 | FPRFRELVSEI | Her2/neu.966.F1I11 | 229 | 240 | 167 | 127 | 28 | 5 |
| 10 | FPASPLDSTF | Her2/neu.995.F1 | 3.7 | 10 | 579 | 10 | 200 | 4 |
| 10 | FPASPLDSTI | Her2/neu.995.F1I10 | 20 | 514 | 20 | 30 | 14 | 5 |
| 11 | FPLDSTFYRSI | Her2/neu.998.F1I11 | 229 | 2667 | 46 | 620 | 2.4 | 3 |
| 11 | FPLDSTFYRSL | Her2/neu.998.F1 | 42 | 400 | 324 | 1192 | 2.7 | 4 |
| 9 | FPLAPSEGI | Her2/neu.1073.F1I9 | 100 | 554 | 204 | 239 | 5.9 | 4 |
| 9 | FPTHDPSPI | Her2/neu.1101.F1I9 | 108 | 600 | 56 | 274 | 50 | 4 |
| 9 | FPTHDPSPL | Her2/neu.1101.F1 | 10 | 72 | 3438 | 1632 | 208 | 3 |
| 9 | FPSETDGYI | Her2/neu.1120.F1I9 | 220 | 655 | 31 | 37 | 156 | 4 |
| 9 | FPSETDGYV | Her2/neu.1120.F1 | 204 | 809 | 32 | 517 | 26 | 3 |
| 11 | PPSPREGPLPI | Her2/neu.1149.I11 | 22 | -- | 423 | 85 | -- | 3 |
| 11 | FPSPREGPLPI | Her2/neu.1149.F1I11 | 190 | -- | 262 | 5167 | 263 | 3 |
| 9 | FPREGPLPI | Her2/neu.1151.F1I9 | 0.30 | -- | 14 | 2818 | 4.0 | 3 |
| 10 | FPREGPLPAI | Her2/neu.1151.F1I10 | 20 | 7200 | 20 | 620 | 3.4 | 3 |
| 9 | FPLPAARPA | Her2/neu.1155.F1 | 34 | 277 | 1897 | -- | 1.5 | 3 |
| 9 | FPLPAARPI | Her2/neu.1155.F1I9 | 6.5 | 600 | 7.1 | 282 | 3.8 | 4 |
| 9 | FPAARPAGI | Her2/neu.1157.F1I9 | 9.3 | -- | 131 | 4227 | 48 | 3 |
| 11 | FPAARPAGATI | Her2/neu.1157.F1I11 | 39 | 4235 | 13 | 332 | 50 | 4 |
| 11 | FPAARPAGATL | Her2/neu.1157.F1 | 3.9 | 360 | 239 | 2906 | 133 | 4 |
| 8 | FPGKNGVI | Her2/neu.1174.F1I8 | 458 | -- | 177 | -- | 385 | 3 |

EP 1 568 373 A2

**Table XXIV. B7 Supermotif Peptides**

| AA | Sequence | Source | B*0702 nM | B*3501 nM | B*5101 nM | B*5301 nM | B*5401 nM | No. of B7 Alleles Crossbound |
|---|---|---|---|---|---|---|---|---|
| 8 | FPGKNGVV | Her2/neu.1174.F1 | 80 | -- | 393 | 9300 | 35 | 3 |
| 9 | FPQPHPPPA | Her2/neu.1204.F1 | 104 | 360 | 3667 | -- | 1.9 | 3 |
| 9 | FPQPHPPPI | Her2/neu.1204.F1I9 | 75 | 655 | 15 | 300 | 12 | 4 |
| 10 | FPQPHPPPAF | Her2/neu.1204.F1 | 212 | 9.4 | 290 | 19 | 24 | 5 |
| 10 | FPQPHPPPAI | Her2/neu.1204.F1I10 | 500 | 343 | 34 | 72 | 5.0 | 5 |
| 9 | FPPPAFSPA | Her2/neu.1208.F1 | 46 | 514 | 183 | -- | 1.2 | 4 |
| 9 | FPPPAFSPI | Her2/neu.1208.F1I9 | 13 | 4800 | 7.5 | 3577 | 3.7 | 3 |
| 10 | FPPPAFSPAF | Her2/neu.1208.F1 | 275 | 120 | 344 | 5167 | 9.1 | 4 |
| 9 | SPAFDNLYI | Her2/neu.1214 | 290 | -- | 229 | 155 | -- | 3 |

160

Table XXV:

| HLA-A1 Motif-Bearing Peptides | | | |
|---|---|---|---|
| AA | Sequence | Source | A*0101 nM |
| 11 | GTDMKLRLPY | Her2/neu.28.Y10 | 50 |
| 11 | ETHLDMLRHLY | Her2/neu.40 | 89 |
| 9 | HLDMLRHLY | Her2/neu.42 | 2.7 |
| 9 | HTDMLRHLY | Her2/neu.42.T2 | 1.9 |
| 9 | GTQLFEDNY | Her2/neu.104 | 139 |
| 9 | GTDLFEDNY | Her2/neu.104.D3 | 0.90 |
| 10 | PTDCCHEQCA | Her2/neu.232 | 125 |
| 10 | PTDCCHEQCY | Her2/neu.232.Y10 | 46 |
| 11 | PTDCCHEQCAA | Her2/neu.232 | 58 |
| 11 | PTDCCHEQCAY | Her2/neu.232.Y11 | 18 |
| 10 | ESMPNPEGRY | Her2/neu.280 | 139 |
| 10 | ETMPNPEGRY | Her2/neu.280.T2 | 3.9 |
| 9 | ASCVTACPY | Her2/neu.293 | 455 |
| 11 | ASCVTACPYNY | Her2/neu.293 | 132 |
| 9 | ATCVTACPY | Her2/neu.293.T2 | 49 |
| 8 | VTACPYNY | Her2/neu.296 | 250 |
| 11 | VFETLEEITGY | Her2/neu.399 | 5556 |
| 11 | ETLEEITGYLY | Her2/neu.401 | 57 |
| 9 | ETDEEITGY | Her2/neu.401.D3 | 17 |
| 10 | TLEEITGYLY | Her2/neu.402 | 23 |
| 10 | TLDEITGYLY | Her2/neu.402.D3 | 3.4 |
| 11 | EADQCVACAHY | Her2/neu.580 | 250 |
| 9 | VMDGVGSPY | Her2/neu.773.D3 | 40 |
| 10 | CMQIAKGMSY | Her2/neu.826 | 83 |
| 10 | CTQIAKGMSY | Her2/neu.826.T2 | 19 |
| 9 | LLDIDETEY | Her2/neu.869 | 3.3 |
| 9 | LTDIDETEY | Her2/neu.869.T2 | 5.7 |
| 10 | FTHQSDVWSY | Her2/neu.899 | 9.3 |
| 10 | FTDQSDVWSY | Her2/neu.899.D3 | 0.60 |
| 10 | PADPLDSTFY | Her2/neu.996.D3 | 19 |
| 9 | ATPLDSTFY | Her2/neu.997.T2 | 36 |
| 10 | MTDLVDAEEY | Her2/neu.1014.T2 | 2.3 |
| 9 | LTCSPQPEY | Her2/neu.1131 | 192 |
| 9 | LTDSPQPEY | Her2/neu.1131.D3 | 32 |
| 10 | FSPAFDNLYY | Her2/neu.1213 | 4.5 |
| 10 | FTPAFDNLYY | Her2/neu.1213.T2 | 0.80 |
| 9 | SPDFDNLYY | Her2/neu1214.D3 | 73.50 |
| 10 | GTPTAENPEY | Her2/neu.1239 | 397 |
| 10 | GTDTAENPEY | Her2/neu.1239.D3 | 26 |

Table XXVI:

| HLA-A24 Motif-Bearing Peptides | | | |
|---|---|---|---|
| AA | Sequence | Source | A*2402 nM |
| 8 | RWGLLLAL | Her2/neu.8 | 480 |
| 9 | RWGLLLALL | Her2/neu.8 | 9.2 |
| 9 | RYGLLALF | Her2/neu8.Y2F9 | 1.3 |

Table XXVI: (continued)

| HLA-A24 Motif-Bearing Peptides | | | |
|---|---|---|---|
| AA | Sequence | Source | A*2402 nM |
| 9 | TYLPTNASL | Her2/neu.63 | 316 |
| 11 | TYLPTNASLSF | Her2/neu.63 | 1.3 |
| 9 | TYLPTNASF | Her2/neu.63.F9 | 44 |
| 9 | CYGLGMEHF | Her2/neu.342.F9 | 164 |
| 10 | LYISAWPDSL | Her2/neu.410 | 143 |
| 10 | LYISAWPDSF | Her2/neu,410.F10 | 10 |
| 9 | AYPDSLPDF | Herw/neu414.Y2F9 | 24 |
| 9 | AYSLTLQGL | Her2/neu.440 | 92 |
| 9 | AYSLTLQGF | Her2/neu.440.F9 | 52 |
| 9 | EYVNARHCF | Her2/neu.553.F9 | 150 |
| 8 | SYMPIWKF | Her2/neu.609 | 38 |
| 9 | PYVSRLLGI | Her2/neu.780 | 71 |
| 11 | PYVSRLLGICL | Her2/neu.780 | 375 |
| 9 | PYVSRLLGF | Her2/neu.780.F9 | 9.2 |
| 10 | GYSYLEDVRF | Her2/neu.832.Y2F10 | 235.0 |
| 9 | KYMALESIF | Her2/neu.887.Y2F9 | 19.0 |
| 9 | RYTHQSDVF | Her2/neu.898.Y2F9 | 60.0 |
| 9 | VWSYGVTVW | Her2/neu.905 | 150 |
| 9 | VYSYGVTVF | Her2/neu.905.Y2F9 | 16 |
| 11 | VWSYGVTVWEL | Her2/neu.905 | 130 |
| 9 | SYGVTVWEL | Her2/neu.907 | 100 |
| 9 | SYGVTVWEF | Her2/neu.907.F9 | 26 |
| 9 | VYMIMVKCW | Her2/neu.951 | 75 |
| 11 | VYMIMVKCWMI | Her2/neu.951 | 6.7 |
| 9 | VYMIMVKCF | Her2/neu.951.F9 | 19 |
| 9 | RYRELVSEF | Her2/neu.968.Y2 | 36 |
| 9 | RYARDPQRF | Her2/neu.978.Y2 | 120 |

## Table XXVII. HLA-A2 Supermotif-bearing Peptides

| AA | Sequence | Source | A*0201 nM | A*0202 nM | A*0203 nM | A*0206 nM | A*6802 nM | No. of A2 Alleles Crossbound | CTL Wildtype [1] | CTL Tumor [1] | CTL Wildtype [2] | CTL Tumor [2] |
|---|---|---|---|---|---|---|---|---|---|---|---|---|
| 9 | ALCRWGLLL | Her2/neu.5 | 100 | -- | 278 | -- | -- | 2 | 2/2 | 2/2 | | |
| 9 | ALBRWGLLV | Her2/neu.5.B3V9 | 18 | 33 | 4.2 | 285 | -- | 4 | | | | |
| 9 | HLYQGCQVV | Her2/neu.48 | 139 | 307 | 13 | 514 | 1143 | 3 | 1/2 | 0/2 | 2/2 | 1/2 |
| 9 | VLIQRNPQL | Her2/neu.153 | 23 | 3909 | 3.3 | 1057 | -- | 2 | | | | |
| 9 | VLIQRNPQV | Her2/neu.153.V9 | 55 | 768 | 135 | 385 | -- | 3 | | | | |
| 9 | KIFGSLAFL | Her2/neu.369 | 36 | 9.0 | 19 | 23 | 3333 | 4 | 6/7 | 4/7 | | |
| 9 | KLFGSLAFV | Her2/neu.369.L2V9 | 5.8 | 7.5 | 19 | 17 | 1270 | 4 | | | | |
| 9 | KVFGSLAFV | Her2/neu.369.V2V9 | 20 | 19 | 769 | 15 | 29 | 4 | | | | |
| 9 | KTFGSLAFV | Her2/neu.369.T2V9 | 35 | 13 | 1010 | 14 | 17 | 4 | | | | |
| 10 | RILHNGAYSL | Her2/neu 434 | 278 | 1000 | 6667 | 1276 | -- | 1 | | | | |
| 9 | ILHNGAYSL | Her2/neu.435 | 75 | 358 | 100 | 569 | -- | 3 | 3/3 | 1/3 | 2/2 | 2/2 |
| 9 | SLISAVVGV | Her2/neu.653.L2V9 | 7.1 | 10 | 16 | 20 | 110 | 5 | | | | |
| 9 | VVLGVVFGI | Her2/neu.665 | 14 | -- | 2500 | 430 | 2000 | 2 | | | | |
| 9 | VLLGVVFGV | Her2/neu.665.L2V9 | 2.4 | 19 | 14 | 6.0 | 8000 | 4 | | | | |
| 9 | RLLQETELV | Her2/neu.689 | 21 | -- | 625 | 34 | -- | 2 | | | | |
| 10 | VMAGVGSPYV | Her2/neu.773 | 200 | 391 | 13 | 3700 | -- | 3 | 1/2 | 0/2 | 1/2 | 1/2 |
| 9 | CLTSTVQLV | Her2/neu.789 | 208 | 457 | 6.7 | 308 | 8000 | 4 | 1/4 | 0/4 | 1/2 | |
| 10 | YMIMVKCWMI | Her2/neu.952 | 20 | 307 | 83 | 116 | 267 | 5 | 0/1 | 0/1 | 2/2 | 2/2 |
| 10 | YLIMVKCWMV | Her2/neu.952.L2V10 | 13 | 56 | 116 | 18 | 84 | 5 | | | | |

1. Number of donors yielding a positive response / total tested.

2. Data from ovarian cancer patients.

Table XXVIII:

| Her2/neu DR supertype primary binding | | | | | | |
|---|---|---|---|---|---|---|
| DR147 Algo Sum | Sequence | Source | DR1 nM nM | DR4w4 | DR7 nM | DR147 Cross-binding |
| 2 | LCRWGLLLALLPPGA | Her2/neu.6 | 53 | -- | -- | 1 |
| 2 | RWGLLLALLPPGAAS | Her2/neu.8 | 0.42 | 161 | -- | 2 |
| 2 | WGLLLALLPPGAAST | Her2/neu.9 | 0.98 | 35 | -- | 2 |
| 2 | GTDMKLRLPASPETH | Her2/neu.28 | 5000 | -- | -- | 0 |
| 2 | DMKLRLPASPETHLD | Her2/neu.30 | 5000 | -- | -- | 0 |
| 2 | NLELTYLPTNASLSF | Her2/neu.59 | 11 | 118 | 368 | 3 |
| 3 | LTYLPTNASLSFLQD | Her2/neu.62 | 10 | 136 | 78 | 3 |
| 2 | TQLFEDNYALAVLDN | Her2/neu.105 | 94 | -- | 1563 | 1 |
| 2 | VCPLHNQEVTAEDGT | Her2/neu.314 | -- | -- | -- | 0 |
| 2 | CKKIFGSLAFLPESF | Her2/neu.367 | 21 | -- | 926 | 2 |
| 2 | LSVFQNLQVIRGRIL | Her2/neu.422 | 28 | 672 | 86 | 3 |
| 2 | LRELGSGLALIHHNT | Her2/neu.458 | 161 | -- | -- | 1 |
| 3 | KPDLSYMPIWKFPDE | Her2/neu.605 | 152 | -- | 8621 | 1 |
| 3 | ASPLTSIISAVVGIL | Her2/neu.648 | 56 | -- | 714 | 2 |
| 2 | LTSIISAVVGILLVV | Her2/neu.651 | 26 | -- | 5102 | 1 |
| 3 | VVGILLVVVLGVVFG | Her2/neu.658 | -- | -- | -- | 0 |
| 3 | LLVVVLGVVFGILIK | Her2/neu.662 | >6250 | -- | -- | 0 |
| 2 | VLGVVFGILIKRRQQ | Her2/neu.666 | 71 | -- | 781 | 2 |
| 2 | ETELVEPLTPSGAMP | Her2/neu.693 | 833 | -- | -- | 1 |
| 2 | VEPLTPSGAMPNQAQ | Her2/neu.697 | >6250 | -- | -- | 0 |
| 2 | ETELRKVKVLGSGAF | Her2/neu.717 | 313 | 1286 | 658 | 2 |
| 2 | GENVKIPVAIKVLRE | Her2/neu.743 | 79 | -- | 807 | 2 |
| 2 | IKVLRENTSPKANKE | Her2/neu.752 | -- | -- | -- | 0 |
| 3 | KEILDEAYVMAGVGS | Her2/neu.765 | -- | 6164 | -- | 0 |
| 3 | DEAYVMAGVGSPYVS | Her2/neu.769 | 100 | 196 | 125 | 3 |
| 2 | SRLLGICLTSTVQLV | Her2/neu.783 | 14 | 375 | 45 | 3 |
| 2 | TVQLVTQLMPYGCLL | Her2/neu.793 | 22 | 978 | 2500 | 2 |
| 3 | LLNWCMQIAKGMSYL | Her2/neu.822 | 6.0 | -- | 208 | 2 |
| 2 | ITDFGLARLLDIDET | Her2/neu.861 | 1042 | -- | -- | 0 |
| 3 | KVPIKWMALESILRR | Her2/neu.883 | 2.3 | 652 | 1316 | 2 |
| 3 | PIKWMALESILRRRF | Her2/neu.885 | 6.3 | 1286 | 3205 | 1 |
| 2 | IKWMALESILRRRFT | Her2/neu.886 | 5.3 | 1125 | 6250 | 1 |
| 2 | GVTVWELMTFGAKPY | Her2/neu.909 | 3.6 | 1364 | 1471 | 1 |
| 3 | VWELMTFGAKPYDGI | Her2/neu.912 | 58 | 818 | 676 | 3 |
| 2 | GERLPQPPICTIDVY | Her2/neu.938 | -- | -- | -- | 0 |
| 2 | QPPICTIDVYMIMVK | Her2/neu.943 | 75 | 7500 | 250 | 2 |
| 2 | DVYMIMVKCWMIDSE | Her2/neu.950 | 179 | 790 | 192 | 3 |
| 2 | QGFFCPDPAPGAGGM | Her2/neu. 1028 | -- | 1957 | -- | 0 |
| 3 | TDGYVAPLTCSPQPE | Her2/neu. 1124 | -- | -- | -- | 0 |
| 2 | QPDVRPQPPSPREGP | Her2/neu. 1142 | 7143 | -- | -- | 0 |
| 2 | PSTFKGTPTAENPEY | Her2/neu. 1234 | -- | -- | -- | 0 |

## Table XXIX. DR supertype crossbinding

| Sequence | Source | DR1 nM | DR4w4 nM | DR7 nM | DR2w2ß1 nM | DR2w2ß2 nM | DR6w19 nM | DR5w11 nM | DR8w2 nM | DR147 Binding | Broad Binding (5/8) |
|---|---|---|---|---|---|---|---|---|---|---|---|
| RWGLLLALLPPGAAS | Her2/neu.8 | 0.40 | 161 | -- | 70 | 741 | -- | 282 | 408 | 2 | 6 |
| WGLLLALLPPGAAST | Her2/neu.9 | 1.0 | 35 | -- | 43 | 1818 | -- | 80 | 109 | 2 | 5 |
| NLELTYLPTNASLSF | Her2/neu.59 | 11 | 118 | 368 | 325 | 2222 | 2059 | 4000 | 2227 | 3 | 4 |
| LTYLPTNASLSFLQD | Her2/neu.62 | 10 | 136 | 78 | 910 | 357 | 125 | 4878 | 9074 | 3 | 6 |
| CKKIFGSLAFLPESF | Her2/neu.367 | 21 | -- | 926 | 1300 | -- | 1029 | -- | -- | 2 | 2 |
| LSVFQNLQVIRGRIL | Her2/neu.422 | 28 | 672 | 86 | 325 | 270 | 614 | 2000 | 1485 | 3 | 6 |
| ASPLTSIISAVVGIL | Her2/neu.648 | 56 | -- | 714 | 96 | 5405 | 73 | -- | -- | 2 | 4 |
| VLGVVFGILIKRRQQ | Her2/neu.666 | 71 | -- | 781 | 827 | 323 | 233 | 43 | 77 | 2 | 7 |
| ETELRKVKVLGSGAF | Her2/neu.717 | 313 | 1286 | 658 | 4790 | 3846 | 2500 | 3279 | 1960 | 2 | 2 |
| GENVKIPVAIKVLRE | Her2/neu.743 | 79 | -- | 807 | 1936 | 5882 | 8750 | -- | -- | 2 | 2 |
| DEAYVMAGVGSPYVS | Her2/neu.769 | 100 | 196 | 125 | 3138 | 833 | 1750 | 7407 | 860 | 3 | 5 |
| SRLLGICLTSTVQLV | Her2/neu.783 | 14 | 375 | 45 | 414 | -- | 10 | 1429 | -- | 3 | 5 |
| TVQLVTQLMPYGCLL | Her2/neu.793 | 22 | 978 | 2500 | 12 | -- | 1129 | -- | 7101 | 2 | 3 |
| LLNWCMQIAKGMSYL | Her2/neu.822 | 6.0 | -- | 208 | 1597 | 17 | 90 | 50 | 120 | 2 | 6 |
| KVPIKWMALESILRR | Her2/neu.883 | 2.3 | 652 | 1316 | 3.4 | 9.5 | 1129 | 2740 | 6203 | 2 | 4 |
| VWELMTFGAKPYDGI | Her2/neu.912 | 58 | 818 | 676 | 92 | 200 | 8750 | 3704 | 5506 | 3 | 5 |
| QPPICTIDVYMIMVK | Her2/neu.943 | 75 | 7500 | 250 | 169 | 7407 | 2692 | 4348 | 9608 | 2 | 3 |
| DVYMIMVKCWMIDSE | Her2/neu.950 | 179 | 790 | 192 | 1936 | 4762 | -- | 909 | 1089 | 3 | 4 |

Table XXX:

| DR3 binding | | |
|---|---|---|
| Sequence | Source | DR3 nM |
| RLPASPETHLDMLRH | Her2/neu.34 | -- |
| SLSFLQDIQEVQGYV | Her2/neu.70 | 5769 |
| VLIAHNQVRQVPLQR | Her2/neu.84 | -- |
| GTQLFEDNYALAVLD | Her2/neu.104 | 1364 |
| DTILWKDIFHKNNQL | Her2/neu.165 | -- |
| ALTLIDTNRSRACHP | Her2/neu.180 | 8571 |
| KGPLPTDCCHEQCAA | Her2/neu.228 | -- |
| LVTYNTDTFESMPNP | Her2/neu.271 | -- |
| YNYLSTDVGSCTLVC | Her2/neu.301 | -- |
| NQEVTAEDGTQRCEK | Her2/neu.319 | -- |
| CYGLGMEHLREVRAV | Her2/neu.342 | -- |
| SLAFLPESFDGDPAS | Her2/neu.373 | -- |
| PESFDGDPASNTAPL | Her2/neu.378 | -- |
| TAPLQPEQLQVFETL | Her2/neu.389 | -- |
| LALIHHNTHLCFVHT | Her2/neu.465 | 968 |
| VHTVPWDQLFRNPHQ | Her2/neu.477 | -- |
| WDQLFRNPHQALLHT | Her2/neu.482 | 333 |
| LQGLPREYVNARHCL | Her2/neu.547 | -- |
| VTCFGPEADQCVACA | Her2/neu.574 | -- |
| PSGVKPDLSYMPIWK | Her2/neu.601 | -- |
| IWKFPDEEGACQPCP | Her2/neu.613 | -- |
| HSCVDLDDKGCPAEQ | Her2/neu.632 | -- |
| MRRLLQETELVEPLT | Her2/neu.687 | -- |
| QMRILKETELRKVKV | Her2/neu.711 | 938 |
| AIKVLRENTSPKANK | Her2/neu.751 | -- |
| NKEILDEAYVMAGVG | Her2/neu.764 | -- |
| GMSYLEDVRLVHRDL | Her2/neu.832 | 1667 |
| VRLVHRDLAARNVLV | Her2/neu.839 | 882 |
| ARLLDIDETEYHADG | Her2/neu.867 | 968 |
| ETEYHADGGKVPIKW | Her2/neu.874 | -- |
| IKWMALESILRRRFT | Her2/neu.886 | 682 |
| CWMIDSECRPRFREL | Her2/neu.958 | 667 |
| FRELVSEFSRMARDP | Her2/neu.969 | 4225 |
| FSRMARDPQRFVVIQ | Her2/neu.976 | 1875 |
| FVVIQNEDLGPASPL | Her2/neu.986 | -- |
| YRSLLEDDDMGDLVD | Her2/neu.1005 | 4762 |
| RSLLEDDDMGDLVDA | Her2/neu.1006 | -- |
| GDLVDAEEYLVPQQG | Her2/neu.1015 | -- |
| QGFFCPDPAPGAGGM | Her2/neu.1028 | -- |
| DLTLGLEPSEEEAPR | Her2/neu.1058 | -- |
| SDVFDGDLGMGAAKG | Her2/neu.1083 | -- |
| LQRYSEDPTVPLPSE | Ner2/neu. 1109 | -- |
| TVPLPSETDGYVAPL | Her2/neu. 1117 | -- |
| KNGVVKDVFAFGGAV | Her2/neu.1177 | -- |
| QGGAAPQPHPPPAFS | Her2/neu.1200 | -- |
| DNLYYWDQDPPERGA | Her2/neu.1218 | -- |

**TABLE XXXI. HLA Class II Supermotif and Motif-Bearing Epitopes**

| Sequence | Source | DRB1 *0101 nM | DRB1 *0301 nM | DRB1 *0401 nM | DRB1 *0701 nM | DRB1 *0802 nM | DRB1 *1101 nM | DRB1 *1302 nM | DRB1 *1501 nM | DRB5 *0101 nM | No. of DR Alleles Crossbound |
|---|---|---|---|---|---|---|---|---|---|---|---|
| VLGVVFGILIKRRQQ | Her2/neu.666 | 71 | -- | -- | 781 | 77 | 43 | 233 | 827 | 323 | 7 |
| QMRILKETELRKVKV | Her2/neu.711 | 119 | 938 | >8182 | 1923 | 7656 | 4878 | 4375 | 607 | 34 | 3 |
| DEAYVMAGVGSPYVS | Her2/neu.769 | 100 | -- | 196 | 125 | 860 | 7407 | 1750 | 3138 | 833 | 5 |
| SRLLGICLTSTVQLV | Her2/neu.783 | 14 | -- | 375 | 45 | -- | 1429 | 10 | 414 | -- | 5 |
| LLNWCMQIAKGMSYL | Her2/neu.822 | 6.0 | -- | -- | 208 | 120 | 50 | 90 | 1597 | 17 | 6 |
| VRLVHRDLAARNVLV | Her2/neu.839 | 147 | 882 | 3058 | 1087 | 490 | 74 | 81 | 1422 | 6061 | 4 |
| ARLLDIDETEYHADG | Her2/neu.867 | -- | 968 | >8182 | -- | -- | -- | -- | -- | -- | 0 |
| IKWMALESILRRRFT | Her2/neu.886 | 17 | 682 | 3224 | 4098 | 731 | 370 | 2500 | 11 | 2.5 | 5 |
| VWELMTFGAKPYDGI | Her2/neu.912 | 58 | -- | 818 | 676 | 5506 | 3704 | 8750 | 92 | 200 | 5 |
| CWMIDSECRPRFREL | Her2/neu.958 | 1389 | 667 | >8182 | -- | -- | -- | -- | -- | 1333 | 0 |

**Claims**

1. An isolated HER2/neu peptide of 13 residues or less, comprising a sequence selected from the group consisting of:

   KVFGSLAFV;
   KLFGSLAFV;
   KTFGSLAFV;
   VVLGVVFGV;
   ALBRWGLLV; and
   ALBRWGLLL.

2. An isolated HER2/neu peptide of 13 residues or less, comprising a sequence selected from the group consisting of:

   VLLGVVFGV;
   SLISAVVGV;
   YLIMVKCWMV;
   YLIMVKBWMV;
   AMBRWGLLV; and
   VLIQRNPQV.

3. A composition comprising the peptide of claim 1 or claim 2.

4. The composition of claim 3, further comprising a CTL-inducing peptide.

5. The composition of claim 4, wherein said CTL-inducing peptide is 13 residues or less and comprises a sequence selected from the group set out in claim 1 or claim 2.

6. The composition of claim 3, further comprising a T helper peptide.

7. The composition of claim 6, wherein said T helper peptide is a pan-DR binding epitope.

8. A composition comprising at least three peptides, wherein each of said peptides comprises a sequence selected from the group set out in claim 1 or claim 2.

9. The composition of claim 3, further comprising a liposome, wherein said peptide is on or within said liposome.

10. The composition of claim 3, further comprising a lipid.

11. The composition of claim 3, wherein said peptide is joined to a linker.

12. The composition of claim 3, wherein said peptide is bound to an HLA heavy chain, β2-microglobulin, and streptavidin complex, whereby a tetramer is formed.

13. The composition of claim 3, further comprising an antigen presenting cell, wherein said peptide is on or within said antigen presenting cell.

14. The composition of claim 13, wherein said antigen presenting cell is a dendritic cell.

15. The composition of claim 13, wherein said peptide is bound to an HLA molecule on said antigen presenting cell, and wherein a cytotoxic lymphocyte (CTL) is present that is restricted to said HLA molecule, and wherein a receptor on said CTL binds to a complex of said HLA molecule and said peptide.

16. A clonal cytotoxic T lymphocyte (CTL), wherein said CTL binds to a complex of a peptide of claim 1 or claim 2 and to an HLA molecule.

17. A peptide comprising at least a first epitope and a second epitope, wherein said first epitope comprises a sequence selected from the group set out in claim 1 or claim 2; and wherein said peptide comprises less than 50 contiguous amino acids that have 100% identity with a native peptide sequence.

18. A composition comprising the peptide of claim 17, wherein each of said first epitope and said second epitope comprises a sequence selected from the group set out in claim 1 or claim 2.

19. The composition of claim 18, further comprising a third epitope which comprises a sequence selected from the group set out in claim 1 or claim 2.

20. A composition comprising the peptide of claim 17, wherein said peptide is a heteropolymer.

21. A composition comprising the peptide of claim 17, wherein said peptide is a homopolymer.

22. A composition comprising the peptide of claim 17, wherein said second epitope is a CTL-inducing peptide.

23. A composition comprising the peptide of claim 17, wherein said second epitope is a T helper peptide.

24. The composition of claim 23, wherein said T helper peptide is a pan-DR binding epitope.

25. A vaccine composition comprising:

a unit dose of a peptide that comprises less than 50 contiguous amino acids that have 100% identity with a native peptide sequence of HER2/neu, said peptide comprising at least a first epitope comprising a sequence selected from the group set out in claim 1 or claim 2; and;

a pharmaceutical excipient.

26. The vaccine composition of claim 25, further comprising a second epitope.

27. The vaccine composition of claim 26, wherein said second epitope is a CTL-inducing peptide.

28. The vaccine composition of claim 26, wherein said second epitope is a T helper peptide.

29. The vaccine composition of claim 28, wherein said T helper peptide is a pan-DR binding epitope.

30. The vaccine composition of claim 25, wherein said pharmaceutical excipient comprises an adjuvant.

31. An isolated nucleic acid encoding the peptide of claim 1 or claim 2.

32. An isolated nucleic acid encoding the peptide of claim 17.

33. The isolated nucleic acid of claim 32, wherein each of said first epitope and said second epitope comprises a sequence selected from the group set out in claim 1 or claim 2.

34. The isolated nucleic acid of claim 32, wherein said second epitope is a CTL-inducing peptide.

35. The isolated nucleic acid of claim 32, wherein said second epitope is a T helper peptide.

36. The isolated nucleic acid of claim 35, wherein said T helper peptide is a pan-DR binding epitope.

37. The use of the peptide of claim 1, claim 2, or claim 17 in the manufacture of a medicament for the prevention or treatment of cancer.

38. The use of the nucleic acid of claim 31 or claim 32 in the manufacture of a medicament for the prevention or treatment of cancer.